# EUROPEAN PATENT APPLICATION

(11) **EP 4 613 780 A1**
(43) Date of publication of application: **10.09.2025**
(21) Application number: 23896754.1
(22) Date of filing: 28.11.2023
(51) Int. Cl.: C07K 19/00, C07K 16/28, C12N 5/10, C12N 15/63, C12N 15/62, A61K 39/00, A61K 39/395, A61P 35/00, A61K 38/17

(54) **FUSION POLYPEPTIDE AND USE THEREOF**

(30) Priority: 29.11.2022 CN 202211507001; 20.04.2023 CN 202310444884
(71) Applicant: Adlai Nortye Biopharma Co., Ltd., Hangzhou, Zhejiang 311121 (CN)
(72) Inventor: HE, Nanhai, Hangzhou, Zhejiang 311100 (CN); WANG, Youping, Hangzhou, Zhejiang 311100 (CN); CHEN, Wanwan, Hangzhou, Zhejiang 311100 (CN); ZHU, Kun, Hangzhou, Zhejiang 311100 (CN)
(74) Representative: Office Freylinger
(86) International application number: PCT/CN2023/134527
(87) International publication number: WO 2024/114605

(57) **Abstract**

The present application relates to a fusion polypeptide and uses thereof. Specifically, the fusion polypeptide of the present application can block PD-1/PD-L1 signals and/or can have the function of activating immune responses. The present application also provides uses of the polypeptide.

## Description

### Technical Field

The present application relates to the field of biomedicine, and specifically to a fusion polypeptide and its use.

### Background Art

Immune checkpoints are a class of immune-related molecules that contain inhibitory signaling molecules. They mainly achieve their functions by regulating immune responses in peripheral tissues, including participating in immune defense, immune tolerance, and immune tissue damage. Targeted immune checkpoint-related treatment methods are aimed at regulating immune responses, including innate immune responses and adaptive immune responses, to intervene in the immune system and achieve the purpose of treating diseases.Tumor cells can exploit immune checkpoint inhibitory signaling pathways to achieve immune escape. The binding of programmed death-1 (PD-1) to its ligand programmed death receptor protein ligand-1 (PD-L1) is an important event in inhibiting anti-tumor immune response. PD-L1 is expressed in a variety of tumor cells including colon cancer, lung cancer, ovarian cancer and multiple myelomas, and the expression of PD-L1 is closely related to the prognosis of various cancers. The binding of PD-L1 to PD-1 can lead to T cell apoptosis, immune signal inhibition, cell exhaustion, and secretion of immunosuppressive factors, thereby inactivating the function of T cells involved in tumor infiltration and helping tumor cells evade the surveillance of the immune system.

Currently, the blocking antibodies targeting PD-L1 on the market include Atezolizumab developed by Roche, Avelumab jointly developed by Merck KGaA/Pfizer, and Durvalumab developed by AstraZeneca; the blocking antibodies targeting PD-1 competing for marketing approval include Nivolumab developed by BMS, Permbrolizumab developed by Merck, Carrelizumab developed by Hengrui Medicine, Tislelizumab developed by BeiGene, and Sintilimab developed by Innovent Biologics; although these antibodies have shown the effect of tumor treatment, their average treatment efficiency is only about 20%, and a considerable number of tumor patients still do not respond to the treatment of anti-PD-L1 antibodies and anti-PD-1 antibodies. Therefore, enhancing the effectiveness of tumor treatment remains a major issue that urgently needs to be addressed in current tumor treatment.

### Summary of the invention

Due to the current limited effectiveness of PD-1/PD-L1 pathway blockade in regulating T cell activation to treat tumors, this application proposes the development of a new agent that can synergize PD-1/PD-L1 pathway blockade to enhance T cell activation for clinical tumor treatment. The development of new agents that can synergize PD-1/PD-L1 pathway blockade, enhance T cell activation and/or improve antigen-presenting cell activation for clinical treatment of tumors can potentially bring more benefits to more cancer patients.

The present application provides a fusion polypeptide, which may comprise (i) a fusion polypeptide derived from an anti-programmed death receptor protein ligand-1 (PD-L1) antibody/anti-programmed death receptor protein-1 (PD-1) antibody-related binding antigen fragment, or/and (ii) an immunoglobulin Fc domain, or/and (iii) a fusion polypeptide of a CD80 extracellular domain (ECD), or/and (iv) a fusion polypeptide composed of a LAG3 extracellular domain, and the present application provides a polynucleotide expressing the fusion polypeptide; the present application provides a method for inducing and/or enhancing immunity using the fusion polypeptide and a method for treating a disease (e.g., cancer).

The present application provides a class of dual and/or multifunctional fusion polypeptides that simultaneously interfere with, inhibit or block the PD-1/PD-L1 signal transduction pathway and/or co-stimulate antigen presenting cell activation and/or co-stimulate T cell activation. The fusion polypeptide can effectively excite T cells to enhance immune response and/or excite antigen presenting cells, and can have a potential tumor-killing effect, thereby improving the therapeutic effect in the treatment of diseases caused by suppressed T cell function, such as tumors, especially in tumor patients who have no response or weak response to anti-PD-1 antibodies and/or anti-PD-L1 antibodies.

On the one hand, the present application provides a fusion polypeptide, which comprises a first domain and a second domain, wherein the first domain is capable of blocking PD-1/PD-L1 signal, and the second domain comprises CD86 or a functionally active fragment thereof.

In one embodiment of the fusion polypeptide, the first domain is capable of binding to PD-L1 and/or PD-1.

In one embodiment of the fusion polypeptide, the first domain comprises HCDR1, HCDR2 and/or HCDR3 of an antibody heavy chain, and the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15 and SEQ ID NO: 17.

In one embodiment of the fusion polypeptide, the first domain comprises the heavy chain variable region VH of an antibody heavy chain, and the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15, and SEQ ID NO: 17.

In one embodiment of the fusion polypeptide, the first domain comprises an antibody heavy chain, and the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15 and SEQ ID NO: 17.

In one embodiment of the fusion polypeptide, the first domain comprises LCDR1, LCDR2 and/or LCDR3 of the light chain of an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

In one embodiment of the fusion polypeptide, the first domain comprises an antibody or an antigen-binding fragment thereof. In one embodiment of the fusion polypeptide, the first domain comprises the light chain variable region VL of an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

In one embodiment of the fusion polypeptide, the first domain comprises an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

In one embodiment of the fusion polypeptide, the antibody is selected from the group consisting of a recombinant antibody, a single domain antibody, a heavy chain antibody, a chimeric antibody and a bispecific antibody.

In one embodiment of the fusion polypeptide, the antigen binding fragment is selected from one or more of the group consisting of: Fab, Fab', Fv fragment, F(ab')2, F(ab)2, scFv, di-scFv, VHH and dAb.

In one embodiment of the fusion polypeptide, the first domain comprises an antibody or antigen-binding fragment thereof selected from the group consisting of Sugemalimab, Durvalumab, Atezolizumab, Avelumab, Envafolimab, Permbrolizumab and Nivolumab.

In one embodiment of the fusion polypeptide, the first domain comprises Sugemalimab or an antigen-binding fragment thereof.

In one embodiment of the fusion polypeptide, the second domain is selected from the group consisting of CD86 derived from humans or a functionally active fragment thereof and CD86 derived from mice or a functionally active fragment thereof.

In one embodiment of the fusion polypeptide, the second domain is capable of binding to CD28 and/or CTLA4.

In one embodiment of the fusion polypeptide, the second domain comprises the IgV domain of CD86 or a functionally active fragment thereof.

In one embodiment of the fusion polypeptide, the second domain comprises the extracellular domain of CD86 or a functionally active fragment thereof.

In one embodiment of the fusion polypeptide, the second domain comprises the amino acid sequence shown in SEQ ID NO: 2 or SEQ ID NO: 3.

In one embodiment of the fusion polypeptide, the first domain is directly or indirectly linked to the second domain.

In one embodiment of the fusion polypeptide, the first domain heavy chain is directly or indirectly linked to the second domain.

In one embodiment of the fusion polypeptide, the C-terminus of the heavy chain of the first domain is directly or indirectly connected to the N-terminus of the second domain.

In one embodiment of the fusion polypeptide, the N-terminus of the heavy chain of the first domain is directly or indirectly connected to the C-terminus of the second domain.

In one embodiment of the fusion polypeptide, the first domain light chain is directly or indirectly linked to the second domain.

In one embodiment of the fusion polypeptide, the C-terminus of the light chain of the first domain is directly or indirectly connected to the N-terminus of the second domain.

In one embodiment of the fusion polypeptide, the N-terminus of the light chain of the first domain is directly or indirectly connected to the C-terminus of the second domain.

In one embodiment of the fusion polypeptide, the indirect connection comprises connection through a linker.

In one embodiment of the fusion polypeptide, the linker comprises an amino acid sequence selected from the group consisting of SEQ ID NO: 31, SEQ ID NO: 32, SEQ ID NO: 33, SEQ ID NO: 34 and SEQ ID NO: 35.

In one embodiment, the fusion polypeptide comprises an amino acid sequence selected from the group consisting of SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83. SEQ ID NO: 84, SEQ ID NO: 85, SEQ ID NO: 86 and SEQ ID NO: 87.

In one embodiment, the fusion polypeptide comprises an amino acid sequence selected from the group consisting of SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44 and SEQ ID NO: 77.

On the other hand, the present application provides a fusion polypeptide, which comprises a first domain, a second domain and a third domain, wherein the first domain can block PD-1/PD-L1 signals, the second domain comprises CD86 or a functionally active fragment thereof, and the third domain can activate an innate immune response.

In one embodiment of the fusion polypeptide, the third domain is capable of binding to an MHCII molecule on an antigen presenting cell.

In one embodiment of the fusion polypeptide, the third domain comprises LAG3 or a functionally active fragment thereof.

In one embodiment of the fusion polypeptide, the first domain is capable of binding to PD-L1 and/or PD-1.

In one embodiment of the fusion polypeptide, the first domain comprises HCDR1, HCDR2 and/or HCDR3 of an antibody heavy chain, and the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15 and SEQ ID NO: 17.

In one embodiment of the fusion polypeptide, the first domain comprises the heavy chain variable region VH of an antibody heavy chain, and the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15 and SEQ ID NO: 17.

In one embodiment of the fusion polypeptide, the first domain comprises an antibody heavy chain, and the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15 and SEQ ID NO: 17.

In one embodiment of the fusion polypeptide, the first domain comprises LCDR1, LCDR2 and/or LCDR3 of the light chain of an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

In one embodiment of the fusion polypeptide, the first domain comprises the light chain variable region VL of an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

In one embodiment of the fusion polypeptide, the first domain comprises an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

In one embodiment of the fusion polypeptide, the first domain comprises an antibody or an antigen-binding fragment thereof.

In one embodiment of the fusion polypeptide, the antibody is selected from the group consisting of a recombinant antibody, a single domain antibody, a heavy chain antibody, a chimeric antibody and a bispecific antibody.

In one embodiment of the fusion polypeptide, the antigen binding fragment is selected from one or more of the group consisting of: Fab, Fab', Fv fragment, F(ab')2, F(ab)2, scFv, di-scFv, VHH and dAb.

In one embodiment of the fusion polypeptide, the first domain comprises an antibody or antigen-binding fragment thereof selected from the group consisting of Sugemalimab, Durvalumab, Atezolizumab, Avelumab, Envafolimab, Permbrolizumab and Nivolumab.

In one embodiment of the fusion polypeptide, the first domain comprises Sugemalimab or an antigen-binding fragment thereof.

In one embodiment of the fusion polypeptide, the second domain is selected from the group consisting of CD86 derived from humans or a functionally active fragment thereof and CD86 derived from mice or a functionally active fragment thereof.

In one embodiment of the fusion polypeptide, the second domain is capable of binding to CD28 and/or CTLA4.

In one embodiment of the fusion polypeptide, the second domain comprises the IgV domain of CD86 or a functionally active fragment thereof.

In one embodiment of the fusion polypeptide, the second domain comprises the extracellular domain of CD86 or a functionally active fragment thereof.

In one embodiment of the fusion polypeptide, the second domain comprises the amino acid sequence shown in SEQ ID NO: 2 or SEQ ID NO: 3.

In one embodiment of the fusion polypeptide, the third domain is selected from the group consisting of LAG3 derived from humans or a functionally active fragment thereof and LAG3 derived from mice or a functionally active fragment thereof.

In one embodiment of the fusion polypeptide, the third domain comprises the extracellular domain of LAG3 or a functionally active fragment thereof.

In one embodiment of the fusion polypeptide, the third domain comprises IgD1, IgD2, IgD3 and/or IgD4 of LAG3 or a functionally active fragment thereof.

In one embodiment of the fusion polypeptide, the third domain comprises IgD1, IgD1-IgD2, IgD1-IgD2-IgD3, and/or IgD1-IgD2-IgD3-IgD4 of LAG3 or a functionally active fragment thereof.

In one embodiment of the fusion polypeptide, the third domain comprises an amino acid sequence selected from the group consisting of SEQ ID NO: 21, SEQ ID NO: 22, SEQ ID NO: 23, SEQ ID NO: 24, SEQ ID NO: 25, SEQ ID NO: 26, SEQ ID NO: 27 and SEQ ID NO: 28.

In one embodiment of the fusion polypeptide, the first domain and the second domain are directly or indirectly linked.

In one embodiment of the fusion polypeptide, the first domain heavy chain is directly or indirectly linked to the second domain.

In one embodiment of the fusion polypeptide, the C-terminus of the heavy chain of the first domain is directly or indirectly connected to the N-terminus of the second domain.

In one embodiment of the fusion polypeptide, the N-terminus of the heavy chain of the first domain is directly or indirectly connected to the C-terminus of the second domain.

In one embodiment of the fusion polypeptide, the first domain light chain is directly or indirectly linked to the second domain.

In one embodiment of the fusion polypeptide, the C-terminus of the light chain of the first domain is directly or indirectly connected to the N-terminus of the second domain.

In one embodiment of the fusion polypeptide, the N-terminus of the light chain of the first domain is directly or indirectly connected to the C-terminus of the second domain.

In one embodiment of the fusion polypeptide, the C-terminus of the heavy chain of the first domain is connected to the N-terminus of the second domain, and the C-terminus of the light chain of the first domain is connected to the N-terminus of the second domain.

In one embodiment of the fusion polypeptide, the N-terminus of the heavy chain of the first domain is connected to the C-terminus of the second domain, and the N-terminus of the light chain of the first domain is connected to the C-terminus of the second domain.

In one embodiment of the fusion polypeptide, the first domain is directly or indirectly linked to the third domain.

In one embodiment of the fusion polypeptide, the first domain heavy chain is directly or indirectly connected to the third domain.

In one embodiment of the fusion polypeptide, the C-terminus of the heavy chain of the first domain is directly or indirectly connected to the N-terminus of the third domain.

In one embodiment of the fusion polypeptide, the N-terminus of the heavy chain of the first domain is directly or indirectly connected to the C-terminus of the third domain.

In one embodiment of the fusion polypeptide, the first domain light chain is directly or indirectly linked to the third domain.

In one embodiment of the fusion polypeptide, the C-terminus of the light chain of the first domain is directly or indirectly connected to the N-terminus of the third domain.

In one embodiment of the fusion polypeptide, the N-terminus of the light chain of the first domain is directly or indirectly connected to the C-terminus of the third domain.

In one embodiment of the fusion polypeptide, the second domain and the third domain are directly or indirectly connected.

In one embodiment of the fusion polypeptide, the C-terminus of the second domain is directly or indirectly connected to the N-terminus of the third domain.

In one embodiment of the fusion polypeptide, the N-terminus of the second domain is directly or indirectly connected to the C-terminus of the third domain.

In one embodiment of the fusion polypeptide, the first domain is directly or indirectly connected to the second domain, and the second domain is directly or indirectly connected to the third domain.

In one embodiment of the fusion polypeptide, the C-terminus of the first domain is directly or indirectly connected to the N-terminus of the second domain, and the C-terminus of the second domain is directly or indirectly connected to the N-terminus of the third domain.

In one embodiment of the fusion polypeptide, the heavy chain C-terminus of the first domain is directly or indirectly connected to the N-terminus of the second domain, and the C-terminus of the second domain is directly or indirectly connected to the N-terminus of the third domain.

In one embodiment of the fusion polypeptide, the C-terminus of the light chain of the first domain is directly or indirectly connected to the N-terminus of the second domain, and the C-terminus of the second domain is directly or indirectly connected to the N-terminus of the third domain.

In one embodiment of the fusion polypeptide, the N-terminus of the first domain is directly or indirectly connected to the C-terminus of the second domain, and the N-terminus of the second domain is directly or indirectly connected to the C-terminus of the third domain.

In one embodiment of the fusion polypeptide, the N-terminus of the heavy chain of the first domain is directly or indirectly connected to the C-terminus of the second domain, and the N-terminus of the second domain is directly or indirectly connected to the C-terminus of the third domain.

In one embodiment of the fusion polypeptide, the N-terminus of the light chain of the first domain is directly or indirectly connected to the C-terminus of the second domain, and the N-terminus of the second domain is directly or indirectly connected to the C-terminus of the third domain.

In one embodiment of the fusion polypeptide, the first domain is directly or indirectly connected to the third domain, and the third domain is directly or indirectly connected to the second domain.

In one embodiment of the fusion polypeptide, the C-terminus of the first domain is directly or indirectly connected to the N-terminus of the third domain, and the C-terminus of the third domain is directly or indirectly connected to the N-terminus of the second domain.

In one embodiment of the fusion polypeptide, the C-terminus of the heavy chain of the first domain is directly or indirectly connected to the N-terminus of the third domain, and the C-terminus of the third domain is directly or indirectly connected to the N-terminus of the second domain.

In one embodiment of the fusion polypeptide, the C-terminus of the light chain of the first domain is directly or indirectly connected to the N-terminus of the third domain, and the C-terminus of the third domain is directly or indirectly connected to the N-terminus of the second domain.

In one embodiment of the fusion polypeptide, the N-terminus of the first domain is directly or indirectly connected to the C-terminus of the third domain, and the N-terminus of the third domain is directly or indirectly connected to the C-terminus of the second domain.

In one embodiment of the fusion polypeptide, the N-terminus of the heavy chain of the first domain is directly or indirectly connected to the C-terminus of the third domain, and the N-terminus of the third domain is directly or indirectly connected to the C-terminus of the second domain.

In one embodiment of the fusion polypeptide, the N-terminus of the light chain of the first domain is directly or indirectly connected to the C-terminus of the third domain, and the N-terminus of the third domain is directly or indirectly connected to the C-terminus of the second domain.

In one embodiment of the fusion polypeptide, the first domain is directly or indirectly connected to the second domain, and the first domain is directly or indirectly connected to the third domain.

In one embodiment of the fusion polypeptide, the C-terminus of the first domain is directly or indirectly connected to the N-terminus of the second domain, and the N-terminus of the first domain is directly or indirectly connected to the C-terminus of the third domain.

In one embodiment of the fusion polypeptide, the C-terminus of the heavy chain of the first domain is directly or indirectly connected to the N-terminus of the second domain, and the N-terminus of the heavy chain of the first domain is directly or indirectly connected to the C-terminus of the third domain.

In one embodiment of the fusion polypeptide, the C-terminus of the light chain of the first domain is directly or indirectly connected to the N-terminus of the second domain, and the N-terminus of the light chain of the first domain is directly or indirectly connected to the C-terminus of the third domain.

In one embodiment of the fusion polypeptide, the C-terminus of the heavy chain of the first domain is directly or indirectly connected to the N-terminus of the second domain, and the N-terminus of the light chain of the first domain is directly or indirectly connected to the C-terminus of the third domain.

In one embodiment of the fusion polypeptide, the C-terminus of the light chain of the first domain is directly or indirectly connected to the N-terminus of the second domain, and the N-terminus of the heavy chain of the first domain is directly or indirectly connected to the C-terminus of the third domain.

In one embodiment of the fusion polypeptide, the N-terminus of the first domain is directly or indirectly connected to the C-terminus of the second domain, and the C-terminus of the first domain is directly or indirectly connected to the N-terminus of the third domain.

In one embodiment of the fusion polypeptide, the N-terminus of the heavy chain of the first domain is directly or indirectly connected to the C-terminus of the second domain, and the C-terminus of the heavy chain of the first domain is directly or indirectly connected to the N-terminus of the third domain.

In one embodiment of the fusion polypeptide, the N-terminus of the light chain of the first domain is directly or indirectly connected to the C-terminus of the second domain, and the C-terminus of the light chain of the first domain is directly or indirectly connected to the N-terminus of the third domain.

In one embodiment of the fusion polypeptide, the N-terminus of the heavy chain of the first domain is directly or indirectly connected to the C-terminus of the second domain, and the C-terminus of the light chain of the first domain is directly or indirectly connected to the N-terminus of the third domain.

In one embodiment of the fusion polypeptide, the N-terminus of the light chain of the first domain is directly or indirectly connected to the C-terminus of the second domain, and the C-terminus of the heavy chain of the first domain is directly or indirectly connected to the N-terminus of the third domain.

In one embodiment of the fusion polypeptide, the indirect connection comprises connection through a linker.

In one embodiment of the fusion polypeptide, the linker comprises an amino acid sequence selected from the group consisting of SEQ ID NO: 31, SEQ ID NO: 32, SEQ ID NO: 33, SEQ ID NO: 34 and SEQ ID NO: 35.

In one embodiment, the fusion polypeptide comprises an amino acid sequence selected from the group consisting of SEQ ID NO: 88, SEQ ID NO: 89, SEQ ID NO: 90, SEQ ID NO: 91, SEQ ID NO: 92, SEQ ID NO: 93, SEQ ID NO: 94, SEQ ID NO: 95, SEQ ID NO: 96, SEQ ID NO: 97, SEQ ID NO: 98, SEQ ID NO: 99, SEQ ID NO: 100, SEQ ID NO: 101, SEQ ID NO: 102, SEQ ID NO: 103, SEQ ID NO: 104, SEQ ID NO: 105, SEQ ID NO: 106, SEQ ID NO: 107, SEQ ID NO: 108, SEQ ID NO: 109, SEQ ID NO: 110, SEQ ID NO: 111, SEQ ID NO: 112, SEQ ID NO: 113, SEQ ID NO: 114, SEQ ID NO: 115, SEQ ID NO: 116, SEQ ID NO: 117, SEQ ID NO: 118, SEQ ID NO: 119, SEQ ID NO: 120, SEQ ID NO: 121, SEQ ID NO: 122, SEQ ID NO: 123, SEQ ID NO: 124, SEQ ID NO: 125, SEQ ID NO: 126, SEQ ID NO: 127, SEQ ID NO: 128, SEQ ID NO: 129, SEQ ID NO: 130, SEQ ID NO: 131, SEQ ID NO: 132, SEQ ID NO: 133, SEQ ID NO: 134, SEQ ID NO: 135, SEQ ID NO: 171, SEQ ID NO: 173.

In one embodiment, the fusion polypeptide comprises an amino acid sequence selected from the group consisting of SEQ ID NO: 104, SEQ ID NO: 107, SEQ ID NO: 108, SEQ ID NO: 112, SEQ ID NO: 114, SEQ ID NO: 115, SEQ ID NO: 116, SEQ ID NO: 171, and SEQ ID NO: 173.

On the other hand, the present application provides a CD86 variant polypeptide, which comprises an amino acid substitution mutation of human CD86.

In one embodiment, the CD86 variant polypeptide comprises an amino acid substitution mutant of the human CD86 extracellular domain IgV domain.

In one embodiment, the CD86 variant polypeptide comprises an amino acid substitution mutant of the IgV domain of CD86, and the mutation site of the amino acid substitution mutant of the IgV domain of CD86 comprises one or more of the amino acid mutations selected from the group consisting of A13I, A13L, A13F, A13M, Q25A, Q25I, Q25V, Q25F, Q25M, F33A, F33L, F33V, F33M, M60R, I89A, I89L, I89V, I89F, I89M, H90I, H90V, H90M, and H90F. In the present invention, unless otherwise explicitly stated, the representation of mutation sites is based on the wild-type sequence, for example, "mutation site A13I of the amino acid substitution mutant of the IgV domain of CD86" means that the A at amino acid site 13 of the IgV domain of wild-type CD86 is mutated to I;

In one embodiment, the CD86 variant polypeptide comprises an amino acid substitution mutant of the IgV domain of CD86, wherein the amino acid substitution mutant comprises a combination selected from the group consisting of Q25I/F33L/H90I, Q25V/F33L/H90I, Q25I/F33V/H90I, Q25V/F33V/H90I, Q25I/F33L/H90V, Q25V/F33L/H90V, Q25I/F33V/H90V, Q25V/F33V/H90V, A13L/Q25I/F33L/H90I, A13I/Q25I/F33L/H90I, A13L/Q25V/F33L/H90I, A13I/Q25V/F33L/H90I, Q25I/F33L/I89L/H90I, Q25I/F33L/M60R/H90I. Q25V/F33L/I89L/H90I, Q25V/F33L/M60R/H90I, Q25F/F33L/H90I, Q25I/F33L/H90F, Q25I /F33A/H90I, Q25I/H90I, Q25I, H90I, Q25V/H90V, Q25V/H90I, Q25F/H901, Q25F/H90V, A13F/Q25I/F33L/H90I, A13M/Q25I/F33L/H90I, Q25A/F33L/H90I, Q25M/F33L/H90I, Q25I/F33M/H90I, Q25I/F33L/I89V/H90I, Q25I/F33L/I89F/H90I, Q25I/F33L/I89M/H90I and Q25I/F33L/H90M.

In one embodiment, the CD86 variant polypeptide comprises the IgV domain amino acid substitution mutant Q25I/F33L/H90I of CD86.

In one embodiment, the CD86 variant polypeptide comprises an amino acid substitution mutant of the extracellular domain of human CD86.

In one embodiment, the CD86 variant polypeptide comprises an amino acid substitution mutant of the extracellular domain of CD86, and the amino acid substitution mutant of the extracellular domain of CD86 comprises one or more amino acid mutations selected from the group consisting of A13I, A13L, A13F, A13M, Q25A, Q25I, Q25V, Q25F, Q25M, F33A, F33L, F33V, F33M, M60R, I89A, I89L, I89V, I89F, I89M, H90I, H90V, H90M, and H90F.

In one embodiment, the CD86 variant polypeptide comprises an amino acid substitution mutant of the extracellular domain of CD86, wherein the amino acid substitution mutant comprises a combination selected from the group consisting of Q25I/F33L/H90I, 25V/F33L/H90I, Q25I/F33V/H90I, Q25V/F33V/H90I, Q25I/F33L/H90V, Q25V/F33L/H90V, Q25I/F33V/H90V, Q25V/F33V/H90V, A13L/Q25I/F33L/H90I, A13I/Q25I/F33L/H90I, A13L/Q25V/F33L/H90I, A13I/Q25V/F33L/H90I, Q25I/F33L/I89L/H90I, Q25I/F33L/M60R/H90I, Q25V/F33L/I89L/H90I, Q25V/F33L/M60R/H90I, Q25F/F33L/H90I, Q25I/F33L/H90F, Q25I/F33A/H90I, Q25I/H90I, Q25I, H90I, Q25V/H90V, Q25V/H90I, Q25F/H90I, Q25F/H90V, A13F/Q25I/F33L/H90I, A13M/Q25I/F33L/H90I, Q25A/F33L/H90I, Q25M/F33L/H90I, Q25I/F33M/H90I, Q25I/F33L/I89V/H90I, Q25I/F33L/I89F/H90I, Q25I/F33L/I89M/H90I and Q25I/F33L/H90M.

In one embodiment, the CD86 variant polypeptide comprises the extracellular domain amino acid substitution mutant Q25I/F33L/H90I of CD86.

In one embodiment, the CD86 variant polypeptide comprises an amino acid sequence selected from the group consisting of SEQ ID NO: 178 to SEQ ID NO: 243.

On the other hand, the present application provides a fusion polypeptide, which comprises a first domain and a second domain, wherein the first domain comprises the CD86 variant polypeptide described in the present application, and the second domain comprises an antibody or an antigen-binding fragment thereof or an immunoglobulin domain.

In one embodiment of the fusion polypeptide, the second domain comprises an antibody or an antigen-binding fragment thereof.

In one embodiment of the fusion polypeptide, the second domain is capable of binding to one or more of the targets shown in the following group: PD-L1, PD-L2, PD-1, OX40, 4-1BB, ICOS, TIGIT, CTLA4, LAG3, CD3, VEGF, VEGFR, CD47, HGF, Trop2, EpCAM, CCR8, CCR4, CCR5, GPRC5D, BCMA, CD19, CD20, HER-2 neu, DLL1, HER-3, HER-4, EGFR, PSMA, CEA, MUC-1 (mucin), MUC2, MUC3, MUC4, MUCSAC, MUC5B, MUC7, CD123, CD33, CD30, CD38, NKG2A, Nkp36 and Tim3.

In one embodiment of the fusion polypeptide, the second domain is capable of binding to PD-L1, PD-1 and/or PD-L2.

In one embodiment of the fusion polypeptide, the second domain comprises HCDR1, HCDR2 and/or HCDR3 of an antibody heavy chain, wherein the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15 and SEQ ID NO: 17.

In one embodiment of the fusion polypeptide, the second domain comprises the heavy chain variable region VH of an antibody heavy chain, and the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15, and SEQ ID NO: 17.

In one embodiment of the fusion polypeptide, the second domain comprises an antibody heavy chain, which comprises an amino acid sequence selected from the group consisting of

SEQ ID NO: 4, SEQ ID NO: 6, SEQ ID NO: 8, SEQ ID NO: 10, SEQ ID NO: 12, SEQ ID NO: 14, SEQ ID NO: 15 and SEQ ID NO: 17.

In one embodiment of the fusion polypeptide, the second domain comprises LCDR1, LCDR2 and/or LCDR3 of an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

In one embodiment of the fusion polypeptide, the second domain comprises the light chain variable region VL of an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

In one embodiment of the fusion polypeptide, the second domain comprises an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

In one embodiment of the fusion polypeptide, the second domain comprises an antibody or an antigen-binding fragment thereof selected from the group consisting of Sugemalimab, Durvalumab, Atezolizumab, Avelumab, Envafolimab, Permbrolizumab and Nivolumab.

In one embodiment of the fusion polypeptide, the second domain comprises Sugemalimab or an antigen-binding fragment thereof.

In one embodiment of the fusion polypeptide, the second domain is capable of binding to CD3.

In one embodiment of the fusion polypeptide, the second domain comprises CD3B219 antibody or an antigen-binding fragment thereof.

In one embodiment of the fusion polypeptide, the second domain comprises HCDR1, HCDR2 and/or HCDR3 of an antibody heavy chain, and the antibody heavy chain comprises the amino acid sequence shown in SEQ ID NO:616.

In one embodiment of the fusion polypeptide, the second domain comprises the heavy chain variable region VH of an antibody, and the antibody heavy chain comprises the amino acid sequence shown in SEQ ID NO:616.

In one embodiment of the fusion polypeptide, the second domain comprises an antibody heavy chain, and the antibody heavy chain comprises the amino acid sequence shown in SEQ ID NO:616.

In one embodiment of the fusion polypeptide, the second domain comprises LCDR1, LCDR2 and/or LCDR3 of an antibody light chain, and the antibody light chain comprises the amino acid sequence shown in SEQ ID NO:617.

In one embodiment of the fusion polypeptide, the second domain comprises the light chain variable region VL of an antibody, and the antibody light chain comprises the amino acid sequence shown in SEQ ID NO:617.

In one embodiment of the fusion polypeptide, the second domain comprises an antibody light chain, and the antibody light chain comprises the amino acid sequence shown in SEQ ID NO:617.

In one embodiment of the fusion polypeptide, the antigen binding fragment is selected from one or more of the group consisting of: Fab, Fab', Fv fragment, F(ab')2, F(ab)2, scFv, di-scFv, VHH and dAb.

In one embodiment of the fusion polypeptide, the second domain comprises an immunoglobulin domain.

In one embodiment of the fusion polypeptide, the second domain comprises an immunoglobulin IgG antibody.

In one embodiment of the fusion polypeptide, the immunoglobulin IgG antibody comprises one or more selected from human IgG1, human IgG2, human IgG3, human IgG4, mouse IgG1, mouse IgG2a, mouse IgG2b and mouse IgG3.

In one embodiment of the fusion polypeptide, the second domain comprises an immunoglobulin Fc domain.

In one embodiment of the fusion polypeptide, the second domain comprises the Fc domain of an immunoglobulin IgG antibody.

In one embodiment of the fusion polypeptide, the second domain comprises the Fc domain of an immunoglobulin IgG antibody, and the Fc domain of the immunoglobulin IgG antibody comprises a human IgG1 Fc domain, a human IgG2 Fc domain, a human IgG3 Fc domain, a human IgG4 Fc domain, a mouse IgG1 Fc domain, a mouse IgG2a Fc domain, a mouse IgG2b Fc domain or a mouse IgG3 Fc domain.

In one embodiment of the fusion polypeptide, the second domain comprises the Fc domain of human immunoglobulin IgG4.

In one embodiment of the fusion polypeptide, the second domain is selected from the group consisting of amino acid sequences shown in SEQ ID NO: 4 to SEQ ID NO: 18, SEQ ID NO: 29 to SEQ ID NO: 30, SEQ ID NO: 634 to SEQ ID NO: 639, SEQ ID NO: 616 and SEQ ID NO: 617.

In one embodiment of the fusion polypeptide, the first domain is directly or indirectly linked to the second domain.

In one embodiment of the fusion polypeptide, the first domain is directly or indirectly connected to the N-terminus of the second domain.

In one embodiment of the fusion polypeptide, the first domain is directly or indirectly connected to the C-terminus of the second domain.

In one embodiment of the fusion polypeptide, the indirect connection comprises connection through a linker.

In one embodiment of the fusion polypeptide, the linker comprises an amino acid sequence selected from the group consisting of SEQ ID NO: 31, SEQ ID NO: 32, SEQ ID NO: 33, SEQ ID NO: 34 and SEQ ID NO: 35.

In one embodiment, the fusion polypeptide comprises an amino acid sequence selected from the group consisting of SEQ ID NO: 350 to SEQ ID NO: 382, SEQ ID NO: 384 to SEQ ID NO: 416, SEQ ID NO: 418 to SEQ ID NO: 450, SEQ ID NO: 451 to SEQ ID NO: 483, SEQ ID NO: 484 to SEQ ID NO: 615, SEQ ID NO: 618 to SEQ ID NO: 624 and SEQ ID NO: 626 to SEQ ID NO: 632.

On the other hand, the present application provides a fusion polypeptide, which comprises a first domain, a second domain and a third domain, wherein the first domain comprises the CD86 variant described in the present application, the second domain comprises an antibody or an antigen-binding fragment thereof or an immunoglobulin antibody, and the third domain is an antibody or an antigen-binding fragment thereof or a functional protein or an active fragment thereof that is the same or different from the second domain.

In one embodiment of the fusion polypeptide, the second domain comprises an antibody or an antigen-binding fragment thereof.

In one embodiment of the fusion polypeptide, the second domain is capable of binding to one or more of the targets shown in the following group: PD-L1, PD-L2, PD-1, OX40, 4-1BB, ICOS, TIGIT, CTLA4, LAG3, CD3, VEGF, VEGFR, CD47, HGF, Trop2, EpCAM, CCR8, CCR4, CCR5, GPRC5D, BCMA, CD19, CD20, HER-2 neu, DLL1, HER-3, HER-4, EGFR, PSMA, CEA, MUC-1 (mucin), MUC2, MUC3, MUC4, MUCSAC, MUC5B, MUC7, CD123, CD33, CD30, CD38, NKG2A, Nkp36 and Tim3.

In one embodiment of the fusion polypeptide, the second domain is capable of binding to PD-L1, PD-1 and/or PD-L2.

In one embodiment of the fusion polypeptide, the second domain comprises HCDR1, HCDR2 and/or HCDR3 of an antibody heavy chain, wherein the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15 and SEQ ID NO: 17.

In one embodiment of the fusion polypeptide, the second domain comprises the heavy chain variable region VH of an antibody heavy chain, and the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15, and SEQ ID NO: 17.

In one embodiment of the fusion polypeptide, the second domain comprises an antibody heavy chain, which comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15 and SEQ ID NO: 17.

In one embodiment of the fusion polypeptide, the second domain comprises LCDR1, LCDR2 and/or LCDR3 of the light chain of an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

In one embodiment of the fusion polypeptide, the second domain comprises the light chain variable region VL of an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

In one embodiment of the fusion polypeptide, the second domain comprises an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

In one embodiment of the fusion polypeptide, the second domain comprises an antibody or an antigen-binding fragment thereof selected from the group consisting of Sugemalimab, Durvalumab, Atezolizumab, Avelumab, Envafolimab, Permbrolizumab and Nivolumab.

In one embodiment of the fusion polypeptide, the second domain comprises Sugemalimab or an antigen-binding fragment thereof.

In one embodiment of the fusion polypeptide, the second domain is capable of binding to CD3.

In one embodiment of the fusion polypeptide, the second domain comprises CD3B219 antibody or an antigen-binding fragment thereof.

In one embodiment of the fusion polypeptide, the second domain comprises HCDR1, HCDR2 and/or HCDR3 of an antibody heavy chain, and the antibody heavy chain comprises the amino acid sequence shown in SEQ ID NO:616.

In one embodiment of the fusion polypeptide, the second domain comprises the heavy chain variable region VH of an antibody, and the antibody heavy chain comprises the amino acid sequence shown in SEQ ID NO:616.

In one embodiment of the fusion polypeptide, the second domain comprises an antibody heavy chain, and the antibody heavy chain comprises the amino acid sequence shown in SEQ ID NO:616.

In one embodiment of the fusion polypeptide, the second domain comprises LCDR1, LCDR2 and/or LCDR3 of an antibody light chain, and the antibody light chain comprises the amino acid sequence shown in SEQ ID NO:617.

In one embodiment of the fusion polypeptide, the second domain comprises the light chain variable region VL of an antibody, and the antibody light chain comprises the amino acid sequence shown in SEQ ID NO:617.

In one embodiment of the fusion polypeptide, the second domain comprises an antibody light chain, and the antibody light chain comprises the amino acid sequence shown in SEQ ID NO:617. In one embodiment of the fusion polypeptide, the antibody is selected from the group consisting of an immunoglobulin antibody, a recombinant antibody, a chimeric antibody, a heavy chain antibody, a single domain antibody, and a bispecific antibody.

In one embodiment of the fusion polypeptide, the antigen binding fragment is selected from one or more of the group consisting of: Fab, Fab', Fv fragment, F(ab')2, F(ab)2, scFv, di-scFv, VHH and dAb.

In one embodiment of the fusion polypeptide, the second domain comprises an immunoglobulin domain.

In one embodiment of the fusion polypeptide, the second domain comprises an immunoglobulin IgG antibody, and the immunoglobulin IgG antibody comprises one or more selected from human IgG1, human IgG2, human IgG3, human IgG4, mouse IgG1, mouse IgG2a, mouse IgG2b and mouse IgG3.

In one embodiment of the fusion polypeptide, the second domain comprises an immunoglobulin Fc domain.

In one embodiment of the fusion polypeptide, the second domain comprises the Fc domain of an immunoglobulin IgG antibody.

In one embodiment of the fusion polypeptide, the second domain comprises the Fc domain of an immunoglobulin IgG antibody, and the Fc domain of the immunoglobulin IgG antibody comprises a human IgG1 Fc domain, a human IgG2 Fc domain, a human IgG3 Fc domain, a human IgG4 Fc domain, a mouse IgG1 Fc domain, a mouse IgG2a Fc domain, a mouse IgG2b Fc domain or a mouse IgG3 Fc domain.

In one embodiment of the fusion polypeptide, the second domain comprises the Fc domain of human immunoglobulin IgG4.

In one embodiment of the fusion polypeptide, the third domain is a functional protein or an active fragment thereof, and the functional protein or the active fragment thereof can activate the innate immune response.

In one embodiment of the fusion polypeptide, the third domain is capable of binding to an MHCII molecule on an antigen presenting cell.

In one embodiment of the fusion polypeptide, the third domain comprises LAG3 or a functionally active fragment thereof.

In one embodiment of the fusion polypeptide, the third domain is selected from the group consisting of LAG3 derived from humans or a functionally active fragment thereof and LAG3 derived from mice or a functionally active fragment thereof.

In one embodiment of the fusion polypeptide, the third domain comprises the extracellular domain of LAG3 or a functionally active fragment thereof.

In one embodiment of the fusion polypeptide, the third domain comprises IgD1, IgD2, IgD3 and/or IgD4 of LAG3 or a functionally active fragment thereof.

In one embodiment of the fusion polypeptide, the third domain comprises IgD1, IgD1-IgD2, IgD1-IgD2-IgD3, and/or IgD1-IgD2-IgD3-IgD4 of LAG3 or a functionally active fragment thereof.

In one embodiment of the fusion polypeptide, the third domain comprises a LAG3 variant polypeptide.

In one embodiment of the fusion polypeptide, the third domain comprises a LAG3 variant polypeptide comprising a truncation and/or mutation based on human LAG3.

In one embodiment of the fusion polypeptide, the third domain comprises a LAG3 variant polypeptide, which comprises a truncation based on a wild-type human LAG3 extracellular region polypeptide.

In one embodiment of the fusion polypeptide, the third domain comprises a LAG3 variant polypeptide, which has 0-124 amino acids truncated at the N-terminus based on the wild-type human LAG3 extracellular region polypeptide.

In one embodiment of the fusion polypeptide, the third domain comprises a LAG3 variant polypeptide, which has 0, 5, 21, 37, 45, 60, 71, 74, 79, 84, 89, 94, 99, 104, 109 or 114 amino acids truncated at the N-terminus based on the wild-type human LAG3 extracellular region polypeptide.

In one embodiment of the fusion polypeptide, the third domain comprises a LAG3 variant polypeptide, which has 0, 5, 21, 37, 45, 60, 71 or 74 amino acids truncated at the N-terminus based on the wild-type human LAG3 extracellular region polypeptide.

In one embodiment of the fusion polypeptide, the third domain comprises a LAG3 variant polypeptide, which terminates at amino acid positions 122-167 at the C-terminus based on the wild-type human LAG3 extracellular region polypeptide.

In one embodiment of the fusion polypeptide, the third domain comprises a LAG3 variant polypeptide, which terminates at amino acid position 122, 129, 136, 146, 156, 157, 158, 159, 160, 161, 162, 163, 164, 165, 166 or 167 at the C-terminus based on the wild-type human LAG3 extracellular region polypeptide.

In one embodiment of the fusion polypeptide, the third domain comprises a LAG3 variant polypeptide, which terminates at amino acid position 122, 129, 136, 146, 156, 161 or 167 at the C-terminus based on the wild-type human LAG3 extracellular region polypeptide.

In one embodiment of the fusion polypeptide, the third domain comprises a LAG3 variant polypeptide, which terminates at amino acid position 156, 161 or 167 at the C-terminus based on the wild-type human LAG3 extracellular region polypeptide.

In one embodiment of the fusion polypeptide, the third domain comprises a LAG3 variant polypeptide, which has 0, 5, 21, 37, 45, 60, 71 or 74 amino acids truncated at the N-terminus based on the wild-type human LAG3 extracellular region polypeptide and terminates at amino acid position 122, 129, 136, 146, 156, 157, 158, 159, 160, 161, 162, 163, 164, 165, 166 or 167 at the C-terminus based on the wild-type human LAG3 extracellular region polypeptide.

In one embodiment of the fusion polypeptide, the third domain comprises a LAG3 variant polypeptide, and the LAG3 variant polypeptide comprises an amino acid mutation.

In one embodiment of the fusion polypeptide, the third domain comprises a LAG3 variant polypeptide, and the LAG3 variant polypeptide comprises an amino acid mutation, and the amino acid mutation site is the Arg amino acid at position 97.

In one embodiment of the fusion polypeptide, the third domain comprises a LAG3 variant polypeptide, and the LAG3 variant polypeptide comprises an amino acid mutation, wherein the amino acid mutation site is a mutation of the Arg amino acid at site 97 to a Glu amino acid.

In one embodiment of the fusion polypeptide, the third domain comprises an amino acid sequence selected from the group consisting of SEQ ID NO: 20 to SEQ ID NO: 28 and SEQ ID NO: 244 to SEQ ID NO: 349.

In one embodiment of the fusion polypeptide, the first domain is directly or indirectly linked to the second domain.

In one embodiment of the fusion polypeptide, the first domain is directly or indirectly linked to the third domain.

In one embodiment of the fusion polypeptide, the second domain is directly or indirectly connected to the third domain.

In one embodiment of the fusion polypeptide, the first domain is directly or indirectly connected to the second domain, and the first domain is directly or indirectly connected to the third domain.

In one embodiment of the fusion polypeptide, the first domain is directly or indirectly connected to the second domain, and the second domain is directly or indirectly connected to the third domain.

In one embodiment of the fusion polypeptide, the first domain is directly or indirectly connected to the third domain, and the second domain is directly or indirectly connected to the third domain.

In one embodiment of the fusion polypeptide, the indirect connection comprises connection through a linker.

In one embodiment of the fusion polypeptide, the linker comprises an amino acid sequence selected from the group consisting of SEQ ID NO: 31, SEQ ID NO: 32, SEQ ID NO: 33, SEQ ID NO: 34 and SEQ ID NO: 35.

In one embodiment, the fusion polypeptide comprises an amino acid sequence selected from the group consisting of SEQ ID NO: 751 to SEQ ID NO: 1024, 1249, 1251, 1253 and 1255.

On the other hand, the present application provides a LAG3 variant polypeptide, which comprises a truncation and/or mutation based on human LAG3.The present application provides an innovative modification of LAG3 polypeptide to obtain a functional LAG3 variant polypeptide, and a method for using the same to regulate the immune response of antigen presenting cells, thereby achieving the treatment or prevention of diseases such as infection and tumors caused by the inhibition of immune cell function.

In one embodiment, the LAG3 variant polypeptide comprises a truncation based on the wild-type human LAG3 extracellular region polypeptide.

In one embodiment, the LAG3 variant polypeptide has 0-124 amino acids truncated, such as 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123 or 124 amino acids truncated based on the wild-type human LAG3 extracellular region polypeptide; and the C-terminus terminates at any of the following position based on the wild-type LAG3 extracellular region polypeptide: amino acid positions 122-167 of the peptide, e.g., amino acid positions 122-167, 123-167, 124-167, 125-167, 126-167, 127-167, 128-167, 129-167, 130-167, 131-167, 132-167, 133-167, 134-167, 135-167, 136-167, 137-167, 138 -167, 139-167, 140-167, 141-167, 142-167, 143-167, 144-167, 145-167, 146-167, 147-167, 148-167, 149-167, 150-167, 151-167, 152-167, 153-1 67, 154-167, 155-167, 156-167, 157-1 67, 158-167, 159-167, 160-167, 161-167, 162-167, 163-167, 164-167, 165-167, 166-167, for example, the C-terminus terminates at the following positions: positions corresponding to amino acid positions 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166 or 167 of the wild-type LAG3 extracellular domain polypeptide.

In certain preferred embodiments, the LAG3 variant polypeptide has 0, 5, 21, 37, 45, 60, 71, 74, 79, 84, 89, 94, 99, 104, 109 or 114 amino acids truncated at the N-terminus based on the wild-type human LAG3 extracellular region polypeptide, and the C-terminus terminates at the following positions based on the wild-type LAG3 extracellular region polypeptide: 122, 129, 136, 146, 156, 157, 158, 159, 160, 161, 162, 163, 164, 165, 166 or 167.

In one embodiment of the LAG3 variant, the LAG3 variant polypeptide comprises an amino acid mutation, wherein the amino acid mutation site is the Arg amino acid at position 97.

In one embodiment of the LAG3 variant, the LAG3 variant polypeptide comprises an amino acid mutation, wherein the amino acid mutation site is a mutation of the Arg amino acid at position 97 to a Glu amino acid.

In certain preferred embodiments, based on the wild-type LAG3 extracellular region polypeptide, the truncated LAG3 variant polypeptide variant has at least one feature selected from the followingbased on:
(1) 74 amino acids is truncated at the N-terminus, and the C-terminus terminates at the following positions: positions corresponding to amino acid positions 122, 129, 136, 146, 156, 157, 158, 159, 160, 161, 162, 163, 164, 165, 166 or 167 based on the wild-type LAG3 extracellular region polypeptide; wherein, preferably, the C-terminus terminates at the following positions: positions corresponding to amino acid positions 122, 129, 136, 146, 156, 161 of the wild-type LAG3 extracellular region polypeptide.
(2) 74 amino acids is truncated at the N-terminus, the Arg amino acid at position 97 is mutated to a Glu amino acid, and the C-terminus terminates at the following positions: positions corresponding to amino acid positions 122, 129, 136, 146, 156, 157, 158, 159, 160, 161, 162, 163, 164, 165, 166 or 167 of the wild-type LAG3 extracellular region polypeptide; wherein, preferably, the C-terminus terminates at the following positions: positions corresponding to amino acid positions 122, 129, 136, 146, 156, 161 of the wild-type LAG3 extracellular region polypeptide.
(3) 5 amino acids is truncated at the N-terminus, and the C-terminus terminates at the following positions: the position corresponding to amino acid position 156, 161 or 167 of the wild-type LAG3 extracellular domain polypeptide.
(4) 5 amino acids is truncated at the N-terminus, the Arg amino acid at position 97 is mutated to a Glu amino acid, and the C-terminus terminates at the following positions: the position corresponding to amino acid position 156, 161 or 167 of the wild-type LAG3 extracellular domain polypeptide.
(5) 21 amino acids is truncated at the N-terminus, and the C-terminus terminates at the following positions: the position corresponding to amino acid position 156, 161 or 167 of the wild-type LAG3 extracellular domain polypeptide.
(6) 21 amino acids is truncated at the N-terminus, the Arg amino acid at position 97 is mutated to a Glu amino acid, and the C-terminus terminates at the following positions: the position corresponding to amino acid position 156, 161 or 167 of the wild-type LAG3 extracellular domain polypeptide.
(7) 37 amino acids is truncated at the N-terminus, and the C-terminus terminates at the following positions: the position corresponding to amino acid position 156, 161 or 167 of the wild-type LAG3 extracellular domain polypeptide.
(8) 37 amino acids is truncated at the N-terminus, the Arg amino acid at position 97 is mutated to a Glu amino acid, and the C-terminus terminates at the following positions: the position corresponding to amino acid position 156, 161 or 167 of the wild-type LAG3 extracellular domain polypeptide.
(9) 45 amino acids is truncated at the N-terminus, and the C-terminus terminates at the following positions: the position corresponding to amino acid position 156, 161 or 167 of the wild-type LAG3 extracellular domain polypeptide.
(10) 45 amino acids is truncated at the N-terminus, the Arg amino acid at position 97 is mutated to a Glu amino acid, and the C-terminus terminates at the following positions: the position corresponding to amino acid position 156, 161 or 167 of the wild-type LAG3 extracellular domain polypeptide.
(11) 60 amino acids is truncated at the N-terminus, and the C-terminus terminates at the following positions: the position corresponding to amino acid position 156, 161 or 167 of the wild-type LAG3 extracellular domain polypeptide.
(12) 60 amino acids is truncated at the N-terminus, the Arg amino acid at position 97 is mutated to a Glu amino acid, and the C-terminus terminates at the following positions: the position corresponding to amino acid position 156, 161 or 167 of the wild-type LAG3 extracellular domain polypeptide.
(13) 71 amino acids is truncated at the N-terminus, and the C-terminus terminates at the following positions: the position corresponding to amino acid position 156, 161 or 167 of the wild-type LAG3 extracellular domain polypeptide.
(14) 71 amino acids is truncated at the N-terminus, the Arg amino acid at position 97 is mutated to a Glu amino acid, and the C-terminus terminates at the following positions: the position corresponding to amino acid position 156, 161 or 167 of the wild-type LAG3 extracellular domain polypeptide.
(15) 79 amino acids is truncated at the N-terminus, and the C-terminus terminates at the following positions: the position corresponding to amino acid position 156, 161 or 167 of the wild-type LAG3 extracellular domain polypeptide.
(16) 84 amino acids is truncated at the N-terminus, and the C-terminus terminates at the following positions: the position corresponding to amino acid position 156, 161 or 167 of the wild-type LAG3 extracellular domain polypeptide.
(17) 89 amino acids is truncated at the N-terminus, and the C-terminus terminates at the following positions: the position corresponding to amino acid position 156, 161 or 167 of the wild-type LAG3 extracellular domain polypeptide.
(18) 94 amino acids is truncated at the N-terminus, and the C-terminus terminates at the following positions: the position corresponding to amino acid position 156, 161 or 167 of the wild-type LAG3 extracellular domain polypeptide.
(19) 99 amino acids is truncated at the N-terminus, and the C-terminus terminated at the following positions: the position corresponding to amino acid position 156, 161, or 167 of the wild-type LAG3 extracellular domain polypeptide.
(20) 104 amino acid is truncated at the N-terminus s, and the C-terminus terminates at the following positions: the position corresponding to amino acid position 156, 161 or 167 of the wild-type LAG3 extracellular domain polypeptide.
(21) 109 amino acids is truncated at the N-terminus, and the C-terminus terminates at the following positions: the position corresponding to amino acid position 156, 161 or 167 of the wild-type LAG3 extracellular domain polypeptide.
(22) 114 amino acids is truncated at the N-terminus, and the C-terminus terminates at the following positions: the position corresponding to amino acid position 156, 161 or 167 of the wild-type LAG3 extracellular domain polypeptide.
(23) 0 amino acids is truncated at the N-terminus, and the C-terminus terminates at the following positions: positions corresponding to amino acid positions 122, 129, 136, 146, 156, 161, or 167 of the wild-type LAG3 extracellular domain polypeptide.
(24) 0 amino acids is truncated at the N-terminus, the Arg amino acid at position 97 was mutated to a Glu amino acid, and the C-terminus terminated at the following positions: the position corresponding to amino acid position 122, 129, 136, 146, 156, 161, or 167 of the wild-type LAG3 extracellular domain polypeptide.

In one embodiment of the LAG3 variant, the LAG3 variant polypeptide is selected from the amino acid sequence shown in SEQ ID NO: 244 to SEQ ID NO: 349.

On the other hand, the present application provides a LAG3 variant polypeptide, which comprises a truncation and/or mutation based on human LAG3.

In certain embodiments, the LAG3 variant polypeptide comprises a truncation based on a wild-type human LAG3 extracellular region polypeptide.

In certain embodiments, the LAG3 variant polypeptide has 0-124 amino acids truncated at the N-terminus based on the wild-type human LAG3 extracellular region polypeptide, and terminates at amino acid positions 121-167 at the C-terminus based on the wild-type human LAG3 extracellular region polypeptide.

In certain embodiments, the LAG3 variant polypeptide has 0, 5, 21, 37, 45, 60, 71, 74, 79, 81, 84, 89, 94, 99, 104, 109 or 121 amino acids truncated at the N-terminus based on the wild-type human LAG3 extracellular region polypeptide.

In certain embodiments, the LAG3 variant polypeptide has 0, 5, 21, 37, 45, 60, 71, 74, 81 or 99 amino acids truncated at the N-terminus based on the wild-type human LAG3 extracellular region polypeptide.

In certain embodiments, the LAG3 variant polypeptide terminates at amino acid position 121, 122, 129, 136, 146, 156, 157, 158, 159, 160, 161, 162, 163, 164, 165, 166 or 167 at the C-terminus based on the wild-type human LAG3 extracellular region polypeptide.

In certain embodiments, the LAG3 variant polypeptide terminates atamino acid position 121, 122, 129, 136, 146, 156, 161 or 167 at the C-terminus based on the wild-type human LAG3 extracellular region polypeptide.

In certain embodiments, the LAG3 variant polypeptide terminates at amino acid position 121, 146, 156, 161 or 167 at the C-terminus based on the wild-type human LAG3 extracellular region polypeptide.

In certain embodiments, the LAG3 variant polypeptide has 0, 5, 21, 37, 45, 60, 71, 74, 81 or 99 amino acids truncated at the N-terminus based on the wild-type human LAG3 extracellular region polypeptide, and terminates at amino acid position 121, 122, 129, 136, 146, 156, 157, 158, 159, 160, 161, 162, 163, 164, 165, 166 or 167 at the C-terminus based on the wild-type human LAG3 extracellular region polypeptide.

In certain embodiments, the LAG3 variant polypeptide comprises an amino acid mutation.

In certain embodiments, the LAG3 mutant polypeptide comprises an amino acid mutation, and the amino acid mutation site is R110, R113, R119, R129, G130, and/or R141.

In certain embodiments, the LAG3 variant polypeptide has 81 amino acids truncated at the N-terminus and terminates at amino acid position 121 at the C-terminus, and mutations are introduced at site R110, and/or R113, and/or R119.

In certain embodiments, the LAG3 variant polypeptide has 99 amino acids truncated at the N-terminus and terminates at amino acid position 146 at the C-terminus, and mutations are introduced at site R129, and/or G130, and/or R141.

In certain embodiments, the LAG3 variant polypeptide has 81 amino acids truncated at the N-terminus and terminates at amino acid position 146 at the C-terminus, and mutations are introduced at site R110, and/or R113, and/or R119, and/or R129, and/or G130, and/or R141.

In certain embodiments, the LAG3 variant polypeptide comprises an amino acid mutation, and the R110 site is mutated to K110.

In certain embodiments, the LAG3 variant polypeptide comprises an amino acid mutation, wherein the R113 site is mutated to K113.

In certain embodiments, the LAG3 variant polypeptide comprises an amino acid mutation, wherein the R119 site is mutated to K119.

In certain embodiments, the LAG3 variant polypeptide comprises an amino acid mutation, and the R129 site is mutated to K129.

In certain embodiments, the LAG3 variant polypeptide comprises an amino acid mutation, wherein the G130 site is mutated to P130, or A130, or T130, or Y130, or S130.

In certain embodiments, the LAG3 variant polypeptide comprises an amino acid mutation, and the R141 site is mutated to K141.

In certain embodiments, the amino acid sequence of the LAG3 variant polypeptide is selected from SEQ ID NO:1285-SEQ ID NO:1318.

On the other hand, the present application provides a fusion polypeptide, which comprises a first domain and a second domain, wherein the first domain comprises the LAG3 variant polypeptide described in the present application, and the second domain comprises an antibody or an antigen-binding fragment thereof or an immunoglobulin domain.

In one embodiment of the fusion polypeptide, the second domain comprises an antibody or an antigen-binding fragment thereof.

In one embodiment of the fusion polypeptide, the second domain is capable of binding to one or more of the targets shown in the following group: PD-L1, PD-L2, PD-1, OX40, 4-1BB, ICOS, TIGIT, CTLA4, LAG3, CD3, VEGF, VEGFR, CD47, HGF, Trop2, EpCAM, CCR8, CCR4, CCR5, GPRC5D, BCMA, CD19, CD20, HER-2 neu, DLL1, HER-3, HER-4, EGFR, PSMA, CEA, MUC-1 (mucin), MUC2, MUC3, MUC4, MUC5AC, MUC5B, MUC7, CD123, CD33, CD30, CD38, NKG2A, Nkp36 and Tim3.

In one embodiment of the fusion polypeptide, the second domain is capable of binding to PD-L1, PD-1 and/or PD-L2.

In one embodiment of the fusion polypeptide, the second domain comprises HCDR1, HCDR2 and/or HCDR3 of an antibody heavy chain, wherein the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15 and SEQ ID NO: 17.

In one embodiment of the fusion polypeptide, the second domain comprises the heavy chain variable region VH of an antibody heavy chain, and the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15, and SEQ ID NO: 17.

In one embodiment of the fusion polypeptide, the second domain comprises an antibody heavy chain, which comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15 and SEQ ID NO: 17.

In one embodiment of the fusion polypeptide, the second domain comprises LCDR1, LCDR2 and/or LCDR3 of the light chain of an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

In one embodiment of the fusion polypeptide, the second domain comprises the light chain variable region VL of an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

In one embodiment of the fusion polypeptide, the second domain comprises an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

In one embodiment of the fusion polypeptide, the second domain comprises an antibody or an antigen-binding fragment thereof selected from the group consisting of Sugemalimab, Durvalumab, Atezolizumab, Avelumab, Envafolimab, Permbrolizumab and Nivolumab.

In one embodiment of the fusion polypeptide, the second domain comprises Sugemalimab or an antigen-binding fragment thereof.

In one embodiment of the fusion polypeptide, the antigen binding fragment is selected from one or more of the group consisting of: Fab, Fab', Fv fragment, F(ab')2, F(ab)2, scFv, di-scFv, VHH and dAb.

In one embodiment of the fusion polypeptide, the second domain comprises an immunoglobulin domain.

In one embodiment of the fusion polypeptide, the second domain comprises an immunoglobulin IgG antibody, and the immunoglobulin IgG antibody comprises one or more selected from human IgG1, human IgG2, human IgG3, human IgG4, mouse IgG1, mouse IgG2a, mouse IgG2b and mouse IgG3.

In one embodiment of the fusion polypeptide, the second domain comprises an immunoglobulin Fc domain.

In one embodiment of the fusion polypeptide, the second domain comprises the Fc domain of an immunoglobulin IgG antibody.

In one embodiment of the fusion polypeptide, the second domain comprises the Fc domain of an immunoglobulin IgG antibody, and the Fc domain of the immunoglobulin IgG antibody comprises a human IgG1 Fc domain, a human IgG2 Fc domain, a human IgG3 Fc domain, a human IgG4 Fc domain, a mouse IgG1 Fc domain, a mouse IgG2a Fc domain, a mouse IgG2b Fc domain or a mouse IgG3 Fc domain.

In one embodiment of the fusion polypeptide, the second domain comprises the Fc domain of human immunoglobulin IgG4.

In one embodiment of the fusion polypeptide, the second domain is selected from the group consisting of amino acid sequences shown in SEQ ID NO: 4 to SEQ ID NO: 18, SEQ ID NO: 29 to SEQ ID NO: 30, and SEQ ID NO: 634 to SEQ ID NO: 639.

In one embodiment of the fusion polypeptide, the first domain is directly or indirectly linked to the second domain.

In one embodiment of the fusion polypeptide, the first domain is directly or indirectly connected to the N-terminus of the second domain.

In one embodiment of the fusion polypeptide, the first domain is directly or indirectly connected to the C-terminus of the second domain.

In one embodiment of the fusion polypeptide, the indirect connection comprises connection through a linker.

In one embodiment of the fusion polypeptide, the linker comprises an amino acid sequence selected from the group consisting of SEQ ID NO: 31, SEQ ID NO: 32, SEQ ID NO: 33, SEQ ID NO: 34 and SEQ ID NO: 35.

In one embodiment of the fusion polypeptide, the fusion polypeptide comprises an amino acid sequence selected from the group consisting of SEQ ID NO: 640 to SEQ ID NO: 745 and SEQ ID NO: 747 to SEQ ID NO: 750.

In certain embodiments, the fusion polypeptide comprises an antibody heavy chain, preferably the antibody heavy chain polypeptide comprises the amino acid sequence as shown in SEQ ID NO: 4; the amino acid sequence comprising the antibody heavy chain is selected from SEQ ID NO: 1353 to SEQ ID NO: 1386.

In certain embodiments, the fusion polypeptide comprises a light chain, and the light chain comprises an antibody light chain polypeptide, preferably the antibody light chain polypeptide comprising the amino acid sequence shown in SEQ ID NO:5.

In certain embodiments, the fusion polypeptide heavy chain and the fusion polypeptide light chain are assembled into the fusion polypeptide.

In certain embodiments, the first domain is directly or indirectly linked to the second domain.

In certain embodiments, the first domain is directly or indirectly connected to the N-terminus of the second domain.

In certain embodiments, the first domain is directly or indirectly connected to the C-terminus of the second domain.

In certain embodiments, the second domain may comprise an antibody heavy chain, and the LAG3 variant polypeptide may be directly or indirectly linked to the antibody heavy chain of the second domain.

In certain embodiments, the second domain may comprise an antibody heavy chain, and the LAG3 variant polypeptide may be directly or indirectly linked to the C-terminus of the antibody heavy chain of the second domain.

In certain embodiments, the second domain may comprise an antibody heavy chain, and the N-terminus of the LAG3 variant polypeptide may be directly or indirectly linked to the C-terminus of the antibody heavy chain of the second domain.

In certain embodiments, the second domain may comprise an antibody heavy chain, and the LAG3 variant polypeptide may be directly or indirectly linked to the N-terminus of the antibody heavy chain of the second domain.

In certain embodiments, the second domain may comprise an antibody heavy chain, and the C-terminus of the LAG3 variant polypeptide may be directly or indirectly linked to the N-terminus of the antibody heavy chain of the second domain.

In certain embodiments, the second domain may comprise an antibody light chain, and the LAG3 variant polypeptide may be directly or indirectly linked to the antibody light chain of the second domain.

In certain embodiments, the second domain may comprise an antibody light chain, and the LAG3 variant polypeptide may be directly or indirectly linked to the antibody light chain C-terminus of the second domain.

In certain embodiments, the second domain may comprise an antibody light chain, and the N-terminus of the LAG3 variant polypeptide may be directly or indirectly linked to the C-terminus of the antibody light chain of the second domain.

In certain embodiments, the second domain may comprise an antibody light chain, and the LAG3 variant polypeptide may be directly or indirectly linked to the N-terminus of the antibody light chain of the second domain.

In certain embodiments, the second domain may comprise an antibody light chain, and the C-terminus of the LAG3 variant polypeptide may be directly or indirectly linked to the N-terminus of the antibody light chain of the second domain.

In certain embodiments, the indirect connection comprises connection through a linker.

In certain embodiments, the second domain comprises an immunoglobulin Fc domain.

In certain embodiments, the second domain comprises the Fc domain of an immunoglobulin IgG antibody.

In certain embodiments, the second domain comprises the Fc domain of an immunoglobulin IgG antibody, and the Fc domain of the immunoglobulin IgG antibody comprises a human IgG1 Fc domain, a human IgG2 Fc domain, a human IgG3 Fc domain, a human IgG4 Fc domain, a mouse IgG1 Fc domain, a mouse IgG2a Fc domain, a mouse IgG2b Fc domain, or a mouse IgG3 Fc domain.

In certain embodiments, the second domain comprises the Fc domain of human immunoglobulin IgG4.

In certain embodiments, the LAG3 variant polypeptide is covalently linked to the second domain with or without a linker peptide.

In certain embodiments, the second domain is selected from the group consisting of amino acid sequences shown in SEQ ID NO: 1313 to SEQ ID NO: 1352.

In certain embodiments, the fusion polypeptide further comprises a third domain, and the third domain is an antibody or antigen-binding fragment or a functional protein or an active fragment thereof that is the same as or different from the second domain.

On the other hand, the present application provides a fusion polypeptide, which comprises a first domain, a second domain and a third domain, wherein the first domain comprises the LAG3 variant polypeptide described in the present application, the second domain comprises an antibody or an antigen-binding fragment thereof or an immunoglobulin domain, and the third domain is an antibody or antigen-binding fragment or a functional protein or an active fragment thereof that is the same or different from the second domain.

In one embodiment of the fusion polypeptide, the second domain comprises an antibody or an antigen-binding fragment thereof.

In one embodiment of the fusion polypeptide, the second domain is capable of binding to one or more of the targets shown in the following group: PD-L1, PD-L2, PD-1, OX40, 4-1BB, ICOS, TIGIT, CTLA4, LAG3, CD3, VEGF, VEGFR, CD47, HGF, Trop2, EpCAM, CCR8, CCR4, CCR5, GPRC5D, BCMA, CD19, CD20, HER-2 neu, DLL1, HER-3, HER-4, EGFR, PSMA, CEA, MUC-1 (mucin), MUC2, MUC3, MUC4, MUC5AC, MUC5B, MUC7, CD123, CD33, CD30, CD38, NKG2A, Nkp36 and Tim3.

In one embodiment of the fusion polypeptide, the second domain is capable of binding to PD-L1, PD-1 and/or PD-L2. In one embodiment of the fusion polypeptide, the second domain comprises HCDR1, HCDR2 and/or HCDR3 of an antibody heavy chain, wherein the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15 and SEQ ID NO: 17.

In one embodiment of the fusion polypeptide, the second domain comprises the heavy chain variable region VH of an antibody heavy chain, and the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15, and SEQ ID NO: 17.

In one embodiment of the fusion polypeptide, the second domain comprises an antibody heavy chain, which comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15 and SEQ ID NO: 17.

In one embodiment of the fusion polypeptide, the second domain comprises LCDR1, LCDR2 and/or LCDR3 of the light chain of an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

In one embodiment of the fusion polypeptide, the second domain comprises the light chain variable region VL of an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

In one embodiment of the fusion polypeptide, the second domain comprises an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

In one embodiment of the fusion polypeptide, the second domain comprises an antibody or an antigen-binding fragment thereof selected from the group consisting of Sugemalimab, Durvalumab, Atezolizumab, Avelumab, Envafolimab, Permbrolizumab and Nivolumab.

In one embodiment of the fusion polypeptide, the second domain comprises Sugemalimab or an antigen-binding fragment thereof.

In one embodiment of the fusion polypeptide, the antibody is selected from the group consisting of an immunoglobulin antibody, a recombinant antibody, a chimeric antibody, a heavy chain antibody, a single domain antibody, and a bispecific antibody.

In one embodiment of the fusion polypeptide, the antigen binding fragment is selected from one or more of the group consisting of: Fab, Fab', Fv fragment, F(ab')2, F(ab)2, scFv, di-scFv, VHH and dAb.

In one embodiment of the fusion polypeptide, the second domain comprises an immunoglobulin domain.

In one embodiment of the fusion polypeptide, the second domain comprises an immunoglobulin IgG antibody, and the immunoglobulin IgG comprises one or more selected from human IgG1, human IgG2, human IgG3, human IgG4, mouse IgG1, mouse IgG2a, mouse IgG2b and mouse IgG3.

In one embodiment of the fusion polypeptide, the second domain comprises an immunoglobulin Fc domain.

In one embodiment of the fusion polypeptide, the second domain comprises an immunoglobulin IgG Fc domain, and the immunoglobulin Fc domain comprises a human IgG1 Fc domain, a human IgG2 Fc domain, a human IgG3 Fc domain, a human IgG4 Fc domain, a mouse IgG1 Fc domain, a mouse IgG2a Fc domain, a mouse IgG2b Fc domain or a mouse IgG3 Fc domain.

In one embodiment of the fusion polypeptide, the immunoglobulin Fc domain comprises the Fc domain of human immunoglobulin IgG4.

In one embodiment of the fusion polypeptide, the second domain is selected from the group consisting of amino acid sequences shown in SEQ ID NO: 4 to SEQ ID NO: 18, SEQ ID NO: 29 to SEQ ID NO: 30, and SEQ ID NO: 634 to SEQ ID NO: 639.

In one embodiment of the fusion polypeptide, the third domain is a functional protein or an active fragment thereof, and the functional protein or the active fragment thereof can activate the innate immune response.

In one embodiment of the fusion polypeptide, the third domain is capable of binding to CD28 and/or CTLA4.

In one embodiment of the fusion polypeptide, the third domain comprises CD80, CD86 or an active fragment thereof.

In one embodiment of the fusion polypeptide, the third domain is selected from the following group: CD86 derived from humans or a functionally active fragment thereof, CD86 derived from mice or a functionally active fragment thereof, CD80 derived from humans or a functionally active fragment thereof, and CD80 derived from mice or a functionally active fragment thereof.

In one embodiment of the fusion polypeptide, the third domain comprises the IgV domain of CD80, the IgV domain of CD86 or an active fragment thereof.

In one embodiment of the fusion polypeptide, the third domain comprises the extracellular domain of CD80, the extracellular domain of CD86 or an active fragment thereof.

In one embodiment of the fusion polypeptide, the third domain comprises a CD86 variant polypeptide.

In one embodiment of the fusion polypeptide, the third domain comprises a CD86 variant polypeptide, and the mutant comprises an amino acid substitution mutation of human CD86.

In one embodiment of the fusion polypeptide, the third domain comprises a CD86 variant polypeptide, which comprises an amino acid substitution mutant of the IgV domain of CD86 and/or an amino acid substitution mutant of the extracellular domain of human CD86.

In one embodiment of the fusion polypeptide, the third domain comprises a CD86 variant polypeptide, which comprises an amino acid substitution mutant of the IgV domain of CD86, and the mutation site of the amino acid substitution mutant of the IgV domain of CD86 comprises one or more of the amino acid mutations selected from the group consisting of A13I, A13L, A13F, A13M, Q25A, Q25I, Q25V, Q25F, Q25M, F33A, F33L, F33V, F33M, M60R, I89A, I89L, I89V, I89F, I89M, H90I, H90V, H90M, and H90F.

In one embodiment of the fusion polypeptide, the third domain comprises a CD86 variant polypeptide, which comprises an amino acid substitution mutant of the extracellular domain of CD86, and the amino acid substitution mutant of the extracellular domain of CD86 comprises one or more amino acid mutations selected from the group consisting of A13I, A13L, A13F, A13M, Q25A, Q25I, Q25V, Q25F, Q25M, F33A, F33L, F33V, F33M, M60R, I89A, I89L, I89V, I89F, I89M, H90I, H90V, H90M, and H90F.

In one embodiment of the fusion polypeptide, the third domain is selected from the group consisting of amino acid sequences shown in: SEQ ID NO: 2 to SEQ ID NO: 3, SEQ ID NO: 178 to SEQ ID NO: 243.

In certain embodiments, the IgV or extracellular domain amino acid substitution mutant of CD86 can be selected from the combination of amino acid mutation sites shown in the following group: Q25I/F33L/H90I, Q25V/F33L/H90I, Q25I/F33V/H90I, Q25V/F33V/H90I, Q251/F33L/H90V, Q25V/F33L/H90V, Q25I/F33V/H90V, Q25V/F33V/H90V, A13L/Q25I/F33L/H90I, A13I/Q25I/F33L/H90I, A13L/Q25V/F33L/H90I, A13I/Q25V/F33L/H90I, Q25I/F33L/I89L/H90I,
Q25I/F33L/M60R/H90I, Q25V/F33L/I89L/H90I, Q25V/F33L/M60R/H90I, Q25F/F33L/H90I, Q25I/F33L/H90F, Q25I/F33A/H90I, Q25I/H90I, Q25I, H90I, Q25V/H90V, Q25V/H90I, Q25F/H901, Q25F/H90V, A13F/Q25I/F33L/H90I, A13M/Q25I/F33L/H90I, Q25A/F33L/H90I, Q25M/F33L/H90I, Q25I/F33M/H90I, Q25I/F33L/I89V/H90I, Q25I/F33L/I89F/H90I, Q25I/F33L/I89M/H90I and Q25I/F33L/H90M.

In certain embodiments, the IgV or extracellular domain amino acid substitution mutant of CD86 includes the following amino acid mutation sites: Q25I/F33L/H90I.

In one embodiment of the fusion polypeptide, the first domain and the second domain are directly or indirectly linked.

In one embodiment of the fusion polypeptide, the first domain is directly or indirectly linked to the third domain.

In one embodiment of the fusion polypeptide, the second domain and the third domain are directly or indirectly connected.

In one embodiment of the fusion polypeptide, the first domain is directly or indirectly connected to the second domain, and the first domain is directly or indirectly connected to the third domain.

In one embodiment of the fusion polypeptide, the first domain is directly or indirectly connected to the second domain, and the second domain is directly or indirectly connected to the third domain.

In one embodiment of the fusion polypeptide, the first domain is directly or indirectly connected to the third domain, and the second domain is directly or indirectly connected to the third domain.

In one embodiment of the fusion polypeptide, the indirect connection comprises connection through a linker.

In one embodiment of the fusion polypeptide, the linker comprises an amino acid sequence selected from the group consisting of SEQ ID NO: 31, SEQ ID NO: 32, SEQ ID NO: 33, SEQ ID NO: 34 and SEQ ID NO: 35.

In one embodiment, the fusion polypeptide comprises an amino acid sequence selected from the group consisting of SEQ ID NO: 751 to SEQ ID NO: 1024, 1249, 1251, 1253 and 1255.

In certain embodiments, the fusion polypeptide comprises the heavy chain of the multifunctional LAG3 variant fused Sugemalimab-CD86 IgV Q25I/F33L/H90I antibody polypeptide complex, and the heavy chain amino acid sequence is selected from SEQ ID NO: 1387-SEQ ID NO: 1420.

In certain embodiments, the fusion polypeptide comprises the heavy chain of the multifunctional LAG3 variant fused Sugemalimab-CD86 IgV Q25I/F33L/H90I antibody polypeptide complex, and the heavy chain amino acid sequence is selected from SEQ ID NO: 1421 and SEQ ID NO: 1426.

In certain embodiments, the nucleic acid encoding the fusion polypeptide comprises the nucleotide sequence shown in any one of SEQ ID NOs: 1422-1425, 1427-1433.

On the other hand, the present application provides an immunoconjugate comprising the fusion polypeptide described in the present application, the CD86 variant polypeptide described in the present application and/or the LAG3 variant described in the present application.

On the other hand, the present application provides a nucleic acid molecule encoding the fusion polypeptide described in the present application, the CD86 variant polypeptide described in the present application and/or the LAG3 variant described in the present application.

In another aspect, the present application provides a vector comprising the nucleic acid molecule described in the present application.

On the other hand, the present application provides a cell, which contains and/or expresses the fusion polypeptide described in the present application, the CD86 variant polypeptide described in the present application, the LAG3 variant described in the present application, the immunoconjugate described in the present application, the nucleic acid molecule described in the present application, and/or the vector described in the present application.

On the other hand, the present application provides a composition comprising the fusion polypeptide described herein, the immunoconjugate described herein, the nucleic acid molecule described herein, the vector described herein, and/or the cell described herein, and optionally a pharmaceutically acceptable carrier.

On the other hand, the present application provides a method for preparing the fusion polypeptide described in the present application, the CD86 variant polypeptide described in the present application, and the LAG3 variant described in the present application, which comprises culturing the cell described in the present application under conditions that allow the fusion polypeptide to be expressed.

On the other hand, the present application provides a method for blocking the interaction between PD-L1 protein and PD-1, which comprises administering an effective amount of the fusion polypeptide described in the present application, the CD86 variant polypeptide described in the present application, the LAG3 variant described in the present application, the immunoconjugate described in the present application, the nucleic acid molecule described in the present application, the vector described in the present application, the cell described in the present application, and/or the composition described in the present application.

On the other hand, the present application provides a method for stimulating antigen-presenting cells and/or activating T cells, which comprises administering an effective amount of the fusion polypeptide described herein, the CD86 variant polypeptide described herein, the LAG3 variant described herein, the immunoconjugate described herein, the nucleic acid molecule described herein, the vector described herein, the cell described herein, and/or the composition described herein.

According to the method described in the present application, the stimulation of antigen presenting cells comprises a step selected from the group consisting of: increasing the expression of co-stimulatory molecules in antigen presenting cells, causing morphological changes and maturation of antigen presenting cells, increasing the secretion of chemokines in antigen presenting cells, and enhancing the phagocytic ability of antigen presenting cells.

On the other hand, the present application provides a method for inhibiting the growth and/or proliferation of tumors or tumor cells, which comprises administering an effective amount of the fusion polypeptide described herein, the CD86 variant polypeptide described herein, the LAG3 variant described herein, the immunoconjugate described herein, the nucleic acid molecule described herein, the vector described herein, the cell described herein, and/or the composition described herein.

On the other hand, the present application provides the use of the fusion polypeptide described in the present application, the CD86 variant polypeptide described in the present application, the LAG3 variant described in the present application, the immunoconjugate described in the present application, the nucleic acid molecule described in the present application, the vector described in the present application, the cell described in the present application, and/or the composition described in the present application in the preparation of a medicament, wherein the medicament is used to prevent, improve and/or treat tumors.

According to the use described in the present application, the tumor comprises a solid tumor and/or a hematological tumor.

According to the use described in the present application, the tumor is selected from the following group: colon tumor, breast tumor, lung tumor, gastric tumor, melanoma, head and neck tumor, lymphoma, nasopharyngeal tumor, cervical tumor, esophageal tumor, kidney tumor, skin squamous cell carcinoma, endometrial tumor, liver tumor, bladder tumor, urothelial tumor and skin tumor.

On the other hand, the present application provides the fusion polypeptide described in the present application, the immunoconjugate described in the present application, the nucleic acid molecule described in the present application, the vector described in the present application, the cell described in the present application, and/or the composition described in the present application, which are used to prevent, improve and/or treat tumors.

On the other hand, the present application provides the fusion polypeptide described in the present application, the immunoconjugate described in the present application, the nucleic acid molecule described in the present application, the vector described in the present application, the cell described in the present application, and/or the composition described in the present application, which are used to prevent, improve and/or treat tumors, wherein the tumors include solid tumors and hematological tumors.

On the other hand, the present application provides the fusion polypeptide described in the present application, the immunoconjugate described in the present application, the nucleic acid molecule described in the present application, the vector described in the present application, the cell described in the present application, and/or the composition described in the present application, which are used to prevent, improve and/or treat tumors, wherein the tumor is selected from the following group: colon tumors, breast tumors, lung tumors, gastric tumors, melanoma, head and neck tumors, lymphomas, nasopharyngeal tumors, cervical tumors, esophageal tumors, kidney tumors, skin squamous cell carcinoma, endometrial tumors, liver tumors, bladder tumors, urothelial tumors and skin tumors.

On the other hand, the present application provides a method for preventing, improving and/or treating tumors, which may comprise administering the fusion polypeptide described herein, the immunoconjugate described herein, the nucleic acid molecule described herein, the vector described herein, the cell described herein, and/or the composition described herein to a subject in need thereof.

According to the method described in the present application, the tumor includes solid tumors and hematological tumors.

According to the method described in the present application, the tumor is selected from the following group: colon tumor, breast tumor, lung tumor, gastric tumor, melanoma, head and neck tumor, lymphoma, nasopharyngeal tumor, cervical tumor, esophageal tumor, kidney tumor, skin squamous cell carcinoma, endometrial tumor, liver tumor, bladder tumor, urothelial tumor and skin tumor.

Those skilled in the art can easily discern other aspects and advantages of the present application from the following detailed description. Only exemplary embodiments of the present application are shown and described in the following detailed description. As those skilled in the art will appreciate, the teachings of this application enable those skilled in the art to make modifications to the specific embodiments disclosed without departing from the spirit and scope of the invention to which this application relates. Accordingly, the drawings and descriptions in the specification of the present application are merely illustrative and not restrictive.

### BRIEF DESCRIPTION OF THE DRAWINGS

The particular features of the invention to which this application relates are set forth in the appended claims. The features and advantages of the invention involved in this application can be better understood by referring to the exemplary embodiments described in detail below and the accompanying drawings. A brief description of the accompanying drawings is as follows:

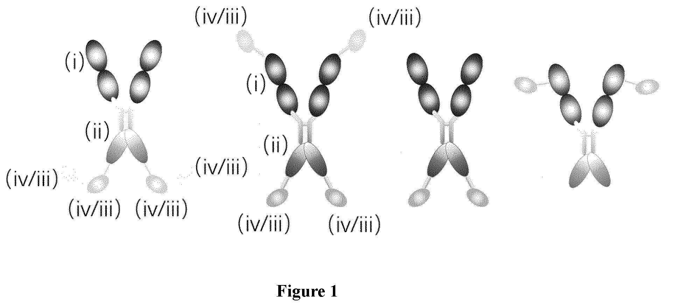

FIG. 1 shows an exemplary structural diagram of the fusion polypeptide described in the present application. The present application provides an exemplary structure that may include any one or more subunits in the first domain derived from any anti-PD-L1 and/or anti-PD-1 antibody antigen recognition region (i), an immunoglobulin Fc region (ii), the second domain (iii) and/or the third domain (iv) (the second domain (iii) and/or the third domain (iv) having 1 subunit are shown here, although two or more tandem connections of the same subunit may be considered, and although it may extend to any subunit mixed combination in tandem in (iv); the first domain is connected to the second domain via a linker or covalently through the immunoglobulin Fc region, and similarly, the subunits of the third domain and the second domain may be connected via a linker or covalently.
FIG. 2 shows the result of using SDS-PAGE to detect the expression of the fusion polypeptide complex of the present application.
FIG3 shows the use of ELISA to detect the binding of the fusion polypeptide complex of the present application to the human PDL1 protein.
FIG. 4 shows the results of using ELISA to detect the binding of the fusion polypeptide complex of the present application to the human CTLA4 protein.
FIG. 5 shows the results of using ELISA to detect the binding between the fusion polypeptide complex of the present application and the human CD28 protein.
FIG. 6 shows the results of the regulation of Jurkat T cell activation by the fusion polypeptide complex of the present application.
FIG. 7 shows the result of the fusion polypeptide complex of the present application regulating the up-regulation of the expression of co-stimulatory molecules in antigen presenting cells.
FIG 8 shows the result of the fusion polypeptide complex of the present application regulating the morphological differentiation of antigen presenting cells.
FIG. 9 shows the result of the fusion polypeptide complex of the present application regulating antigen presentation and secretion of CCL4.
FIG. 10 shows the expression of CD86 mutant fusion polypeptide complex detected by SDS-PAGE.
FIG. 11 shows the expression of CD86 variant polypeptide fusion polypeptide complex detected by AKTA.
FIG. 12 shows the result of the fusion polypeptide complex of the present application regulating antigen presentation and secretion of CCL4.
FIG. 13 shows the effect of the fusion polypeptide complex of the present application on inhibiting tumor growth.
FIG. 14 shows the result of using SDS-PAGE to detect the expression of the LAG3 variant polypeptide complex of the present application.
FIG. 15 shows the result of the fusion polypeptide complex of the present application regulating the secretion of CCL4 by antigen presenting cells.
FIG. 16 shows the result of the fusion polypeptide complex of the present application regulating the secretion of CCL4 by primary PBMC cells.
FIG. 17 shows the results of the fusion polypeptide complex of the present application regulating the morphological differentiation of primary DCs cells.
FIG. 18 shows the result of the fusion polypeptide complex of the present application regulating the up-regulation of the expression of co-stimulatory molecules in primary DCs cells.
FIG. 19 shows the result of the fusion polypeptide complex of the present application regulating the secretion of TNFa by primary DCs cells.
FIG. 20 shows the results of the fusion polypeptide complex of the present application regulating T cell activation.
FIG. 21 shows the results of the LAG3 variant polypeptide complex of the present application regulating the morphological differentiation of primary DCs cells.
FIG. 22 shows the results of the LAG3 variant polypeptide complex of the present application regulating the up-regulation of co-stimulatory molecule expression in primary DCs cells.
FIG. 23 shows the results of the LAG3 variant polypeptide complex of the present application regulating the secretion of TNFa by primary DCs cells.
FIG. 24 shows the desugar-reduced LC-MS mass analysis of the LAG3 variant polypeptide complex of the present application.

### Embodiments

The following is an explanation of the implementation of the present invention by means of specific embodiments. Those skilled in the art can easily understand other advantages and effects of the present invention from the contents disclosed in this specification.

### Definition of terms

In the present application, the term "regulation of T cell immune response" generally refers to the regulation of lymphatic T cell action by the dual and/or multifunctional fusion polypeptide disclosed in this application. For example, it can be extended to any other related T cell regulators that affect the expression of T cell NFAT transcription factors, the secretion of cytokines such as IL-2, IFN-γ, TNFα and Granzyme B, cell proliferation and effects such as cell killing of target cells including tumor cells, The T cells can be expanded to T cell lines and/or primary T cells including but not limited to CD4 T cells and CD4 T cell subsets Th1, Th2, Th9, Th17, TFH and/or Treg cells, etc.; also includes CD8 T cells include but are not limited to tumor tissue infiltrating CD8 T cells, effector CD8 T cells, immune memory CD8 T cells, etc.

In the present application, the term "stimulating antigen-presenting cells" generally refers to the regulation of antigen-presenting cell activation by the dual and/or multifunctional fusion polypeptides disclosed in this application. For example, it can also be extended to any other antigen-presenting cell regulator that affects the expression of antigen-presenting cells after treating the antigen-presenting cells, including but not limited to the costimulatory molecule CD40 or/and CD80 or/and CD83 or/and CD86. , activation and differentiation and maturation of antigen-presenting cells, antigen phagocytosis and presentation, production of chemokines CCL4, CCL22, CCL17, etc., expression of cytokines IL-6, IL-12, TNFa, IL-1β, etc., recruitment of T cells Migrate to secondary lymphoid organs and enhance T cell immune responses and other effects; the antigen-presenting cells can be expanded to but are not limited to B lymphocyte lines and primary B lymphocytes, monocyte lines and primary monocytes , macrophage cell lines and primary macrophages, dendritic cell (DC) cell lines and primary DC cells, etc.

In the present application, the term "relieving the inhibition of T cells by PD-L1/PD-1"generally refers to the dual and/or multifunctional fusion polypeptides disclosed in this application that bind to the PDL1 protein and block the binding of PD-L1 to PD-1, thereby improving the inhibition of T cells by PD-L1/PD-1, including but not limited to expressing the NFAT transcription factor on T cells, secreting cytokines such as IL-2, IFN-γ, TNFα and Granzyme B, and having effects on cell proliferation and cell killing of target cells.

In this application, the term "anti-tumor activity" generally refers to any biological activity that reduces or prevents the proliferation or viability of tumor cells in vivo and/or in vitro. In one embodiment, the anti-tumor activity is the anti-tumor effect possessed by the dual and/or multifunctional fusion polypeptide described in the present application.

In this application, the term "CD80" generally refers to a class of molecules that activate cells. For example, CD86 in this application includes its full length, variants, and/or functionally active fragments. For example, CD80 can refer to a polypeptide or fragment thereof that has at least about 85% amino acid identity with the protein encoded by the NCBI accession number Gene ID: 942 gene and has binding activity to CD28 (protein encoded by NCBI accession number Gene ID: 940 gene) and/or CTLA4 (protein encoded by NCBI accession number Gene ID: 1493 gene). Provided below is exemplary human CD80amino acid sequence (SEQ ID NO: 1); term "CD86 extracellular region" generally refers to a polypeptide or fragment thereof that has amino acid sequence of CD86 protein extracellular region or amino acid sequence that has at least about 85% amino acid identity, and has binding activity to CD28 and/or CTLA4; provided below is exemplary CD86 extracellular region amino acid sequence (SEQ ID NO: 2). The term "CD86 extracellular domain IgV domain" and "CD86 IgV domain" refer to the amino acid sequence of the IgV-like domain within the extracellular region of the CD86 protein, which exhibits at least about 85% amino acid identity and possesses binding activity to CD28 and/or CTLA4, or a polypeptide fragment thereof. An exemplary amino acid sequence of the CD86 extracellular domain IgV domain is provided below (SEQ ID NO: 3). The number of tandem combinations disclosed in this application is not limited to the number shown in the example, and any tandem combination including this functional domain can be regarded as within the scope of this application.

In this application, the term "anti-PD-L1 antibody" generally refers to an antibody that selectively binds and has the activity of blocking PD-L1 polypeptide. For example, Chinese Patent No. CN102245640(B), U.S. Patent Application Publication No. 7,943,743, U.S. Patent Application Publication No. 8,779,108 and U.S. Patent Application Publication No. 7,943,743, which are incorporated herein by reference; including but not limited to anti-PD-L1 antibodies such as Aspen Durvalumab (MEDI4736) developed by AstraZeneca, sugemalimab (CS1001) developed by CStone Pharmaceuticals, JS003 developed by Junshi Pharmaceuticals, Envafolimab (KN035) developed by Corning Jirui, Atezolizumab (MPDL3280A) developed by Roche and Germany Avelumab (MSB0010718C) jointly developed by Merck KGaA/Pfizer. The following provides an exemplary anti-PD-L1 antibody sugemalimab (CS1001) heavy chain polypeptide amino acid sequence (SEQ ID NO: 4) and light chain polypeptide amino acid sequence Sequence (SEQ ID NO: 5), JS003 heavy chain polypeptide amino acid sequence (SEQ ID NO: 6) and light chain polypeptide amino acid sequence (SEQ ID NO: 7), Durvalumab (MEDI4736) heavy chain polypeptide amino acid sequence (SEQ ID NO: 8) and light chain polypeptide amino acid sequence (SEQ ID NO: 9), atezolizumab (MPDL3280A) heavy chain polypeptide amino acid sequence (SEQ ID NO: 10) and light chain polypeptide amino acid sequence (SEQ ID NO: 11), Avelumab (MSB0010718C) The amino acid sequence of the heavy chain polypeptide (SEQ ID NO: 12), the amino acid sequence of the light chain polypeptide (SEQ ID NO: 13) and the amino acid sequence of Envafolimab (KN035) (SEQ ID NO: 14).

In this application, the term "anti-PD-1 antibody" generally refers to an antibody that selectively binds and has the activity of blocking PD-1 polypeptide. For example, U.S. Patent Application Publication No. 8,354,509 and U.S. Patent Application Publication No. 7,488,802, which are incorporated herein by reference; including but not limited to anti-PD-1 antibodies such as Permbrolizumab (MK-3475) developed by Merck and Bristol- For Nivolumab (BMS-936558) developed by BMS, the amino acid sequence of the heavy chain polypeptide (SEQ ID NO: 15) and light chain polypeptide amino acid sequence of an exemplary anti-PD-1 antibody Permbrolizumab (MK-3475) are provided below (SEQ ID NO: 16) and Nivolumab (BMS-936558) heavy chain polypeptide amino acid sequence (SEQ ID NO: 17) and light chain polypeptide amino acid sequence (SEQ ID NO: 18).

In this application, the term "LAG3" generally refers to a class of proteins or polypeptides. For example, LAG3 in this application includes its full length, variants, and/or functionally active fragments. For example, LAG3 may refer to a polypeptide or its fragment that has at least about 85% amino acid identity with the protein encoded by the NCBI accession number Gene ID: 3902 gene of LAG3 (Lymphocyte activation gene 3): and has binding activity and/or activates antigen presenting cells to a complex protein MHCII (or HLA-DR) assembled from alpha subunit (the protein encoded by the NCBI accession number Gene ID: 3122 gene) and the β subunit (the protein encoded by the NCBI accession number Gene ID: 3123 gene, or the NCBI accession number Gene ID: 3125 gene) and/or FGL1 (Protein encoded by NCBI accession number Gene ID: 2267 gene); provided below is an exemplary human LAG3 amino acid sequence (SEQ ID NO: 19); the term "LAG3 extracellular region" usually refers to a polypeptide or fragment thereof that has the amino acid sequence of the extracellular region of the full-length LAG3 protein (a polypeptide of NCBI number 23-450 amino acids in the LAG3 protein; the extracellular region starts at amino acid position 23, so for example, if 74 amino acids is truncated, the truncation can start at position 97) or the amino acid sequence that has at least about 85% amino acid identity, and has binding activity to MHCII and/or FGL1; provided below is an exemplary human LAG3 extracellular region amino acid sequence (SEQ ID NO: 20) and a tandem human LAG3 extracellular region amino acid sequence (SEQ ID NO: 21) covalently linked by a linker peptide; the number of tandems disclosed in this application is not limited to the number shown in the example, any tandem combination comprising this functional domain all can be regarded as the category of this application.

In this application, the term "LAG3-IgD1" generally refers to a polypeptide or fragment thereof that has an amino acid sequence of the Ig-like V-type domain in the extracellular region of human LAG3 (or an amino acid sequence has at least about 85% amino acid identity). Provided below is exemplary human LAG3-IgD1 amino acid sequence (SEQ ID NO: 22); term "LAG3-IgD2" generally refers to a polypeptide or fragment thereof that has an amino acid sequence of Ig-like C2-type 1 domain of human LAG3 extracellular region (or an amino acid sequence has at least about 85%amino acid identity); provided below is exemplary human LAG3-IgD2 amino acid sequence (SEQ ID NO: 23); term "LAG3-IgD3" generally refers to amino acid sequence of human LAG3 extracellular region Ig-like C2-type 2 domain (or an amino acid sequence has at least about 85% amino acid identity); provided below is exemplary human LAG3-IgD3amino acid sequence (SEQ ID NO: 24); term "LAG3-IgD4" generally refers to amino acid sequence of human extracellular region LAG3 Ig-like C2-type 3 (or an amino acid sequence has at least about 85% amino acid identity); provided below is exemplary human LAG3-IgD4amino acid sequence (SEQ ID NO: 25); term "LAG3-IgD1/IgD2" generally refers to a polypeptide or fragment thereof that has amino acid sequence of human LAG3-IgD1 and human LAG3-IgD2 (or an amino acid sequence has at least about 85%amino acid identity), and has binding activity to MHCII and/or FGL1; provided below is exemplary human LAG3-IgD1/IgD2 amino acid sequence (SEQ ID NO: 26); The number of tandem combinations disclosed in this application is not limited to the number shown in the example, any tandem combination comprising LAG3-IgD1/IgD2 functional domain can be deemed as within the scope of this application; term "LAG3-IgD1/IgD2/IgD3" generally refers to a polypeptide or fragment thereof that has amino acid sequence of human LAG3-IgD1, human LAG3-IgD2 and human LAG3-IgD3 (or an amino acid sequence has at least about 85% amino acid identity) and has binding activity to MHCII and/or FGL1; provided below is exemplary human LAG3-IgD1/IgD2/IgD3 amino acid sequence (SEQ ID NO: 27); The number of tandem combinations disclosed in this application is not limited to the number shown in the example, any tandem combinationcan comprising LAG3-IgD1/IgD2/IgD3 functional domain can be deemed as within the scope of this application; term "LAG3-IgD1/IgD2/IgD3/IgD4" generally refers to polypeptide or fragment thereof that has amino acid sequence of human LAG3-IgD1, human LAG3-IgD2, human LAG3-IgD3 and human LAG3-IgD4 (or an amino acid sequence has at least about 85% amino acid identity), and has MHCII and/or FGL1 binding activity; provided below is human LAG3-IgD1/IgD2/IgD3/IgD4 amino acid sequence (SEQ ID NO: 28); The number of tandem combinations disclosed in this application is not limited to the number shown in the example, any tandem combination containing functional domain of LAG3-IgD1/IgD2/IgD3/IgD4 can be deemed as within the scope of this application

In this application, the term "fusion polypeptide" generally refers to a polypeptide obtained by fusion of two or more proteins or polypeptides. Fusion polypeptides can be artificially produced by recombinant DNA techniques. For example, the genes or nucleic acid molecules encoding the two or more proteins or polypeptides can be linked to each other to form a fusion gene or a fused nucleic acid molecule which can encode the fusion polypeptide. Translation of the fusion gene may result in a single polypeptide, which may have the properties of at least one, or even each, of the two or more proteins or polypeptides prior to fusion.

In this application, the term "dual and/or multifunctional fusion polypeptide" generally refers to a polypeptide or protein fused from one or more sources of polypeptide or its domains. Anti-PD-L1 antibody and/or anti-PD-1 antibody via the linker peptide is covalently linked to that at least comprising the CD86 extracellular domain, and/or that at least comprising the LAG3 extracellular domain, and/or that at least comprising one functional domain of LAG3 extracellular domain to form the functional fusion polypeptide, wherein the disclosed two or more LAG3 extracellular domain or/and functional domain comprising at least one LAG3 extracellular domain and LAG3 extracellular domain or/and functional domain comprising at least one LAG3 extracellular domain is linked in series to form two or more LAG3 extracellular domain; or/and functional domain comprising at least one LAG3 extracellular domainis linked via the linker peptide; the dual and/or multifunctional fusion polypeptide disclosed in this application includes but are not limited to the anti-PD-L1 antibody and/or the anti-PD-1 antibody; functional fusion polypeptide or combination thereof wherein any form of anti-PD-L1 antibody and/or anti-PD-1 antibody via a linker peptide is covalently linked to at least one subunit functional domain comprising CD86 extracellular domain, and/or LAG3 extracellular domain, and/or LAG3 extracellular domain should be deemed as within the scope of the present application.

In this application, the term "antibody-associated antigen-binding fragment" generally refers to an antibody comprising an amino acid fragment responsible for specific binding to an antigen. It can be a fragment that determines the key difference in an antibody molecule, and can also be called an antigen-binding domain, or an "epitope" or "antigenic determinant"; an antigen-binding domain is usually composed of an antibody heavy chain variable region (VH ) and antibody light chain variable region (VL), however, it does not necessarily include both, and the antigen-binding domain of the antibody disclosed in this application is not limited to the traditional domain consisting of VH and VL, but also includes any Antigen-binding domains contained in other types of antibodies such as but not limited to recombinant antibodies, single domain antibodies, heavy chain antibodies, chimeric antibodies, bispecific antibodies and other unconventional antibodies and combinations thereof.

Furthermore, the anti-PD-1 antibody and/or anti-PD-L1 antibody disclosed in this application are not limited to traditional natural antibodies, and should include any other antibodies with anti-PD-1 antibody and/or anti-PD-L1 properties such as but not limited to recombinant antibodies, single domain antibodies, heavy chain antibodies, chimeric antibodies, bispecific antibodies and other unconventional antibodies and combinations thereof.

In this application, the term "Fab" generally refers to an antibody fragment consisting of VL, VH, CL and CH1 domains.

In this application, the term "Fab'" generally refers to an antibody fragment that has several additional residues at the carboxy-terminus of the CH1 domain based on the Fab fragment. For example, a Fab' may include one or more cysteines from the antibody hinge region.

In this application, the term "F(ab)₂" generally refers to an antigen-binding fragment obtained from a pair of Fab fragments linked by cysteines.

In this application, the term "dAb fragment" generally refers to an antibody fragment consisting of a VH domain (Ward et al., Nature 341:544546 (1989)).

In this application, the term "complementarity determining region CDR" usually refers to the three hypervariable regions (HVR) of the light chain variable region (VL) and the heavy chain variable region (VH). It forms a precise complementarity with the antigenic determinant, so the hypervariable region is also called complementarity-determining region.

In this application, the term "Fv fragment" generally refers to an antibody fragment consisting of the VL and VH domains of a single arm of an antibody.

In this application, the term "scFv" generally refers to a molecule formed by linking the variable region of the heavy chain and the variable region of the light chain of an antibody through a short peptide linker (linker), also known as a single-chain antibody.

In the present application, the term "immunoglobulin" refers to an antibody comprising an antigen-specific binding amino acid fragment and a constant region fragment. The antigen-specific binding region, which determines the critical differences in immunoglobulins, is also referred to as the antigen-binding domain, "epitope," or "antigenic determinant." The antigen-binding domain typically consists of the variable region of the antibody heavy chain (VH) and the variable region of the antibody light chain (VL). However, it is not necessarily limited to both. The antigen-binding domain of the antibodies disclosed in this invention is not restricted to the traditional VH and VL domains but also includes antigen-binding domains from other types of antibodies, such as recombinant antibodies, single-domain antibodies, heavy-chain antibodies, chimeric antibodies, bispecific antibodies, and other unconventional antibodies, as well as their combinations. The constant region refers to the common structural region of immunoglobulins, comprising the constant region of the antibody light chain and the constant region of the antibody heavy chain.

In the present application, the term "immunoglobulin Fc domain" typically refers to the Fc fragment obtained from a conventional IgG antibody after papain digestion, resulting in one Fc fragment and two identical Fab fragments. The Fc domain may include the CH2, CH3, and hinge region fragments of the antibody heavy chain. Traditional Fc fragments have functions related to binding Fc receptor-mediated biological effects. Site-specific mutations can alter their binding affinity to corresponding target receptors, thereby affecting their biological functions. The immunoglobulin Fc domains disclosed in this application include, but are not limited to, traditional Fc fragments and any form of Fc mutants. The biological functions that immunoglobulin Fc domains can provide include, but are not limited to, extending half-life, enhancing molecular stability, facilitating the expression and detection of fusion proteins, mediating transplacental and mucosal barrier passage, mediating antibody-dependent cellular cytotoxicity (ADCC), mediating inflammatory responses, mediating antibody-dependent cellular phagocytosis (ADCP), mediating complement-dependent cytotoxicity (CDC), promoting dendritic cell (DC) maturation, regulating cytokine secretion, and modulating B-cell proliferation and differentiation. Exemplary human immunoglobulin IgG1 Fc domain (SEQ ID NO: 20) and human immunoglobulin IgG4 Fc domain (SEQ ID NO: 21) are provided below.

In the present invention, the terms "polypeptide" and "protein" have the same meaning and can be used interchangeably. And in the present invention, amino acids are generally represented by single-letter and three-letter abbreviations known in the art. For example, alanine can be represented by A or Ala.

The protein, polypeptide and/or amino acid sequence involved in this application should also be understood to include at least the following scope: variants or homologues having the same or similar functions as the protein or polypeptide.

In the present application, the variant may be that one or more amino acids are substituted, deleted or added to the amino acid sequence of the protein or the polypeptide (for example, an antibody or fragment thereof that specifically binds the protein). For example, the functional variant may comprise proteins or polypeptides with amino acid changes by at least 1, such as 1-30, 1-20 or 1-10, further such as 1, 2, 3, 4 or 5 amino acid substitutions, deletion and/or insertion. The functional variant may substantially maintain the biological properties of the protein or polypeptide prior to alteration (eg, substitution, deletion or addition). For example, the functional variant may retain at least 60%, 70%, 80%, 90%, or 100% of the biological activity (eg, antigen binding ability) of the protein or polypeptide prior to the alteration. For example, the substitutions may be conservative substitutions.

In the present application, the homologue may be a protein or polypeptide having at least about 85% (for example, having at least about 85%, about 90%, about 91%, about 92%, about 93%, about 94%, about 95%, about 96%, about 97%, about 98%, about 99% or more) sequence homology with the amino acid sequence of the protein and/or the polypeptide.

In this application, the homology generally refers to the similarity or association between two or more sequences. "Percent sequence homology" can be calculated by comparing the two sequences to be aligned in a comparison window and determining the presence of identical nucleic acid bases (e.g., A, T, C, G, I) in both sequences. ) or the same amino acid residue (e.g., Ala, Pro, Ser, Thr, Gly, Val, Leu, Ile, Phe, Tyr, Trp, Lys, Arg, His, Asp, Glu, Asn, Gln, Cys, and Met) Number of positions To obtain the number of matching positions, divide the number of matching positions by the total number of positions in the comparison window (i.e., window size), and multiply the result by 100 to produce percent sequence homology. Alignment to determine percent sequence identity can be accomplished in various ways known in the art, for example, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared or over a region of sequence of interest. The homology can also be determined by the following methods: FASTA and BLAST.

Usually, in a polypeptide chain, the amino group is connected to another carboxyl group in the polypeptide chain to make it a chain, but at the two ends of the protein, the remaining amino acid residues that do not form a peptide bond are respectively carrying the free end of the polypeptide chain with an amino group and the end of the polypeptide chain with a carboxyl group. In this application, the term "N-terminal" generally refers to the end of a polypeptide chain whose amino acid residues bear a free amino group. In this application, the term "C-terminal" generally refers to the end of the polypeptide chain whose amino acid residue bears a free carboxyl group.

In this application, the term "nucleic acid molecule" generally refers to an isolated form of nucleotides, deoxyribonucleotides or ribonucleotides or analogs thereof of any length isolated from their natural environment or artificially synthesized.

In this application, the term "immunoconjugate" generally refers to a polypeptide molecule conjugated to one or more heterologous molecules, including but not limited to cytotoxins.

In the present invention, the term "vector" refers to a nucleic acid delivery tool into which a polynucleotide encoding a protein can be inserted and the protein can be expressed.

In this application, the term "linker peptide" generally refers to a linker molecule that links one or more polypeptides or domains thereof. For example, the linker peptide may have conformational flexibility and be a short peptide chain formed by a combination of amino acid Gly(G) and Ser(S) residues, wherein the ratio of the number of amino acid Gly to the number of amino acid Ser may be ≥1. The linker peptide as disclosed in this application can be extended to any short peptide with this property. The following provides exemplary linker peptide amino acid sequences including but not limited to GGGGS (SEQ ID NO: 22), GGGGSGGGS (SEQ ID NO: 23), GGGGSGGGGSGGGGS (SEQ ID NO :24), GGGGSGGGSGGGGSGGGGS (SEQ ID NO:25) and GGGGSGGGS (SEQ ID NO:26).

In the present application, the term "CD3" generally refers to a protein complex composed of four distinct chains. In mammals, this complex comprises one CD3γ chain, one CD3δ chain, and two CD3ε chains. These chains associate with the T-cell receptor (TCR) and the ζ-chain to generate activation signals in T lymphocytes. The TCR, ζ-chain, and CD3 molecules together form the TCR complex. As used herein, the term "CD3" refers to any natural CD3 from any human source. This term also encompasses the "full-length" and unprocessed proteins, as well as any form of protein or one or more CD3 chains (polypeptides) derived from cellular processing (e.g., mature polypeptides). Additionally, the term includes naturally occurring variants and isoforms of CD3, such as splice variants or allelic variants. For example, descriptions and sequences of the CD3γ chain, CD3δ chain, and CD3ε chain can be found at www.uniprot.org/uniprot/P04234, www.uniprot.org/uniprot/P07766, and www.uniprot.org/uniprot/P09693, respectively.

In the present application, the term "anti-CD3 antibody" includes antibodies and their antigen-binding fragments that specifically recognize a single CD3 subunit (e.g., B, δ, γ, or ζ) or a dimeric complex of two CD3 subunits (e.g., γ/ε, δ/ε, or ζ/ζ CD3 dimers). For example, monoclonal antibodies, including human, humanized, chimeric, or murine antibodies, targeting the CD3 receptor in the T-cell antigen receptor of mature T cells, fall under this term. An anti-CD3 antibody can be, for instance, the CD3B219 antibody or its antigen-binding fragment.

In this application, the term "amino acid mutation" generally refers to amino acid substitutions, deletions, insertions, and modifications. Any combination of substitutions, deletions, insertions, and modifications can be made to achieve the desired construct, as long as the final construct exhibits the desired properties. In one embodiment, the amino acid mutation is a substitution. The term "amino acid mutation at a specific position" refers to the substitution or deletion of a specified residue or the insertion of at least one amino acid residue adjacent to the specified residue. Amino acid substitutions can be conservative or non-conservative. For example, "A13I" indicates the substitution of alanine (A, Ala) at position 13 with isoleucine (I, Ile).

In the present application, the term "active fragment" generally refers to a nucleic acid or amino acid fragment or variant that possesses certain biological activity or function. For example, a functional active fragment may retain or partially retain the ability of the full-length protein to bind another molecule.

The term "CD86 active fragment" in this application refers to polypeptides or fragments of the CD86 IgV or CD86 ECD proteins described in this invention, including their derivative variants, that exhibit binding activity with CD28 and/or CTLA4. These include, but are not limited to, characteristics such as regulating T-cell activity, immune system activation, and antitumor activity.

The term "LAG3 active fragment" in this application refers to polypeptides or fragments of the LAG3 protein described in this invention, including their derivative variants, that activate antigen-presenting cells. These include, but are not limited to, characteristics such as regulating antigen-presenting cell activity, immune system activation, and antitumor activity.

In the present application, the term "CD86 variant polypeptide" refers to a protein or polypeptide with one or more amino acid substitutions, deletions, or additions in the wild-type CD86 amino acid sequence. For example, the functional variant may include a protein or polypeptide with at least one, such as 1-30, 1-20, or 1-10, and specifically 1, 2, 3, 4, or 5 amino acid substitutions, deletions, and/or insertions resulting in amino acid alterations. The functional variant may substantially retain the biological properties of CD86 before the alteration (e.g., substitution, deletion, or addition). For instance, the functional variant may retain at least 60%, 70%, 80%, 90%, or 100% of the biological activity of the protein or polypeptide before alteration (e.g., antigen-binding capability). The substitution may, for example, be a conservative substitution. The term "CD86 variant polypeptide" refers to an active fragment derived from the CD86 protein that retains the functional characteristics of CD86 protein, including but not limited to binding activity with CD28 and/or CTLA4.

The term "fusion polypeptide complex of variant polypeptide" is also referred to in this invention as "variant polypeptide complex," "polypeptide complex," "fusion polypeptide complex," or "fusion complex." A "CD86 mutant fusion polypeptide complex" refers to a functional fusion polypeptide complex in which a CD86 ECD mutant or CD86 IgV mutant is linked, with or without a linker peptide, to an antibody or its antigen-binding fragment, or to an immunoglobulin Fc domain or a functional polypeptide fragment. The disclosed CD86 mutant fusion polypeptide complexes include, but are not limited to, those where the CD86 mutant is covalently linked, with or without a linker peptide, to an antibody or its antigen-binding fragment. The antibodies include but are not limited to anti-PD-L1 antibodies, anti-PD-1 antibodies, anti-TIGIT antibodies, anti-CTLA4 antibodies, anti-CEA antibodies, anti-BCMAantibodies, anti-LAG3 antibodies, anti-CD3 antibodies, anti-Her2 antibodies, anti-Her3 antibodies, anti-VEGF antibodies, anti-VEGFR antibodies, anti-EGFR antibodies, anti-c-Met antibodies, anti-CD19 antibodies, anti-CD20 antibodies, anti-CD38 antibodies, anti-TROP-2 antibodies, anti-CD40 antibodies, anti-4-1BB antibodies, anti-CD30 antibodies, and so forth. Any form of antibody covalently linked to the CD86 ECD mutant or CD86 IgV mutant disclosed in this invention, with or without a linker peptide, is within the scope of this invention.

The term "LAG3 variant polypeptide" refers to a protein or polypeptide with one or more amino acid substitutions, deletions, or additions in the wild-type LAG3 amino acid sequence. For example, the functional variant may include a protein or polypeptide with at least one, such as 1-30, 1-20, or 1-10, and specifically 1, 2, 3, 4, or 5 amino acid substitutions, deletions, and/or insertions resulting in amino acid alterations. The functional variant may substantially retain the biological properties of LAG3 before the alteration (e.g., substitution, deletion, or addition). For instance, the functional variant may retain at least 60%, 70%, 80%, 90%, or 100% of the biological activity of the protein or polypeptide before alteration (e.g., antigen-binding capability). The substitution may, for example, be a conservative substitution. The term "LAG3 variant polypeptide" refers to an active fragment derived from the LAG3 protein that retains the functional characteristics of LAG3 protein, including but not limited to promoting the activation and maturation of antigen-presenting cells. The functional characteristics of LAG3 protein include but are not limited to binding with MHCII (or HLA-DR) and/or FGL1 and promoting the activation and maturation of antigen-presenting cells.

In this application, the term "LAG3 variant fusion polypeptide complex" generally refers to a polypeptide or protein formed by fusing one or more polypeptides or domains. The term "fusion polypeptide complex of variant polypeptide" is also referred to in this invention as "variant polypeptide complex," "polypeptide complex," "fusion polypeptide complex," or "fusion complex." LAG3 variant polypeptides may be linked, with or without a linker peptide, to at least the Fc domain and/or at least an antibody or its antigen-binding fragment. The LAG3 variant fusion polypeptide complexes disclosed in this application include, but are not limited to, functional fusion polypeptides or their combinations in which LAG3 variant polypeptides are covalently linked to any form of antibody, functional protein fragments, and/or Fc domains. These are all considered within the scope of this application.

The activation and maturation of antigen-presenting cells include but are not limited to the upregulation of co-stimulatory receptor expression on antigen-presenting cells, enhanced antigen phagocytosis, and increased secretion of cytokines and chemokines.

The co-stimulatory receptors include but are not limited to ICOSL, CD40L, CD137L, OX40L, CD80, CD83 and CD86.

The antigen phagocytosis includes but is not limited to the phagocytosis of bacteria, viruses, proteins, polysaccharides and the like.

The cytokines include but are not limited to IL-1beta, TNFa, IFNγ, IL-6, IL-12 and the like.

The chemokines include but are not limited to CCL1, CCL2, CCL3, CCL4, CCL5, CXCL1, CXCL2, CXCL3, CXCL4, CXCL5 and the like.

In the present application, the expression "X amino acids is truncated at the N-terminus" means that the amino acid residues 1 to X at the N-terminus of the extracellular segment of the protein are deleted. For example, the expression "LAG3 extracellular region polypeptide having 74 amino acids truncated at the N-terminus" may refer to a variant polypeptide obtained by deleting amino acid residues 23-96 at the N-terminus of the wild-type LAG3 extracellular region polypeptide according to NCBI numbering. For example, the expression "LAG3 extracellular region polypeptide having 74 amino acids truncated at the N-terminus" may refer to an obtained wild-type human LAG3 polypeptide based polypeptide starting from amino acid site 97 of the wild-type human LAG3 polypeptide. For example, the expression "LAG3 extracellular region polypeptide with 74 amino acids truncated at the N-terminus" can be expressed as "LAG3 97-450".

In the present application, the expression "the C-terminus ends at amino acid position X" means that all amino acid residues after amino acid position X (ie, starting from amino acid position X+1) are deleted. For example, the expression "the C-terminus terminates at amino acid position 156 of the LAG3 extracellular region polypeptide" may refer to a variant polypeptide obtained by deleting all the amino acid residues after amino acid position NCBI number 156 (i.e., starting from amino acid position 157) of the wild-type LAG3 extracellular region polypeptide. For example, the expression "the C-terminus terminates at amino acid position 156 of the LAG3 extracellular region polypeptide" may mean that 294 amino acids are truncated at the C-terminus of the wild-type LAG3 extracellular region polypeptide. For example, the expression "the C-terminus terminates at amino acid position 156 of the LAG3 extracellular domain polypeptide" can be expressed as "LAG3 23-156". For example, the expression "74 amino acids are truncated at the N-terminus is truncated and the C-terminus terminates at amino acid position 156" can be expressed as "LAG3 97-156".

In this application, the term "comprising" generally means the inclusion of explicitly specified features, but not the exclusion of other elements.

In this application, the term "about" generally refers to a range of 0.5%-10% above or below the specified value, such as 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

### DETAILED DESCRIPTION OF THE invention

On the one hand, the present application provides a fusion polypeptide, which may include a first domain and a second domain, wherein the first domain can block PD-1/PD-L1 signal, and the second domain includes CD86 or a functionally active fragment thereof.

For example, the fusion polypeptide may comprise one or more of the first domain and one or more of the second domain.

For example, the first domain is capable of binding to PD-L1 and/or PD-1.

For example, the first domain may comprise HCDR3 of an antibody heavy chain, and the antibody heavy chain may comprise an amino acid sequence selected from the group consisting of SEQ ID NO:4, SEQ ID NO:6, SEQ ID NO:8, SEQ ID NO:10, SEQ ID NO:12, SEQ ID NO: 14, SEQ ID NO: 15 and SEQ ID NO: 17.

For example, the first domain may comprise HCDR2 of an antibody heavy chain, and the antibody heavy chain may comprise an amino acid sequence selected from the group consisting of SEQ ID NO: SEQ ID NO:4, SEQ ID NO:6, SEQ ID NO:8, SEQ ID NO:10, SEQ ID NO:12, SEQ ID NO:14, SEQ ID NO:15 and SEQ ID NO:17.

For example, the first domain may comprise HCDR1 of an antibody heavy chain, and the antibody heavy chain may comprise an amino acid sequence selected from the group consisting of SEQ ID NO: SEQ ID NO:4, SEQ ID NO:6, SEQ ID NO:8, SEQ ID NO: 10, SEQ ID NO: 12, SEQ ID NO:14, SEQ ID NO:15 and SEQ ID NO:17.

For example, the first domain may comprise a heavy chain variable region VH of an antibody heavy chain, and the antibody heavy chain variable region may comprise an amino acid sequence selected from the group consisting of SEQ ID NO:4, SEQ ID NO:6, SEQ ID NO:8, SEQ ID NO: 10, SEQ ID NO: 12, SEQ ID NO: 14, SEQ ID NO: 15 and SEQ ID NO: 17.

For example, the first domain may comprise an antibody heavy chain, which may comprise an amino acid sequence selected from the group consisting of SEQ ID NO:4, SEQ ID NO:6, SEQ ID NO:8, SEQ ID NO:10, SEQ ID NO:12, SEQ ID NO:14, SEQ ID NO:15 and SEQ ID NO: 17.

For example, the first domain may comprise LCDR3 of an antibody light chain, and the antibody light chain may comprise an amino acid sequence selected from the group consisting of SEQ ID NO:5, SEQ ID NO:7, SEQ ID NO:9, SEQ ID NO: 11, SEQ ID NO: 13, SEQ ID NO:16 and SEQ ID NO:18.

For example, the first domain may comprise LCDR2 of an antibody light chain, and the antibody light chain may comprise an amino acid sequence selected from the group consisting of SEQ ID NO:5, SEQ ID NO:7, SEQ ID NO:9, SEQ ID NO: 11, SEQ ID NO: 13, SEQ ID NO:16 and SEQ ID NO:18.

For example, the first domain may comprise LCDR1 of an antibody light chain, and the antibody light chain may comprise an amino acid sequence selected from the group consisting of SEQ ID NO:5, SEQ ID NO:7, SEQ ID NO:9, SEQ ID NO: 11, SEQ ID NO: 13, SEQ ID NO:16 and SEQ ID NO:18.

For example, the first domain may comprise a light chain variable region VL of an antibody light chain, and the antibody light chain variable region may comprise an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

For example, the first domain may comprise an antibody light chain, which may comprise an amino acid sequence selected from the group consisting of SEQ ID NO:5, SEQ ID NO:7, SEQ ID NO:9, SEQ ID NO:11, SEQ ID NO:13, SEQ ID NO:16 and SEQ ID NO:18.

For example, the first domain may comprise HCDR3, HCDR2 and HCDR1 of an antibody heavy chain, wherein the antibody heavy chain may comprise the amino acid sequence shown in SEQ ID NO:4. For example, the antibody can be Sugemalimab.

For example, the first domain may comprise LCDR3, LCDR2 and LCDR1 of an antibody light chain, wherein the antibody light chain may comprise the amino acid sequence shown in SEQ ID NO:5. For example, the antibody can be Sugemalimab.

For example, the first domain may comprise HCDR3, HCDR2 and HCDR1 of an antibody heavy chain, wherein the antibody heavy chain may comprise the amino acid sequence shown in SEQ ID NO:4, and the first domain may comprise LCDR3, LCDR2 and LCDR1 of an antibody light chain, wherein the antibody light chain may comprise the amino acid sequence shown in SEQ ID NO:5. For example, the antibody can be Sugemalimab.

For example, the first domain may comprise the heavy chain variable region VH of an antibody heavy chain, and the antibody heavy chain may comprise the amino acid sequence shown in SEQ ID NO:4, and the first domain may comprise the light chain variable region VL of an antibody light chain, and the antibody light chain may comprise the amino acid sequence shown in SEQ ID NO:5. For example, the antibody can be Sugemalimab.

For example, the first domain may comprise HCDR3, HCDR2 and HCDR1 of an antibody heavy chain, wherein the antibody heavy chain may comprise the amino acid sequence shown in SEQ ID NO:6. For example, the antibody may be JS003.

For example, the first domain may comprise LCDR3, LCDR2 and LCDR1 of an antibody light chain, wherein the antibody light chain may comprise the amino acid sequence shown in SEQ ID NO:7. For example, the antibody may be JS003.

For example, the first domain may comprise HCDR3, HCDR2 and HCDR1 of an antibody heavy chain, wherein the antibody heavy chain may comprise the amino acid sequence shown in SEQ ID NO:6, and the first domain may comprise LCDR3, LCDR2 and LCDR1 of an antibody light chain, wherein the antibody light chain may comprise the amino acid sequence shown in SEQ ID NO:7. For example, the antibody may be JS003.

For example, the first domain may comprise the heavy chain variable region VH of an antibody heavy chain, and the antibody heavy chain may comprise the amino acid sequence shown in SEQ ID NO:6, and the first domain may comprise the light chain variable region VL of an antibody light chain, and the antibody light chain may comprise the amino acid sequence shown in SEQ ID NO:7. For example, the antibody may be JS003.

For example, the first domain may comprise HCDR3, HCDR2 and HCDR1 of an antibody heavy chain, wherein the antibody heavy chain may comprise the amino acid sequence shown in SEQ ID NO:8. For example, the antibody may be Durvalumab.

For example, the first domain may comprise LCDR3, LCDR2 and LCDR1 of an antibody light chain, wherein the antibody light chain may comprise the amino acid sequence shown in SEQ ID NO:9. For example, the antibody may be Durvalumab.

For example, the first domain may comprise HCDR3, HCDR2 and HCDR1 of an antibody heavy chain, wherein the antibody heavy chain may comprise the amino acid sequence shown in SEQ ID NO:8, and the first domain may comprise LCDR3, LCDR2 and LCDR1 of an antibody light chain, wherein the antibody light chain may comprise the amino acid sequence shown in SEQ ID NO:9. For example, the antibody may be Durvalumab.

For example, the first domain may comprise the heavy chain variable region VH of an antibody heavy chain, and the antibody heavy chain may comprise the amino acid sequence shown in SEQ ID NO:8, and the first domain may comprise the light chain variable region VL of an antibody light chain, and the antibody light chain may comprise the amino acid sequence shown in SEQ ID NO:9. For example, the antibody may be Durvalumab.

For example, the first domain may comprise HCDR3, HCDR2 and HCDR1 of an antibody heavy chain, wherein the antibody heavy chain may comprise the amino acid sequence shown in SEQ ID NO: 10. For example, the antibody can be Atezolizumab.

For example, the first domain may comprise LCDR3, LCDR2 and LCDR1 of an antibody light chain, wherein the antibody light chain may comprise the amino acid sequence shown in SEQ ID NO: 11. For example, the antibody can be Atezolizumab.

For example, the first domain may comprise HCDR3, HCDR2 and HCDR1 of an antibody heavy chain, wherein the antibody heavy chain may comprise the amino acid sequence shown in SEQ ID NO:10, and the first domain may comprise LCDR3, LCDR2 and LCDR1 of an antibody light chain, wherein the antibody light chain may comprise the amino acid sequence shown in SEQ ID NO: 11. For example, the antibody can be Atezolizumab.

For example, the first domain may comprise the heavy chain variable region VH of an antibody heavy chain, and the antibody heavy chain may comprise the amino acid sequence shown in SEQ ID NO:10, and the first domain may comprise the light chain variable region VL of an antibody light chain, and the antibody light chain may comprise the amino acid sequence shown in SEQ ID NO: 11. For example, the antibody can be Atezolizumab.

For example, the first domain may comprise HCDR3, HCDR2 and HCDR1 of an antibody heavy chain, wherein the antibody heavy chain may comprise the amino acid sequence shown in SEQ ID NO: 12. For example, the antibody can be Avelumab.

For example, the first domain may comprise LCDR3, LCDR2 and LCDR1 of an antibody light chain, wherein the antibody light chain may comprise the amino acid sequence shown in SEQ ID NO:13. For example, the antibody can be Avelumab.

For example, the first domain may comprise HCDR3, HCDR2 and HCDR1 of an antibody heavy chain, wherein the antibody heavy chain may comprise the amino acid sequence shown in SEQ ID NO: 12, and the first domain may comprise LCDR3, LCDR2 and LCDR1 of an antibody light chain, wherein the antibody light chain may comprise the amino acid sequence shown in SEQ ID NO: 13. For example, the antibody can be Avelumab.

For example, the first domain may comprise the heavy chain variable region VH of an antibody heavy chain, and the antibody heavy chain may comprise the amino acid sequence shown in SEQ ID NO: 12, and the first domain may comprise the light chain variable region VL of an antibody light chain, and the antibody light chain may comprise the amino acid sequence shown in SEQ ID NO: 13. For example, the antibody can be Avelumab.

For example, the first domain may comprise HCDR3, HCDR2 and HCDR1 of an antibody, wherein the antibody may comprise the amino acid sequence shown in SEQ ID NO:14. For example, the antibody can be Envafolimab.

For example, the first domain may comprise HCDR3, HCDR2 and HCDR1 of an antibody heavy chain, wherein the antibody heavy chain may comprise the amino acid sequence shown in SEQ ID NO:15. For example, the antibody can be Permbrolizumab.

For example, the first domain may comprise LCDR3, LCDR2 and LCDR1 of an antibody light chain, wherein the antibody light chain may comprise the amino acid sequence shown in SEQ ID NO: 16. For example, the antibody can be Permbrolizumab.

For example, the first domain may comprise HCDR3, HCDR2 and HCDR1 of an antibody heavy chain, wherein the antibody heavy chain may comprise the amino acid sequence shown in SEQ ID NO:15, and the first domain may comprise LCDR3, LCDR2 and LCDR1 of an antibody light chain, wherein the antibody light chain may comprise the amino acid sequence shown in SEQ ID NO: 16. For example, the antibody can be Permbrolizumab.

For example, the first domain may comprise the heavy chain variable region VH of an antibody heavy chain, and the antibody heavy chain may comprise the amino acid sequence shown in SEQ ID NO:15, and the first domain may comprise the light chain variable region VL of an antibody light chain, and the antibody light chain may comprise the amino acid sequence shown in SEQ ID NO:16. For example, the antibody can be Permbrolizumab.

For example, the first domain may comprise HCDR3, HCDR2 and HCDR1 of an antibody heavy chain, wherein the antibody heavy chain may comprise the amino acid sequence shown in SEQ ID NO: 17. For example, the antibody can be Nivolumab.

For example, the first domain may comprise LCDR3, LCDR2 and LCDR1 of an antibody light chain, wherein the antibody light chain may comprise the amino acid sequence shown in SEQ ID NO:18. For example, the antibody can be Nivolumab.

For example, the first domain may comprise HCDR3, HCDR2 and HCDR1 of an antibody heavy chain, wherein the antibody heavy chain may comprise the amino acid sequence shown in SEQ ID NO: 17, and the first domain may comprise LCDR3, LCDR2 and LCDR1 of an antibody light chain, wherein the antibody light chain may comprise the amino acid sequence shown in SEQ ID NO:18. For example, the antibody can be Nivolumab.

For example, the first domain may comprise the heavy chain variable region VH of an antibody heavy chain, and the antibody heavy chain may comprise the amino acid sequence shown in SEQ ID NO: 17, and the first domain may comprise the light chain variable region VL of an antibody light chain, and the antibody light chain may comprise the amino acid sequence shown in SEQ ID NO: 18. For example, the antibody can be Nivolumab.

For example, the first domain may comprise an antibody or an antigen-binding fragment thereof.

For example, the antibody may be selected from the group consisting of a recombinant antibody, a single domain antibody, a heavy chain antibody, a chimeric antibody, and a bispecific antibody.

For example, the antigen-binding fragment may be selected from one or more of the group consisiting of: Fab, Fab', Fv fragment, F(ab')2, F(ab)2, scFv, di-scFv, VHH and dAb.

For example, the first domain may comprise an antibody or an antigen-binding fragment thereof selected from the group consisting of
: Sugemalimab, Durvalumab, Atezolizumab, Avelumab, Envafolimab, Permbrolizumab and Nivolumab.

For example, the first domain can comprise Sugemalimab or an antigen-binding fragment thereof.

For example, the second domain may comprise CD86 or a functionally active fragment thereof.

For example, the second domain can be selected from the group consisting of CD86 derived from humans or a functionally active fragment thereof and CD86 derived from mice or a functionally active fragment thereof.

For example, the second domain may bind to CD28, and/or CTLA4.

For example, the second domain may comprise the IgV domain of CD86 or a functionally active fragment thereof.

For example, the second domain may comprise the extracellular domain of CD86 or a functionally active fragment thereof.

For example, the second domain may comprise an amino acid sequence as shown in SEQ ID NO:2 or SEQ ID NO:3.

For example, the first domain and the second domain may be linked directly or indirectly.

For example, the first domain and the N-terminus of the second domain may be directly or indirectly connected.

For example, the first domain and the C-terminus of the second domain may be directly or indirectly connected.

For example, the first domain may comprise an antibody heavy chain, the antibody heavy chain of the first domain being directly or indirectly linked to the second domain.

For example, the first domain may comprise an antibody heavy chain, and the C-terminus of the first domain antibody heavy chain is directly or indirectly connected to the N-terminus of the second domain.

For example, the first domain may comprise an antibody heavy chain, and the N-terminus of the antibody heavy chain of the first domain is directly or indirectly connected to the C-terminus of the second domain.

For example, the first domain may comprise an antibody light chain, and the first domain antibody light chain and the second domain may be directly or indirectly connected.

For example, the first domain may comprise an antibody light chain, and the C-terminus of the antibody light chain of the first domain is directly or indirectly connected to the N-terminus of the second domain.

For example, the first domain may comprise an antibody light chain, and the N-terminus of the antibody light chain of the first domain is directly or indirectly connected to the C-terminus of the second domain.

For example, the indirect connection may comprise connection via a linker.

For example, the linker may comprise an amino acid sequence selected from the group consisting of SEQ ID NO:31, SEQ ID NO:32, SEQ ID NO:33, SEQ ID NO:34, and SEQ ID NO:35.

For example, the fusion polypeptide can comprise an amino acid sequence selected from the group consisting of SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64, SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78, SEQ ID NO:79, SEQ ID NO:80, SEQ ID NO:81, SEQ ID NO:82, SEQ ID NO:83, SEQ ID NO:84, SEQ ID NO:85, SEQ ID NO:86 and SEQ ID NO:87.

For example, the fusion polypeptide can comprise an amino acid sequence selected from the group consisting of SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, and SEQ ID NO:77.

On the one hand, the present application also provides a fusion polypeptide, which may comprise a first domain, a second domain and a third domain, wherein the first domain can block PD-1/PD-L1 signals, the second domain comprises CD86 or a functionally active fragment thereof, and the third domain can activate an innate immune response. For example, the fusion polypeptide may comprise one or more of the first domains, one or more of the second domains, and one or more of the third domains.

For example, the first domain is capable of binding to PD-L1 and/or PD-1.

For example, the first domain may comprise HCDR3 of an antibody heavy chain, and the antibody heavy chain may comprise an amino acid sequence selected from the group consisting of SEQ ID NO:4, SEQ ID NO:6, SEQ ID NO:8, SEQ ID NO:10, SEQ ID NO:12, SEQ ID NO: 14, SEQ ID NO:15 and SEQ ID NO: 17.

For example, the first domain may comprise HCDR2 of an antibody heavy chain, and the antibody heavy chain may comprise an amino acid sequence selected from the group consisting of SEQ ID NO: SEQ ID NO:4, SEQ ID NO:6, SEQ ID NO:8, SEQ ID NO:10, SEQ ID NO:12, SEQ ID NO:14, SEQ ID NO:15 and SEQ ID NO:17.

For example, the first domain may comprise HCDR1 of an antibody heavy chain, and the antibody heavy chain may comprise an amino acid sequence selected from the group consisting of SEQ ID NO: SEQ ID NO:4, SEQ ID NO:6, SEQ ID NO:8, SEQ ID NO:10, SEQ ID NO:12, SEQ ID NO:14, SEQ ID NO:15 and SEQ ID NO:17.

For example, the first domain may comprise a heavy chain variable region VH of an antibody heavy chain, and the antibody heavy chain variable region may comprise an amino acid sequence selected from the group consisting of SEQ ID NO:4, SEQ ID NO:6, SEQ ID NO:8, SEQ ID NO:10, SEQ ID NO:12, SEQ ID NO:14, SEQ ID NO:15 and SEQ ID NO:17.

For example, the first domain may comprise an antibody heavy chain, which may comprise an amino acid sequence selected from the group consisting of SEQ ID NO:4, SEQ ID NO:6, SEQ ID NO:8, SEQ ID NO:10, SEQ ID NO:12, SEQ ID NO:14, SEQ ID NO:15 and SEQ ID NO: 17.

For example, the first domain may comprise LCDR3 of an antibody light chain, and the antibody light chain may comprise an amino acid sequence selected from the group consisting of SEQ ID NO:5, SEQ ID NO:7, SEQ ID NO:9, SEQ ID NO: 11, SEQ ID NO: 13, SEQ ID NO:16 and SEQ ID NO:18.

For example, the first domain may comprise LCDR2 of an antibody light chain, and the antibody light chain may comprise an amino acid sequence selected from the group consisting of SEQ ID NO:5, SEQ ID NO:7, SEQ ID NO:9, SEQ ID NO: 11, SEQ ID NO: 13, SEQ ID NO:16 and SEQ ID NO:18.

For example, the first domain may comprise LCDR1 of an antibody light chain, and the antibody light chain may comprise an amino acid sequence selected from the group consisting of SEQ ID NO:5, SEQ ID NO:7, SEQ ID NO:9, SEQ ID NO: 11, SEQ ID NO: 13, SEQ ID NO:16 and SEQ ID NO:18.

For example, the first domain may comprise a light chain variable region VL of an antibody light chain, and the antibody light chain variable region may comprise an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

For example, the first domain may comprise an antibody light chain, which may comprise an amino acid sequence selected from the group consisting of SEQ ID NO:5, SEQ ID NO:7, SEQ ID NO:9, SEQ ID NO: 11, SEQ ID NO: 13, SEQ ID NO:16 and SEQ ID NO:18.

For example, the first domain may comprise HCDR3, HCDR2 and HCDR1 of an antibody heavy chain, wherein the antibody heavy chain may comprise the amino acid sequence shown in SEQ ID NO:4. For example, the antibody can be Sugemalimab.

For example, the first domain may comprise LCDR3, LCDR2 and LCDR1 of an antibody light chain, wherein the antibody light chain may comprise the amino acid sequence shown in SEQ ID NO:5. For example, the antibody can be Sugemalimab.

For example, the first domain may comprise HCDR3, HCDR2 and HCDR1 of an antibody heavy chain, wherein the antibody heavy chain may comprise the amino acid sequence shown in SEQ ID NO:4, and the first domain may comprise LCDR3, LCDR2 and LCDR1 of an antibody light chain, wherein the antibody light chain may comprise the amino acid sequence shown in SEQ ID NO:5. For example, the antibody can be Sugemalimab.

For example, the first domain may comprise the heavy chain variable region VH of an antibody heavy chain, and the antibody heavy chain may comprise the amino acid sequence shown in SEQ ID NO:4, and the first domain may comprise the light chain variable region VL of an antibody light chain, and the antibody light chain may comprise the amino acid sequence shown in SEQ ID NO:5. For example, the antibody can be Sugemalimab.

For example, the first domain may comprise HCDR3, HCDR2 and HCDR1 of an antibody heavy chain, wherein the antibody heavy chain may comprise the amino acid sequence shown in SEQ ID NO:6. For example, the antibody may be JS003.

For example, the first domain may comprise LCDR3, LCDR2 and LCDR1 of an antibody light chain, wherein the antibody light chain may comprise the amino acid sequence shown in SEQ ID NO:7. For example, the antibody may be JS003.

For example, the first domain may comprise HCDR3, HCDR2 and HCDR1 of an antibody heavy chain, wherein the antibody heavy chain may comprise the amino acid sequence shown in SEQ ID NO:6, and the first domain may comprise LCDR3, LCDR2 and LCDR1 of an antibody light chain, wherein the antibody light chain may comprise the amino acid sequence shown in SEQ ID NO:7. For example, the antibody may be JS003.

For example, the first domain may comprise the heavy chain variable region VH of an antibody heavy chain, and the antibody heavy chain may comprise the amino acid sequence shown in SEQ ID NO:6, and the first domain may comprise the light chain variable region VL of an antibody light chain, and the antibody light chain may comprise the amino acid sequence shown in SEQ ID NO:7. For example, the antibody may be JS003.

For example, the first domain may comprise HCDR3, HCDR2 and HCDR1 of an antibody heavy chain, wherein the antibody heavy chain may comprise the amino acid sequence shown in SEQ ID NO:8. For example, the antibody may be Durvalumab.

For example, the first domain may comprise LCDR3, LCDR2 and LCDR1 of an antibody light chain, wherein the antibody light chain may comprise the amino acid sequence shown in SEQ ID NO:9. For example, the antibody may be Durvalumab.

For example, the first domain may comprise HCDR3, HCDR2 and HCDR1 of an antibody heavy chain, wherein the antibody heavy chain may comprise the amino acid sequence shown in SEQ ID NO:8, and the first domain may comprise LCDR3, LCDR2 and LCDR1 of an antibody light chain, wherein the antibody light chain may comprise the amino acid sequence shown in SEQ ID NO:9. For example, the antibody may be Durvalumab.

For example, the first domain may comprise the heavy chain variable region VH of an antibody heavy chain, and the antibody heavy chain may comprise the amino acid sequence shown in SEQ ID NO:8, and the first domain may comprise the light chain variable region VL of an antibody light chain, and the antibody light chain may comprise the amino acid sequence shown in SEQ ID NO:9. For example, the antibody may be Durvalumab.

For example, the first domain may comprise HCDR3, HCDR2 and HCDR1 of an antibody heavy chain, wherein the antibody heavy chain may comprise the amino acid sequence shown in SEQ ID NO:10. For example, the antibody can be Atezolizumab.

For example, the first domain may comprise LCDR3, LCDR2 and LCDR1 of an antibody light chain, wherein the antibody light chain may comprise the amino acid sequence shown in SEQ ID NO: 11. For example, the antibody can be Atezolizumab.

For example, the first domain may comprise HCDR3, HCDR2 and HCDR1 of an antibody heavy chain, wherein the antibody heavy chain may comprise the amino acid sequence shown in SEQ ID NO:10, and the first domain may comprise LCDR3, LCDR2 and LCDR1 of an antibody light chain, wherein the antibody light chain may comprise the amino acid sequence shown in SEQ ID NO: 11. For example, the antibody can be Atezolizumab.

For example, the first domain may comprise the heavy chain variable region VH of an antibody heavy chain, and the antibody heavy chain may comprise the amino acid sequence shown in SEQ ID NO:10, and the first domain may comprise the light chain variable region VL of an antibody light chain, and the antibody light chain may comprise the amino acid sequence shown in SEQ ID NO: 11. For example, the antibody can be Atezolizumab.

For example, the first domain may comprise HCDR3, HCDR2 and HCDR1 of an antibody heavy chain, wherein the antibody heavy chain may comprise the amino acid sequence shown in SEQ ID NO: 12. For example, the antibody can be Avelumab.

For example, the first domain may comprise LCDR3, LCDR2 and LCDR1 of an antibody light chain, wherein the antibody light chain may comprise the amino acid sequence shown in SEQ ID NO:13. For example, the antibody can be Avelumab.

For example, the first domain may comprise HCDR3, HCDR2 and HCDR1 of an antibody heavy chain, wherein the antibody heavy chain may comprise the amino acid sequence shown in SEQ ID NO: 12, and the first domain may comprise LCDR3, LCDR2 and LCDR1 of an antibody light chain, wherein the antibody light chain may comprise the amino acid sequence shown in SEQ ID NO: 13. For example, the antibody can be Avelumab.

For example, the first domain may comprise the heavy chain variable region VH of an antibody heavy chain, and the antibody heavy chain may comprise the amino acid sequence shown in SEQ ID NO: 12, and the first domain may comprise the light chain variable region VL of an antibody light chain, and the antibody light chain may comprise the amino acid sequence shown in SEQ ID NO: 13. For example, the antibody can be Avelumab.

For example, the first domain may comprise HCDR3, HCDR2 and HCDR1 of an antibody, wherein the antibody may comprise the amino acid sequence shown in SEQ ID NO:14. For example, the antibody can be Envafolimab.

For example, the first domain may comprise HCDR3, HCDR2 and HCDR1 of an antibody heavy chain, wherein the antibody heavy chain may comprise the amino acid sequence shown in SEQ ID NO:15. For example, the antibody can be Permbrolizumab.

For example, the first domain may comprise LCDR3, LCDR2 and LCDR1 of an antibody light chain, wherein the antibody light chain may comprise the amino acid sequence shown in SEQ ID NO: 16. For example, the antibody can be Permbrolizumab.

For example, the first domain may comprise HCDR3, HCDR2 and HCDR1 of an antibody heavy chain, wherein the antibody heavy chain may comprise the amino acid sequence shown in SEQ ID NO:15, and the first domain may comprise LCDR3, LCDR2 and LCDR1 of an antibody light chain, wherein the antibody light chain may comprise the amino acid sequence shown in SEQ ID NO: 16. For example, the antibody can be Permbrolizumab.

For example, the first domain may comprise the heavy chain variable region VH of an antibody heavy chain, and the antibody heavy chain may comprise the amino acid sequence shown in SEQ ID NO:15, and the first domain may comprise the light chain variable region VL of an antibody light chain, and the antibody light chain may comprise the amino acid sequence shown in SEQ ID NO: 16. For example, the antibody can be Permbrolizumab.

For example, the first domain may comprise HCDR3, HCDR2 and HCDR1 of an antibody heavy chain, wherein the antibody heavy chain may comprise the amino acid sequence shown in SEQ ID NO: 17. For example, the antibody can be Nivolumab.

For example, the first domain may comprise LCDR3, LCDR2 and LCDR1 of an antibody light chain, wherein the antibody light chain may comprise the amino acid sequence shown in SEQ ID NO:18. For example, the antibody can be Nivolumab.

For example, the first domain may comprise HCDR3, HCDR2 and HCDR1 of an antibody heavy chain, wherein the antibody heavy chain may comprise the amino acid sequence shown in SEQ ID NO: 17, and the first domain may comprise LCDR3, LCDR2 and LCDR1 of an antibody light chain, wherein the antibody light chain may comprise the amino acid sequence shown in SEQ ID NO: 18. For example, the antibody can be Nivolumab.

For example, the first domain may comprise the heavy chain variable region VH of an antibody heavy chain, and the antibody heavy chain may comprise the amino acid sequence shown in SEQ ID NO: 17, and the first domain may comprise the light chain variable region VL of an antibody light chain, and the antibody light chain may comprise the amino acid sequence shown in SEQ ID NO: 18. For example, the antibody can be Nivolumab.

For example, the first domain may comprise an antibody or an antigen-binding fragment thereof.

For example, the antibody may be selected from the group consisting of a recombinant antibody, a single domain antibody, a heavy chain antibody, a chimeric antibody, and a bispecific antibody.

For example, the antigen-binding fragment may be selected from one or more of the group consisiting of: Fab, Fab', Fv fragment, F(ab')2, F(ab)2, scFv, di-scFv, VHH and dAb.

For example, the first domain may comprise an antibody or an antigen-binding fragment thereof selected from the group consisting of Sugemalimab, Durvalumab, Atezolizumab, Avelumab, Envafolimab, Permbrolizumab and Nivolumab.

For example, the first domain can comprise Sugemalimab or an antigen-binding fragment thereof.

For example, the second domain may comprise CD86 or a functionally active fragment thereof.

For example, the second domain can be selected from the group consisting of CD86 derived from humans or a functionally active fragment thereof and CD86 derived from mice or a functionally active fragment thereof.

For example, the second domain can bind to CD28, and/or CTLA4.

For example, the second domain may comprise the IgV domain of CD86 or a functionally active fragment thereof.

For example, the second domain may comprise the extracellular domain of CD86 or a functionally active fragment thereof.

For example, the second domain may comprise an amino acid sequence as shown in SEQ ID NO:2 or SEQ ID NO:3.

For example, the third domain is capable of binding to an MHCII molecule on an antigen presenting cell, and/or activating antigen presentation.

For example, the third domain may comprise LAG3 or a functionally active fragment thereof.

For example, the third domain can be selected from the group consisting of LAG3 derived from humans or a functionally active fragment thereof and LAG3 derived from mice or a functionally active fragment thereof.

For example, the third domain may comprise the extracellular domain of LAG3 or a functionally active fragment thereof.

For example, the third domain may comprise IgD1, IgD2, IgD3 and/or IgD4 of LAG3 or a functionally active fragment thereof.

For example, the third domain may comprise IgD1, IgD1-IgD2, IgD1-IgD2-IgD3, and/or IgD1-IgD2-IgD3-IgD4 of LAG3 or a functionally active fragment thereof.

For example, the third domain may comprise an amino acid sequence selected from the group consisting of SEQ ID NO:21, SEQ ID NO:22, SEQ ID NO:23, SEQ ID NO:24, SEQ ID NO:25, SEQ ID NO:26, SEQ ID NO:27 and SEQ ID NO:28.

For example, the first domain and the second domain may be linked directly or indirectly.

For example, the first domain comprises an antibody heavy chain, and the first domain antibody heavy chain and the second domain may be directly or indirectly connected.

For example, the first domain comprises an antibody heavy chain, and the C-terminus of the antibody heavy chain of the first domain and the N-terminus of the second domain can be directly or indirectly connected.

For example, the first domain comprises an antibody heavy chain, and the N-terminus of the antibody heavy chain of the first domain and the C-terminus of the second domain can be directly or indirectly connected.

For example, the first domain comprises an antibody light chain, and the first domain antibody light chain and the second domain may be directly or indirectly connected.

For example, the first domain comprises an antibody light chain, and the C-terminus of the antibody light chain of the first domain and the N-terminus of the second domain may be directly or indirectly connected.

For example, the first domain comprises an antibody light chain, and the N-terminus of the antibody light chain of the first domain and the C-terminus of the second domain may be directly or indirectly connected.

For example, the first domain comprises an antibody heavy chain and an antibody light chain, the C-terminus of the antibody heavy chain of the first domain may be directly or indirectly connected to the N-terminus of the second domain, and the C-terminus of the antibody light chain of the first domain may be directly or indirectly connected to the N-terminus of the second domain.

For example, the first domain comprises an antibody heavy chain and an antibody light chain, the N-terminus of the antibody heavy chain of the first domain may be directly or indirectly connected to the C-terminus of the second domain, and the N-terminus of the antibody light chain of the first domain may be directly or indirectly connected to the C-terminus of the second domain.

For example, the first domain and the third domain may be directly or indirectly connected.

For example, the first domain comprises an antibody heavy chain, and the first domain antibody heavy chain and the third domain may be directly or indirectly connected.

For example, the first domain comprises an antibody heavy chain, and the C-terminus of the antibody heavy chain of the first domain can be directly or indirectly connected to the N-terminus of the third domain.

For example, the first domain comprises an antibody heavy chain, and the N-terminus of the antibody heavy chain of the first domain and the C-terminus of the third domain may be directly or indirectly connected.

For example, the first domain comprises an antibody light chain, and the first domain light chain and the third domain may be directly or indirectly connected.

For example, the first domain comprises an antibody light chain, and the C-terminus of the light chain of the first domain and the N-terminus of the third domain may be directly or indirectly connected.

For example, the first domain comprises an antibody light chain, and the N-terminus of the light chain of the first domain and the C-terminus of the third domain may be directly or indirectly connected.

For example, the second domain and the third domain may be directly or indirectly linked.

For example, the C-terminus of the second domain and the N-terminus of the third domain may be directly or indirectly connected.

For example, the N-terminus of the second domain and the C-terminus of the third domain may be directly or indirectly connected.

For example, the first domain and the second domain may be directly or indirectly connected, and the second domain and the third domain may be directly or indirectly connected.

For example, the C-terminus of the first domain may be directly or indirectly connected to the N-terminus of the second domain, and the C-terminus of the second domain may be directly or indirectly connected to the N-terminus of the third domain.

For example, the first domain comprises an antibody heavy chain, the C-terminus of the antibody heavy chain of the first domain may be directly or indirectly connected to the N-terminus of the second domain, and the C-terminus of the second domain may be directly or indirectly connected to the N-terminus of the third domain.

For example, the first domain comprises an antibody light chain, the C-terminus of the antibody light chain of the first domain may be directly or indirectly connected to the N-terminus of the second domain, and the C-terminus of the second domain may be directly or indirectly connected to the N-terminus of the third domain.

For example, the N-terminus of the first domain and the C-terminus of the second domain may be directly or indirectly connected, and the N-terminus of the second domain and the C-terminus of the third domain may be directly or indirectly connected.

For example, the first domain comprises an antibody heavy chain, the N-terminus of the antibody heavy chain of the first domain may be directly or indirectly connected to the C-terminus of the second domain, and the N-terminus of the second domain may be directly or indirectly connected to the C-terminus of the third domain.

For example, the first domain comprises an antibody light chain, the N-terminus of the antibody light chain of the first domain may be directly or indirectly connected to the C-terminus of the second domain, and the N-terminus of the second domain may be directly or indirectly connected to the C-terminus of the third domain.

For example, the first domain and the third domain may be directly or indirectly connected, and the third domain and the second domain may be directly or indirectly connected.

For example, the C-terminus of the first domain may be directly or indirectly connected to the N-terminus of the third domain, and the C-terminus of the third domain may be directly or indirectly connected to the N-terminus of the second domain.

For example, the first domain comprises an antibody heavy chain, the C-terminus of the antibody heavy chain of the first domain may be directly or indirectly connected to the N-terminus of the third domain, and the C-terminus of the third domain may be directly or indirectly connected to the N-terminus of the second domain.

For example, the first domain comprises an antibody light chain, the C-terminus of the antibody light chain of the first domain may be directly or indirectly connected to the N-terminus of the third domain, and the C-terminus of the third domain may be directly or indirectly connected to the N-terminus of the second domain.

For example, the N-terminus of the first domain and the C-terminus of the third domain may be directly or indirectly connected, and the N-terminus of the third domain and the C-terminus of the second domain may be directly or indirectly connected.

For example, the first domain comprises an antibody heavy chain, the N-terminus of the antibody heavy chain of the first domain may be directly or indirectly connected to the C-terminus of the third domain, and the N-terminus of the third domain may be directly or indirectly connected to the C-terminus of the second domain.

For example, the first domain comprises an antibody light chain, the N-terminus of the antibody light chain of the first domain may be directly or indirectly connected to the C-terminus of the third domain, and the N-terminus of the third domain may be directly or indirectly connected to the C-terminus of the second domain.

For example, the first domain and the second domain may be directly or indirectly connected, and the first domain and the third domain may be directly or indirectly connected.

For example, the C-terminus of the first domain and the N-terminus of the second domain may be directly or indirectly connected, and the N-terminus of the first domain and the C-terminus of the third domain may be directly or indirectly connected.

For example, the first domain comprises an antibody heavy chain, the C-terminus of the antibody heavy chain of the first domain may be directly or indirectly connected to the N-terminus of the second domain, and the N-terminus of the heavy chain of the first domain may be directly or indirectly connected to the C-terminus of the third domain.

For example, the first domain comprises an antibody light chain, the C-terminus of the first domain antibody light chain and the N-terminus of the second domain may be directly or indirectly connected, and the N-terminus of the first domain light chain and the C-terminus of the third domain may be directly or indirectly connected.

For example, the first domain comprises an antibody heavy chain, the C-terminus of the antibody heavy chain of the first domain may be directly or indirectly connected to the N-terminus of the second domain, and the N-terminus of the light chain of the first domain may be directly or indirectly connected to the C-terminus of the third domain.

For example, the first domain comprises an antibody light chain, the C-terminus of the antibody light chain of the first domain may be directly or indirectly connected to the N-terminus of the second domain, and the N-terminus of the heavy chain of the first domain may be directly or indirectly connected to the C-terminus of the third domain.

For example, the N-terminus of the first domain may be directly or indirectly connected to the C-terminus of the second domain, and the C-terminus of the first domain may be directly or indirectly connected to the N-terminus of the third domain.

For example, the first domain comprises an antibody heavy chain, the N-terminus of the antibody heavy chain of the first domain may be directly or indirectly connected to the C-terminus of the second domain, and the C-terminus of the heavy chain of the first domain may be directly or indirectly connected to the N-terminus of the third domain.

For example, the first domain comprises an antibody light chain, the N-terminus of the first domain antibody light chain and the C-terminus of the second domain may be directly or indirectly connected, and the C-terminus of the first domain light chain and the N-terminus of the third domain may be directly or indirectly connected.

For example, the first domain comprises an antibody heavy chain and an antibody light chain, the N-terminus of the antibody heavy chain of the first domain may be directly or indirectly connected to the C-terminus of the second domain, and the C-terminus of the antibody light chain of the first domain may be directly or indirectly connected to the N-terminus of the third domain.

For example, the first domain comprises an antibody heavy chain and an antibody light chain, the N-terminus of the antibody light chain of the first domain may be directly or indirectly connected to the C-terminus of the second domain, and the C-terminus of the antibody heavy chain of the first domain may be directly or indirectly connected to the N-terminus of the third domain.

For example, the indirect connection may comprise connection via a linker.

For example, the linker may comprise an amino acid sequence selected from the group consisting of SEQ ID NO:31, SEQ ID NO:32, SEQ ID NO:33, SEQ ID NO:34 and SEQ ID NO:35.

For example, the fusion polypeptide can comprise an amino acid sequence selected from the group consisting of SEQ ID NO: 88, SEQ ID NO: 89, SEQ ID NO: 90, SEQ ID NO: 91, SEQ ID NO: 92, SEQ ID NO: 93, SEQ ID NO: 94, SEQ ID NO: 95, SEQ ID NO: 96, SEQ ID NO: 97, SEQ ID NO: 98, SEQ ID NO: 99, SEQ ID NO: 100, SEQ ID NO: 101, SEQ ID NO: 102, SEQ ID NO: 103, SEQ ID NO: 104, SEQ ID NO: 105, SEQ ID NO: 106, SEQ ID NO: 107, SEQ ID NO: 108, SEQ ID NO: 109, SEQ ID NO: 110, SEQ ID NO: 111, SEQ ID NO: 112, SEQ ID NO: 113, SEQ ID NO: 114, SEQ ID NO: 115, SEQ ID NO: 116, SEQ ID NO: 117, SEQ ID NO: 118, SEQ ID NO: 119, SEQ ID NO: 120, SEQ ID NO: 121, SEQ ID NO: 122, SEQ ID NO: 123, SEQ ID NO: 124, SEQ ID NO: 125, SEQ ID NO: 126, SEQ ID NO: 127, SEQ ID NO: 128, SEQ ID NO: 129, SEQ ID NO: 130, SEQ ID NO: 131, SEQ ID NO: 132, SEQ ID NO: 133, SEQ ID NO: 134, SEQ ID NO: 135, SEQ ID NO: 171, SEQ ID NO: 173.

For example, the fusion polypeptide may comprise an amino acid sequence selected from the group consisting of SEQ ID NO: 104, SEQ ID NO: 107, SEQ ID NO: 108, SEQ ID NO: 112, SEQ ID NO: 114, SEQ ID NO: 115, SEQ ID NO: 116, SEQ ID NO: 171, and SEQ ID NO: 173.

In one aspect, the present application discloses a dual and/or multifunctional fusion polypeptide comprising a double-chain fusion polypeptide, wherein the fusion polypeptide includes (i) an antigen binding fragment that has specific binding to a first cell target, the presence or absence of (ii) a first domain composed of an Fc domain and/or (iii) a second domain that has specific binding to a second cell target and/or (iv) a third domain that has specific binding to a third cell target, wherein the second cell target binds to the second domain through the C-terminus of the first domain polypeptide, or through the N-terminus of the first domain polypeptide fragment via or without a linker, and wherein the third cell target binds to the third domain through the C-terminus of the first domain polypeptide, or through the C-terminus of the second domain, or through the N-terminus of the first domain polypeptide, or through the N-terminus of the second domain via or without a linker; it should be clear that the first domain disclosed in the present invention can be extended to certain anti-PD-L1 and/or anti-PD-1 antibodies without an Fc domain, including but not limited to single domain antibodies, recombinant antibodies and single chain antibodies. For example, the C-terminus of the heavy chain polypeptide or light chain polypeptide of the first domain is covalently linked to the second domain and/or the third domain by a linker, which should be regarded as the scope of the claims of the present invention; similarly, the N-terminus of the heavy chain polypeptide or light chain polypeptide of the first domain is covalently linked to the second domain and/or the third domain by a linker, which should also be regarded as the scope of the claims of the present invention. The dual and/or multifunctional fusion polypeptide is thus capable of binding to the first cell target, the second cell target and/or the third cell target simultaneously.

In one aspect, the present invention provides a specific polypeptide complex constituting a dual and/or multifunctional fusion polypeptide, wherein the specific polypeptide complex comprises a first polypeptide and a second polypeptide constituting the polypeptide complex. It should be understood that the second polypeptide may not be present in certain specific antibodies.

On the other hand, the second polypeptide of the polypeptide complex of the present invention is the light chain of the first domain; the first polypeptide of the polypeptide complex of the present invention includes, from the N-terminus to the C-terminus of the polypeptide, the third domain and/or the second domain, a linker, the heavy chain of the first domain, a linker, and the third domain and/or the second domain covalently connected, and the first polypeptide and the second polypeptide constitute a specific polypeptide complex of a dual and/or multifunctional fusion polypeptide; wherein the linker can exist or not exist independently of each other; in some cases, only one of the second domain or the third domain exists.

On the other hand, the second polypeptide of the polypeptide complex of the present invention is the light chain of the first domain; the first polypeptide of the polypeptide complex of the present invention includes, from the N-terminus to the C-terminus of the polypeptide, the heavy chain of the first domain, a linker, the third domain and/or the second domain, the linker and the third domain and/or the second domain covalently connected, and the first polypeptide and the second polypeptide constitute a specific polypeptide complex of a dual and/or multifunctional fusion polypeptide; wherein the linker can exist or not exist independently of each other; in some cases, only one of the second domain or the third domain exists.

On the other hand, the second polypeptide of the polypeptide complex of the present invention is the light chain of the first domain; the first polypeptide of the polypeptide complex of the present invention includes, from the N-terminus to the C-terminus of the polypeptide, the third domain and/or the second domain, a linker, the third domain and/or the second domain, a linker and the first domain heavy chain covalently connected, and the first polypeptide and the second polypeptide constitute a specific polypeptide complex of a dual and/or multifunctional fusion polypeptide; wherein the linker can exist or not exist independently of each other; in some cases, only one of the second domain or the third domain exists.

On the other hand, the first polypeptide of the polypeptide complex of the present invention is the heavy chain of the first domain; the second polypeptide of the polypeptide complex of the present invention includes, from the N-terminus to the C-terminus of the polypeptide, the light chain of the first domain, a linker, the third domain and/or the second domain, and the linker and the third domain and/or the second domain covalently connected, and the first polypeptide and the second polypeptide constitute a specific polypeptide complex of a dual and/or multifunctional fusion polypeptide; wherein the linker can exist or not exist independently of each other; in some cases, only one of the second domain or the third domain exists.

On the other hand, the first polypeptide of the polypeptide complex of the present invention is the heavy chain of the first domain; the second polypeptide of the polypeptide complex of the present invention includes, from the N-terminus to the C-terminus of the polypeptide, the light chain of the first domain, a linker, the third domain and/or the second domain, and the linker and the third domain and/or the second domain covalently connected, and the first polypeptide and the second polypeptide constitute a specific polypeptide complex of a dual and/or multifunctional fusion polypeptide; wherein the linker can exist or not exist independently of each other; in some cases, only one of the second domain or the third domain exists.

On the other hand, the first polypeptide of the polypeptide complex of the present invention is the heavy chain of the first domain; the second polypeptide of the polypeptide complex of the present invention includes, from the N-terminus to the C-terminus of the polypeptide, the third domain and/or the second domain, a linker, the third domain and/or the second domain, a linker and the light chain of the first domain covalently connected, and the first polypeptide and the second polypeptide constitute a specific polypeptide complex of a dual and/or multifunctional fusion polypeptide; wherein the linker can exist or not exist independently of each other; in some cases, only one of the second domain or the third domain exists.

In one aspect, the first polypeptide of the polypeptide complex of the present invention comprises, from the N-terminus to the C-terminus of the polypeptide, a third domain and/or a second domain, a linker and a heavy chain of the first domain covalently linked; the second polypeptide of the polypeptide complex of the present invention comprises, from the N-terminus to the C-terminus of the polypeptide, a light chain of the first domain, a linker and a third domain and/or a second domain covalently linked; wherein the linker may exist independently or not; in some cases, only one of the second domain or the third domain exists.

In one aspect, the first polypeptide of the polypeptide complex of the present invention comprises, from the N-terminus to the C-terminus of the polypeptide, a third domain and/or a second domain, a linker and a heavy chain of the first domain covalently linked; the second polypeptide of the polypeptide complex of the present invention comprises, from the N-terminus to the C-terminus of the polypeptide, a third domain and/or a second domain, a linker and a light chain of the first domain covalently linked; wherein the linker may exist or not exist independently of each other; in some cases, only one of the second domain or the third domain exists.

In one aspect, the first polypeptide of the polypeptide complex of the present invention comprises, from the N-terminus to the C-terminus of the polypeptide, a first domain heavy chain, a linker, and a third domain and/or a second domain covalently connected; the second polypeptide of the polypeptide complex of the present invention comprises, from the N-terminus to the C-terminus of the polypeptide, a third domain and/or a second domain, a linker, and a first domain light chain covalently connected; wherein the linker may exist or not exist independently of each other; in some cases, only one of the second domain or the third domain exists.

In one aspect, the first polypeptide of the polypeptide complex of the present invention comprises, from the N-terminus to the C-terminus of the polypeptide, a first domain heavy chain, a linker, and a third domain and/or a second domain covalently connected; the second polypeptide of the polypeptide complex of the present invention comprises, from the N-terminus to the C-terminus of the polypeptide, a first domain light chain, a linker, and a third domain and/or a second domain covalently connected; wherein the linker may exist or not exist independently of each other; in some cases, only one of the second domain or the third domain exists.

On the other hand, the polypeptide complex of the present invention only comprises the structure of the first polypeptide, and its structure includes, from the N-terminus to the C-terminus of the polypeptide, the first domain heavy chain, the linker, the third domain and/or the second domain, and the linker and the third domain and/or the second domain covalently linked to form a specific polypeptide complex of a dual and/or multifunctional fusion polypeptide; wherein the linkers may exist or not exist independently of each other; in some cases, only one of the second domain or the third domain exists.

On the other hand, the polypeptide complex of the present invention only comprises the structure of the first polypeptide, and its structure includes, from the N-terminus to the C-terminus of the polypeptide, the first domain heavy chain, the linker, the third domain and/or the second domain, and the linker and the third domain and/or the second domain covalently linked to form a specific polypeptide complex of a dual and/or multifunctional fusion polypeptide; wherein the linkers may exist or not exist independently of each other; in some cases, only one of the second domain or the third domain exists.

On the other hand, the polypeptide complex of the present invention only comprises the structure of the first polypeptide, and its structure includes, from the N-terminus to the C-terminus of the polypeptide, the third domain and/or the second domain, the linker, the third domain and/or the second domain, the linker and the first domain heavy chain covalently connected to form a specific polypeptide complex of a dual and/or multifunctional fusion polypeptide; wherein the linker may exist or not exist independently of each other; in some cases, only one of the second domain or the third domain exists.

The (a) antigen-binding fragment contained in the first domain in the dual and/or multifunctional fusion polypeptide of the present invention can be derived from any anti-PD-L1 and/or anti-PD-1 antibody, including any antibody that can block or reduce the binding of PD-L1 to its receptor and then relieve the immunosuppressive effect mediated by PD-L1, including the currently published anti-PD-L1 and/or anti-PD-1 antibodies and the anti-PD-L1 antibodies and/or anti-PD-1 antibodies to be developed in the future.

The dual and/or multifunctional fusion polypeptide of the present invention comprises the second domain comprising the extracellular region of one or more ligands, which at least includes a polypeptide consisting of the CD86 extracellular domain or the IgV region of the CD86 extracellular domain. For example, CD86 is a member protein of the B7 molecule family that has the function of costimulating and activating T cells. The mature CD86 molecule is composed of an extracellular domain (ECD), a transmembrane domain and an intracellular domain. The extracellular domain (ECD) is the key region for these molecules to bind to the corresponding receptors on T cells. The CD86 extracellular domain contains an immunoglobulin-like V (IgV) region and an immunoglobulin-like C2 (IgC2) region, and the IgV domain is the key domain that is directly involved in its receptor binding. Both the CD86 extracellular domain and IgV can bind to CD28 to participate in inducing T cell activation, proliferation and effector function. CTLA4 can also bind to the IgV region of CD86 to participate in immunosuppressive regulation.

The dual and/or multifunctional fusion polypeptide of the present invention comprises an extracellular region in which the third domain comprises one and/or multiple ligands, which at least includes the LAG3 extracellular domain or one or more subunits of a polypeptide composed of IgD1, IgD2, IgD3 and IgD4 in the LAG3 extracellular domain. For example, LAG3 can be a type 1 transmembrane protein in immune checkpoints and can be mainly expressed on activated NK cells and T cells. LAG3 is expressed on exhausted CD8+ T cells in tumors and may be an effective target for tumor immunotherapy. For example, the function of LAG3 can be dual-sided. On the one hand, LAG3 can play a negative regulatory role in T cell proliferation and activation. After binding to its ligand, major histocompatibility complex II (MHCII) or ligand fibrinogen-like protein 1 (FGL1), LAG3 can participate in inhibiting T cell activation and function. On the other hand, LAG3 can participate in inducing the activation of antigen-presenting cells by binding to MHCII molecules on antigen-presenting cells, such as upregulating the expression of CD80/CD83/CD86, enhancing antigen presentation, and secreting chemokines to recruit T cells, thereby assisting T cell activation. Antibodies that antagonize LAG3 have the ability to directly relieve LAG3-mediated T cell inhibition, but they also block LAG3-mediated antigen-presenting cell activation, and therefore cannot fully enhance LAG3-related immune activation effects. The LAG3 extracellular domain chimeric protein provided in the present application can simultaneously take into account the effects of T cell immunosuppression relief and antigen presenting cell activation, and may have potential prospects for tumor treatment.

Provided below is: an exemplary anti-PD-L1 antibody Sugemalimab is linked through the Fc domain to a linker peptide covalently linked to the CD86 extracellular domain to form the bifunctional fusion polypeptide complex first polypeptide amino acid sequence (SEQ ID NO: 36), and the first polypeptide and the Sugemalimab light chain second polypeptide (SEQ ID NO: 5) constitute the bifunctional fusion polypeptide complex; Sugemalimab is linked through the Fc domain to a linker peptide covalently linked to CD86 extracellular domain IgV domain to form the bifunctional fusion polypeptide complex first polypeptide amino acid sequence (SEQ ID NO: 37), and the first polypeptide and Sugemalimab light chain second polypeptide constitute the bifunctional antibody polypeptide complex; Sugemalimab is linked through the antigen binding region heavy chain N-terminus to a linker peptide covalently linked to CD86 extracellular domain to form the bifunctional fusion polypeptide complex first polypeptide amino acid sequence (SEQ ID NO: 38), and the first polypeptide and Sugemalimab light chain second polypeptide constitute the bifunctional fusion polypeptide complex; Sugemalimab is linked through the antigen binding region heavy chain N-terminus to a linker peptide covalently linked to CD86 extracellular domain IgV domain to form the bifunctional fusion polypeptide complex first polypeptide amino acid sequence (SEQ ID NO: 39), and the first polypeptide and Sugemalimab light chain second polypeptide constitute the bifunctional fusion polypeptide complex; Sugemalimab is linked through the antigen binding region light chain C-terminus to a linker peptide covalently linked to CD86 extracellular domain to form bifunctional fusion polypeptide complex second polypeptide amino acid sequence (SEQ ID NO: 40), and the second polypeptide and the Sugemalimab heavy chain first polypeptide (SEQ ID NO: 4) constitute the bifunctional fusion polypeptide complex; Sugemalimab is linked through the antigen binding region light chain C-terminus to a linker peptide covalently linked to CD86 extracellular domain IgV domain to form the bifunctional fusion polypeptide complex second polypeptide amino acid sequence (SEQ ID NO: 41), and the second polypeptide and the Sugemalimab heavy chain first polypeptide constitute the bifunctional fusion polypeptide complex; Sugemalimab is linked through antigen binding region light chain N-terminus to a linker peptide covalently linked to CD86 extracellular domain to form the bifunctional fusion polypeptide complex second polypeptide amino acid sequence (SEQ ID NO: 42), and the second polypeptide and the Sugemalimab heavy chain first polypeptide constitute the bifunctional fusion polypeptide complex; Sugemalimab is linked through antigen binding region light chain N-terminus to a linker peptide covalently linked to CD86 extracellular domain IgV domain to form the bifunctional fusion polypeptide complex second polypeptide amino acid sequence (SEQ ID NO: 43), and the second polypeptide and the Sugemalimab heavy chain first polypeptide constitute the bifunctional fusion polypeptide complex.

Provided below is: an exemplary anti-PD-L1 antibody Durvalumab is linked through the Fc domain to a linker peptide covalently linked to the CD86 extracellular domain to form the bifunctional fusion polypeptide complex first polypeptide amino acid sequence (SEQ ID NO: 44), and the first polypeptide and Durvalumab light chain second polypeptide (SEQ ID NO: 9) constitute the bifunctional fusion polypeptide complex; Durvalumab is linked through the Fc domain to a linker peptide covalently linked to CD86 extracellular domain IgV domain to form the to form the bifunctional fusion polypeptide complex first polypeptide amino acid sequence (SEQ ID NO: 45), and the first polypeptide and Durvalumab light chain second polypeptide constitute the bifunctional antibody polypeptide complex; Durvalumab is linked through the antigen binding region heavy chain N-terminus to a linker peptide covalently linked to CD86 extracellular domain to form the bifunctional fusion polypeptide complex first polypeptide amino acid sequence (SEQ ID NO: 46), and the first polypeptide and Durvalumab light chain second polypeptide constitute the bifunctional fusion polypeptide complex; Durvalumab is linked through the antigen binding region heavy chain N-terminus to a linker peptide covalently linked to CD86 extracellular domain IgV domain to form the to form the bifunctional fusion polypeptide complex first polypeptide amino acid sequence (SEQ ID NO: 47), and the first polypeptide and Durvalumab light chain second polypeptide constitute the bifunctional fusion polypeptide complex; Durvalumab is linked through the antigen binding region light chain C-terminus to a linker peptide covalently linked to CD86 extracellular domain to form the bifunctional fusion polypeptide complex second polypeptide amino acid sequence (SEQ ID NO: 48), and the second polypeptide and the Durvalumab heavy chain first polypeptide (SEQ ID NO: 8) constitute the bifunctional fusion polypeptide complex; Durvalumab is linked through the antigen binding region light chain C-terminus to a linker peptide covalently linked to CD86 extracellular domain IgV domain to form the to form the bifunctional fusion polypeptide complex second polypeptide amino acid sequence (SEQ ID NO: 49), and the second polypeptide and the Durvalumab heavy chain first polypeptide constitute the bifunctional fusion polypeptide complex; the second polypeptide and Durvalumab heavy chain first polypeptide constitute the bifunctional antibody polypeptide complex; Durvalumab is linked through the antigen binding region light chain N-terminus to a linker peptide covalently linked to CD86 extracellular domain to form the bifunctional fusion polypeptide complex second polypeptide amino acid sequence (SEQ ID NO: 50), and the second polypeptide and the Durvalumab heavy chain first polypeptide constitute the bifunctional fusion polypeptide complex; Durvalumab is linked through the antigen binding region light chain N-terminus to a linker peptide covalently linked to CD86 extracellular domain IgV domain to form the bifunctional fusion polypeptide complex second polypeptide amino acid sequence (SEQ ID NO: 51), and the second polypeptide and the Durvalumab heavy chain first polypeptide constitute the bifunctional fusion polypeptide complex.

Provided below is an exemplary anti-PD-L1 antibody Atezolizumab is linked through the Fc domain to a linker peptide covalently linked to the CD86 extracellular domain to form the bifunctional fusion polypeptide complex first polypeptide amino acid sequence (SEQ ID NO: 52), and the first polypeptide and Atezolizumab light chain second polypeptide (SEQ ID NO: 11) constitute the bifunctional fusion polypeptide complex; Atezolizumab is linked through the Fc domain to a linker peptide covalently linked to CD86 extracellular domain IgV domain to form the bifunctional fusion polypeptide complex first polypeptide amino acid sequence (SEQ ID NO: 53), and the first polypeptide and Atezolizumab light chain second polypeptide constitute the bifunctional antibody polypeptide complex; Atezolizumab is linked through the antigen binding region heavy chain N-terminus to a linker peptide covalently linked to CD86 extracellular domain to form the bifunctional fusion polypeptide complex first polypeptide amino acid sequence (SEQ ID NO: 54), and the first polypeptide and Atezolizumab light chain second polypeptide constitute the bifunctional fusion polypeptide complex; Atezolizumab is linked through the antigen binding region heavy chain N-terminus to a linker peptide covalently linked to CD86 extracellular domain IgV domain to form the bifunctional fusion polypeptide complex first polypeptide amino acid sequence (SEQ ID NO: 55), and the first polypeptide and Atezolizumab light chain second polypeptide constitute the bifunctional fusion polypeptide complex; Atezolizumab is linked through the antigen binding region light chain C-terminus to a linker peptide covalently linked to CD86 extracellular domain to form the bifunctional fusion polypeptide complex second polypeptide amino acid sequence (SEQ ID NO: 56), and the second polypeptide and the Atezolizumab heavy chain first polypeptide (SEQ ID NO: 10) constitute the bifunctional fusion polypeptide complex; Atezolizumab is linked through the antigen binding region light chain C-terminus to a linker peptide covalently linked to CD86 extracellular domain IgV domain to form the bifunctional fusion polypeptide complex second polypeptide amino acid sequence (SEQ ID NO: 57), and the second polypeptide and the Atezolizumab heavy chain first polypeptide constitute the bifunctional fusion polypeptide complex; Atezolizumab is linked through the antigen binding region light chain N-terminus to a linker peptide covalently linked to CD86 extracellular domain to form the bifunctional fusion polypeptide complex second polypeptide amino acid sequence (SEQ ID NO: 58), and the second polypeptide and the Atezolizumab heavy chain first polypeptide constitute the bifunctional fusion polypeptide complex; Atezolizumab is linked through the antigen binding region light chain N-terminus to a linker peptide covalently linked to CD86 extracellular domain IgV domainto form the bifunctional fusion polypeptide complex second polypeptide amino acid sequence (SEQ ID NO: 59), and the second polypeptide and the Atezolizumab heavy chain first polypeptide constitute the bifunctional fusion polypeptide complex.

Provided below is an exemplary anti-PD-L1 antibody Avelumab is linked through the Fc domain to a linker peptide covalently linked to the CD86 extracellular domain to form the bifunctional fusion polypeptide complex first polypeptide amino acid sequence (SEQ ID NO: 60), and the first polypeptide and Avelumab light chain second polypeptide (SEQ ID NO: 13) constitute the bifunctional fusion polypeptide complex; Avelumab is linked through the Fc domain to a linker peptide covalently linked to CD86 extracellular domain IgV domain to form the bifunctional fusion polypeptide complex first polypeptide amino acid sequence (SEQ ID NO: 61), and the first polypeptide and Avelumab light chain second polypeptide constitute the bifunctional antibody polypeptide complex; Avelumab is linked through the antigen binding region heavy chain N-terminus to a linker peptide covalently linked to CD86 extracellular domain to form bifunctional fusion polypeptide complex first polypeptide amino acid sequence (SEQ ID NO: 62), and the first polypeptide and Avelumab light chain second polypeptide constitute the bifunctional fusion polypeptide complex; Avelumab is linked through the antigen binding region heavy chain N-terminus to a linker peptide covalently linked to CD86 extracellular domain IgV domain to form the bifunctional fusion polypeptide complex first polypeptide amino acid sequence (SEQ ID NO: 63), and the first polypeptide and Avelumab light chain second polypeptide constitute the bifunctional fusion polypeptide complex; Avelumab is linked through the antigen binding region light chain C-terminus to a linker peptide covalently linked to CD86 extracellular domain to form bifunctional fusion polypeptide complex second polypeptide amino acid sequence (SEQ ID NO: 64), and the second polypeptide and the Avelumab heavy chain first polypeptide (SEQ ID NO: 12) constitute the bifunctional fusion polypeptide complex; Avelumab is linked through the antigen binding region light chain C-terminus to a linker peptide covalently linked to CD86 extracellular domain IgV domain to form the bifunctional fusion polypeptide complex second polypeptide amino acid sequence (SEQ ID NO: 65), and the second polypeptide and the Avelumab heavy chain first polypeptide constitute the bifunctional fusion polypeptide complex; Avelumab is linked through the antigen binding region light chain N-terminus to a linker peptide covalently linked to CD86 extracellular domain to form the bifunctional fusion polypeptide complex second polypeptide amino acid sequence (SEQ ID NO: 66), and the second polypeptide and the Avelumab heavy chain first polypeptide constitute the bifunctional fusion polypeptide complex; Avelumab is linked through the antigen binding region light chain N-terminus to a linker peptide covalently linked to CD86 extracellular domain IgV domain to form the bifunctional fusion polypeptide complex second polypeptide amino acid sequence (SEQ ID NO: 67), and the second polypeptide and the Avelumab heavy chain first polypeptide constitute the bifunctional fusion polypeptide complex.

Provided below is: an exemplary anti-PD-L1 antibody Envafolimab is linked through the Fc domain to a linker peptide covalently linked to the CD86 extracellular domain to form the bifunctional fusion polypeptide complex first polypeptide amino acid sequence (SEQ ID NO: 68), and the first polypeptide constitute the bifunctional fusion polypeptide complex; Envafolimab is linked through the Fc domain to a linker peptide covalently linked to CD86 extracellular domain IgV domain to form the bifunctional fusion polypeptide complex first polypeptide amino acid sequence (SEQ ID NO: 69 ), and the first polypeptide constitute the bifunctional antibody polypeptide complex; Envafolimab is linked through the antigen binding region heavy chain N-terminus to a linker peptide covalently linked to CD86 extracellular domain to form the bifunctional fusion polypeptide complex first polypeptide amino acid sequence (SEQ ID NO: 70), and the first polypeptide constitute the bifunctional fusion polypeptide complex; Envafolimab is linked through the antigen binding region heavy chain N-terminus to a linker peptide covalently linked to CD86 extracellular domain IgV domain to form the bifunctional fusion polypeptide complex first polypeptide amino acid sequence (SEQ ID NO: 71), and the first polypeptide constitute the bifunctional fusion polypeptide complex;

Provided below is: an exemplary anti-PD-1 antibody Permbrolizumab is linked through the Fc domain to a linker peptide covalently linked to the CD86 extracellular domain to form the bifunctional fusion polypeptide complex first polypeptide amino acid sequence (SEQ ID NO: 72), and the first polypeptide and Permbrolizumab light chain second polypeptide (SEQ ID NO: 16) constitute the bifunctional fusion polypeptide complex; Permbrolizumab is linked through the Fc domain to a linker peptide covalently linked to CD86 extracellular domain IgV domain to form the bifunctional fusion polypeptide complex first polypeptide amino acid sequence (SEQ ID NO: 73), and the first polypeptide and Permbrolizumab light chain second polypeptide constitute the bifunctional antibody polypeptide complex; Permbrolizumab is linked through the antigen binding region heavy chain N-terminus to a linker peptide covalently linked to CD86 extracellular domain to form the bifunctional fusion polypeptide complex first polypeptide amino acid sequence (SEQ ID NO: 74), and the first polypeptide and Permbrolizumab light chain second polypeptide constitute the bifunctional fusion polypeptide complex; Permbrolizumab is linked through the antigen binding region heavy chain N-terminus to a linker peptide covalently linked to CD86 extracellular domain IgV domain to form the bifunctional fusion polypeptide complex first polypeptide amino acid sequence (SEQ ID NO: 75), and the first polypeptide and Permbrolizumab light chain second polypeptide constitute the bifunctional fusion polypeptide complex; Permbrolizumab is linked through the antigen binding region light chain C-terminus to a linker peptide covalently linked to CD86 extracellular domain to form the bifunctional fusion polypeptide complex second polypeptide amino acid sequence (SEQ ID NO: 76), and the second polypeptide and the Permbrolizumab heavy chain first polypeptide (SEQ ID NO: 15) constitute the bifunctional fusion polypeptide complex; Permbrolizumab is linked through the antigen binding region light chain C-terminus to a linker peptide covalently linked to CD86 extracellular domain IgV domain to form the bifunctional fusion polypeptide complex second polypeptide amino acid sequence (SEQ ID NO: 77), and the second polypeptide and the Permbrolizumab heavy chain first polypeptide constitute the bifunctional fusion polypeptide complex; Permbrolizumab is linked through the antigen binding region light chain N-terminus to a linker peptide covalently linked to CD86 extracellular domain to form the bifunctional fusion polypeptide complex second polypeptide amino acid sequence (SEQ ID NO: 78), and the second polypeptide and the Permbrolizumab heavy chain first polypeptide constitute the bifunctional fusion polypeptide complex; Permbrolizumab is linked through the antigen binding region light chain N-terminus to a linker peptide covalently linked to CD86 extracellular domain IgV domain to form the bifunctional fusion polypeptide complex second polypeptide amino acid sequence (SEQ ID NO: 79), and the second polypeptide and the Permbrolizumab heavy chain first polypeptide constitute the bifunctional fusion polypeptide complex.

Provided below is: an exemplary anti-PD-1 antibody Nivolumab is linked through the Fc domain to a linker peptide covalently linked to the CD86 extracellular domain to form the bifunctional fusion polypeptide complex first polypeptide amino acid sequence (SEQ ID NO: 80), and the first polypeptide and Nivolumab light chain second polypeptide (SEQ ID NO: 18) constitute the bifunctional fusion polypeptide complex; Nivolumab is linked through the Fc domain to a linker peptide covalently linked to CD86 extracellular domain IgV domain to form the bifunctional fusion polypeptide complex first polypeptide amino acid sequence (SEQ ID NO: 81), and the first polypeptide and Nivolumab light chain second polypeptide constitute the bifunctional antibody polypeptide complex; Nivolumab is linked through the antigen binding region heavy chain N-terminus to a linker peptide covalently linked to CD86 extracellular domain to form the bifunctional fusion polypeptide complex first polypeptide amino acid sequence (SEQ **ID** NO: 82), and the first polypeptide and Nivolumab light chain second polypeptide constitute the bifunctional fusion polypeptide complex; Nivolumab is linked through the antigen binding region heavy chain N-terminus to a linker peptide covalently linked to CD86 extracellular domain IgV domain to form the bifunctional fusion polypeptide complex first polypeptide amino acid sequence (SEQ ID NO: 83), and the first polypeptide and Nivolumab light chain second polypeptide constitute the bifunctional fusion polypeptide complex; Nivolumab is linked through the antigen binding region light chain C-terminus to a linker peptide covalently linked to CD86 extracellular domain to form the bifunctional fusion polypeptide complex second polypeptide amino acid sequence (SEQ ID NO: 84, and the second polypeptide and the Nivolumab heavy chain first polypeptide (SEQ ID NO: 17) constitute the bifunctional fusion polypeptide complex; Nivolumab is linked through the antigen binding region light chain C-terminus to a linker peptide covalently linked to CD86 extracellular domain IgV domain to form the bifunctional fusion polypeptide complex second polypeptide amino acid sequence (SEQ ID NO: 85), and the second polypeptide and the Nivolumab heavy chain first polypeptide constitute the bifunctional fusion polypeptide complex; Nivolumab is linked through the antigen binding region light chain N-terminus to a linker peptide covalently linked to CD86 extracellular domain to form the bifunctional fusion polypeptide complex second polypeptide amino acid sequence (SEQ ID NO: 86), and the second polypeptide and the Nivolumab heavy chain first polypeptide constitute the bifunctional fusion polypeptide complex; Nivolumab is linked through the antigen binding region light chain N-terminus to a linker peptide covalently linked to CD86 extracellular domain IgV domain to form the bifunctional fusion polypeptide complex second polypeptide amino acid sequence (SEQ ID NO: 87), and the second polypeptide and the Nivolumab heavy chain first polypeptide constitute the bifunctional fusion polypeptide complex.

Provided below is: an exemplary anti-PD-L1 antibody Sugemalimab heavy chain is linked through the Fc domain C-terminus to a linker peptide covalently linked to CD86 extracellular domain, and CD86 extracellular domain C-terminus is through a linker peptide covalently linked to LAG3 extracellular domain to form the multifunctional fusion polypeptide complex first polypeptide amino acid sequence (SEQ ID NO: 88), and the first polypeptide and Sugemalimab light chain second polypeptide constitute the multifunctional fusion polypeptide complex; Sugemalimab heavy chain is linked through the Fc domain C-terminus to a linker peptide covalently linked to CD86 extracellular domain, and CD86 extracellular domain C-terminus through a linker peptide is covalently linked to LAG3 extracellular domain D1/D2/D3 to form the multifunctional fusion polypeptide complex first polypeptide amino acid sequence (SEQ ID NO: 89), and the first polypeptide and Sugemalimab light chain second polypeptide constitute the multifunctional fusion polypeptide complex; Sugemalimab heavy chain is linked through the Fc domain C-terminus to a linker peptide covalently linked to CD86 extracellular domain, and CD86 extracellular domain C-terminus through a linker peptide is covalently linked to LAG3 extracellular domain D1/D2 to form the multifunctional fusion polypeptide complex first polypeptide amino acid sequence (SEQ ID NO: 90), and the first polypeptide and Sugemalimab light chain second polypeptide constitute the multifunctional fusion polypeptide complex; Sugemalimab heavy chain is linked through the Fc domain C-terminus to a linker peptide covalently linked to CD86 extracellular domain, and CD86 extracellular domain C-terminus through a linker peptide is covalently linked to LAG3 extracellular domain D1 to form the multifunctional fusion polypeptide complex first polypeptide amino acid sequence (SEQ ID NO: 91 ), and the first polypeptide and Sugemalimab light chain second polypeptide constitute the multifunctional fusion polypeptide complex; Sugemalimab heavy chain is linked through the Fc domain C-terminus to a linker peptide covalently linked to LAG3 extracellular domain , and LAG3 extracellular domain C-terminus through a linker peptide is covalently linked to CD86 extracellular domain to form the multifunctional fusion polypeptide complex first polypeptide amino acid sequence (SEQ ID NO: 92), and the first polypeptide and Sugemalimab light chain second polypeptide constitute the multifunctional fusion polypeptide complex; Sugemalimab heavy chain is linked through the Fc domain C-terminus to a linker peptide covalently linked to LAG3 extracellular domain D1/D2/D3, and LAG3 extracellular domain D1/D2/D3 C-terminus through a linker peptide is covalently linked to CD86 extracellular domain to form the multifunctional fusion polypeptide complex first polypeptide amino acid sequence (SEQ ID NO: 93), and the first polypeptide and Sugemalimab light chain second polypeptide constitute the multifunctional fusion polypeptide complex; Sugemalimab heavy chain is linked through the Fc domain C-terminus to a linker peptide covalently linked to LAG3 extracellular domain D1/D2, and LAG3 extracellular domain D1/D2 C-terminus through a linker peptide is covalently linked to CD86 extracellular domain to form the multifunctional fusion polypeptide complex first polypeptide amino acid sequence (SEQ ID NO: 94), and the first polypeptide and Sugemalimab light chain second polypeptide constitute the multifunctional fusion polypeptide complex; Sugemalimab heavy chain is linked through the Fc domain C-terminus to a linker peptide covalently linked to LAG3 extracellular domain D1, and LAG3 extracellular domain D1 C-terminus through a linker peptide is covalently linked to CD86 extracellular domain to form the multifunctional fusion polypeptide complex first polypeptide amino acid sequence (SEQ ID NO: 95), and the first polypeptide and Sugemalimab light chain second polypeptide constitute the multifunctional fusion polypeptide complex.

Provided below is an exemplary anti-PD-L1 antibody Sugemalimab heavy chain is linked through the Fc domain C-terminus to a linker peptide covalently linked to CD86 extracellular domain IgV domain, and CD86 extracellular domain IgV domain C-terminus is through a linker peptide covalently linked to LAG3 extracellular domain to form the multifunctional fusion polypeptide complex first polypeptide amino acid sequence (SEQ ID NO: 96), and the first polypeptide and Sugemalimab light chain second polypeptide constitute the multifunctional fusion polypeptide complex; Sugemalimab heavy chain is linked through the Fc domain C-terminus to a linker peptide covalently linked to CD86 extracellular domain IgV domain, and CD86 extracellular domain IgV domain C-terminus is through a linker peptide covalently linked to LAG3 extracellular domain D1/D2/D3 to form the multifunctional fusion polypeptide complex first polypeptide amino acid sequence (SEQ ID NO: 97), and the first polypeptide and Sugemalimab light chain second polypeptide constitute the multifunctional fusion polypeptide complex; Sugemalimab heavy chain is linked through the Fc domain C-terminus to a linker peptide covalently linked to CD86 extracellular domain IgV domain, and CD86 extracellular domain IgV domain C-terminus is through a linker peptide covalently linked to LAG3 extracellular domain D1/D2 to form the multifunctional fusion polypeptide complex first polypeptide amino acid sequence (SEQ ID NO: 98), and the first polypeptide and Sugemalimab light chain second polypeptide constitute the multifunctional fusion polypeptide complex; Sugemalimab heavy chain is linked through the Fc domain C-terminus to a linker peptide covalently linked to CD86 extracellular domain IgV domain, and CD86 extracellular domain IgV domain C-terminus is through a linker peptide covalently linked to LAG3 extracellular domain D1 to form the multifunctional fusion polypeptide complex first polypeptide amino acid sequence (SEQ ID NO: 99), and the first polypeptide and Sugemalimab light chain second polypeptide constitute the multifunctional fusion polypeptide complex; Sugemalimab heavy chain is linked through the Fc domain C-terminus to a linker peptide covalently linked to LAG3 extracellular domain, LAG3 extracellular domain C-terminus is through a linker peptide covalently linked to CD86 extracellular domain IgV domain to form the multifunctional fusion polypeptide complex first polypeptide amino acid sequence (SEQ ID NO: 100), and the first polypeptide and Sugemalimab light chain second polypeptide constitute the multifunctional fusion polypeptide complex; Sugemalimab heavy chain is linked through the Fc domain C-terminus to a linker peptide covalently linked to LAG3 extracellular domain D1/D2/D3, amd LAG3 extracellular domain D1/D2/D3 C-terminus is through a linker peptide covalently linked to CD86 extracellular domain IgV domain to form the multifunctional fusion polypeptide complex first polypeptide amino acid sequence (SEQ ID NO: 101), and the first polypeptide and Sugemalimab light chain second polypeptide constitute the multifunctional fusion polypeptide complex; Sugemalimab heavy chain is linked through the Fc domain C-terminus to a linker peptide covalently linked to LAG3 extracellular domain D1/D2, LAG3 extracellular domain D1/D2 C-terminus is through a linker peptide covalently linked to CD86 extracellular domain IgV domain to form the multifunctional fusion polypeptide complex first polypeptide amino acid sequence (SEQ ID NO: 102), and the first polypeptide and Sugemalimab light chain second polypeptide constitute the multifunctional fusion polypeptide complex; Sugemalimab heavy chain is linked through the Fc domain C-terminus to a linker peptide covalently linked to LAG3 extracellular domain D1, LAG3 extracellular domain D1 C-terminus is through a linker peptide covalently linked to CD86 extracellular domain IgV domain to form the multifunctional fusion polypeptide complex first polypeptide amino acid sequence (SEQ ID NO: 103), and the first polypeptide and Sugemalimab light chain second polypeptide constitute the multifunctional fusion polypeptide complex.

Provided below is an exemplary CD86 extracellular domain C-terminus is through a linker peptide covalently linked to anti-PD-L1 antibody Sugemalimab heavy chain, and Sugemalimab C-terminus is through a linker peptide covalently linked to LAG3 extracellular domain to form the multifunctional fusion polypeptide complex first polypeptide amino acid sequence (SEQ ID NO: 104), and the first polypeptide and Sugemalimab light chain second polypeptide constitute the multifunctional fusion polypeptide complex; CD86 extracellular domain C-terminus is through a linker peptide covalently linked to anti-PD-L1 antibody Sugemalimab heavy chain, amd Sugemalimab heavy chain C-terminus is through a linker peptide covalently linked to LAG3 extracellular domain D1/D2/D3 to form the multifunctional fusion polypeptide complex first polypeptide amino acid sequence (SEQ ID NO: 105), and the first polypeptide and Sugemalimab light chain second polypeptide constitute the multifunctional fusion polypeptide complex; CD86 extracellular domain C-terminus is through a linker peptide covalently linked to anti-PD-L1 antibody Sugemalimab heavy chain, and Sugemalimab heavy chain C-terminus is through a linker peptide covalently linked to LAG3 extracellular domain D1/D2 to form the multifunctional fusion polypeptide complex first polypeptide amino acid sequence (SEQ ID NO: 106), and the first polypeptide and Sugemalimab light chain second polypeptide constitute the multifunctional fusion polypeptide complex; CD86 extracellular domain C-terminus is through a linker peptide covalently linked to anti-PD-L1 antibody Sugemalimab heavy chain, and Sugemalimab heavy chain C-terminus is through a linker peptide covalently linked to LAG3 extracellular domain D1 to form the multifunctional fusion polypeptide complex first polypeptide amino acid sequence (SEQ ID NO: 107), and the first polypeptide and Sugemalimab light chain second polypeptide constitute the multifunctional fusion polypeptide complex; LAG3 extracellular domain C-terminus is through a linker peptide covalently linked to anti-PD-L1 antibody Sugemalimab heavy chain, and Sugemalimab C-terminus is through a linker peptide covalently linked to CD86 extracellular domain to form the multifunctional fusion polypeptide complex first polypeptide amino acid sequence (SEQ ID NO: 108), and the first polypeptide and Sugemalimab light chain second polypeptide constitute the multifunctional fusion polypeptide complex; LAG3 extracellular domain D1/D2/D3 C-terminus is through a linker peptide covalently linked to anti-PD-L1 antibody Sugemalimab heavy chain, and Sugemalimab C-terminus is through a linker peptide covalently linked to CD86 extracellular domain to form the multifunctional fusion polypeptide complex first polypeptide amino acid sequence (SEQ ID NO: 109), and the first polypeptide and Sugemalimab light chain second polypeptide constitute the multifunctional fusion polypeptide complex; LAG3 extracellular domain D1/D2 C-terminus is through a linker peptide covalently linked to anti-PD-L1 antibody Sugemalimab heavy chain, and Sugemalimab C-terminus is through a linker peptide covalently linked to CD86 extracellular domain to form the multifunctional fusion polypeptide complex first polypeptide amino acid sequence (SEQ ID NO: 110), and the first polypeptide and Sugemalimab light chain second polypeptide constitute the multifunctional fusion polypeptide complex; LAG3 extracellular domain D1 C-terminus is through a linker peptide covalently linked to anti-PD-L1 antibody Sugemalimab heavy chain, and Sugemalimab C-terminus is through a linker peptide covalently linked to CD86 extracellular domain to form the multifunctional fusion polypeptide complex first polypeptide amino acid sequence (SEQ ID NO: 111), and the first polypeptide and Sugemalimab light chain second polypeptide constitute the multifunctional fusion polypeptide complex.

Provided below is an exemplary CD86 extracellular domain IgV domain C-terminus is through a linker peptide covalently linked to anti-PD-L1 antibody Sugemalimab heavy chain, and Sugemalimab C-terminus is through a linker peptide covalently linked to LAG3 extracellular domain to form the multifunctional fusion polypeptide complex first polypeptide amino acid sequence (SEQ ID NO: 112), and the first polypeptide and Sugemalimab light chain second polypeptide constitute the multifunctional fusion polypeptide complex; CD86 extracellular domain IgV domain C-terminus is through a linker peptide covalently linked to anti-PD-L1 antibody Sugemalimab heavy chain, and Sugemalimab heavy chain C-terminus is through a linker peptide covalently linked to LAG3 extracellular domain D1/D2/D3 to form the multifunctional fusion polypeptide complex first polypeptide amino acid sequence (SEQ ID NO: 113), and the first polypeptide and Sugemalimab light chain second polypeptide constitute the multifunctional fusion polypeptide complex; CD86 extracellular domain IgV domain C-terminus is through a linker peptide covalently linked to anti-PD-L1 antibody Sugemalimab heavy chain, and Sugemalimab heavy chain C-terminus is through a linker peptide covalently linked to LAG3 extracellular domain D1/D2 to form the multifunctional fusion polypeptide complex first polypeptide amino acid sequence (SEQ ID NO: 114), and the first polypeptide and Sugemalimab light chain second polypeptide constitute the multifunctional fusion polypeptide complex; CD86 extracellular domain IgV domain C-terminus is through a linker peptide covalently linked to anti-PD-L1 antibody Sugemalimab heavy chain, and Sugemalimab heavy chain C-terminus is through a linker peptide covalently linked to LAG3 extracellular domain D1 to form the multifunctional fusion polypeptide complex first polypeptide amino acid sequence (SEQ ID NO: 115), and the first polypeptide and Sugemalimab light chain second polypeptide constitute the multifunctional fusion polypeptide complex; LAG3 extracellular domain C-terminus is through a linker peptide covalently linked to anti-PD-L1 antibody Sugemalimab heavy chain, and Sugemalimab C-terminus is through a linker peptide covalently linked to CD86 extracellular domain IgV domain to form the multifunctional fusion polypeptide complex first polypeptide amino acid sequence (SEQ ID NO: 116), and the first polypeptide and Sugemalimab light chain second polypeptide constitute the multifunctional fusion polypeptide complex; LAG3 extracellular domain D1/D2/D3 C-terminus is through a linker peptide covalently linked to anti-PD-L1 antibody Sugemalimab heavy chain, and Sugemalimab C-terminus is through a linker peptide covalently linked to CD86 extracellular domain IgV domain to form the multifunctional fusion polypeptide complex first polypeptide amino acid sequence (SEQ ID NO: 117), and the first polypeptide and Sugemalimab light chain second polypeptide constitute the multifunctional fusion polypeptide complex; LAG3 extracellular domain D1/D2 C-terminus is through a linker peptide covalently linked to anti-PD-L1 antibody Sugemalimab heavy chain, and Sugemalimab C-terminus is through a linker peptide covalently linked to CD86 extracellular domain IgV domain to form the multifunctional fusion polypeptide complex first polypeptide amino acid sequence (SEQ ID NO: 118), and the first polypeptide and Sugemalimab light chain second polypeptide constitute the multifunctional fusion polypeptide complex; LAG3 extracellular domain D1 C-terminus is through a linker peptide covalently linked to anti-PD-L1 antibody Sugemalimab heavy chain, and Sugemalimab C-terminus is through a linker peptide covalently linked to CD86 extracellular domain IgV domain to form the multifunctional fusion polypeptide complex first polypeptide amino acid sequence (SEQ ID NO: 119), and the first polypeptide and Sugemalimab light chain second polypeptide constitute the multifunctional fusion polypeptide complex.

Provided below is: an exemplary CD86 extracellular domain C-terminus is through a linker peptide covalently linked to LAG3 extracellular domain, and LAG3 extracellular domain C-terminus is through a linker peptide covalently linked to anti-PD-L1 antibody Sugemalimab heavy chain to form multifunctional fusion polypeptide complex first polypeptide amino acid sequence (SEQ ID NO: 120), and the first polypeptide and Sugemalimab light chain second polypeptide constitute the multifunctional fusion polypeptide complex; CD86 extracellular domain C-terminus is through a linker peptide covalently linked to LAG3 extracellular domain D1/D2/D3, and LAG3 extracellular domain D1/D2/D3 C-terminus is through a linker peptide covalently linked to anti-PD-L1 antibody Sugemalimab heavy chain to form multifunctional fusion polypeptide complex first polypeptide amino acid sequence (SEQ ID NO: 121), and the first polypeptide and Sugemalimab light chain second polypeptide constitute the multifunctional fusion polypeptide complex; CD86 extracellular domain C-terminus is through a linker peptide covalently linked to LAG3 extracellular domain D1/D2, and LAG3 extracellular domain D1/D2 C-terminus is through a linker peptide covalently linked to anti-PD-L1 antibody Sugemalimab heavy chain to form the multifunctional fusion polypeptide complex first polypeptide amino acid sequence (SEQ ID NO: 122), and the first polypeptide and Sugemalimab light chain second polypeptide constitute the multifunctional fusion polypeptide complex; CD86 extracellular domain C-terminus is through a linker peptide covalently linked to LAG3 extracellular domain D1, and LAG3 extracellular domain D1 C-terminus is through a linker peptide covalently linked to anti-PD-L1 antibody Sugemalimab heavy chain to form multifunctional fusion polypeptide complex first polypeptide amino acid sequence (SEQ ID NO: 123), and the first polypeptide and Sugemalimab light chain second polypeptide constitute the multifunctional fusion polypeptide complex; LAG3 extracellular domain C-terminus is through a linker peptide covalently linked to CD86 extracellular domain, and CD86 extracellular domain C-terminus is through a linker peptide covalently linked to anti-PD-L1 antibody Sugemalimab heavy chain to form multifunctional fusion polypeptide complex first polypeptide amino acid sequence (SEQ ID NO: 124), and the first polypeptide and Sugemalimab light chain second polypeptide constitute the multifunctional fusion polypeptide complex; LAG3 extracellular domain D1/D2/D3 C-terminus is through a linker peptide covalently linked to CD86 extracellular domain, and CD86 extracellular domain C-terminus is through a linker peptide covalently linked to anti-PD-L1 antibody Sugemalimab heavy chain to form multifunctional fusion polypeptide complex first polypeptide amino acid sequence (SEQ ID NO: 125), and the first polypeptide and Sugemalimab light chain second polypeptide constitute the multifunctional fusion polypeptide complex; LAG3 extracellular domain D1/D2 C-terminus is through a linker peptide covalently linked to CD86 extracellular domain, and CD86 extracellular domain C-terminus is through a linker peptide covalently linked to anti-PD-L1 antibody Sugemalimab heavy chain to form multifunctional fusion polypeptide complex first polypeptide amino acid sequence (SEQ ID NO: 126), and the first polypeptide and Sugemalimab light chain second polypeptide constitute the multifunctional fusion polypeptide complex; LAG3 extracellular domain D1 C-terminus is through a linker peptide covalently linked to CD86 extracellular domain, and CD86 extracellular domain C-terminus is through a linker peptide covalently linked to anti-PD-L1 antibody Sugemalimab heavy chain to form multifunctional fusion polypeptide complex first polypeptide amino acid sequence (SEQ ID NO: 127), and the first polypeptide and Sugemalimab light chain second polypeptide constitute the multifunctional fusion polypeptide complex;

Provided below is an exemplary CD86 extracellular domain IgV domain C-terminus is through a linker peptide covalently linked to LAG3 extracellular domain, and LAG3 extracellular domain C-terminus is through a linker peptide covalently linked to anti-PD-L1 antibody Sugemalimab heavy chain to form multifunctional fusion polypeptide complex first polypeptide amino acid sequence (SEQ ID NO: 128), and the first polypeptide and Sugemalimab light chain second polypeptide constitute the multifunctional fusion polypeptide complex; CD86 extracellular domain IgV domain C-terminus is through a linker peptide covalently linked to LAG3 extracellular domain D1/D2/D3, LAG3 extracellular domain D1/D2/D3 C-terminus is through a linker peptide covalently linked to anti-PD-L1 antibody Sugemalimab heavy chain to form multifunctional fusion polypeptide complex first polypeptide amino acid sequence (SEQ ID NO: 129), and the first polypeptide and Sugemalimab light chain second polypeptide constitute the multifunctional fusion polypeptide complex; CD86 extracellular domain IgV domain C-terminus is through a linker peptide covalently linked to LAG3 extracellular domain D1/D2, LAG3 extracellular domain D1/D2 C-terminus is through a linker peptide covalently linked to anti-PD-L1 antibody Sugemalimab heavy chain to form multifunctional fusion polypeptide complex first polypeptide amino acid sequence (SEQ ID NO: 130), and the first polypeptide and Sugemalimab light chain second polypeptide constitute the multifunctional fusion polypeptide complex; CD86 extracellular domain IgV domain C-terminus is through a linker peptide covalently linked to LAG3 extracellular domain D1, and LAG3 extracellular domain D1 C-terminus is through a linker peptide covalently linked to anti-PD-L1 antibody Sugemalimab heavy chain to form multifunctional fusion polypeptide complex first polypeptide amino acid sequence (SEQ ID NO: 131), and the first polypeptide and Sugemalimab light chain second polypeptide constitute the multifunctional fusion polypeptide complex; LAG3 extracellular domain C-terminus is through a linker peptide covalently linked to CD86 extracellular domain IgV domain, and CD86 extracellular domain IgV domain C-terminus is through a linker peptide covalently linked to anti-PD-L1 antibody Sugemalimab heavy chain to form multifunctional fusion polypeptide complex first polypeptide amino acid sequence (SEQ ID NO: 132), and the first polypeptide and Sugemalimab light chain second polypeptide constitute the multifunctional fusion polypeptide complex; LAG3 extracellular domain D1/D2/D3 C-terminus is through a linker peptide covalently linked to CD86 extracellular domain IgV domain, and CD86 extracellular domain IgV domain C-terminus is through a linker peptide covalently linked to anti-PD-L1 antibody Sugemalimab heavy chain to form multifunctional fusion polypeptide complex first polypeptide amino acid sequence (SEQ ID NO: 133), and the first polypeptide and Sugemalimab light chain second polypeptide constitute the multifunctional fusion polypeptide complex; LAG3 extracellular domain D1/D2 C-terminus is through a linker peptide covalently linked to CD86 extracellular domain IgV domain, and CD86 extracellular domain IgV domain C-terminus is through a linker peptide covalently linked to anti-PD-L1 antibody Sugemalimab heavy chain to form multifunctional fusion polypeptide complex first polypeptide amino acid sequence (SEQ ID NO: 134), and the first polypeptide and Sugemalimab light chain second polypeptide constitute the multifunctional fusion polypeptide complex; LAG3 extracellular domain D1 C-terminus is through a linker peptide covalently linked to CD86 extracellular domain IgV domain, and CD86 extracellular domain IgV domain C-terminus is through a linker peptide covalently linked to anti-PD-L1 antibody Sugemalimab heavy chain to form multifunctional fusion polypeptide complex first polypeptide amino acid sequence (SEQ ID NO: 135), and the first polypeptide and Sugemalimab light chain second polypeptide constitute the multifunctional fusion polypeptide complex.

Provided below is an exemplary CD86 extracellular domain C-terminus is through a linker peptide covalently linked to anti-PD-L1 antibody Sugemalimab heavy chain to form multifunctional fusion polypeptide complex first polypeptide amino acid sequence (SEQ ID NO: 38), and the first polypeptide and the second polypeptide (SEQ ID NO: 136) (formed by LAG3 extracellular domain C-terminus through a linker peptide covalently linked to Sugemalimab light chain) constitute the multifunctional fusion polypeptide complex; CD86 extracellular domain IgV domain C-terminus is through a linker peptide covalently linked to anti-PD-L1 antibody Sugemalimab heavy chain to form multifunctional fusion polypeptide complex first polypeptide amino acid sequence (SEQ ID NO: 39), and the first polypeptide and the second polypeptide (formed by LAG3 extracellular domain C-terminus through a linker peptide covalently linked to Sugemalimab light chain) constitute the multifunctional fusion polypeptide complex; PD-L1 antibody Sugemalimab heavy chain C-terminus is through a linker peptide covalently linked to CD86 extracellular domain to form the multifunctional fusion polypeptide complex first polypeptide amino acid sequence (SEQ ID NO: 40), and the first polypeptide and the second polypeptide (formed by LAG3 extracellular domain C-terminus through a linker peptide covalently linked to Sugemalimab light chain) constitute the multifunctional fusion polypeptide complex; PD-L1 antibody Sugemalimab heavy chain C-terminus is through a linker peptide covalently linked to CD86 extracellular domain IgV domain to form the multifunctional fusion polypeptide complex first polypeptide amino acid sequence (SEQ ID NO: 41), and the first polypeptide and second polypeptide (formed by LAG3 extracellular domain C-terminus through a linker peptide covalently linked to Sugemalimab light chain) constitute the multifunctional fusion polypeptide complex.

Provided below is an exemplary CD86 extracellular domain C-terminus is through a linker peptide covalently linked to anti-PD-L1 antibody Sugemalimab heavy chainto form the multifunctional fusion polypeptide complex first polypeptide amino acid sequence, and the first polypeptide and the second polypeptide (SEQ ID NO: 137) (formed by LAG3 extracellular domain D1/D2/D3 C-terminus through a linker peptide covalently linked to Sugemalimab light chain) constitute the multifunctional fusion polypeptide complex; CD86 extracellular domain IgV domain C-terminus is through a linker peptide covalently linked to anti-PD-L1 antibody Sugemalimab heavy chain to form the multifunctional fusion polypeptide complex first polypeptide amino acid sequence, and the first polypeptide and the second polypeptide(formed by LAG3 extracellular domain D1/D2/D3 C-terminus through a linker peptide covalently linked to Sugemalimab light chain) constitute the multifunctional fusion polypeptide complex; PD-L1 antibody Sugemalimab heavy chain C-terminus is through a linker peptide covalently linked to CD86 extracellular domain to form the multifunctional fusion polypeptide complex first polypeptide amino acid sequence, and the first polypeptide and the second polypeptide (formed by LAG3 extracellular domain D1/D2/D3 C-terminus through a linker peptide covalently linked to Sugemalimab light chain) constitute the multifunctional fusion polypeptide complex; PD-L1 antibody Sugemalimab heavy chain C-terminus is through a linker peptide covalently linked to CD86 extracellular domain IgV domain to form the multifunctional fusion polypeptide complex first polypeptide amino acid sequence, and the first polypeptide and the second polypeptide (formed by LAG3 extracellular domain D1/D2/D3 C-terminus through a linker peptide covalently linked to Sugemalimab light chain) constitute the multifunctional fusion polypeptide complex.

Provided below is an exemplary CD86 extracellular domain C-terminus is through a linker peptide covalently linked to anti-PD-L1 antibody Sugemalimab heavy chain to form the multifunctional fusion polypeptide complex first polypeptide amino acid sequence, and the first polypeptide and the second polypeptide (SEQ ID NO: 138) (formed by LAG3 extracellular domain D1/D2 C-terminus through a linker peptide covalently linked to Sugemalimab light chain) constitute the multifunctional fusion polypeptide complex; CD86 extracellular domain IgV domain C-terminus is through a linker peptide covalently linked to anti-PD-L1 antibody Sugemalimab heavy chain to form the multifunctional fusion polypeptide complex first polypeptide amino acid sequence, and the first polypeptide and the second polypeptide (formed by LAG3 extracellular domain D1/D2 C-terminus through a linker peptide covalently linked to Sugemalimab light chain) constitute the multifunctional fusion polypeptide complex; PD-L1 antibody Sugemalimab heavy chain C-terminus is through a linker peptide covalently linked to CD86 extracellular domain to form the multifunctional fusion polypeptide complex first polypeptide amino acid sequence, and the first polypeptide and the second polypeptide (formed by LAG3 extracellular domain D1/D2 C-terminus through a linker peptide covalently linked to Sugemalimab light chain) constitute the multifunctional fusion polypeptide complex; PD-L1 antibody Sugemalimab heavy chain C-terminus is through a linker peptide covalently linked to CD86 extracellular domain IgV domain to form the multifunctional fusion polypeptide complex first polypeptide amino acid sequence, and the first polypeptide and the second polypeptide (formed by LAG3 extracellular domain D1/D2 C-terminus through a linker peptide covalently linked to Sugemalimab light chain) constitute the multifunctional fusion polypeptide complex_{∘}

Provided below is an exemplary CD86 extracellular domain C-terminus is through a linker peptide covalently linked to anti-PD-L1 antibody Sugemalimab heavy chain to form the multifunctional fusion polypeptide complex first polypeptide amino acid sequence, and the first polypeptide and the second polypeptide (SEQ ID NO: 139) (formed by LAG3 extracellular domain D1 C-terminus through a linker peptide covalently linked to Sugemalimab light chain) constitute the multifunctional fusion polypeptide complex; CD86 extracellular domain IgV domain C-terminus is through a linker peptide covalently linked to anti-PD-L1 antibody Sugemalimab heavy chain to form the multifunctional fusion polypeptide complex first polypeptide amino acid sequence, and the first polypeptide and the second polypeptide (formed by LAG3 extracellular domain D1 C-terminus through a linker peptide covalently linked to Sugemalimab light chain) constitute the multifunctional fusion polypeptide complex; PD-L1 antibody Sugemalimab heavy chain C-terminus is through a linker peptide covalently linked to CD86 extracellular domain to form the multifunctional fusion polypeptide complex first polypeptide amino acid sequence, and the first polypeptide and the second polypeptide (formed by LAG3 extracellular domain D1 C-terminus through a linker peptide covalently linked to Sugemalimab light chain) constitute the multifunctional fusion polypeptide complex; PD-L1 antibody Sugemalimab heavy chain C-terminus is through a linker peptide covalently linked to CD86 extracellular domain IgV domain to form the multifunctional fusion polypeptide complex first polypeptide amino acid sequence, and the first polypeptide and the second polypeptide (formed by LAG3 extracellular domain D1 C-terminus through a linker peptide covalently linked to Sugemalimab light chain) constitute the multifunctional fusion polypeptide complex.

Provided below is an exemplary CD86 extracellular domain C-terminus is through a linker peptide covalently linked to anti-PD-L1 antibody Sugemalimab heavy chain to form the multifunctional fusion polypeptide complex first polypeptide amino acid sequence, and the first polypeptide and the second polypeptide (SEQ ID NO: 140) (formed by Sugemalimablight chain C-terminus through a linker peptide covalently linked to LAG3 extracellular domain) constitute the multifunctional fusion polypeptide complex; CD86 extracellular domain IgV domain C-terminus is through a linker peptide covalently linked to anti-PD-L1 antibody Sugemalimab heavy chain to form the multifunctional fusion polypeptide complex first polypeptide amino acid sequence, and the first polypeptide and the second polypeptide (formed by Sugemalimablight chain C-terminus through a linker peptide covalently linked to LAG3 extracellular domain) constitute the multifunctional fusion polypeptide complex; PD-L1 antibody Sugemalimab heavy chain C-terminus is through a linker peptide covalently linked to CD86 extracellular domain to form the multifunctional fusion polypeptide complex first polypeptide amino acid sequence, and the first polypeptide and the second polypeptide (formed by Sugemalimablight chain C-terminus through a linker peptide covalently linked to LAG3 extracellular domain) constitute the multifunctional fusion polypeptide complex; PD-L1 antibody Sugemalimab heavy chain C-terminus is through a linker peptide covalently linked to CD86 extracellular domain IgV domain to form the multifunctional fusion polypeptide complex first polypeptide amino acid sequence, and the first polypeptide and the second polypeptide (formed by Sugemalimablight chain C-terminus through a linker peptide covalently linked to LAG3 extracellular domain) constitute the multifunctional fusion polypeptide complex.

Provided below is an exemplary CD86 extracellular domain C-terminus is through a linker peptide covalently linked to anti-PD-L1 antibody Sugemalimab heavy chain to form the multifunctional fusion polypeptide complex first polypeptide amino acid sequence, and the first polypeptide and the second polypeptide (SEQ ID NO: 141) (formed by Sugemalimablight chain C-terminus through a linker peptide covalently linked to LAG3 extracellular domain D1/D2/D3) constitute the multifunctional fusion polypeptide complex; CD86 extracellular domain IgV domain C-terminus is through a linker peptide covalently linked to anti-PD-L1 antibody Sugemalimab heavy chain to form the multifunctional fusion polypeptide complex first polypeptide amino acid sequence, and the first polypeptide and the second polypeptide (formed by Sugemalimablight chain C-terminus through a linker peptide covalently linked to LAG3 extracellular domain D1/D2/D3) constitute the multifunctional fusion polypeptide complex; PD-L1 antibody Sugemalimab heavy chain C-terminus is through a linker peptide covalently linked to CD86 extracellular domain to form the multifunctional fusion polypeptide complex first polypeptide amino acid sequence, and the first polypeptide and the second polypeptide (formed by Sugemalimab light chain C-terminus through a linker peptide covalently linked to LAG3 extracellular domain D1/D2/D3) constitute the multifunctional fusion polypeptide complex; PD-L1 antibody Sugemalimab heavy chain C-terminus is through a linker peptide covalently linked to CD86 extracellular domain IgV domain to form the multifunctional fusion polypeptide complex first polypeptide amino acid sequence, and the first polypeptide and the second polypeptide (formed by Sugemalimablight chain C-terminus through a linker peptide covalently linked to LAG3 extracellular domain D1/D2/D3) constitute the multifunctional fusion polypeptide complex.

Provided below is an exemplary CD86 extracellular domain C-terminus is through a linker peptide covalently linked to anti-PD-L1 antibody Sugemalimab heavy chain to form multifunctional fusion polypeptide complex first polypeptide amino acid sequence, and the first polypeptide and the second polypeptide (SEQ ID NO: 142) (formed by Sugemalimab light chain C-terminus through a linker peptide covalently linked to LAG3 extracellular domain D1/D2) constitute the multifunctional fusion polypeptide complex; CD86 extracellular domain IgV domain C-terminus is through a linker peptide covalently linked to anti-PD-L1 antibody Sugemalimab heavy chain to form the multifunctional fusion polypeptide complex first polypeptide amino acid sequence, and the first polypeptide and the second polypeptide (formed by Sugemalimablight chain C-terminus through a linker peptide covalently linked to LAG3 extracellular domain D1/D2) constitute the multifunctional fusion polypeptide complex; PD-L1 antibody Sugemalimab heavy chain C-terminus is through a linker peptide covalently linked to CD86 extracellular domain to form multifunctional fusion polypeptide complex first polypeptide amino acid sequence, and the first polypeptide and the second polypeptide (formed by Sugemalimablight chain C-terminus through a linker peptide covalently linked to LAG3 extracellular domain D1/D2) constitute the multifunctional fusion polypeptide complex; PD-L1 antibody Sugemalimab heavy chain C-terminus through a linker peptide covalently linked to CD86 extracellular domain IgV domain to form the multifunctional fusion polypeptide complex first polypeptide amino acid sequence, and the first polypeptide and the second polypeptide (formed by Sugemalimablight chain C-terminus is through a linker peptide covalently linked to LAG3 extracellular domain D1/D2) constitute the multifunctional fusion polypeptide complex.

Provided below is an exemplary CD86 extracellular domain C-terminus through a linker peptide covalently linked to anti-PD-L1 antibody Sugemalimab heavy chain to form the multifunctional fusion polypeptide complex first polypeptide amino acid sequence, and the first polypeptide and the second polypeptide (SEQ ID NO: 143) (formed by Sugemalimab light chain C-terminus through a linker peptide covalently linked to LAG3 extracellular domain D1) constitute the multifunctional fusion polypeptide complex; CD86 extracellular domain IgV domain C-terminus is through a linker peptide covalently linked to anti-PD-L1 antibody Sugemalimab heavy chain to form the multifunctional fusion polypeptide complex first polypeptide amino acid sequence, and the first polypeptide and the second polypeptide (formed by Sugemalimabl ight chain C-terminus through a linker peptide covalently linked to LAG3 extracellular domain D1) constitute the multifunctional fusion polypeptide complex; PD-L1 antibody Sugemalimab heavy chain C-terminus is through a linker peptide covalently linked to CD86 extracellular domain to form the multifunctional fusion polypeptide complex first polypeptide amino acid sequence, and the first polypeptide and the second polypeptide (formed by Sugemalimablight chain C-terminus through a linker peptide covalently linked to LAG3 extracellular domain D1) constitute the multifunctional fusion polypeptide complex; PD-L1 antibody Sugemalimab heavy chain C-terminus is through a linker peptide covalently linked to CD86 extracellular domain IgV domain to form the multifunctional fusion polypeptide complex first polypeptide amino acid sequence, and the first polypeptide and the second polypeptide (formed by Sugemalimablight chain C-terminus through a linker peptide covalently linked to LAG3 extracellular domain D1) constitute the multifunctional fusion polypeptide complex.

Provided below is an exemplary CD86 extracellular domain C-terminus is through a linker peptide covalently linked to anti-PD-L1 antibody Sugemalimab light chain to form the multifunctional fusion polypeptide complex second polypeptide amino acid sequence (SEQ ID NO: 42), and the second polypeptide and the first polypeptide (SEQ ID NO: 144) (formed by LAG3 extracellular domain C-terminus is through a linker peptide covalently linked to Sugemalimab heavy chain) constitute the multifunctional fusion polypeptide complex; CD86 extracellular domain IgV domain C-terminus is through a linker peptide covalently linked to anti-PD-L1 antibody Sugemalimab light chain to form the multifunctional fusion polypeptide complex second polypeptide amino acid sequence (SEQ ID NO: 43), and the second polypeptide and the first polypeptide (formed by LAG3 extracellular domain C-terminus is through a linker peptide covalently linked to Sugemalimab heavy chain) constitute the multifunctional fusion polypeptide complex; anti-PD-L1 antibody Sugemalimab light chain C-terminus is through a linker peptide covalently linked to CD86 extracellular domain to form the multifunctional fusion polypeptide complex second polypeptide amino acid sequence (SEQ ID NO: 40), and the second polypeptide and the first polypeptide (formed by LAG3 extracellular domain C-terminus through a linker peptide covalently linked to Sugemalimab heavy chain) constitute the multifunctional fusion polypeptide complex; anti-PD-L1 antibody Sugemalimab light chain C-terminus is through a linker peptide covalently linked to CD86 extracellular domain IgV domain to form the multifunctional fusion polypeptide complex second polypeptide amino acid sequence (SEQ ID NO: 41), and the second polypeptide and the first polypeptide (formed by LAG3 extracellular domain C-terminus is through a linker peptide covalently linked to Sugemalimab heavy chain) constitute the multifunctional fusion polypeptide complex.

Provided below is an exemplary CD86 extracellular domain C-terminus is through a linker peptide covalently linked to anti-PD-L1 antibody Sugemalimab light chain to form the multifunctional fusion polypeptide complex second polypeptide amino acid sequence, and the second polypeptide and the first polypeptide (SEQ ID NO: 145 ) (formed by LAG3 extracellular domain D1/D2/D3 C-terminus through a linker peptide covalently linked to Sugemalimab heavy chain) constitute the multifunctional fusion polypeptide complex; CD86 extracellular domain IgV domain C-terminus is through a linker peptide covalently linked to anti-PD-L1 antibody Sugemalimab light chain to form the multifunctional fusion polypeptide complex second polypeptide amino acid sequence, and the second polypeptide and the first polypeptide (formed by LAG3 extracellular domain D1/D2/D3 C-terminus is through a linker peptide covalently linked to Sugemalimab heavy chain) constitute the multifunctional fusion polypeptide complex; anti-PD-L1 antibody Sugemalimab light chain C-terminus is through a linker peptide covalently linked to CD86 extracellular domain to form the multifunctional fusion polypeptide complex second polypeptide amino acid sequence, the second polypeptide (formed by LAG3 extracellular domain D1/D2/D3 C-terminus is through a linker peptide covalently linked to Sugemalimab heavy chain) first polypeptide constitute the multifunctional fusion polypeptide complex; anti-PD-L1 antibody Sugemalimablight chain C-terminus is through a linker peptide covalently linked to CD86 extracellular domain IgV domain to form the multifunctional fusion polypeptide complex second polypeptide amino acid sequence, the second polypeptide (formed by LAG3 extracellular domain D1/D2/D3 C-terminus is through a linker peptide covalently linked to Sugemalimab heavy chain first polypeptide constitute the multifunctional fusion polypeptide complex.

Provided below is an exemplary CD86 extracellular domain C-terminus is through a linker peptide covalently linked to anti-PD-L1 antibody Sugemalimab light chain to form the multifunctional fusion polypeptide complex second polypeptide amino acid sequence, and the second polypeptide and the first polypeptide (SEQ ID NO: 146) (formed by LAG3 extracellular domain D1/D2 C-terminus is through a linker peptide covalently linked to Sugemalimab heavy chain) constitute the multifunctional fusion polypeptide complex; CD86 extracellular domain IgV domain C-terminus is through a linker peptide covalently linked to anti-PD-L1 antibody Sugemalimab light chain to form the multifunctional fusion polypeptide complex second polypeptide amino acid sequence, and the second polypeptide and the first polypeptide (formed by LAG3 extracellular domain D1/D2 C-terminus through a linker peptide covalently linked to Sugemalimab heavy chain) constitute the multifunctional fusion polypeptide complex; anti-PD-L1 antibody Sugemalimab light chain C-terminus is through a linker peptide covalently linked to CD86 extracellular domain to form the multifunctional fusion polypeptide complex second polypeptide amino acid sequence, the second polypeptide (formed by LAG3 extracellular domain D1/D2 C-terminus is through a linker peptide covalently linked to Sugemalimab heavy chain first polypeptide constitute the multifunctional fusion polypeptide complex; anti-PD-L1 antibody Sugemalimab light chain C-terminus is through a linker peptide covalently linked to CD86 extracellular domain IgV domain to form the multifunctional fusion polypeptide complex second polypeptide amino acid sequence, the second polypeptide (formed by LAG3 extracellular domain D1/D2 C-terminus is through a linker peptide covalently linked to Sugemalimab heavy chain first polypeptide constitute the multifunctional fusion polypeptide complex.

Provided below is an exemplary CD86 extracellular domain C-terminus is through a linker peptide covalently linked to anti-PD-L1 antibody Sugemalimab light chain to form the multifunctional fusion polypeptide complex second polypeptide amino acid sequence, and the second polypeptide and the first polypeptide (SEQ ID NO: 147) (formed by LAG3 extracellular domain D1 C-terminus is through a linker peptide covalently linked to Sugemalimab heavy chain) constitute the multifunctional fusion polypeptide complex; CD86 extracellular domain IgV domain C-terminus is through a linker peptide covalently linked to anti-PD-L1 antibody Sugemalimab light chain to form the multifunctional fusion polypeptide complex second polypeptide amino acid sequence, and the second polypeptide and the first polypeptide (formed by LAG3 extracellular domain D1 C-terminus is through a linker peptide covalently linked to Sugemalimab heavy chain) constitute the multifunctional fusion polypeptide complex; anti-PD-L1 antibody Sugemalimab light chain C-terminus is through a linker peptide covalently linked to CD86 extracellular domain to form the multifunctional fusion polypeptide complex second polypeptide amino acid sequence, and the second polypeptide and the first polypeptide (formed by LAG3 extracellular domain D1 C-terminus through a linker peptide covalently linked to Sugemalimab heavy chain) constitute the multifunctional fusion polypeptide complex; anti-PD-L1 antibody Sugemalimab light chain C-terminus is through a linker peptide covalently linked to CD86 extracellular domain IgV domain to form the multifunctional fusion polypeptide complex second polypeptide amino acid sequence, and the second polypeptide and the first polypeptide (formed by LAG3 extracellular domain D1 C-terminus is through a linker peptide covalently linked to Sugemalimab heavy chain) constitute the multifunctional fusion polypeptide complex.

Provided below is an exemplary CD86 extracellular domain C-terminus is through a linker peptide covalently linked to anti-PD-L1 antibody Sugemalimab light chain to form the multifunctional fusion polypeptide complex second polypeptide amino acid sequence, the second polypeptide and the first polypeptide (SEQ ID NO: 148) (formed by Sugemalimab heavy chain C-terminus is through a linker peptide covalently linked to LAG3 extracellular domain) constitute the multifunctional fusion polypeptide complex; CD86 extracellular domain IgV domain C-terminus is through a linker peptide covalently linked to anti-PD-L1 antibody Sugemalimab light chain to form the multifunctional fusion polypeptide complex second polypeptide amino acid sequence, the second polypeptide and the first polypeptide (formed by Sugemalimab heavy chain C-terminus is through a linker peptide covalently linked to LAG3 extracellular domain) constitute the multifunctional fusion polypeptide complex; anti-PD-L1 antibody Sugemalimab light chain C-terminus is through a linker peptide covalently linked to CD86 extracellular domain to form the multifunctional fusion polypeptide complex second polypeptide amino acid sequence, the second polypeptide and the first polypeptide (formed by Sugemalimab heavy chainC-terminus is through a linker peptide covalently linked to LAG3 extracellular domain) constitute the multifunctional fusion polypeptide complex; anti-PD-L1 antibody Sugemalimab light chain C-terminus is through a linker peptide covalently linked to CD86 extracellular domain IgV domain to form the multifunctional fusion polypeptide complex second polypeptide amino acid sequence, and the second polypeptide and the first polypeptide (formed by Sugemalimab heavy chain C-terminus is through a linker peptide covalently linked to LAG3 extracellular domain) constitute the multifunctional fusion polypeptide complex_{∘}

Provided below is: an exemplary CD86 extracellular domain C-terminus is through a linker peptide covalently linked to anti-PD-L1 antibody Sugemalimab light chain to form multifunctional fusion polypeptide complex second polypeptide amino acid sequence, and the second polypeptide and the first polypeptide (SEQ ID NO: 149) (formed by Sugemalimab heavy chain C-terminus through a linker peptide covalently linked to LAG3 extracellular domain D1/D2/D3) constitute the multifunctional fusion polypeptide complex; CD86 extracellular domain IgV domain C-terminus is through a linker peptide covalently linked to anti-PD-L1 antibody Sugemalimab light chain to form multifunctional fusion polypeptide complex second polypeptide amino acid sequence, and the second polypeptide and the first polypeptide (formed by Sugemalimab heavy chain C-terminus through a linker peptide covalently linked to LAG3 extracellular domain D1/D2/D3) constitute the multifunctional fusion polypeptide complex; anti-PD-L1 antibody Sugemalimab light chain C-terminus is through a linker peptide covalently linked to CD86 extracellular domain to form multifunctional fusion polypeptide complex second polypeptide amino acid sequence, and the second polypeptide and the first polypeptide (formed by Sugemalimab heavy chain C-terminus through a linker peptide covalently linked to LAG3 extracellular domain D1/D2/D3) constitute the multifunctional fusion polypeptide complex; anti-PD-L1 antibody Sugemalimab light chain C-terminus is through a linker peptide covalently linked to CD86 extracellular domain IgV domain to form the multifunctional fusion polypeptide complex second polypeptide amino acid sequence, and the second polypeptide and the first polypeptide (formed by Sugemalimab heavy chain C-terminus through a linker peptide covalently linked to LAG3 extracellular domain D1/D2/D3) constitute the multifunctional fusion polypeptide complex.

Provided below is an exemplary CD86 extracellular domain C-terminus is through a linker peptide covalently linked to anti-PD-L1 antibody Sugemalimab light chain to form the multifunctional fusion polypeptide complex second polypeptide amino acid sequence, and the second polypeptide and the first polypeptide (SEQ ID NO: 150) (formed by Sugemalimab heavy chain C-terminus through a linker peptide covalently linked to LAG3 extracellular domain D1/D2) constitute the multifunctional fusion polypeptide complex; CD86 extracellular domain IgV domain C-terminus is through a linker peptide covalently linked to anti-PD-L1 antibody Sugemalimab light chain to form the multifunctional fusion polypeptide complex second polypeptide amino acid sequence, and the second polypeptide and the first polypeptide (formed by Sugemalimab heavy chain C-terminus through a linker peptide covalently linked to LAG3 extracellular domain D1/D2) constitute the multifunctional fusion polypeptide complex; anti-PD-L1 antibody Sugemalimab light chain C-terminus is through a linker peptide covalently linked to CD86 extracellular domain to form the multifunctional fusion polypeptide complex second polypeptide amino acid sequence, and the second polypeptide and the first polypeptide (formed by Sugemalimab heavy chain C-terminus through a linker peptide covalently linked to LAG3 extracellular domain D1/D2) constitute the multifunctional fusion polypeptide complex; anti-PD-L1 antibody Sugemalimab light chain C-terminus is through a linker peptide covalently linked to CD86 extracellular domain IgV domain to form the multifunctional fusion polypeptide complex second polypeptide amino acid sequence, and the second polypeptide and the first polypeptide(formed by Sugemalimab heavy chain C-terminus through a linker peptide covalently linked to LAG3 extracellular domain D1/D2) constitute the multifunctional fusion polypeptide complex.

Provided below is: an exemplary CD86 extracellular domain C-terminus is through a linker peptide covalently linked to anti-PD-L1 antibody Sugemalimab light chain to form multifunctional fusion polypeptide complex second polypeptide amino acid sequence, and the second polypeptide and the first polypeptide (SEQ ID NO: 151) (formed by Sugemalimab heavy chain C-terminus through a linker peptide covalently linked to LAG3 extracellular domain D1) constitute the multifunctional fusion polypeptide complex; CD86 extracellular domain IgV domain C-terminus is through a linker peptide covalently linked to anti-PD-L1 antibody Sugemalimab light chain to form multifunctional fusion polypeptide complex second polypeptide amino acid sequence, and the second polypeptide and the first polypeptide (formed by Sugemalimab heavy chain C-terminus through a linker peptide covalently linked to LAG3 extracellular domain D1) constitute the multifunctional fusion polypeptide complex; anti-PD-L1 antibody Sugemalimab light chain C-terminus is through a linker peptide covalently linked to CD86 extracellular domain to form multifunctional fusion polypeptide complex second polypeptide amino acid sequence, and the second polypeptide and the first polypeptide (formed by Sugemalimab heavy chain C-terminus through a linker peptide covalently linked to LAG3 extracellular domain D1) constitute the multifunctional fusion polypeptide complex; anti-PD-L1 antibody Sugemalimab light chain C-terminus is through a linker peptide covalently linked to CD86 extracellular domain IgV domain to form the multifunctional fusion polypeptide complex second polypeptide amino acid sequence, and the second polypeptide and the first polypeptide (formed by Sugemalimab heavy chain C-terminus through a linker peptide covalently linked to LAG3 extracellular domain D1) constitute the multifunctional fusion polypeptide complex.

For example, the antibody AN_Bif_Abs_0016 can be composed of a Sugemalimab heavy chain antibody AN_Bif_Abs_0016 first polypeptide (SEQ ID NO: 4) and Sugemalimab light chain second polypeptide (SEQ ID NO: 5). AN_Bif_Abs_0016 antibody is assembled by introducing a polynucleotide sequence (SEQ ID NO: 152) vector expressing AN_Bif_Abs_0016 first polypeptide and a polynucleotide sequence (SEQ ID NO: 153) vector expressing AN_Bif_Abs_0016 second polypeptide into a host cell for expression.

For example, the bifunctional antibody AN_Bif_Abs_0220 can be composed of a chimeric antibody AN_Bif_Abs_0220 first polypeptide (SEQ ID NO: 38) (formed by CD86 extracellular domain C-terminus connecting to a linker peptide (SEQ ID NO: 34) covalently linked to Sugemalimab heavy chain N-terminus) and Sugemalimab light chain second polypeptide (SEQ ID NO: 5). AN_Bif_Abs_0220 bifunctional antibody is assembled by introducing a polynucleotide sequence (SEQ ID NO: 154) vector expressing AN_Bif_Abs_0220 first polypeptide and a polynucleotide sequence (SEQ ID NO: 153) vector expressing AN_Bif_Abs_0220 second polypeptide into a host cell for expression.

For example, the bifunctional antibody AN_Bif_Abs_0222 can be composed of a chimeric antibody AN_Bif_Abs_0222 first polypeptide (SEQ ID NO: 36) (formed by a Sugemalimab heavy chain Fc domain via a linker peptide (SEQ ID NO: 34) covalently linked to CD86 extracellular domain N-terminus) and a Sugemalimab light chain second polypeptide (SEQ ID NO: 5). AN_Bif_Abs_0222 bifunctional antibody is assembled by introducing a polynucleotide sequence (SEQ ID NO: 155) vector expressing AN_Bif_Abs_0222 first polypeptide and a polynucleotide sequence (SEQ ID NO: 153) vector expressing AN_Bif_Abs_0222 second polypeptide into a host cell for expression.

For example, the bifunctional antibody AN_Bif_Abs_0703 can be composed of a chimeric antibody AN_Bif_Abs_0703 first polypeptide (SEQ ID NO: 37) (formed by a Sugemalimab heavy chain Fc domain via a linker peptide (SEQ ID NO: 34) covalently linked to CD86 extracellular domain IgV domain) and a Sugemalimab light chain second polypeptide (SEQ ID NO: 5). AN_Bif_Abs_0703 bifunctional antibody is assembled by introducing a polynucleotide sequence (SEQ ID NO: 156) vector expressing AN_Bif_Abs_0703 first polypeptide and a polynucleotide sequence (SEQ ID NO: 153) vector expressing AN_Bif_Abs_0703 second polypeptide into a host cell for expression.

For example, the bifunctional antibody AN_Bif_Abs_0158 can be composed of a chimeric antibody AN_Bif_Abs_0158 first polypeptide (SEQ ID NO: 39) (formed by CD86 extracellular domain IgV domain C-terminus connecting to a linker peptide (SEQ ID NO: 34) covalently linked to Sugemalimab heavy chain N-terminus) and Sugemalimab light chain second polypeptide (SEQ ID NO: 5). AN_Bif_Abs_0158 bifunctional antibody is assembled by introducing a polynucleotide sequence (SEQ ID NO: 157) vector expressing AN_Bif_Abs_0158 first polypeptide and a polynucleotide sequence (SEQ ID NO: 153) vector expressing AN_Bif_Abs_0158 second polypeptide into a host cell for expression.

For example, the bifunctional antibody AN_Bif_Abs_0675 can be composed of a Sugemalimab heavy chain first polypeptide (SEQ ID NO: 4) and a chimeric antibody AN_Bif_Abs_0675 second polypeptide (SEQ ID NO: 43) (formed by CD86 extracellular domain IgV domain connecting to a linker peptide (SEQ ID NO: 34) covalently linked to Sugemalimablight chain). AN_Bif_Abs_0675 bifunctional antibody is assembled by introducing a polynucleotide sequence (SEQ ID NO: 152) vector expressing AN_Bif_Abs_0675 first polypeptide and a polynucleotide sequence (SEQ ID NO: 158) vector expressing AN_Bif_Abs_0675 second polypeptide into a host cell for expression.

For example, the bifunctional antibody AN_Bif_Abs_0676 can be composed of a Sugemalimab heavy chain first polypeptide (SEQ ID NO: 4) and a chimeric antibody AN_Bif_Abs_0676 second polypeptide (SEQ ID NO: 41) (formed by Sugemalimablight chain connecting to a linker peptide (SEQ ID NO: 34) covalently linked to CD86 extracellular domain IgV domain). AN_Bif_Abs_0676 bifunctional antibody is assembled by introducing a polynucleotide sequence (SEQ ID NO: 152) vector expressing AN_Bif_Abs_0676 first polypeptide and a polynucleotide sequence (SEQ ID NO: 159) vector expressing AN_Bif_Abs_0676 second polypeptide into a host cell for expression.

For example, the multifunctional antibody AN_Bif_Abs_0159 can be composed of a chimeric antibody AN_Bif_Abs_0159 first polypeptide (SEQ ID NO: 39) (formed by CD86 extracellular domain IgV domain C-terminus connecting to a linker peptide (SEQ ID NO: 34) covalently linked to Sugemalimab heavy chain N-terminus) and second polypeptide (SEQ ID NO: 138) (formed by LAG3 D1/D2 C-terminus through a linker peptide (SEQ ID NO: 34) covalently linked to Sugemalimab light chain). AN_Bif_Abs_0159 multifunctional antibody is assembled by introducing a polynucleotide sequence (SEQ ID NO: 157) vector expressing AN_Bif_Abs_0159 first polypeptide and a polynucleotide sequence (SEQ ID NO: 160) vector expressing AN_Bif_Abs_0159 second polypeptide into a host cell for expression.

For example, the multifunctional antibody AN_Bif_Abs_0160 can be composed of a chimeric antibody AN_Bif_Abs_0160 first polypeptide (SEQ ID NO: 39) (formed by CD86 extracellular domain IgV domain C-terminus through a linker peptide (SEQ ID NO: 34) covalently linked to Sugemalimab heavy chain N-terminus) and second polypeptide (SEQ ID NO: 142) (formed by Sugemalimab light chain C-terminus through a linker peptide (SEQ ID NO: 34) covalently linked to LAG3 D1/D2). AN_Bif_Abs_0160 multifunctional antibody is assembled by introducing a polynucleotide sequence (SEQ ID NO: 157) vector expressing AN_Bif_Abs_0160 first polypeptide and a polynucleotide sequence (SEQ ID NO: 161) vector expressing AN_Bif_Abs_0160 second polypeptide into a host cell for expression.

For example, the multifunctional antibody AN_Bif_Abs_0161 can be composed of a chimeric antibody AN_Bif_Abs_0161 first polypeptide (SEQ ID NO: 114) (formed by CD86 extracellular domain IgV domain C-terminus through a linker peptide (SEQ ID NO: 34) covalently linked to Sugemalimab heavy chain N-terminus, and a sugemalimab heavy chain C-terminus connecting to a linker peptide (SEQ ID NO: 34) covalently linked to LAG3 D1/D2) and a Sugemalimab light chain second polypeptide (SEQ ID NO: 5). AN_Bif_Abs_0161 multifunctional antibody is assembled by introducing a polynucleotide sequence (SEQ ID NO: 162) vector expressing AN_Bif_Abs_0161 first polypeptide and polynucleotide sequence (SEQ ID NO: 153) vector expressing AN_Bif_Abs_0161 second polypeptide into a host cell for expression.

For example, the multifunctional antibody AN_Bif_Abs_0644 can be composed of a chimeric antibody AN_Bif_Abs_0644 first polypeptide (SEQ ID NO: 107) (formed by a CD86 extracellular domain C-terminus connecting to a linker peptide (SEQ ID NO: 34) covalently linked to Sugemalimab heavy chain N-terminus, and a sugemalimab heavy chain C-terminus connecting to a linker peptide (SEQ ID NO: 34) covalently linked to LAG3 D1) and a Sugemalimab light chain second polypeptide (SEQ ID NO: 5). AN_Bif_Abs_0644 multifunctional antibody is assembled by introducing a polynucleotide sequence (SEQ ID NO: 163) vector expressing AN_Bif_Abs_0644 first polypeptide and a polynucleotide sequence (SEQ ID NO: 153) vector expressing AN_Bif_Abs_0644 second polypeptide into a host cell for expression.

For example, the multifunctional antibody AN_Bif_Abs_0646 can be composed of a chimeric antibody AN_Bif_Abs_0646 first polypeptide (SEQ ID NO: 115) (formed by a CD86 extracellular domain IgV domain C-terminus connecting to a linker peptide (SEQ ID NO: 34) covalently linked to Sugemalimab heavy chain N-terminus, and a sugemalimab heavy chain C-terminus connecting to a linker peptide (SEQ ID NO: 34) covalently linked to LAG3 D1) and a Sugemalimab light chain second polypeptide (SEQ ID NO: 5). AN_Bif_Abs_0646 multifunctional antibody is assembled by introducing a polynucleotide sequence (SEQ ID NO: 164) vector expressing AN_Bif_Abs_0646 first polypeptide and a polynucleotide sequence (SEQ ID NO: 153) vector expressing AN_Bif_Abs_0646 second polypeptide into a host cell for expression.

For example, the multifunctional antibody AN_Bif_Abs_0162 can be composed of a chimeric antibody AN_Bif_Abs_0162 first polypeptide (SEQ ID NO: 112) a CD86 extracellular domain IgV domain C-terminus connecting to a linker peptide (SEQ ID NO: 34) covalently linked to Sugemalimab heavy chain N-terminus, and a sugemalimab heavy chain C-terminus connecting to a linker peptide (SEQ ID NO: 34) covalently linked to LAG3 extracellular domain) and a Sugemalimab light chain second polypeptide (SEQ ID NO: 5). AN_Bif_Abs_0162 multifunctional antibody is assembled by introducing a polynucleotide (SEQ ID NO: 165) vector expressing AN_Bif_Abs_0162 first polypeptide and a polynucleotide (SEQ ID NO: 153) vector expressing AN_Bif_Abs_0162 second polypeptide into a host cell for expression.

For example, the multifunctional antibody AN_Bif_Abs_0163 can be composed of a chimeric antibody AN_Bif_Abs_0163 first polypeptide (SEQ ID NO: 104) (formed by a CD86 extracellular domain C-terminus connecting to a linker peptide (SEQ ID NO: 34) covalently linked to Sugemalimab heavy chain N-terminus, and a sugemalimab heavy chain C-terminus connecting to a linker peptide (SEQ ID NO: 34) covalently linked to LAG3 extracellular domain) and a Sugemalimab light chain second polypeptide (SEQ ID NO: 5). AN_Bif_Abs_0163 multifunctional antibody is assembled by introducing a polynucleotide sequence (SEQ ID NO: 166) vector expressing AN_Bif_Abs_0163 first polypeptide and a polynucleotide sequence (SEQ ID NO: 153) vector expressing AN_Bif_Abs_0163 second polypeptide into a host cell for expression.

For example, the multifunctional antibody AN_Bif_Abs_0164 can be composed of a chimeric antibody AN_Bif_Abs_0164 first polypeptide (SEQ ID NO: 108) (formed by LAG3 extracellular domain C-terminus connecting to a linker peptide (SEQ ID NO: 34) covalently linked to Sugemalimab heavy chain N-terminus, and a sugemalimab heavy chain C-terminus connecting to a linker peptide (SEQ ID NO: 34) covalently linked to CD86 extracellular domain) and a Sugemalimab light chain second polypeptide (SEQ ID NO: 5). AN_Bif_Abs_0164 multifunctional antibody is assembled by introducing a polynucleotide sequence (SEQ ID NO: 167) vector expressing AN_Bif_Abs_0164 first polypeptide and a polynucleotide sequence (SEQ ID NO: 153) vector expressing AN_Bif_Abs_0164 second polypeptide into a host cell for expression.

For example, the multifunctional antibody AN_Bif_Abs_0166 can be composed of a chimeric antibody AN_Bif_Abs_0166 first polypeptide (SEQ ID NO: 116) (formed by LAG3 extracellular domain C-terminus connecting to a linker peptide (SEQ ID NO: 34) covalently linked to Sugemalimab heavy chain N-terminus, and the Sugemalimab heavy chain C-terminus connecting to a linker peptide (SEQ ID NO: 34) covalently linked to CD86 extracellular domain IgV domain) and a Sugemalimab light chain second polypeptide (SEQ ID NO: 5). AN_Bif_Abs_0166 multifunctional antibody is assembled by introducing a polynucleotide (SEQ ID NO: 168) vector expressing AN_Bif_Abs_0166 first polypeptide and a polynucleotide (SEQ ID NO: 153) vector expressing AN_Bif_Abs_0166 second polypeptide into a host cell for expression.

For example, the antibody AN_Bif_Abs_0031 can be composed of an antibody AN_Bif_Abs_0031 first polypeptide (SEQ ID NO: 14) formed by Envafolimab . AN_Bif_Abs_0031 antibody is assembled by introducing a polynucleotide (SEQ ID NO: 169) vector expressing AN_Bif_Abs_0031 first polypeptide polynucleotide into a host cell for expression.

For example, the bifunctional antibody AN_Bif_Abs_0129 can be composed of a chimeric antibody AN_Bif_Abs_0129 first polypeptide (SEQ ID NO: 71) (formed by CD86 extracellular domain IgV domain C-terminus connecting to a linker peptide (SEQ ID NO: 34) covalently linked to Envafolimab N-terminus). AN_Bif_Abs_0129 bifunctional antibody is assembled by introducing a polynucleotide (SEQ ID NO: 170) vector expressing AN_Bif_Abs_0129 first polypeptide into a host cell for expression.

For example, the multifunctional antibody AN_Bif_Abs_0130 can be composed of a chimeric antibody AN_Bif_Abs_0130 first polypeptide (SEQ ID NO: 171) (formed by a CD86 extracellular domain IgV domain C-terminus connecting to a linker peptide (SEQ ID NO: 34) covalently linked to LAG3 D1/D2 N-terminus, and LAG3 D1/D2 C-terminus connecting to a linker peptide (SEQ ID NO: 34) covalently linked to Envafolimab N-terminus). AN_Bif_Abs_0130 multifunctional antibody is assembled by introducing a polynucleotide (SEQ ID NO: 172) vector expressing AN_Bif_Abs_0130 first polypeptide into a host cell for expression.

For example, the multifunctional antibody AN_Bif_Abs_0131 can be composed of a chimeric antibody AN_Bif_Abs_0131 first polypeptide (SEQ ID NO: 173) (formed by a CD86 extracellular domain IgV domain C-terminus connecting to a linker peptide (SEQ ID NO: 34) covalently linked to Envafolimab N-terminus, and Envafolimab C-terminus connecting to a linker peptide (SEQ ID NO: 34) covalently linked to LAG3 D1/D2 C-terminus). AN_Bif_Abs_0131 multifunctional antibody is assembled by introducing a polynucleotide (SEQ ID NO: 174) vector expressing AN_Bif_Abs_0131 first polypeptide into a host cell for expression.

For example, the bifunctional antibody AN_Bif_Abs_0012 can be composed of a chimeric antibody AN_Bif_Abs_0012 first polypeptide (SEQ ID NO: 44) (formed by Durvalumab heavy chain C-terminus connecting to a linker peptide (SEQ ID NO: 34) covalently linked to CD86 extracellular domain) and a Durvalumab light chain second polypeptide (SEQ ID NO: 9). AN_Bif_Abs_0012 multifunctional antibody is assembled by introducing a polynucleotide sequence (SEQ ID NO: 175) vector expressing AN_Bif_Abs_0164 first polypeptide and a polynucleotide sequence (SEQ ID NO: 176) vector expressing AN_Bif_Abs_0012 second polypeptide into a host cell for expression.

For example, the antibody AN_Bif_Abs_0001 can be composed of a Durvalumab heavy chain antibody AN_Bif_ Abs_0001 first polypeptide (SEQ ID NO: 8) and Durvalumab light chain second polypeptide (SEQ ID NO: 9). AN_Bif_ Abs_0001 antibody is assembled by introducing a polynucleotide sequence (SEQ ID NO: 177) vector expressing AN_Bif_ Abs_0001 first polypeptide and a polynucleotide sequence (SEQ ID NO: 176) vector expressing AN _Bif_Abs_0001 second polypeptide into a host cell for expression.

In certain embodiments, the present application also provides a method for separating a polypeptide complex, which is used to separate the polypeptide complex.

In one aspect, the present invention provides a method for producing the polypeptide complex: the method comprises introducing the polynucleotide encoding the antibody polypeptide complex of the present invention into a host cell, expressing the first polypeptide, and/or the second polypeptide, wherein the first polypeptide and the second polypeptide, and the first polypeptide itself can form a stable dimer, and the stable dimer can be maintained by a natural bond or/and a stable dimer comprising at least one non-natural interchain bond, the first polypeptide and the second polypeptide are assembled into a stable polymer in the host cell, and form the stable complex having the ability to bind to the first cell target, the second cell target and/or the third cell target.

In other aspects, the contents disclosed in the present application provide a method for relieving the inhibitory effect of PD-L1/PD-1 on T cells and stimulating the activation of antigen-presenting cells and/or T cells. The method includes using the dual and/or multifunctional fusion polypeptide disclosed in the present application to stimulate the activation of T cells, but is not limited to the scheme used in the examples, and can be expanded to the schemes that have been published or developed in the future and can be used to evaluate the activation of antigen-presenting cells and/or T cells.

On the other hand, the dual and/or multifunctional fusion polypeptides disclosed in the present application have the effect of blocking tumor growth, including but not limited to colon tumors, breast tumors, lung tumors, gastric tumors, melanoma, head and neck tumors, lymphomas, nasopharyngeal tumors, cervical tumors, esophageal tumors, kidney tumors, skin squamous cell carcinoma, endometrial tumors, liver tumors, bladder tumors, urothelial tumors and/or skin tumors.

On the other hand, the present application provides an immunoconjugate, which may comprise the fusion polypeptide described in the present application.

On the other hand, the present application provides a nucleic acid molecule, which can encode the fusion polypeptide described in the present application.

On the other hand, the present application provides a vector, which may contain the nucleic acid molecule described in the present application.

On the other hand, the present application provides a cell, which may contain and/or express the fusion polypeptide described in the present application, the immunoconjugate described in the present application, the nucleic acid molecule described in the present application, and/or the vector described in the present application.

On the other hand, the present application provides a composition, which may include the fusion polypeptide described in the present application, the immunoconjugate described in the present application, the nucleic acid molecule described in the present application, the vector described in the present application, and/or the cell described in the present application, and optionally a pharmaceutically acceptable carrier.

On the other hand, the present application provides a method for preparing the fusion polypeptide described in the present application, which comprises culturing the cell described in the present application under conditions that allow the fusion polypeptide to be expressed.

On the other hand, the present application provides a method for blocking the interaction between PD-L1 protein and PD-1, which may include administering an effective amount of the fusion polypeptide described in the present application, the immunoconjugate described in the present application, the nucleic acid molecule described in the present application, the vector described in the present application, the cell described in the present application, and/or the composition described in the present application.

On the other hand, the present application provides a method for stimulating antigen presenting cells and/or activating T cells, which may include administering an effective amount of the fusion polypeptide described in the present application, the immunoconjugate described in the present application, the nucleic acid molecule described in the present application, the vector described in the present application, the cell described in the present application, and/or the composition described in the present application.

According to the method described in the present application, the stimulation of antigen presenting cells may include a method selected from the following group: increasing the expression of co-stimulatory molecules in antigen presenting cells, causing morphological changes and maturation of antigen presenting cells, increasing the secretion of chemokines in antigen presenting cells, and enhancing the phagocytic ability of antigen presenting cells.

On the other hand, the present application provides a method for inhibiting the growth and/or proliferation of tumors or tumor cells, which may include administering an effective amount of the fusion polypeptide described herein, the immunoconjugate described herein, the nucleic acid molecule described herein, the vector described herein, the cell described herein, and/or the composition described herein.

On the other hand, the present application provides the use of the fusion polypeptide described in the present application, the immunoconjugate described in the present application, the nucleic acid molecule described in the present application, the vector described in the present application, the cell described in the present application, and/or the composition described in the present application in the preparation of a medicament, wherein the medicament can be used to prevent, improve and/or treat tumors.

According to the use described in the present application, the tumor may include solid tumors and hematological tumors.

According to the use described in the present application, the tumor can be selected from the following group: colon tumor, breast tumor, lung tumor, gastric tumor, melanoma, head and neck tumor, lymphoma, nasopharyngeal tumor, cervical tumor, esophageal tumor, kidney tumor, endometrial tumor, liver tumor, bladder tumor, urothelial tumor and skin tumor. For example, a skin tumor may include squamous cell carcinoma of the skin.

On the other hand, the present application provides the fusion polypeptide described in the present application, the immunoconjugate described in the present application, the nucleic acid molecule described in the present application, the vector described in the present application, the cell described in the present application, and/or the composition described in the present application, which can be used to prevent, improve and/or treat tumors.

On the other hand, the present application provides the fusion polypeptide described in the present application, the immunoconjugate described in the present application, the nucleic acid molecule described in the present application, the vector described in the present application, the cell described in the present application, and/or the composition described in the present application, which can be used to prevent, improve and/or treat tumors, wherein the tumors include solid tumors and hematological tumors.

On the other hand, the present application provides the fusion polypeptide described in the present application, the immunoconjugate described in the present application, the nucleic acid molecule described in the present application, the vector described in the present application, the cell described in the present application, and/or the composition described in the present application, which can be used to prevent, improve and/or treat tumors, wherein the tumor can be selected from the following group: colon tumors, breast tumors, lung tumors, gastric tumors, melanoma, head and neck tumors, lymphomas, nasopharyngeal tumors, cervical tumors, esophageal tumors, kidney tumors, endometrial tumors, liver tumors, bladder tumors, urothelial tumors and skin tumors. For example, a skin tumor may include squamous cell carcinoma of the skin.

On the other hand, the present application provides a method for preventing, improving and/or treating tumors, which may comprise administering the fusion polypeptide described herein, the immunoconjugate described herein, the nucleic acid molecule described herein, the vector described herein, the cell described herein, and/or the composition described herein to a subject in need thereof.

According to the method described in the present application, the tumor may include solid tumors and hematological tumors.

According to the method described in the present application, the tumor can be selected from the following group: colon tumor, breast tumor, lung tumor, gastric tumor, melanoma, head and neck tumor, lymphoma, nasopharyngeal tumor, cervical tumor, esophageal tumor, kidney tumor, endometrial tumor, liver tumor, bladder tumor, urothelial tumor and skin tumor. For example, a skin tumor may include squamous cell carcinoma of the skin.

CD86 protein is a type of immune-related molecule with stimulatory immune response. It mainly realizes its function by regulating T cell immune response, including participating in immune defense, immune tolerance and immune tissue damage. CD86 mainly exerts immunomodulatory effects by binding to its ligands CD28 and CTLA4 to regulate immune response. Changing the affinity between CD86 and ligands can regulate CD86-mediated related functions, especially enhancing the body's immune response function and strengthening the control of diseases including tumors and infections. CD86 is one of the members of the B7 family of proteins that can co-stimulate and activate T cells. The mature CD86 molecule is composed of an extracellular domain (ECD), a transmembrane domain and an intracellular domain. The extracellular domain (ECD) is the key region for these molecules to bind to the corresponding receptors on T cells. The CD86 extracellular domain contains an immunoglobulin variable-like V (IgV) region and an immunoglobulin constant-like C2 (IgC2) region, and the IgV domain is the key domain directly involved in its receptor binding. The CD86 extracellular domain IgV can bind to CD28 to participate in inducing T cell activation, proliferation and effector function. CTLA4 can also bind to the IgV region of CD86 to participate in immunosuppressive regulation. In the immune response regulation reaction, the activation of T cells is regulated by the binding of co-stimulatory signal receptors such as CD28 and the corresponding ligand CD86 on the APC surface. However, CD86 is usually not expressed or lowly expressed in the tumor microenvironment, resulting in the inability to effectively activate T cells. This is also one of the key mechanisms of tumor immune escape. In addition, CTLA4 competes with CD28 for binding to CD86 and exhibits higher affinity characteristics, resulting in CD28 being unable to effectively receive immune stimulation signals mediated by CD86, making it impossible for T cells to be effectively activated or immunosuppressed.

Therefore, there is a need in the art for CD86 mutant polypeptides that improve the binding activity of CD86 to its ligand, which can effectively stimulate T cells and enhance immune response, thereby being able to treat or prevent diseases caused by suppressed T cell function, such as infections, tumors, and the like.

The CD86 mutant polypeptide formed by innovatively modifying the CD86 IgV domain in the present application can regulate the binding activity of CD86 and its ligand, so that the CD86 mutant polypeptide can effectively stimulate T cells to enhance the immune response, thereby being able to treat or prevent diseases caused by the inhibition of T cell function, such as infections, tumors and the like.

In one aspect, the present application provides an isolated CD86 variant polypeptide, which may comprise an amino acid substitution mutation of human CD86.

For example, the CD86 variant polypeptide may comprise an amino acid substitution mutant of the humanized IgV domain of CD86 (SEQ ID NO: 3).

For example, the CD86 mutant may comprise an amino acid substitution mutant of the IgV domain of human CD86 (SEQ ID NO: 3), and the amino acid substitution mutation site may comprise one or more of A13I, A13L, A13F, A13M, Q25A, Q25I, Q25V, Q25F, Q25M, F33A, F33L, F33V, F33M, M60R, I89A, I89L, 189V, I89F, I89M, H90I, H90V, H90M, and H90F. For example, the CD86 IgV mutant polypeptide includes a combination of 1, 2, 3, 4, 5 or more of the above-mentioned amino acid mutations.

For example, the amino acid substitution mutant of the IgV domain of CD86 can be selected from the combination of amino acid mutation sites shown in the following group: Q25I/F33L/H90I, Q25V/F33L/H90I, Q25I/F33V/H90I, Q25V/F33V/H90I, Q25I/F33L/H90V, Q25V/F33L/H90V, Q25I/F33V/H90V, Q25V/F33V/H90V, A13L/Q25I/F33L/H90I, A13I/Q25I/F33L/H90I, A13L/Q25V/F33L/H90I. A13I/Q25V/F33L/H90I, Q25I/F33L/I89L/H90I, Q25I/F33L/M60R/H90I, Q25V/F33L/I89L/H90I, Q25V/F33L/M60R/H90I, Q25F/F33L/H90I, Q25I/F33L/H90F, Q25I/F33A/H90I, Q25I/H90I, Q25I, H90I, Q25V/H90V, Q25V/H90I, Q25F/H90I, Q25F/H90V, A13F/Q25I/F33L/H90I, A13M/Q25I/F33L/H90I, Q25A/F33L/H90I, Q25M/F33L/H90I, Q25I/F33M/H90I, Q25I/F33L/I89V/H90I, Q25I/F33L/I89F/H90I, Q25I/F33L/I89M/H90I and Q25I/F33L/H90M.

For example, the variant polypeptide may comprise the IgV domain amino acid substitution mutant Q25I/F33L/H90I of CD86.

For example, the CD86 variant polypeptide may comprise an amino acid substitution mutant of the extracellular domain of human CD86.

For example, the CD86 variant polypeptide may comprise an amino acid substitution mutant of the extracellular domain of human CD86 (SEQ ID NO: 2), and the amino acid substitution mutant may comprise one or more of A13I, A13L, A13F, A13M, Q25A, Q25I, Q25V, Q25F, Q25M, F33A, F33L, F33V, F33M, M60R, I89A, I89L, 189V, I89F, I89M, H90I, H90V, H90M, and H90F. For example, the CD86 extracellular domain mutant polypeptide includes a combination of 1, 2, 3, 4, 5 or more of the above-mentioned amino acid mutations.

The amino acid substitution mutant of the extracellular domain of CD86 (SEQ ID NO: 2) can be selected from the combination of amino acid mutation sites shown in the following group: Q25I/F33L/H90I, Q25V/F33L/H90I, Q25I/F33V/H90I, Q25V/F33V/H90I, Q25I/F33L/H90V, Q25V/F33L/H90V, Q25I/F33V/H90V, Q25V/F33V/H90V, A13L/Q25I/F33L/H90I, A13I/Q25I/F33L/H90I, A13L/Q25V/F33L/H90I, A13I /Q25V/F33L/H90I, Q25I/F33L/I89L/H90I, Q25I/F33L/M60R/H90I, Q25V/F33L/I89L/H90I, Q25V/F33L/M60R/H90I, Q25F/F33L/H90I, Q25I/F33L/H90F, Q25I/F33A/H90I, Q25 I/H90I, Q25I, H90I, Q25V/H90V, Q25V/H90I, Q25F/H90I, Q25F/H90V, A13F/Q25I/F33L/H90I, A13M/Q25I/F33L/H90I, Q25A/F33L/H90I, Q25M/F33L/H90I, Q25I/F33M/H90I, Q25I/F33L/I89V/H90I, Q25I/F33L/I89F/H90I, Q25I/F33L/I89M/H90I and Q25I/F33L/H90M.

For example, the CD86 variant polypeptide may comprise the extracellular domain amino acid substitution mutant Q25I/F33L/H90I of CD86.

For example, the CD86 variant polypeptide may comprise an amino acid sequence selected from the group consisting of SEQ ID NO: 178 to SEQ ID NO: 243.

On the other hand, the present application provides a fusion polypeptide, which may include a first domain and a second domain, wherein the first domain may include a CD86 variant polypeptide, and the second domain may include an antibody or an antigen-binding fragment thereof or other forms of polypeptides.

For example, the first domain may comprise an amino acid substitution mutant of the IgV domain of human CD86.

For example, the first domain may comprise an amino acid substitution mutant of the IgV domain of human CD86 (SEQ ID NO: 3), and the amino acid substitution mutation site may comprise one or more of A13I, A13L, A13F, A13M, Q25A, Q25I, Q25V, Q25F, Q25M, F33A, F33L, F33V, F33M, M60R, I89A, I89L, I89V, I89F, I89M, H90I, H90V, H90M, and H90F.

For example, the CD86 IgV mutant polypeptide includes a combination of 1, 2, 3, 4, 5 or more of the above-mentioned amino acid mutations.

For example, the first domain may comprise an amino acid substitution mutant of the IgV domain of CD86 (SEQ ID NO: 3), and the amino acid substitution mutant may be selected from the combination of amino acid mutation sites shown in the following group: Q25I/F33L/H90I, Q25V/F33L/H90I, Q25I/F33V/H90I, Q25V/F33V/H90I, Q25I/F33L/H90V, Q25V/F33L/H90V, Q25I/F33V/H90V, Q25V/F33V/H90V, A13L/Q25I/F33L/H90I, A13I/Q25I/F33L/H90I, A13L/Q25V/F33L/H90I, A13I/Q25V/F33L/H90I, Q25I/F33L/I89L/H90I, Q25I/F33L/M60R/H90I, Q25V/F33L/I89L/H90I, Q25V/F33L/M60R/H90I, Q25F/F33L/H90I, Q25I/F33L/H90F, Q25I/F33A/H90I, Q25I/H90I, Q25I, H90I, Q25V/H90V, Q25V/H90I, Q25F/H90I, Q25F/H90V, A13F/Q25I/F33L/H90I, A13M/Q25I/F33L/H90I, Q25A/F33L/H90I, Q25M/F33L/H90I, Q25I/F33M/H90I, Q25I/F33L/I89V/H90I, Q25I/F33L/I89F/H90I, Q25I/F33L/I89M/H90I and Q25I/F33L/H90M.

For example, the first domain may comprise the IgV domain amino acid substitution mutant Q25I/F33L/H90I of CD86.

For example, the first domain may comprise an amino acid substitution mutant of the extracellular domain of human CD86 (SEQ ID NO: 2).

For example, the first domain may comprise an amino acid substitution mutant of the extracellular domain of human CD86 (SEQ ID NO: 2), and the amino acid substitution mutant may comprise one or more of A13I, A13L, A13F, A13M, Q25A, Q25I, Q25V, Q25F, Q25M, F33A, F33L, F33V, F33M, M60R, I89A, I89L, I89V, I89F, I89M, H90I, H90V, H90M, and H90F.

For example, the CD86 extracellular domain mutant polypeptide includes a combination of 1, 2, 3, 4, 5 or more of the above-mentioned amino acid mutations.

For example, the first domain may comprise an amino acid substitution mutant of the extracellular domain of human CD86 (SEQ ID NO: 2), and the amino acid substitution mutant of the extracellular domain of CD86 may be selected from the combination of amino acid mutation sites shown in the following group: Q25I/F33L/H90I, Q25V/F33L/H90I, Q25I/F33V/H90I, Q25V/F33V/H90I, Q25I/F33L/H90V,
Q25V/F33L/H90V, Q25I/F33V/H90V, Q25V/F33V/H90V, A13L/Q25I/F33L/H90I, A13I/Q25I/F33L/H90I, A13L/Q25V/F 33L/H90I, A13I/Q25V/F33L/H90I, Q25I/F33L/I89L/H90I, Q25I/F33L/M60R/H90I, Q25V/F33L/I89L/H90I, Q25V/F33L/M60R/H90I, Q25F/F33L/H90I, Q25I/F33L/H90F, Q25I/F33A/ H90I, Q25I/H90I, Q25I, H90I, Q25V/H90V, Q25V/H90I, Q25F/H90I, Q25F/H90V, A13F/Q25I/F33L/H90I, A13M/Q25I/F33L/H90I, Q25A/F33L/H90I, Q25M/F33L/H90I, Q25I/F33M/H90I, Q25I/F33L/I89V/H90I, Q25I/F33L/I89F/H90I, Q25I/F33L/I89M/H90I and Q25I/F33L/H90M.

For example, the first domain may comprise the extracellular domain amino acid substitution mutant Q25I/F33L/H90I of a human CD86 variant polypeptide.

For example, the first domain may comprise an amino acid sequence selected from the group consisting of SEQ ID NO: 178 to SEQ ID NO: 243.

For example, the second domain may comprise an antibody or an antigen-binding fragment thereof.

For example, the second domain can bind to one or more of the targets shown in the following group: PD-L1, PD-L2, PD-1, OX40, 4-1BB, ICOS, TIGIT, CTLA4, LAG3, CD3, VEGF, VEGFR, CD47, HGF, Trop2, EpCAM, CCR8, CCR4, CCR5, GPRC5D, BCMA, CD19, CD20, HER-2 neu, DLL1, HER-3, HER-4, EGFR, PSMA, CEA, MUC-1 (mucin), MUC2, MUC3, MUC4, MUC5AC, MUC5B, MUC7, CD123, CD33, CD30, CD38, NKG2A, Nkp36 and Tim3.

For example, the second domain is capable of binding to PD-L1 and/or PD-1.

For example, the second domain may comprise HCDR3 of an antibody heavy chain, which may comprise an amino acid sequence selected from the group consisting of SEQ ID NO:4, SEQ ID NO:6, SEQ ID NO:8, SEQ ID NO:10, SEQ ID NO:12, SEQ ID NO:14, SEQ ID NO: 15 and SEQ ID NO: 17.

For example, the second domain may comprise HCDR2 of an antibody heavy chain, which may comprise an amino acid sequence selected from the group consisting of SEQ ID NO: SEQ ID NO:4, SEQ ID NO:6, SEQ ID NO:8, SEQ ID NO:10, SEQ ID NO:12, SEQ ID NO: 14, SEQ ID NO: 15 and SEQ ID NO: 17.

For example, the second domain may comprise HCDR1 of an antibody heavy chain, which may comprise an amino acid sequence selected from the group consisting of SEQ ID NO: SEQ ID NO:4, SEQ ID NO:6, SEQ ID NO:8, SEQ ID NO:10, SEQ ID NO:12, SEQ ID NO: 14, SEQ ID NO: 15 and SEQ ID NO: 17.

For example, the second domain may comprise a heavy chain variable region VH of an antibody heavy chain, and the antibody heavy chain variable region may comprise an amino acid sequence selected from the group consisting of SEQ ID NO:4, SEQ ID NO:6, SEQ ID NO:8, SEQ ID NO: 10, SEQ ID NO: 12, SEQ ID NO: 14, SEQ ID NO: 15 and SEQ ID NO: 17.

For example, the second domain may comprise an antibody heavy chain, which may comprise an amino acid sequence selected from the group consisting of SEQ ID NO:4, SEQ ID NO:6, SEQ ID NO:8, SEQ ID NO:10, SEQ ID NO:12, SEQ ID NO:14, SEQ ID NO:15 and SEQ ID NO: 17.

For example, the second domain may comprise LCDR3 of an antibody light chain, which may comprise an amino acid sequence selected from the group consisting of SEQ ID NO:5, SEQ ID NO:7, SEQ ID NO:9, SEQ ID NO: 11, SEQ ID NO: 13, SEQ ID NO:16 and SEQ ID NO:18.

For example, the second domain may comprise LCDR2 of an antibody light chain, which may comprise an amino acid sequence selected from the group consisting of SEQ ID NO:5, SEQ ID NO:7, SEQ ID NO:9, SEQ ID NO: 11, SEQ ID NO: 13, SEQ ID NO:16 and SEQ ID NO:18.

For example, the second domain may comprise LCDR1 of an antibody light chain, and the antibody light chain may comprise an amino acid sequence selected from the group consisting of SEQ ID NO:5, SEQ ID NO:7, SEQ ID NO:9, SEQ ID NO: 11, SEQ ID NO:13, SEQ ID NO:16 and SEQ ID NO:18.

For example, the second domain may comprise a light chain variable region VL of an antibody light chain, and the antibody light chain variable region may comprise an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

For example, the second domain may comprise an antibody light chain, which may comprise an amino acid sequence selected from the group consisting of SEQ ID NO:5, SEQ ID NO:7, SEQ ID NO:9, SEQ ID NO:11, SEQ ID NO:13, SEQ ID NO:16 and SEQ ID NO:18.

For example, the second domain may comprise HCDR3, HCDR2 and HCDR1 of an antibody heavy chain, wherein the antibody heavy chain may comprise the amino acid sequence shown in SEQ ID NO:4. For example, the antibody can be Sugemalimab.

For example, the second domain may comprise LCDR3, LCDR2 and LCDR1 of an antibody light chain, wherein the antibody light chain may comprise the amino acid sequence shown in SEQ ID NO: 5. For example, the antibody can be Sugemalimab.

For example, the second domain may comprise HCDR3, HCDR2 and HCDR1 of an antibody heavy chain, wherein the antibody heavy chain may comprise the amino acid sequence shown in SEQ ID NO:4, and the second domain may comprise LCDR3, LCDR2 and LCDR1 of an antibody light chain, wherein the antibody light chain may comprise the amino acid sequence shown in SEQ ID NO:5. For example, the antibody can be Sugemalimab.

For example, the second domain may comprise the heavy chain variable region VH of the antibody heavy chain, and the antibody heavy chain may comprise the amino acid sequence shown in SEQ ID NO:4, and the second domain may comprise the light chain variable region VL of the antibody light chain, and the antibody light chain may comprise the amino acid sequence shown in SEQ ID NO:5. For example, the antibody can be Sugemalimab.

For example, the second domain may comprise HCDR3, HCDR2 and HCDR1 of an antibody heavy chain, wherein the antibody heavy chain may comprise the amino acid sequence shown in SEQ ID NO:10. For example, the antibody can be Atezolizumab.

For example, the second domain may comprise LCDR3, LCDR2 and LCDR1 of an antibody light chain, wherein the antibody light chain may comprise the amino acid sequence shown in SEQ ID NO:11. For example, the antibody can be Atezolizumab.

For example, the second domain may comprise HCDR3, HCDR2 and HCDR1 of an antibody heavy chain, wherein the antibody heavy chain may comprise the amino acid sequence shown in SEQ ID NO: 10, and the first domain may comprise LCDR3, LCDR2 and LCDR1 of an antibody light chain, wherein the antibody light chain may comprise the amino acid sequence shown in SEQ ID NO: 11. For example, the antibody can be Atezolizumab.

For example, the first domain may comprise the heavy chain variable region VH of an antibody heavy chain, and the antibody heavy chain may comprise the amino acid sequence shown in SEQ ID NO:10, and the first domain may comprise the light chain variable region VL of an antibody light chain, and the antibody light chain may comprise the amino acid sequence shown in SEQ ID NO: 11. For example, the antibody can be Atezolizumab.

For example, the second domain may bind to CD3.

For example, the second domain may comprise a CD3B219 antibody or an antigen-binding fragment thereof.

For example, the second domain may comprise HCDR3, HCDR2 and HCDR1 of an antibody heavy chain, wherein the antibody heavy chain may comprise the amino acid sequence shown in SEQ ID NO:616. For example, the antibody may be a CD3B219 antibody.

For example, the second domain may comprise LCDR3, LCDR2 and LCDR1 of an antibody light chain, wherein the antibody light chain may comprise the amino acid sequence shown in SEQ ID NO:617. For example, the antibody may be a CD3B219 antibody.

For example, the second domain may comprise HCDR3, HCDR2 and HCDR1 of an antibody heavy chain, wherein the antibody heavy chain may comprise the amino acid sequence shown in SEQ ID NO:616, and the second domain may comprise LCDR3, LCDR2 and LCDR1 of an antibody light chain, wherein the antibody light chain may comprise the amino acid sequence shown in SEQ ID NO:617. For example, the antibody may be a CD3B219 antibody.

For example, the second domain may comprise the heavy chain variable region VH of the antibody heavy chain, and the antibody heavy chain may comprise the amino acid sequence shown in SEQ ID NO:616, and the second domain may comprise the light chain variable region VL of the antibody light chain, and the antibody light chain may comprise the amino acid sequence shown in SEQ ID NO:617. For example, the antibody may be a CD3B219 antibody.

For example, the antibody can be selected from immunoglobulin antibodies, recombinant antibodies, chimeric antibodies, heavy chain antibodies, single domain antibodies and/or bispecific antibodies; the antigen-binding fragment can be selected from Fab, Fab', Fv, F(ab)2, F(ab')2, scFv, di-scFv, VHH and/or dAb.

For example, the second domain may comprise an immunoglobulin domain.

For example, the second domain may comprise an immunoglobulin IgG antibody, and the immunoglobulin IgG antibody may comprise one or more selected from human IgG1, human IgG2, human IgG3, human IgG4, mouse IgG1, mouse IgG2a, mouse IgG2b and mouse IgG3.

For example, the second domain may comprise an immunoglobulin Fc domain. For example, the second domain may comprise the Fc domain of an immunoglobulin IgG antibody. For example, the second domain may comprise the Fc domain of an immunoglobulin IgG antibody, wherein the Fc domain of the immunoglobulin IgG antibody comprises a human IgG1 Fc domain, a human IgG2 Fc domain, a human IgG3 Fc domain, a human IgG4 Fc domain, a mouse IgG1 Fc domain, a mouse IgG2a Fc domain, a mouse IgG2b Fc domain or a mouse IgG3 Fc domain. For example, the second domain may be the Fc domain of human immunoglobulin IgG4.

For example, the second domain may comprise an amino acid sequence selected from the group consisting of SEQ ID NO: 4 to SEQ ID NO: 18, SEQ ID NO: 29 to SEQ ID NO: 30, SEQ ID NO: 634 to SEQ ID NO: 639, SEQ ID NO: 616 and SEQ ID NO: 617.

For example, the first domain may be directly or indirectly linked to the second domain.

For example, the second structure may include an immunoglobulin Fc domain, and the first domain may be directly or indirectly connected to the C-terminus of the second domain.

For example, the second structure may include an immunoglobulin Fc domain, and the first domain may be directly or indirectly connected to the N-terminus of the second domain.

For example, the second domain may comprise an antibody heavy chain and the first domain may be directly or indirectly linked to the antibody heavy chain of the second domain.

For example, the second domain may comprise an antibody heavy chain, and the first domain may be directly or indirectly linked to the antibody heavy chain C-terminus of the second domain.

For example, the second domain may comprise an antibody heavy chain, and the N-terminus of the first domain may be directly or indirectly connected to the antibody heavy chain C-terminus of the second domain.

For example, the second domain may comprise an antibody heavy chain, and the first domain may be directly or indirectly linked to the N-terminus of the antibody heavy chain of the second domain.

For example, the second domain may comprise an antibody heavy chain, and the C-terminus of the first domain may be directly or indirectly connected to the N-terminus of the antibody heavy chain of the second domain.

For example, the second domain may comprise an antibody light chain, and the first domain may be directly or indirectly linked to the antibody light chain of the second domain.

For example, the second domain may comprise an antibody light chain, and the first domain may be directly or indirectly connected to the antibody light chain C-terminus of the second domain.

For example, the second domain may comprise an antibody light chain, and the N-terminus of the first domain may be directly or indirectly connected to the antibody light chain C-terminus of the second domain.

For example, the second domain may comprise an antibody light chain, and the first domain may be directly or indirectly linked to the N-terminus of the antibody light chain of the second domain.

For example, the second domain may comprise an antibody light chain, and the C-terminus of the first domain may be directly or indirectly connected to the N-terminus of the antibody light chain of the second domain.

For example, the indirect connection may comprise connection via a linker.

For example, the linker may comprise an amino acid sequence selected from the group consisting of SEQ ID NO:31, SEQ ID NO:32, SEQ ID NO:33, SEQ ID NO:34 and SEQ ID NO:35.

For example, the fusion polypeptide complex can include a first polypeptide and a second polypeptide. For example, the second polypeptide may be absent.

For example, the first polypeptide of the fusion polypeptide complex may comprise a second domain, the second domain may comprise an antibody or an antigen-binding fragment thereof, and the second polypeptide may comprise a light chain of the antibody or an antigen-binding fragment thereof.

For example, the first polypeptide of the fusion polypeptide complex may comprise a second domain, the second domain may comprise a heavy chain of an antibody or an antigen-binding fragment thereof, and the second polypeptide may comprise a light chain of the antibody or an antigen-binding fragment thereof.

For example, the fusion polypeptide complex may comprise a second domain, the second domain may comprise a heavy chain, and the second domain heavy chain may represent an Fc peptide chain or a heavy chain of an antibody or an antigen-binding fragment thereof.

For example, the first polypeptide of the fusion polypeptide complex can be covalently linked from the N-terminus to the C-terminus of the polypeptide by a CD86 mutant polypeptide, a linker peptide, and a heavy chain of an antibody or its antigen-binding fragment, wherein the linker peptides can be the same or different and can exist or not exist independently of each other.

For example, the first polypeptide of the fusion polypeptide complex can be covalently linked in sequence from the N-terminus to the C-terminus of the polypeptide by the heavy chain of the antibody or its antigen-binding fragment, a linker peptide, and a CD86 mutant polypeptide, wherein the linker peptides can be the same or different and can exist or not exist independently of each other. For example, the second polypeptide of the fusion polypeptide complex may include a light chain of an antibody or antigen-binding fragment thereof.

For example, the second polypeptide of the fusion polypeptide complex can be covalently linked in sequence from the N-terminus to the C-terminus of the polypeptide by a CD86 mutant polypeptide, a linker peptide, and a light chain of an antibody or its antigen-binding fragment, wherein the linker peptides can be the same or different and can exist or not exist independently of each other.

For example, the second polypeptide of the fusion polypeptide complex can be covalently linked in sequence from the N-terminus to the C-terminus of the polypeptide by a light chain of an antibody or its antigen-binding fragment, a linker peptide, and a CD86 mutant polypeptide, wherein the linker peptides can be the same or different and can exist or not exist independently of each other.

For example, the fusion polypeptide can comprise an amino acid sequence selected from the group consisting of SEQ ID NO: 350 to SEQ ID NO: 382, SEQ ID NO: 384 to SEQ ID NO: 416, SEQ ID NO: 418 to SEQ ID NO: 450, SEQ ID NO: 451 to SEQ ID NO: 483, SEQ ID NO: 484 to SEQ ID NO: 615, SEQ ID NO: 618 to SEQ ID NO: 624 and SEQ ID NO: 626 to SEQ ID NO: 632.

Provided below is: an exemplary CD86 ECD mutant is respectively covalently linked to the N-terminus of the Fc domain of human immunoglobulin IgG4 to form a functional fusion polypeptide amino acid sequence: Q25I/F33L/H90I (SEQ ID NO: 350), Q25V/F33L/H90I (SEQ ID NO: 351), Q25I/F33V/H90I (SEQ ID NO: 352), Q25V/F33V/H90I (SEQ ID NO: 353), Q25I/F33L/H90V (SEQ ID NO: 354), Q25V/F33L/H90V ( SEQ IDNO: 355), Q25I/F33V/H90V (SEQ ID NO: 356), Q25V/F33V/H90V (SEQ ID NO: 357), A13L/Q25I/F33L/H90I (SEQ ID NO: 358), A13I/Q25I/F33L/H90I (SEQ ID NO: 359), A13L/Q25V/F33L/H90I (SEQ ID NO: 360), A13I/Q25V/F33L/H90I (SEQ ID NO: 361), Q25I/F33L/I89L/H90I (SEQ ID NO: 362), Q25I/F33L/M60R/H90I (SEQ ID NO: 363), Q25V/F33L/I89L/H90I (SEQ ID NO: 364), Q25V/F33L/M60R/H90I (SEQ ID NO: 365), Q25F/F33L/H90I (SEQ ID NO: 366), Q25I/F33L/H90F (SEQ ID NO: 367), Q25I/F33A/H90I (SEQ ID NO: 368), Q25I/H90I (SEQ ID NO: 369), Q25V/H90V (SEQ ID NO: 370), Q25V/H90I (SEQ ID NO: 371), Q25F/H90I (SEQ ID NO: 372), Q25F/H90V (SEQ ID NO: 373), A13F/Q25I/F33L/H90I (SEQ ID NO: 374), A13M/Q25I/F33L/H90I (SEQ ID NO: 375), Q25A/F33L/H90I (SEQ ID NO: 376), Q25M/F33L/H90I (SEQ ID NO: 377), Q25I/F33M/H90I (SEQ ID NO: 378), Q25I/F33L/I89V/H90I (SEQ ID NO: 379), Q25I/F33L/I89F/H90I (SEQ ID NO: 380), Q25I/F33L/I89M/H90I (SEQ ID NO: 381), Q25I/F33L/H90M (SEQ ID NO: 382), and an unmutated CD86 ECD is covalently linked to the N-terminus of the Fc domain of human immunoglobulin IgG4 to form a functional fusion polypeptide amino acid sequence (SEQ ID NO:383 ).

Provided below is: an exemplary CD86 IgV mutant is respectively covalently linked to the N-terminus of the Fc domain of human immunoglobulin IgG4 to form a functional fusion polypeptide amino acid sequence: Q25I/F33L/H90I (SEQ ID NO: 384), Q25V/F33L/H90I (SEQ ID NO: 385), Q25I/F33V/H90I (SEQ ID NO: 386), Q25V/F33V/H90I (SEQ ID NO: 387), Q25I/F33L/H90V (SEQ ID NO: 388), Q25V/F33L/H90V (SEQ ID NO: 389), Q25I/F33V/H90V (SEQ ID NO: 390), Q25V/F33V/H90V (SEQ ID NO: 391), A13L/Q25I/F33L/H90I (SEQ ID NO: 392), A13I/Q25I/F33L/H90I (SEQ ID NO: 393), A13L/Q25V/F33L/H90I (SEQ ID NO: 394), A13I/Q25V/F33L/H90I (SEQ ID NO: 395), Q25I/F33L/I89L/H90I (SEQ ID NO: 396), Q25I/F33L/M60R/H90I (SEQ ID NO: 397), Q25V/F33L/I89L/H90I (SEQ ID NO: 398), Q25V/F33L/M60R/H90I (SEQ ID NO: 399), Q25F/F33L/H90I (SEQ ID NO: 400), Q25I/F33L/H90F (SEQ ID NO: 401), Q25I/F33A/H90I (SEQ ID NO: 402), Q25I/H90I (SEQ ID NO: 403), Q25V/H90V (SEQ ID NO: 404), Q25V/H90I (SEQ ID NO: 405), Q25F/H90I (SEQ ID NO: 406), Q25F/H90V (SEQ ID NO: 407), A13F/Q25I/F33L/H90I (SEQ ID NO: 408), A13M/Q25I/F33L/H90I (SEQ ID NO: 409), Q25A/F33L/H90I (SEQ ID NO: 410), Q25M/F33L/H90I (SEQ ID NO: 411), Q25I/F33M/H90I (SEQ ID NO: 412), Q25I/F33L/I89V/H90I (SEQ ID NO: 413), Q25I/F33L/I89F/H90I (SEQ ID NO: 414), Q25I/F33L/I89M/H90I (SEQ ID NO: 415), Q25I/F33L/H90M (SEQ ID NO: 416), and an unmutated CD86 IgV is covalently linked to the N-terminus of the Fc domain of human immunoglobulin IgG4 to form a functional fusion polypeptide amino acid sequence (SEQ ID NO:417 ).

Provided below is: an exemplary Sugemalimab antibody heavy chain C-terminus through a linker peptide is respectively covalently linked to CD86 ECD mutant to form the functional fusion polypeptide complex first polypeptide amino acid sequence: Q25I/F33L/H90I (SEQ ID NO: 418), Q25V/F33L/H90I (SEQ ID NO: 419), Q25I/F33V/H90I (SEQ ID NO: 420), Q25V/F33V/H90I (SEQ ID NO: 421), Q25I/F33L/H90V (SEQ ID NO: 422) , Q25V/F33L/H90V (SEQ ID NO: 423), Q25I/F33V/H90V (SEQ ID NO: 424), Q25V/F33V/H90V (SEQ ID NO: 425), A13L/Q25I/F33L/H90I (SEQ ID NO: 426), A13I/Q25I/F33L/H90I (SEQ ID NO: 427), A13L/Q25V/F33L/H90I (SEQ ID NO: 428), A13I/Q25V/F33L/H90I (SEQ ID NO: 429), Q25I/F33L/I89L/H90I (SEQ ID NO: 430), Q25I/F33L/M60R/H90I (SEQ ID NO: 431), Q25V/F33L/I89L/H90I (SEQ ID NO: 432), Q25V/F33L/M60R/H90I (SEQ ID NO: 433), Q25F/F33L/H90I (SEQ ID NO: 434), Q25I/F33L/H90F (SEQ ID NO: 435), Q25I/F33A/H90I (SEQ ID NO: 436), Q25I/H90I (SEQ ID NO: 437), Q25V/H90V(SEQ ID NO: 438), Q25V/H90I (SEQ ID NO: 439), Q25F/H90I (SEQ ID NO: 440), Q25F/H90V (SEQ ID NO: 441), A13F/Q25I/F33L/H90I (SEQ ID NO: 442), A13M/Q25I/F33L/H90I (SEQ ID NO: 443), Q25A/F33L/H90I (SEQ ID NO: 444), Q25M/F33L/H90I (SEQ ID NO: 445), Q25I/F33M/H90I (SEQ ID NO: 446), Q25I/F33L/I89V/H90I (SEQ ID NO: 447), Q25I/F33L/I89F/H90I (SEQ ID NO: 448), Q25I/F33L/I89M/H90I (SEQ ID NO: 449), Q25I/F33L/H90M (SEQ ID NO: 450), and sugemalimab antibody heavy chain C-terminus through a linker peptide is respectively covalently linked to a unmutated CD86 ECD to form the functional fusion polypeptide complex first polypeptideamino acid sequence (SEQ ID NO: 36); the above first polypeptide is respectively assembled with Sugemalimab antibody light chain second polypeptide (SEQ ID NO: 5) to form the functional fusion polypeptide complex.

Provided below is: an exemplary CD86 ECD mutant is respectively through a linker peptide covalently linked to Sugemalimab antibody heavy chain N-terminus to form the functional fusion polypeptide complex first polypeptide amino acid sequence: Q25I/F33L/H90I (SEQ ID NO: 451), Q25V/F33L/H90I (SEQ ID NO: 452), Q25I/F33V/H90I (SEQ ID NO: 453), Q25V/F33V/H90I (SEQ ID NO: 454), Q25I/F33L/H90V (SEQ ID NO: 455), Q25V/F33L/H90V (SEQ ID NO: 456), Q25I/F33V/H90V (SEQ ID NO: 457), Q25V/F33V/H90V (SEQ ID NO: 458), A13L/Q25I/F33L/H90I (SEQ ID NO: 459), A13I/Q25I/F33L/H90I (SEQ ID NO: 460), A13L/Q25V/F33L/H90I (SEQ ID NO: 461), A13I/Q25V/F33L/H90I (SEQ ID NO: 462), Q25I/F33L/I89L/H90I (SEQ ID NO: 463), Q25I/F33L/M60R/H90I (SEQ ID NO: 464), Q25V/F33L/I89L/H90I (SEQ ID NO: 465), Q25V/F33L/M60R/H90I (SEQ ID NO: 466), Q25F/F33L/H90I (SEQ ID NO: 467), Q25I/F33L/H90F (SEQ ID NO: 468), Q25I/F33A/H90I (SEQ ID NO: 469), Q25I/H90I (SEQ ID NO: 470), Q25V/H90V (SEQ ID NO: 471), Q25V/H90I (SEQ ID NO: 472), Q25F/H90I (SEQ ID NO: 473), Q25F/H90V (SEQ ID NO: 474), A13F/Q25I/F33L/H90I (SEQ ID NO: 475), A13M/Q25I/F33L/H90I (SEQ ID NO: 476), Q25A/F33L/H90I (SEQ ID NO: 477), Q25M/F33L/H90I (SEQ ID NO: 478), Q25I/F33M/H90I (SEQ ID NO: 479), Q25I/F33L/I89V/H90I (SEQ ID NO: 480), Q25I/F33L/I89F/H90I (SEQ ID NO: 481), Q25I/F33L/I89M/H90I (SEQ ID NO: 482), Q25I/F33L/H90M (SEQ ID NO: 483), and unmutated CD86 ECD is through the linker peptide covalently linked to sugemalimab antibody heavy chain N-terminus to form the functional fusion polypeptide complex first polypeptide amino acid sequence (SEQ ID NO: 38); the above first polypeptide is respectively assembled with the Sugemalimab antibody light chain second polypeptide (SEQ ID NO: 5) to form the functional fusion polypeptide complex.

Provided below is an exemplary sugemalimab antibody heavy chain C-terminus through a linker peptide is respectively covalently linked to a CD86 IgV mutant to form the functional fusion polypeptide complex first polypeptide amino acid sequence: Q25I/F33L/H90I (SEQ ID NO: 484), Q25V/F33L/H90I (SEQ ID NO: 485), Q25I/F33V/H90I (SEQ ID NO: 486), Q25V/F33V/H90I (SEQ ID NO: 487), Q25I/F33L/H90V (SEQ ID NO: 488), Q25V/F33L/H90V (SEQ ID NO: 489), Q25I/F33V/H90V (SEQ ID NO: 490), Q25V/F33V/H90V (SEQ ID NO: 491), A13L/Q25I/F33L/H90I (SEQ ID NO: 492), A13I/Q25I/F33L/H90I (SEQ ID NO: 493), A13L/Q25V/F33L/H90I (SEQ ID NO: 494), A13I/Q25V/F33L/H90I (SEQ ID NO: 495), Q25I/F33L/I89L/H90I (SEQ ID NO: 496), Q25I/F33L/M60R/H90I (SEQ ID NO: 497), Q25V/F33L/I89L/H90I (SEQ ID NO: 498), Q25V/F33L/M60R/H90I (SEQ ID NO: 499), Q25F/F33L/H90I (SEQ ID NO: 500), Q25I/F33L/H90F (SEQ ID NO: 501), Q25I/F33A/H90I (SEQ ID NO: 502), Q25I/H90I (SEQ ID NO: 503), Q25V/H90V (SEQ ID NO: 504), Q25V/H90I (SEQ ID NO: 505), Q25F/H90I (SEQ ID NO: 506), Q25F/H90V (SEQ ID NO: 507), A13F/Q25I/F33L/H90I (SEQ ID NO: 508), A13M/Q25I/F33L/H90I (SEQ ID NO: 509), Q25A/F33L/H90I (SEQ ID NO: 510), Q25M/F33L/H90I (SEQ ID NO: 511), Q25I/F33M/H90I (SEQ ID NO: 512), Q25I/F33L/I89V/H90I (SEQ ID NO: 513), Q25I/F33L/I89F/H90I (SEQ ID NO: 514), Q25I/F33L/I89M/H90I (SEQ ID NO: 515, Q25I/F33L/H90M (SEQ ID NO: 516), and sugemalimab antibody heavy chain C-terminus through a linker peptide is respectively covalently linked to a unmutated CD86 IgV to form the functional fusion polypeptide complex first polypeptide amino acid sequence (SEQ ID NO: 37); the above first polypeptide is respectively assembled with Sugemalimab antibody light chain second polypeptide (SEQ ID NO: 5) to form the functional fusion polypeptide complex.

Provided below is an exemplary CD86 IgV mutant through a linker peptide is respectively covalently linked to sugemalimab antibody heavy chain N-terminus to form the functional fusion polypeptide complex first polypeptide amino acid sequence: Q25I/F33L/H90I (SEQ ID NO: 517), Q25V/F33L/H90I (SEQ ID NO: 518), Q25I/F33V/H90I (SEQ ID NO: 519), Q25V/F33V/H90I (SEQ ID NO: 520), Q25I/F33L/H90V (SEQ ID NO: 521), Q25V/F33L/H90V (SEQ ID NO: 522), Q25I/F33V/H90V (SEQ ID NO: 523), Q25V/F33V/H90V (SEQ ID NO: 524), A13L/Q25I/F33L/H90I (SEQ ID NO: 525), A13I/Q25I/F33L/H90I (SEQ ID NO: 526), A13L/Q25V/F33L/H90I (SEQ ID NO: 527), A13I/Q25V/F33L/H90I (SEQ ID NO: 528), Q25I/F33L/I89L/H90I (SEQ ID NO: 529), Q25I/F33L/M60R/H90I (SEQ ID NO: 530), Q25V/F33L/I89L/H90I (SEQ ID NO: 531), Q25V/F33L/M60R/H90I (SEQ ID NO: 532), Q25F/F33L/H90I (SEQ ID NO: 533), Q25I/F33L/H90F (SEQ ID NO: 534), Q25I/F33A/H90I (SEQ ID NO: 535), Q25I/H90I (SEQ ID NO: 536), Q25V/H90V (SEQ ID NO: 537), Q25V/H90I (SEQ ID NO: 538), Q25F/H90I (SEQ ID NO: 539), Q25F/H90V (SEQ ID NO: 540), A13F/Q25I/F33L/H90I (SEQ ID NO: 541), A13M/Q25I/F33L/H90I (SEQ ID NO: 542), Q25A/F33L/H90I (SEQ ID NO: 543), Q25M/F33L/H90I (SEQ ID NO: 544), Q25I/F33M/H90I (SEQ ID NO: 545), Q25I/F33L/I89V/H90I (SEQ ID NO: 546), Q25I/F33L/I89F/H90I (SEQ ID NO: 547), Q25I/F33L/I89M/H90I (SEQ ID NO: 548), Q25I/F33L/H90M (SEQ ID NO: 549), and unmutated CD86 IgV is through a linker peptide covalently linked to a sugemalimab antibody heavy chain N-terminus to form the functional fusion polypeptide complex first polypeptide amino acid sequence (SEQ ID NO: 39); the above first polypeptide is respectively assembled with the sugemalimab antibody light chain second polypeptide (SEQ ID NO: 5) to form the functional fusion polypeptide complex.

Provided below is: an exemplary Atezolizumab antibody heavy chain C-terminus is through a linker peptide respectively covalently linked to a CD86 IgV mutant to form the functional fusion polypeptide complex first polypeptideamino acid sequence: Q25I/F33L/H90I (SEQ ID NO: 550), Q25V/F33L/H90I (SEQ ID NO: 551), Q25I/F33V/H90I (SEQ ID NO: 552), Q25V/F33V/H90I (SEQ ID NO: 553), Q25I/F33L/H90V (SEQ ID NO: 554), Q25V/F33L/H90V (SEQ ID NO: 555), Q25I/F33V/H90V (SEQ ID NO: 556), Q25V/F33V/H90V (SEQ ID NO: 557), A13L/Q25I/F33L/H90I (SEQ ID NO: 558), A13I/Q25I/F33L/H90I (SEQ ID NO: 559), A13L/Q25V/F33L/H90I (SEQ ID NO: 560), A13I/Q25V/F33L/H90I (SEQ ID NO: 561), Q25I/F33L/I89L/H90I (SEQ ID NO: 562), Q25I/F33L/M60R/H90I (SEQ ID NO: 563), Q25V/F33L/I89L/H90I (SEQ ID NO: 564), Q25V/F33L/M60R/H90I (SEQ ID NO: 565), Q25F/F33L/H90I (SEQ ID NO: 566), Q25I/F33L/H90F (SEQ ID NO: 567), Q25I/F33A/H90I (SEQ ID NO: 568), Q25I/H90I (SEQ ID NO: 569), Q25V/H90V (SEQ ID NO: 570), Q25V/H90I (SEQ ID NO: 571), Q25F/H90I (SEQ ID NO: 572), Q25F/H90V (SEQ ID NO: 573), A13F/Q25I/F33L/H90I (SEQ ID NO: 574), A13M/Q25I/F33L/H90I (SEQ ID NO: 575), Q25A/F33L/H90I (SEQ ID NO: 576), Q25M/F33L/H90I (SEQ ID NO: 577), Q25I/F33M/H90I (SEQ ID NO: 578), Q25I/F33L/I89V/H90I (SEQ ID NO: 579), Q25I/F33L/I89F/H90I (SEQ ID NO: 580), Q25I/F33L/I89M/H90I (SEQ ID NO: 581), Q25I/F33L/H90M (SEQ ID NO: 582), and atezolizumab antibody heavy chain C-terminus is through a linker peptide respectively covalently linked to an unmutated CD86 IgV to form the functional fusion polypeptide complex first polypeptideamino acid sequence (SEQ ID NO: 53); the above first polypeptide is respectively assembled with atezolizumab antibody light chain second polypeptide (SEQ ID NO: 11) to form the functional fusion polypeptide complex.

Provided below is: an exemplary CD86 IgV mutant is through a linker peptide respectively covalently linked to atezolizumab antibody heavy chain N-terminus to form the functional fusion polypeptide complexfirst polypeptideamino acid sequence: Q25I/F33L/H90I (SEQ ID NO: 583), Q25V/F33L/H90I (SEQ ID NO: 584), Q25I/F33V/H90I (SEQ ID NO: 585), Q25V/F33V/H90I (SEQ ID NO: 586), Q25I/F33L/H90V (SEQ ID NO: 587), Q25V/F33L/H90V (SEQ ID NO: 588), Q25I/F33V/H90V (SEQ ID NO: 589), Q25V/F33V/H90V (SEQ ID NO: 590), A13L/Q25I/F33L/H90I (SEQ ID NO: 591), A13I/Q25I/F33L/H90I (SEQ ID NO: 592), A13L/Q25V/F33L/H90I (SEQ ID NO: 593), A13I/Q25V/F33L/H90I (SEQ ID NO: 594), Q25I/F33L/I89L/H90I (SEQ ID NO: 595), Q25I/F33L/M60R/H90I (SEQ ID NO: 596), Q25V/F33L/I89L/H90I (SEQ ID NO: 597), Q25V/F33L/M60R/H90I (SEQ ID NO: 598), Q25F/F33L/H90I (SEQ ID NO: 599), Q25I/F33L/H90F (SEQ ID NO: 600), Q25I/F33A/H90I (SEQ ID NO: 601), Q25I/H90I (SEQ ID NO: 602), Q25V/H90V (SEQ ID NO: 603), Q25V/H90I (SEQ ID NO: 604), Q25F/H90I (SEQ ID NO: 605), Q25F/H90V (SEQ ID NO: 606), A13F/Q25I/F33L/H90I (SEQ ID NO: 607), A13M/Q25I/F33L/H90I (SEQ ID NO: 608), Q25A/F33L/H90I (SEQ ID NO: 609), Q25M/F33L/H90I (SEQ ID NO: 610), Q25I/F33M/H90I (SEQ ID NO: 611), Q25I/F33L/I89V/H90I (SEQ ID NO: 612), Q25I/F33L/I89F/H90I (SEQ ID NO: 613), Q25I/F33L/I89M/H90I (SEQ ID NO: 614), Q25I/F33L/H90M (SEQ ID NO: 615), and unmutated CD86 IgV is through a linker peptide covalently linked to a atezolizumab antibody heavy chain N-terminus to form the functional fusion polypeptide complex first polypeptide amino acid sequence (SEQ ID NO: 63); the above first polypeptide is respectively assembled with the atezolizumab antibody light chain second polypeptide (SEQ ID NO: 11) to form the functional fusion polypeptide complex.

Provided below is: an exemplary CD3 antibody CD3B219 heavy chain C-terminus is through a linker peptide respectively covalently linked to a CD86 ECD mutant to form the functional fusion polypeptide complex first polypeptide amino acid sequence: Q25I/F33L/H90I (SEQ ID NO: 618), Q25V/F33L/H90I (SEQ ID NO: 619), Q25V/F33V/H90I (SEQ ID NO: 620), A13I/Q25I/F33L/H90I (SEQ ID NO: 621), Q25I/H90I (SEQ ID NO: 622), Q25V/H90V (SEQ ID NO: 623), Q25V/H90I (SEQ ID NO: 624), and an unmutated CD86 ECD (SEQ ID NO: 625); the above first polypeptides are respectively assembled with the CD3B219 antibody light chain second polypeptide (SEQ ID NO: 617) to form the functional fusion polypeptide complex.

Provided below is: an exemplary anti-CD3 antibody CD3B219 heavy chain C-terminus is covalently linked to a CD86 IgV mutant via linker peptide to form the functional fusion polypeptide complex first polypeptide amino acid sequence: Q25I/F33L/H90I (SEQ ID NO: 626), Q25V/F33L/H90I (SEQ ID NO: 627), Q25V/F33V/H90I (SEQ ID NO: 628), A13I/Q25I/F33L/H90I (SEQ ID NO: 629), Q25I/H90I (SEQ ID NO: 630), Q25V/H90V (SEQ ID NO: 631), Q25V/H90I (SEQ ID NO: 632), and an unmutated CD86 IgV (SEQ ID NO: 633); the above first polypeptides are respectively assembled with the CD3B219 antibody light chain second polypeptide (SEQ ID NO: 617) to form the functional fusion polypeptide complexes.

For example, the functional antibody Abs-300 is composed of a chimeric polypeptide Abs-(SEQ ID NO: 350) formed by covalently linking the CD86 ECD mutant Q25I/F33L/H90I to the N-terminus of IgG4 Fc, and the Abs-300 functional polypeptide complex is assembled by introducing a polynucleotide sequence (SEQ ID NO: 1025) vector expressing the Abs-300 polypeptide into a host cell for expression.

For example, the functional antibody Abs-301 is composed of a chimeric polypeptide Abs-302 (SEQ ID NO: 351) formed by covalently linking the CD86 ECD mutant Q25V/F33L/H90I to the N-terminus of IgG4 Fc, and the assembly into an Abs-functional polypeptide complex is achieved by introducing a polynucleotide sequence (SEQ ID NO: 1026) vector expressing the Abs-polypeptide into a host cell for expression.

For example, the functional antibody Abs-303 is composed of a chimeric polypeptide Abs-303 (SEQ ID NO: 352) formed by covalently linking the CD86 ECD mutant Q25I/F33V/H90I to the N-terminus of IgG4 Fc, and the assembly into the Abs-303 functional polypeptide complex is achieved by introducing a polynucleotide sequence (SEQ ID NO: 1027) vector expressing the Abs-303 polypeptide into a host cell for expression.

For example, the functional antibody Abs-304 is composed of a chimeric polypeptide Abs-304 (SEQ ID NO: 353) formed by covalently linking the CD86 ECD mutant Q25V/F33V/H90I to the N-terminus of IgG4 Fc, and the assembly into the Abs-304 functional polypeptide complex is achieved by introducing a polynucleotide sequence (SEQ ID NO: 1028) vector expressing the Abs-304 polypeptide into a host cell for expression.

For example, the functional antibody Abs-305 is composed of a chimeric polypeptide Abs-305 (SEQ ID NO: 354) formed by covalently linking the CD86 ECD mutant Q25I/F33L/H90V to the N-terminus of IgG4 Fc, and the assembly into the Abs-305 functional polypeptide complex is achieved by introducing a polynucleotide sequence (SEQ ID NO: 1029) vector expressing the Abs-305 polypeptide into a host cell for expression.

For example, the functional antibody Abs-306 is composed of a chimeric polypeptide Abs-306 (SEQ ID NO: 355) formed by covalently linking the CD86 ECD mutant Q25V/F33L/H90V to the N-terminus of IgG4 Fc, and the assembly into the Abs-306 functional polypeptide complex is achieved by introducing a polynucleotide sequence (SEQ ID NO: 1030) vector expressing the Abs-306 polypeptide into a host cell for expression.

For example, the functional antibody Abs-307 is composed of a chimeric polypeptide Abs-307 (SEQ ID NO: 356) formed by covalently linking the CD86 ECD mutant Q25I/F33V/H90V to the N-terminus of IgG4 Fc, and the assembly into the Abs-307 functional polypeptide complex is achieved by introducing a polynucleotide sequence (SEQ ID NO: 1031) vector expressing the Abs-307 polypeptide into a host cell for expression.

For example, the functional antibody Abs-308 is composed of a chimeric polypeptide Abs-308 (SEQ ID NO: 357) formed by covalently linking the CD86 ECD mutant Q25V/F33V/H90V to the N-terminus of IgG4 Fc, and the assembly into the Abs-308 functional polypeptide complex is achieved by introducing a polynucleotide sequence (SEQ ID NO: 1032) vector expressing the Abs-308 polypeptide into a host cell for expression.

For example, the functional antibody Abs-309 is composed of a chimeric polypeptide Abs-309 (SEQ ID NO: 358) formed by covalently linking the CD86 ECD mutant A13L/Q25I/F33L/H90I to the N-terminus of IgG4 Fc, and the Abs-309 functional polypeptide complex is assembled by introducing a polynucleotide sequence (SEQ ID NO: 1033) vector expressing the Abs-309 polypeptide into a host cell for expression.

For example, the functional antibody Abs-310 is composed of a chimeric polypeptide Abs-310 (SEQ ID NO: 359) formed by covalently linking the CD86 ECD mutant A13I/Q25I/F33L/H90I to the N-terminus of IgG4 Fc, and the Abs-310 functional polypeptide complex is assembled by introducing a polynucleotide sequence (SEQ ID NO: 1034) vector expressing the Abs-310 polypeptide into a host cell for expression.

For example, the functional antibody Abs-311 is composed of a chimeric polypeptide Abs-311 (SEQ ID NO: 360) formed by covalently linking the CD86 ECD mutant A13L/Q25V/F33L/H90I to the N-terminus of IgG4 Fc, and the assembly into the Abs-311 functional polypeptide complex is achieved by introducing a polynucleotide sequence (SEQ ID NO: 1035) vector expressing the Abs-311 polypeptide into a host cell for expression.

For example, the functional antibody Abs-312 is composed of a chimeric polypeptide Abs-312 (SEQ ID NO: 361) formed by covalently linking the CD86 ECD mutant A13I/Q25V/F33L/H90I to the N-terminus of IgG4 Fc, and the assembly into the Abs-312 functional polypeptide complex is achieved by introducing a polynucleotide sequence (SEQ ID NO: 1036) vector expressing the Abs-312 polypeptide into a host cell for expression.

For example, the functional antibody Abs-313 is composed of a chimeric polypeptide Abs-313 (SEQ ID NO: 362) formed by covalently linking the CD86 ECD mutant Q25I/F33L/I89L/H90I to the N-terminus of IgG4 Fc, and the assembly into the Abs-313 functional polypeptide complex is achieved by introducing a polynucleotide sequence (SEQ ID NO: 1037) vector expressing the Abs-313 polypeptide into host cells for expression.

For example, the functional antibody Abs-314 is composed of a chimeric polypeptide Abs-314 (SEQ ID NO: 363) formed by covalently linking the CD86 ECD mutant Q25I/F33L/M60R/H90I to the N-terminus of IgG4 Fc, and the assembly into the Abs-314 functional polypeptide complex is achieved by introducing a polynucleotide sequence (SEQ ID NO: 1038) vector expressing the Abs-314 polypeptide into host cells for expression.

For example, the functional antibody Abs-315 is composed of a chimeric polypeptide Abs-315 (SEQ ID NO: 364) formed by covalently linking the CD86 ECD mutant Q25V/F33L/I89L/H90I to the N-terminus of IgG4 Fc, and the assembly into the Abs-315 functional polypeptide complex is achieved by introducing a polynucleotide sequence (SEQ ID NO: 1039) vector expressing the Abs-315 polypeptide into host cells for expression.

For example, the functional antibody Abs-316 is composed of a chimeric polypeptide Abs-316 (SEQ ID NO: 365) formed by covalently linking the CD86 ECD mutant Q25V/F33L/M60R/H90I to the N-terminus of IgG4 Fc, and the assembly into the Abs-316 functional polypeptide complex is achieved by introducing a polynucleotide sequence (SEQ ID NO: 1040) vector expressing the Abs-316 polypeptide into host cells for expression.

For example, the functional antibody Abs-317 is composed of a chimeric polypeptide Abs-317 (SEQ ID NO: 366) formed by covalently linking the CD86 ECD mutant Q25F/F33L/H90I to the N-terminus of IgG4 Fc, and the assembly into the Abs-317 functional polypeptide complex is achieved by introducing a polynucleotide sequence (SEQ ID NO: 1041) vector expressing the Abs-317 polypeptide into a host cell for expression.

For example, the functional antibody Abs-318 is composed of a chimeric polypeptide Abs-318 (SEQ ID NO: 367) formed by covalently linking the CD86 ECD mutant Q25I/F33L/H90F to the N-terminus of IgG4 Fc, and the Abs-318 functional polypeptide complex is assembled by introducing a polynucleotide sequence (SEQ ID NO: 1042) vector expressing the Abs-318 polypeptide into a host cell for expression.

For example, the functional antibody Abs-319 is composed of a chimeric polypeptide Abs-319 (SEQ ID NO: 368) formed by covalently linking the CD86 ECD mutant Q25I/F33A/H90I to the N-terminus of IgG4 Fc, and the assembly into the Abs-319 functional polypeptide complex is achieved by introducing a polynucleotide sequence (SEQ ID NO: 1043) vector expressing the Abs-319 polypeptide into a host cell for expression.

For example, the functional antibody Abs-320 is composed of a chimeric polypeptide Abs-320 (SEQ ID NO: 369) formed by covalently linking the CD86 ECD mutant Q25I/H90I to the N-terminus of IgG4 Fc, and the assembly into the Abs-320 functional polypeptide complex is achieved by introducing a polynucleotide sequence (SEQ ID NO: 1044) vector expressing the Abs-320 polypeptide into a host cell for expression.

For example, the functional antibody Abs-321 is composed of a chimeric polypeptide Abs-321 (SEQ ID NO: 370) formed by covalently linking the CD86 ECD mutant Q25V/H90V to the N-terminus of IgG4 Fc, and the assembly into the Abs-321 functional polypeptide complex is achieved by introducing a polynucleotide sequence (SEQ ID NO: 1045) vector expressing the Abs-321 polypeptide into a host cell for expression.

For example, the functional antibody Abs-322 is composed of a chimeric polypeptide Abs-322 (SEQ ID NO: 371) formed by covalently linking the CD86 ECD mutant Q25V/H90I to the N-terminus of IgG4 Fc, and the assembly into the Abs-322 functional polypeptide complex is achieved by introducing a polynucleotide sequence (SEQ ID NO: 1046) vector expressing the Abs-322 polypeptide into a host cell for expression.

For example, the functional antibody Abs-323 is composed of a chimeric polypeptide Abs-323 (SEQ ID NO: 372) formed by covalently linking the CD86 ECD mutant Q25F/H90I to the N-terminus of IgG4 Fc, and the assembly into the Abs-323 functional polypeptide complex is achieved by introducing a polynucleotide sequence (SEQ ID NO: 1047) vector expressing the Abs-323 polypeptide into a host cell for expression.

For example, the functional antibody Abs-324 is composed of a chimeric polypeptide Abs-324 (SEQ ID NO: 373) formed by covalently linking the CD86 ECD mutant Q25F/H90V to the N-terminus of IgG4 Fc, and the assembly into the Abs-324 functional polypeptide complex is achieved by introducing a polynucleotide sequence (SEQ ID NO: 1048) vector expressing the Abs-324 polypeptide into a host cell for expression.

For example, the functional antibody Abs-325 is composed of a chimeric polypeptide Abs-325 (SEQ ID NO: 374) formed by covalently linking the CD86 ECD mutant A13F/Q25I/F33L/H90I to the N-terminus of IgG4 Fc, and the assembly into the Abs-325 functional polypeptide complex is achieved by introducing a polynucleotide sequence (SEQ ID NO: 1049) vector expressing the Abs-325 polypeptide into a host cell for expression.

For example, the functional antibody Abs-326 is composed of a chimeric polypeptide Abs-326 (SEQ ID NO: 375) formed by covalently linking the CD86 ECD mutant A13M/Q25I/F33L/H90I to the N-terminus of IgG4 Fc, and the assembly into the Abs-326 functional polypeptide complex is achieved by introducing a polynucleotide sequence (SEQ ID NO: 1050) vector expressing the Abs-326 polypeptide into host cells for expression.

For example, the functional antibody Abs-327 is composed of a chimeric polypeptide Abs-327 (SEQ ID NO: 376) formed by covalently linking the CD86 ECD mutant Q25A/F33L/H90I to the N-terminus of IgG4 Fc, and the assembly into the Abs-327 functional polypeptide complex is achieved by introducing a polynucleotide sequence (SEQ ID NO: 1051) vector expressing the Abs-327 polypeptide into a host cell for expression.

For example, the functional antibody Abs-328 is composed of a chimeric polypeptide Abs-328 (SEQ ID NO: 377) formed by covalently linking the CD86 ECD mutant Q25M/F33L/H90I to the N-terminus of IgG4 Fc, and the assembly into the Abs-328 functional polypeptide complex is achieved by introducing a polynucleotide sequence (SEQ ID NO: 1052) vector expressing the Abs-328 polypeptide into a host cell for expression.

For example, the functional antibody Abs-329 is composed of a chimeric polypeptide Abs-329 (SEQ ID NO: 378) formed by covalently linking the CD86 ECD mutant Q25I/F33M/H90I to the N-terminus of IgG4 Fc, and the assembly into the Abs-329 functional polypeptide complex is achieved by introducing a polynucleotide sequence (SEQ ID NO: 1053) vector expressing the Abs-329 polypeptide into a host cell for expression.

For example, the functional antibody Abs-330 is composed of a chimeric polypeptide Abs-330 (SEQ ID NO: 379) formed by covalently linking the CD86 ECD mutant Q25I/F33L/I89V/H90I to the N-terminus of IgG4 Fc, and the assembly into the Abs-330 functional polypeptide complex is achieved by introducing a polynucleotide sequence (SEQ ID NO: 1054) vector expressing the Abs-330 polypeptide into host cells for expression.

For example, the functional antibody Abs-331 is composed of a chimeric polypeptide Abs-331 (SEQ ID NO: 380) formed by covalently linking the CD86 ECD mutant Q25I/F33L/I89F/H90I to the N-terminus of IgG4 Fc, and the assembly into the Abs-331 functional polypeptide complex is achieved by introducing a polynucleotide sequence (SEQ ID NO: 1055) vector expressing the Abs-331 polypeptide into host cells for expression.

For example, the functional antibody Abs-332 is composed of a chimeric polypeptide Abs-332 (SEQ ID NO: 381) formed by covalently linking the CD86 ECD mutant Q25I/F33L/I89M/H90I to the N-terminus of IgG4 Fc, and the assembly into the Abs-332 functional polypeptide complex is achieved by introducing a polynucleotide sequence (SEQ ID NO: 1056) vector expressing the Abs-332 polypeptide into host cells for expression.

For example, the functional antibody Abs-133 is composed of a chimeric polypeptide Abs-133 (SEQ ID NO: 382) formed by covalently linking the CD86 ECD mutant Q25I/F33L/H90M to the N-terminus of IgG4 Fc, and the assembly into the Abs-133 functional polypeptide complex is achieved by introducing a polynucleotide sequence (SEQ ID NO: 1057) vector expressing the Abs-133 polypeptide into a host cell for expression.

For example, the functional antibody Abs-333 is composed of a chimeric polypeptide Abs-333 (SEQ ID NO: 383) formed by covalently linking an unmutated CD86 ECD to the N-terminus of IgG4 Fc, and the assembly into an Abs-333 functional polypeptide complex is achieved by introducing a polynucleotide sequence (SEQ ID NO: 1058) vector expressing the Abs-333 polypeptide into a host cell for expression.

For example, the functional antibody Abs-334 is composed of a chimeric polypeptide Abs-334 (SEQ ID NO: 384) formed by covalently linking the CD86 IgV mutant Q25I/F33L/H90I to the N-terminus of IgG4 Fc, and the assembly into the Abs-334 functional polypeptide complex is achieved by introducing a polynucleotide sequence (SEQ ID NO: 1059) vector expressing the Abs-334 polypeptide into a host cell for expression.

For example, the functional antibody Abs-335 is composed of a chimeric polypeptide Abs-335 (SEQ ID NO: 385) formed by covalently linking the CD86 IgV mutant Q25V/F33L/H90I to the N-terminus of IgG4 Fc, and the assembly into the Abs-335 functional polypeptide complex is achieved by introducing a polynucleotide sequence (SEQ ID NO: 1060) vector expressing the Abs-335 polypeptide into a host cell for expression.

For example, the functional antibody Abs-336 is composed of a chimeric polypeptide Abs-336 (SEQ ID NO: 386) formed by covalently linking the CD86 IgV mutant Q25I/F33V/H90I to the N-terminus of IgG4 Fc, and the assembly into the Abs-336 functional polypeptide complex is achieved by introducing a polynucleotide sequence (SEQ ID NO: 1061) vector expressing the Abs-336 polypeptide into a host cell for expression.

For example, the functional antibody Abs-337 is composed of a chimeric polypeptide Abs-337 (SEQ ID NO: 387) formed by covalently linking the CD86 IgV mutant Q25V/F33V/H90I to the N-terminus of IgG4 Fc, and the assembly into the Abs-337 functional polypeptide complex is achieved by introducing a polynucleotide sequence (SEQ ID NO: 1062) vector expressing the Abs-337 polypeptide into a host cell for expression.

For example, the functional antibody Abs-338 is composed of a chimeric polypeptide Abs-338 (SEQ ID NO: 388) formed by covalently linking the CD86 IgV mutant Q25I/F33L/H90V to the N-terminus of IgG4 Fc, and the assembly into the Abs-338 functional polypeptide complex is achieved by introducing a polynucleotide sequence (SEQ ID NO: 1063) vector expressing the Abs-338 polypeptide into a host cell for expression.

For example, the functional antibody Abs-339 is composed of a chimeric polypeptide Abs-339 (SEQ ID NO: 389) formed by covalently linking the CD86 IgV mutant Q25V/F33L/H90V to the N-terminus of IgG4 Fc, and the Abs-339 functional polypeptide complex is assembled by introducing a polynucleotide sequence (SEQ ID NO: 1064) vector expressing the Abs-339 polypeptide into a host cell for expression.

For example, the functional antibody Abs-340 is composed of a chimeric polypeptide Abs-340 (SEQ ID NO: 390) formed by covalently linking the CD86 IgV mutant Q25I/F33V/H90V to the N-terminus of IgG4 Fc, and the assembly into the Abs-340 functional polypeptide complex is achieved by introducing a polynucleotide sequence (SEQ ID NO: 1065) vector expressing the Abs-340 polypeptide into a host cell for expression.

For example, the functional antibody Abs-341 is composed of a chimeric polypeptide Abs-341 (SEQ ID NO: 391) formed by covalently linking the CD86 IgV mutant Q25V/F33V/H90V to the N-terminus of IgG4 Fc, and the assembly into the Abs-341 functional polypeptide complex is achieved by introducing a polynucleotide sequence (SEQ ID NO: 1066) vector expressing the Abs-341 polypeptide into a host cell for expression.

For example, the functional antibody Abs-342 is composed of a chimeric polypeptide Abs-342 (SEQ ID NO: 392) formed by covalently linking the CD86 IgV mutant A13L/Q25I/F33L/H90I to the N-terminus of IgG4 Fc, and the assembly into the Abs-342 functional polypeptide complex is achieved by introducing a polynucleotide sequence (SEQ ID NO: 1067) vector expressing the Abs-342 polypeptide into a host cell for expression.

For example, the functional antibody Abs-343 is composed of a chimeric polypeptide Abs-343 (SEQ ID NO: 393) formed by covalently linking the CD86 IgV mutant A13I/Q25I/F33L/H90I to the N-terminus of IgG4 Fc, and the assembly into the Abs-343 functional polypeptide complex is achieved by introducing a polynucleotide sequence (SEQ ID NO: 1068) vector expressing the Abs-343 polypeptide into a host cell for expression.

For example, the functional antibody Abs-344 is composed of a chimeric polypeptide Abs-344 (SEQ ID NO: 394) formed by covalently linking the CD86 IgV mutant A13L/Q25V/F33L/H90I to the N-terminus of IgG4 Fc, and the assembly into the Abs-344 functional polypeptide complex is achieved by introducing a polynucleotide sequence (SEQ ID NO: 1069) vector expressing the Abs-344 polypeptide into a host cell for expression.

For example, the functional antibody Abs-345 is composed of a chimeric polypeptide Abs-345 (SEQ ID NO: 395) formed by covalently linking the CD86 IgV mutant A13I/Q25V/F33L/H90I to the N-terminus of IgG4 Fc, and the assembly into the Abs-345 functional polypeptide complex is achieved by introducing a polynucleotide sequence (SEQ ID NO: 1070) vector expressing the Abs-345 polypeptide into a host cell for expression.

For example, the functional antibody Abs-346 is composed of a chimeric polypeptide Abs-346 (SEQ ID NO: 396) formed by covalently linking the CD86 IgV mutant Q25I/F33L/I89L/H90I to the N-terminus of IgG4 Fc, and the assembly into the Abs-346 functional polypeptide complex is achieved by introducing a polynucleotide sequence (SEQ ID NO: 1071) vector expressing the Abs-346 polypeptide into host cells for expression.

For example, the functional antibody Abs-347 is composed of a chimeric polypeptide Abs-347 (SEQ ID NO: 397) formed by covalently linking the CD86 IgV mutant Q25I/F33L/M60R/H90I to the N-terminus of IgG4 Fc, and the assembly into the Abs-347 functional polypeptide complex is achieved by introducing a polynucleotide sequence (SEQ ID NO: 1072) vector expressing the Abs-347 polypeptide into host cells for expression.

For example, the functional antibody Abs-348 is composed of a chimeric polypeptide Abs-348 (SEQ ID NO: 398) formed by covalently linking the CD86 IgV mutant Q25V/F33L/I89L/H90I to the N-terminus of IgG4 Fc, and the assembly into the Abs-348 functional polypeptide complex is achieved by introducing a polynucleotide sequence (SEQ ID NO: 1073) vector expressing the Abs-348 polypeptide into a host cell for expression.

For example, the functional antibody Abs-349 is composed of a chimeric polypeptide Abs-349 (SEQ ID NO: 399) formed by covalently linking the CD86 IgV mutant Q25V/F33L/M60R/H90I to the N-terminus of IgG4 Fc, and the assembly into the Abs-349 functional polypeptide complex is achieved by introducing a polynucleotide sequence (SEQ ID NO: 1074) vector expressing the Abs-349 polypeptide into a host cell for expression.

For example, the functional antibody Abs-350 is composed of a chimeric polypeptide Abs-350 (SEQ ID NO: 400) formed by covalently linking the CD86 IgV mutant Q25F/F33L/H90I to the N-terminus of IgG4 Fc, and the Abs-350 functional polypeptide complex is assembled by introducing a polynucleotide sequence (SEQ ID NO: 1075) vector expressing the Abs-350 polypeptide into a host cell for expression.

For example, the functional antibody Abs-351 is composed of a chimeric polypeptide Abs-351 (SEQ ID NO: 401) formed by covalently linking the CD86 IgV mutant Q25I/F33L/H90F to the N-terminus of IgG4 Fc, and the assembly into the Abs-351 functional polypeptide complex is achieved by introducing a polynucleotide sequence (SEQ ID NO: 1076) vector expressing the Abs-351 polypeptide into a host cell for expression.

For example, the functional antibody Abs-352 is composed of a chimeric polypeptide Abs-352 (SEQ ID NO: 402) formed by covalently linking the CD86 IgV mutant Q25I/F33A/H90I to the N-terminus of IgG4 Fc, and the assembly into the Abs-352 functional polypeptide complex is achieved by introducing a polynucleotide sequence (SEQ ID NO: 1077) vector expressing the Abs-352 polypeptide into a host cell for expression.

For example, the functional antibody Abs-353 is composed of a chimeric polypeptide Abs-353 (SEQ ID NO: 403) formed by covalently linking the CD86 IgV mutant Q25I/H90I to the N-terminus of IgG4 Fc, and the assembly into the Abs-353 functional polypeptide complex is achieved by introducing a polynucleotide sequence (SEQ ID NO: 1078) vector expressing the Abs-353 polypeptide into a host cell for expression.

For example, the functional antibody Abs-354 is composed of a chimeric polypeptide Abs-354 (SEQ ID NO: 404) formed by covalently linking the CD86 IgV mutant Q25V/H90V to the N-terminus of IgG4 Fc, and the assembly into the Abs-354 functional polypeptide complex is achieved by introducing a polynucleotide sequence (SEQ ID NO: 1079) vector expressing the Abs-354 polypeptide into a host cell for expression.

For example, the functional antibody Abs-355 is composed of a chimeric polypeptide Abs-(SEQ ID NO: 405) formed by covalently linking the CD86 IgV mutant Q25V/H90I to the N-terminus of IgG4 Fc, and the Abs-355 functional polypeptide complex is assembled by introducing a polynucleotide sequence (SEQ ID NO: 1080) vector expressing the Abs-355 polypeptide into a host cell for expression.

For example, the functional antibody Abs-356 is composed of a chimeric polypeptide Abs-356 (SEQ ID NO: 406) formed by covalently linking the CD86 IgV mutant Q25F/H90I to the N-terminus of IgG4 Fc, and the assembly into the Abs-356 functional polypeptide complex is achieved by introducing a polynucleotide sequence (SEQ ID NO: 1081) vector expressing the Abs-356 polypeptide into a host cell for expression.

For example, the functional antibody Abs-357 is composed of a chimeric polypeptide Abs-357 (SEQ ID NO: 407) formed by covalently linking the CD86 IgV mutant Q25F/H90V to the N-terminus of IgG4 Fc, and the assembly into the Abs-357 functional polypeptide complex is achieved by introducing a polynucleotide sequence (SEQ ID NO: 1082) vector expressing the Abs-357 polypeptide into a host cell for expression.

For example, the functional antibody Abs-358 is composed of a chimeric polypeptide Abs-358 (SEQ ID NO: 408) formed by covalently linking the CD86 IgV mutant A13F/Q25I/F33L/H90I to the N-terminus of IgG4 Fc, and the assembly into the Abs-358 functional polypeptide complex is achieved by introducing a polynucleotide sequence (SEQ ID NO: 1083) vector expressing the Abs-358 polypeptide into a host cell for expression.

For example, the functional antibody Abs-359 is composed of a chimeric polypeptide Abs-359 (SEQ ID NO: 409) formed by covalently linking the CD86 IgV mutant A13M/Q25I/F33L/H90I to the N-terminus of IgG4 Fc, and the Abs-359 functional polypeptide complex is assembled by introducing a polynucleotide sequence (SEQ ID NO: 1084) vector expressing the Abs-359 polypeptide into a host cell for expression.

For example, the functional antibody Abs-360 is composed of a chimeric polypeptide Abs-360 (SEQ ID NO: 410) formed by covalently linking the CD86 IgV mutant Q25A/F33L/H90I to the N-terminus of IgG4 Fc, and the Abs-360 functional polypeptide complex is assembled by introducing a polynucleotide sequence (SEQ ID NO: 1085) vector expressing the Abs-360 polypeptide into a host cell for expression.

For example, the functional antibody Abs-361 is composed of a chimeric polypeptide Abs-361 (SEQ ID NO: 411) formed by covalently linking the CD86 IgV mutant Q25M/F33L/H90I to the N-terminus of IgG4 Fc, and the assembly into the Abs-361 functional polypeptide complex is achieved by introducing a polynucleotide sequence (SEQ ID NO: 1086) vector expressing the Abs-361 polypeptide into a host cell for expression.

For example, the functional antibody Abs-362 is composed of a chimeric polypeptide Abs-362 (SEQ ID NO: 412) formed by covalently linking the CD86 IgV mutant Q25I/F33M/H90I to the N-terminus of IgG4 Fc, and the assembly into the Abs-362 functional polypeptide complex is achieved by introducing a polynucleotide sequence (SEQ ID NO: 1087) vector expressing the Abs-362 polypeptide into a host cell for expression.

For example, the functional antibody Abs-363 is composed of a chimeric polypeptide Abs-363 (SEQ ID NO: 413) formed by covalently linking the CD86 IgV mutant Q25I/F33L/I89V/H90I to the N-terminus of IgG4 Fc, and the assembly into the Abs-363 functional polypeptide complex is achieved by introducing a polynucleotide sequence (SEQ ID NO: 1088) vector expressing the Abs-363 polypeptide into host cells for expression.

For example, the functional antibody Abs-364 is composed of a chimeric polypeptide Abs-364 (SEQ ID NO: 414) formed by covalently linking the CD86 IgV mutant Q25I/F33L/I89F/H90I to the N-terminus of IgG4 Fc, and the assembly into the Abs-364 functional polypeptide complex is achieved by introducing a polynucleotide sequence (SEQ ID NO: 1089) vector expressing the Abs-364 polypeptide into host cells for expression.

For example, the functional antibody Abs-365 is composed of a chimeric polypeptide Abs-365 (SEQ ID NO: 415) formed by covalently linking the CD86 IgV mutant Q25I/F33L/I89M/H90I to the N-terminus of IgG4 Fc, and the assembly into the Abs-365 functional polypeptide complex is achieved by introducing a polynucleotide sequence (SEQ ID NO: 1090) vector expressing the Abs-365 polypeptide into host cells for expression.

For example, the functional antibody Abs-366 is composed of a chimeric polypeptide Abs-366 (SEQ ID NO: 416) formed by covalently linking the CD86 IgV mutant Q25I/F33L/H90M to the N-terminus of IgG4 Fc, and the assembly into the Abs-366 functional polypeptide complex is achieved by introducing a polynucleotide sequence (SEQ ID NO: 1091) vector expressing the Abs-366 polypeptide into a host cell for expression.

For example, the functional antibody Abs-367 is composed of a chimeric polypeptide Abs-367 (SEQ ID NO: 417) formed by covalently linking an unmutated CD86 IgV to the N-terminus of IgG4 Fc, and the assembly into an Abs-367 functional polypeptide complex is achieved by introducing a polynucleotide sequence (SEQ ID NO: 1092) vector expressing the Abs-367 polypeptide into a host cell for expression.

For example, the functional antibody Abs-500 comprises a chimeric multifunctional antibody first polypeptide (SEQ ID NO: 484) (formed by CD86 IgV mutant Q25I/F33L/H90I N-terminus connecting to a linker peptide (SEQ ID NO: 34) covalently linked to sugemalimab antibody heavy chain C-terminus). Abs-500 functional antibody is assembled by introducing a polynucleotide sequence (SEQ ID NO: 1093) vector expressing Abs-500first polypeptide and a polynucleotide sequence (SEQ ID NO: 1158) vector expressing Abs-500 second polypeptide into a host cell for expression.

For example, the functional antibody Abs-501 comprises a chimeric multifunctional antibody first polypeptide (SEQ ID NO: 485) (formed by CD86 IgV mutant Q25V/F33L/H90I N-terminus connecting to a linker peptide (SEQ ID NO: 34) covalently linked to sugemalimab antibody heavy chain C-terminus). Abs-501 functional antibody is assembled by introducing a polynucleotide sequence (SEQ ID NO: 1094) vector expressing Abs-501first polypeptide and a polynucleotide sequence (SEQ ID NO: 1160) vector expressing Abs-501 second polypeptide into a host cell for expression.

For example, the functional antibody Abs-502 comprises a chimeric multifunctional antibody first polypeptide (SEQ ID NO: 486) (formed by CD86 IgV mutant Q25I/F33V/H90I N-terminus connecting to a linker peptide (SEQ ID NO: 34) covalently linked to sugemalimab antibody heavy chain C-terminus). Abs-502 functional antibody is assembled by introducing a polynucleotide sequence (SEQ ID NO: 195) vector expressing Abs-502first polypeptide and a polynucleotide sequence (SEQ ID NO: 1160) vector expressing Abs-502 second polypeptide into a host cell for expression.

For example, the functional antibody Abs-503 comprises a chimeric multifunctional antibody first polypeptide (SEQ ID NO: 487) (formed by CD86 IgV mutant Q25V/F33V/H90I N-terminus connecting to a linker peptide (SEQ ID NO: 34) covalently linked to sugemalimab antibody heavy chain C-terminus). Abs-503 functional antibody is assembled by introducing a polynucleotide sequence (SEQ ID NO: 1096) vector expressing Abs-503first polypeptide and a polynucleotide sequence (SEQ ID NO: 1160) vector expressing Abs-503 second polypeptide into a host cell for expression.

For example, the functional antibody Abs-504 comprises a chimeric multifunctional antibody first polypeptide (SEQ ID NO: 488) (formed by CD86 IgV mutant Q25I/F33L/H90V N-terminus connecting to a linker peptide (SEQ ID NO: 34) covalently linked to sugemalimab antibody heavy chain C-terminus). Abs-504 functional antibody is assembled by introducing a polynucleotide sequence (SEQ ID NO: 1097) vector expressing Abs-504 first polypeptide and a polynucleotide sequence (SEQ ID NO: 1160) vector expressing Abs-504 second polypeptide into a host cell for expression.

For example, the functional antibody Abs-505 comprises a chimeric multifunctional antibody first polypeptide (SEQ ID NO: 489) (formed by CD86 IgV mutant Q25V/F33L/H90V N-terminus connecting to a linker peptide (SEQ ID NO: 34) covalently linked to a sugemalimab antibody heavy chain C-terminus). Abs-505 functional antibody is assembled by introducing a polynucleotide sequence (SEQ ID NO: 1098) vector expressing Abs-505 first polypeptide and a polynucleotide sequence (SEQ ID NO: 1160) vector expressing Abs-505 second polypeptide into a host cell for expression.

For example, the functional antibody Abs-506 comprises a chimeric multifunctional antibody first polypeptide (SEQ ID NO: 490) (formed by CD86 IgV mutant Q25I/F33V/H90V N-terminus connecting to a linker peptide (SEQ ID NO: 34) covalently linked to sugemalimab antibody heavy chain C-terminus). Abs-506 functional antibody is assembled by introducing a polynucleotide sequence (SEQ ID NO: 1099) vector expressing Abs-506 first polypeptide and a polynucleotide sequence (SEQ ID NO: 1160) vector expressing Abs-506 second polypeptide into a host cell for expression.

For example, the functional antibody Abs-507 comprises a chimeric multifunctional antibody first polypeptide (SEQ ID NO: 491) (formed by CD86 IgV mutant Q25V/F33V/H90V N-terminus connecting to a linker peptide (SEQ ID NO: 34) covalently linked to sugemalimab antibody heavy chain C-terminus). Abs-507 functional antibody is assembled by introducing a polynucleotide sequence (SEQ ID NO: 1100) vector expressing Abs-507 first polypeptide and a polynucleotide sequence (SEQ ID NO: 1160) vector expressing Abs-507 second polypeptide into a host cell for expression.

For example, the functional antibody Abs-508 comprises a chimeric multifunctional antibody first polypeptide (SEQ ID NO: 492) (formed by CD86 IgV mutant A13L/Q25I/F33L/H90I N-terminus connecting to a linker peptide (SEQ ID NO: 34) covalently linked to sugemalimab antibody heavy chain C-terminus). Abs-508 functional antibody is assembled by introducing a polynucleotide sequence (SEQ ID NO: 1101) vector expressing Abs-508 first polypeptide and a polynucleotide sequence (SEQ ID NO: 1160) vector expressing Abs-508 second polypeptide into a host cell for expression.

For example, the functional antibody Abs-509 comprises a chimeric multifunctional antibody first polypeptide (SEQ ID NO: 493) (formed by CD86 IgV mutant A13I/Q25I/F33L/H90I N-terminus connecting to a linker peptide (SEQ ID NO: 34) covalently linked to sugemalimab antibody heavy chain C-terminus). Abs-509 functional antibody is assembled by introducing a polynucleotide sequence (SEQ ID NO: 1102) vector expressing Abs-509 first polypeptide and a polynucleotide sequence (SEQ ID NO: 1160) vector expressing Abs-509 second polypeptide into a host cell for expression.

For example, the functional antibody Abs-510 comprises a chimeric multifunctional antibody first polypeptide (SEQ ID NO: 494) (formed by CD86 IgV mutant A13L/Q25V/F33L/H90I N-terminus connecting to a linker peptide (SEQ ID NO: 34) covalently linked to sugemalimab antibody heavy chain C-terminus). Abs-510 functional antibody is assembled by introducing a polynucleotide sequence (SEQ ID NO: 1103) vector expressing Abs-510 first polypeptide and a polynucleotide sequence (SEQ ID NO: 1160) vector expressing Abs-510 second polypeptide into a host cell for expression.

For example, the functional antibody Abs-511 comprises a chimeric multifunctional antibody first polypeptide (SEQ ID NO: 495) (formed by CD86 IgV mutant A13I/Q25V/F33L/H90I N-terminus connecting to a linker peptide (SEQ ID NO: 34) covalently linked to sugemalimab antibody heavy chain C-terminus). Abs-511 functional antibody is assembled by introducing a polynucleotide sequence (SEQ ID NO: 1104) vector expressing Abs-511 first polypeptide and a polynucleotide sequence (SEQ ID NO: 1160) vector expressing Abs-511 second polypeptide into a host cell for expression.

For example, the functional antibody Abs-512 comprises a chimeric multifunctional antibody first polypeptide (SEQ ID NO: 496) (formed by CD86 IgV mutant Q25I/F33L/I89L/H90I N-terminus connecting to a linker peptide (SEQ ID NO: 34) covalently linked to sugemalimab antibody heavy chain C-terminus). Abs-512 functional antibody is assembled by introducing a polynucleotide sequence (SEQ ID NO: 1105) vector expressing Abs-512 first polypeptide and a polynucleotide sequence (SEQ ID NO: 1160) vector expressing Abs-512 second polypeptide into a host cell for expression.

For example, the functional antibody Abs-513 comprises a chimeric multifunctional antibody first polypeptide (SEQ ID NO: 497) (formed by CD86 IgV mutant Q25I/F33L/M60R/H90I N-terminus connecting to a linker peptide (SEQ ID NO: 34) covalently linked to sugemalimab antibody heavy chain C-terminus). Abs-513 functional antibody is assembled by introducing a polynucleotide sequence (SEQ ID NO: 1106) vector expressing Abs-513first polypeptide and a polynucleotide sequence (SEQ ID NO: 1160) vector expressing Abs-513 second polypeptide into a host cell for expression.

For example, the functional antibody Abs-514 comprises a chimeric multifunctional antibody first polypeptide (SEQ ID NO: 498) (formed by CD86 IgV mutant Q25V/F33L/I89L/H90I N-terminus connecting to a linker peptide (SEQ ID NO: 34) covalently linked to sugemalimab antibody heavy chain C-terminus). Abs-514 functional antibody is assembled by introducing a polynucleotide sequence (SEQ ID NO: 1107) vector expressing Abs-514 first polypeptide and a polynucleotide sequence (SEQ ID NO: 1160) vector expressing Abs-514 second polypeptide into a host cell for expression.

For example, the functional antibody Abs-515 comprises a chimeric multifunctional antibody first polypeptide (SEQ ID NO: 499) (formed by CD86 IgV mutant Q25V/F33L/M60R/H90I N-terminus connecting to a linker peptide (SEQ ID NO: 34) covalently linked to sugemalimab antibody heavy chain C-terminus). Abs-515 functional antibody is assembled by introducing a polynucleotide sequence (SEQ ID NO: 1108) vector expressing Abs-515 first polypeptide and a polynucleotide sequence (SEQ ID NO: 1160) vector expressing Abs-515 second polypeptide into a host cell for expression.

For example, the functional antibody Abs-516 comprises a chimeric multifunctional antibody first polypeptide (SEQ ID NO: 500) (formed by CD86 IgV mutant Q25F/F33L/H90I N-terminus connecting to a linker peptide (SEQ ID NO: 34) covalently linked to sugemalimab antibody heavy chain C-terminus). Abs-516 functional antibody is assembled by introducing a polynucleotide sequence (SEQ ID NO: 1109) vector expressing Abs-516 first polypeptide and a polynucleotide sequence (SEQ ID NO: 1160) vector expressing Abs-516 second polypeptide into a host cell for expression.

For example, the functional antibody Abs-517 comprises a chimeric multifunctional antibody first polypeptide (SEQ ID NO: 501) (formed by CD86 IgV mutant Q25I/F33L/H90F N-terminus connecting to a linker peptide (SEQ ID NO: 34) covalently linked to sugemalimab antibody heavy chain C-terminus). Abs-517 functional antibody is assembled by introducing a polynucleotide sequence (SEQ ID NO: 1110) vector expressing Abs-517 first polypeptide and a polynucleotide sequence (SEQ ID NO: 1160) vector expressing Abs-517 second polypeptide into a host cell for expression.

For example, the functional antibody Abs-518 comprises a chimeric multifunctional antibody first polypeptide (SEQ ID NO: 502) (formed by CD86 IgV mutant Q25I/F33A/H90I N-terminus connecting to a linker peptide (SEQ ID NO: 34) covalently linked to sugemalimab antibody heavy chain C-terminus). Abs-518 functional antibody is assembled by introducing a polynucleotide sequence (SEQ ID NO: 1111) vector expressing Abs-518first polypeptide and a polynucleotide sequence (SEQ ID NO: 1160) vector expressing Abs-518 second polypeptide into a host cell for expression.

For example, the functional antibody Abs-519 comprises a chimeric multifunctional antibody first polypeptide (SEQ ID NO: 503) (formed by CD86 IgV mutant Q25I/H90I N-terminus connecting to a linker peptide (SEQ ID NO: 34) covalently linked to sugemalimab antibody heavy chain C-terminus). Abs-519 functional antibody is assembled by introducing a polynucleotide sequence (SEQ ID NO: 1112) vector expressing Abs-519first polypeptide and a polynucleotide sequence (SEQ ID NO: 1160) vector expressing Abs-519 second polypeptide into a host cell for expression.

For example, the functional antibody Abs-520 comprises a chimeric multifunctional antibody first polypeptide (SEQ ID NO: 504) (formed by CD86 IgV mutant Q25V/H90V N-terminus connecting to a linker peptide (SEQ ID NO: 34) covalently linked to sugemalimab antibody heavy chain C-terminus). Abs-520 functional antibody is assembled by introducing a polynucleotide sequence (SEQ ID NO: 1113) vector expressing Abs-520 first polypeptide and a polynucleotide sequence (SEQ ID NO: 1160) vector expressing Abs-520 second polypeptide into a host cell for expression.

For example, the functional antibody Abs-521 comprises a chimeric multifunctional antibody first polypeptide (SEQ ID NO: 505) (formed by CD86 IgV mutant Q25V/H90I N-terminus connecting to a linker peptide (SEQ ID NO: 34) covalently linked to sugemalimab antibody heavy chain C-terminus). Abs-521 functional antibody is assembled by introducing a polynucleotide sequence (SEQ ID NO: 1114) vector expressing Abs-521first polypeptide and a polynucleotide sequence (SEQ ID NO: 1160) vector expressing Abs-521 second polypeptide into a host cell for expression.

For example, the functional antibody Abs-522 comprises a chimeric multifunctional antibody first polypeptide (SEQ ID NO: 506) (formed by CD86 IgV mutant Q25F/H90I N-terminus connecting to a linker peptide (SEQ ID NO: 34) covalently linked to sugemalimab antibody heavy chain C-terminus). Abs-522 functional antibody is assembled by introducing a polynucleotide sequence (SEQ ID NO: 1115) vector expressing Abs-522 first polypeptide and a polynucleotide sequence (SEQ ID NO: 1160) vector expressing Abs-522 second polypeptide into a host cell for expression.

For example, the functional antibody Abs-523 comprises a chimeric multifunctional antibody first polypeptide (SEQ ID NO: 507) (formed by CD86 IgV mutant Q25F/H90V N-terminus connecting to a linker peptide (SEQ ID NO: 34) covalently linked to sugemalimab antibody heavy chain C-terminus). Abs-523 functional antibody is assembled by introducing a polynucleotide sequence (SEQ ID NO: 1160) vector expressing Abs-523first polypeptide and a polynucleotide sequence (SEQ ID NO: 1244) vector expressing Abs-523 second polypeptide into a host cell for expression.

For example, the functional antibody Abs-524 comprises a chimeric multifunctional antibody first polypeptide (SEQ ID NO: 508) (formed by CD86 IgV mutant A13F/Q25I/F33L/H90I N-terminus connecting to a linker peptide (SEQ ID NO: 34) covalently linked to a sugemalimab antibody heavy chain C-terminus). Abs-524 functional antibody is assembled by introducing a polynucleotide sequence (SEQ ID NO: 1117) vector expressing Abs-524 first polypeptide and a polynucleotide sequence (SEQ ID NO: 1160) vector expressing Abs-524 second polypeptide into a host cell for expression.

For example, the functional antibody Abs-525 comprises a chimeric multifunctional antibody first polypeptide (SEQ ID NO: 509) (formed by CD86 IgV mutant A13M/Q25I/F33L/H90I N-terminus connecting to a linker peptide (SEQ ID NO: 34) covalently linked to sugemalimab antibody heavy chain C-terminus). Abs-525 functional antibody is assembled by introducing a polynucleotide sequence (SEQ ID NO: 1118) vector expressing Abs-525 first polypeptide and a polynucleotide sequence (SEQ ID NO: 1160) vector expressing Abs-525 second polypeptide into a host cell for expression.

For example, the functional antibody Abs-526 comprises a chimeric multifunctional antibody first polypeptide (SEQ ID NO: 510) (formed by CD86 IgV mutant Q25A/F33L/H90I N-terminus connecting to a linker peptide (SEQ ID NO: 34) covalently linked to sugemalimab antibody heavy chain C-terminus). Abs-526 functional antibody is assembled by introducing a polynucleotide sequence (SEQ ID NO: 1119) vector expressing Abs-526 first polypeptide and a polynucleotide sequence (SEQ ID NO: 1160) vector expressing Abs-526 second polypeptide into a host cell for expression.

For example, the functional antibody Abs-527 comprises a chimeric multifunctional antibody first polypeptide (SEQ ID NO: 511) (formed by CD86 IgV mutant Q25M/F33L/H90I N-terminus connecting to a linker peptide (SEQ ID NO: 34) covalently linked to sugemalimab antibody heavy chain C-terminus). Abs-527 functional antibody is assembled by introducing a polynucleotide sequence (SEQ ID NO: 1120) vector expressing Abs-527first polypeptide and a polynucleotide sequence (SEQ ID NO: 1160) vector expressing Abs-527 second polypeptide into a host cell for expression.

For example, the functional antibody Abs-528 comprises a chimeric multifunctional antibody first polypeptide (SEQ ID NO: 512) (formed by CD86 IgV mutant Q25I/F33M/H90I N-terminus connecting to a linker peptide (SEQ ID NO: 34) covalently linked to sugemalimab antibody heavy chain C-terminus). Abs-528 functional antibody is assembled by introducing a polynucleotide sequence (SEQ ID NO: 1121) vector expressing Abs-528 first polypeptide and a polynucleotide sequence (SEQ ID NO: 1160) vector expressing Abs-528 second polypeptide into a host cell for expression.

For example, the functional antibody Abs-529 comprises a chimeric multifunctional antibody first polypeptide (SEQ ID NO: 513) (formed by CD86 IgV mutant Q25I/F33L/I89V/H90I N-terminus connecting to a linker peptide (SEQ ID NO: 34) covalently linked to sugemalimab antibody heavy chain C-terminus). Abs-529 functional antibody is assembled by introducing a polynucleotide sequence (SEQ ID NO: 1122) vector expressing Abs-529first polypeptide and a polynucleotide sequence (SEQ ID NO: 1160) vector expressing Abs-529 second polypeptide into a host cell for expression.

For example, the functional antibody Abs-530 comprises a chimeric multifunctional antibody first polypeptide (SEQ ID NO: 514) (formed by CD86 IgV mutant Q25I/F33L/I89F/H90I N-terminus connecting to a linker peptide (SEQ ID NO: 34) covalently linked to sugemalimab antibody heavy chain C-terminus). Abs-530 functional antibody is assembled by introducing a polynucleotide sequence (SEQ ID NO: 1123) vector expressing Abs-530 first polypeptide and a polynucleotide sequence (SEQ ID NO: 1160) vector expressing Abs-530 second polypeptide into a host cell for expression.

For example, the functional antibody Abs-531 comprises a chimeric multifunctional antibody first polypeptide (SEQ ID NO: 515) (formed by CD86 IgV mutant Q25I/F33L/I89M/H90I N-terminus connecting to a linker peptide (SEQ ID NO: 34) covalently linked to sugemalimab antibody heavy chain C-terminus). Abs-531 functional antibody is assembled by introducing a polynucleotide sequence (SEQ ID NO: 1124) vector expressing Abs-531first polypeptide and a polynucleotide sequence (SEQ ID NO: 1160) vector expressing Abs-531 second polypeptide into a host cell for expression.

For example, the functional antibody Abs-532 comprises a chimeric multifunctional antibody first polypeptide (SEQ ID NO: 516) (formed by CD86 IgV mutant Q25I/F33L/H90M N-terminus connecting to a linker peptide (SEQ ID NO: 34) covalently linked to sugemalimab antibody heavy chain C-terminus). Abs-532 functional antibody is assembled by introducing a polynucleotide sequence (SEQ ID NO: 1125) vector expressing Abs-532first polypeptide and a polynucleotide sequence (SEQ ID NO: 1160) vector expressing Abs-532 second polypeptide into a host cell for expression. For example, the functional antibody Abs-533 comprises a chimeric multifunctional antibody first polypeptide (SEQ ID NO: 37) (formed by unmutated CD86 IgV N-terminus connecting to a linker peptide (SEQ ID NO: 34) covalently linked to 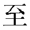 sugemalimab antibody heavy chain C-terminus). Abs-533 functional antibody is assembled by introducing a polynucleotide sequence (SEQ ID NO: 1126) vector expressing Abs-533first polypeptide and a polynucleotide sequence (SEQ ID NO: 1160) vector expressing Abs-533 second polypeptide into a host cell for expression.

For example, the functional antibody Abs-665 comprises a chimeric multifunctional antibody first polypeptide (SEQ ID NO: 517) formed by CD86 IgV mutant Q25I/F33L/H90I C-terminus connecting to linker peptide (SEQ ID NO: 34) covalently linked to a sugemalimab antibody heavy chain N-terminus. Abs-665 functional antibody is assembled by introducing a polynucleotide sequence (SEQ ID NO: 1127) vector expressing Abs-665first polypeptide and a polynucleotide sequence (SEQ ID NO: 1160) vector expressing Abs-665 second polypeptide into a host cell for expression.

For example, the functional antibody Abs-666 comprises a chimeric multifunctional antibody first polypeptide (SEQ ID NO: 518) formed by CD86 IgV mutant Q25V/F33L/H90I C terminus via a linker peptide (SEQ ID NO: 34) covalently linked to sugemalimab antibody heavy chain N-terminus. Abs-666 functional antibody is assembled by introducing a polynucleotide sequence (SEQ ID NO: 1128) vector expressing Abs-666first polypeptide and a polynucleotide sequence (SEQ ID NO: 1160) vector expressing Abs-666 second polypeptide into a host cell for expression.

For example, the functional antibody Abs-667 comprises a chimeric multifunctional antibody first polypeptide (SEQ ID NO: 519) formed by CD86 IgV mutant Q25I/F33V/H90I C terminus via a linker peptide (SEQ ID NO: 34) covalently linked to sugemalimab antibody heavy chain N-terminus. Abs-667 functional antibody is assembled by introducing a polynucleotide sequence (SEQ ID NO: 1129) vector expressing Abs-667first polypeptide and a polynucleotide sequence (SEQ ID NO: 1160) vector expressing Abs-667 second polypeptide into a host cell for expression.

For example, the functional antibody Abs-668 comprises a chimeric multifunctional antibody first polypeptide (SEQ ID NO: 520) formed by CD86 IgV mutant Q25V/F33V/H90I C terminus via a linker peptide (SEQ ID NO: 34) covalently linked to sugemalimab antibody heavy chain N-terminus. Abs-668 functional antibody is assembled by introducing a polynucleotide sequence (SEQ ID NO: 1130) vector expressing Abs-668first polypeptide and a polynucleotide sequence (SEQ ID NO: 1160) vector expressing Abs-668 second polypeptide into a host cell for expression.

For example, the functional antibody Abs-669 comprises a chimeric multifunctional antibody first polypeptide (SEQ ID NO: 521) formed by CD86 IgV mutant Q25I/F33L/H90V C terminus via a linker peptide (SEQ ID NO: 34) covalently linked to sugemalimab antibody heavy chain N-terminus. Abs-669 functional antibody is assembled by introducing a polynucleotide sequence (SEQ ID NO: 1131) vector expressing Abs-669first polypeptide and a polynucleotide sequence (SEQ ID NO: 1160) vector expressing Abs-669 second polypeptide into a host cell for expression.

For example, the functional antibody Abs-670 comprises a chimeric multifunctional antibody first polypeptide (SEQ ID NO: 522) formed by CD86 IgV mutant Q25V/F33L/H90V C terminus via a linker peptide (SEQ ID NO: 34) covalently linked to sugemalimab antibody heavy chain N-terminus. Abs-670 functional antibody is assembled by introducing a polynucleotide sequence (SEQ ID NO: 1132) vector expressing Abs-670first polypeptide and a polynucleotide sequence (SEQ ID NO: 1160) vector expressing Abs-670 second polypeptide into a host cell for expression.

For example, the functional antibody Abs-671 comprises a chimeric multifunctional antibody first polypeptide (SEQ ID NO: 523) formed by CD86 IgV mutant Q25I/F33V/H90V C terminus via a linker peptide (SEQ ID NO: 34) covalently linked to sugemalimab antibody heavy chain N-terminus. expressing Abs-671first polypeptideto form a polynucleotide sequence (SEQ ID NO: 1133) vector and expressing Abs-671 second polypeptide to form a polynucleotide sequence (SEQ ID NO: 1160) vector into a host cell for expression is assembled by introducing Abs-671 functional antibody.

For example, the functional antibody Abs-672 comprises chimeric multifunctional antibody first polypeptide (SEQ ID NO: 524) formed by CD86 IgV mutant Q25V/F33V/H90V C terminus via a linker peptide (SEQ ID NO: 34) covalently linked to sugemalimab antibody heavy chain N-terminus. Abs-672 functional antibody is assembled by introducing a polynucleotide sequence (SEQ ID NO: 1134) vector expressing Abs-672first polypeptide and a polynucleotide sequence (SEQ ID NO: 1160) vector expressing Abs-672 second polypeptide into a host cell for expression.

For example, the functional antibody Abs-673 comprises chimeric multifunctional antibody first polypeptide (SEQ ID NO: 536) formed by CD86 IgV mutant Q25I/H90I C terminus via a linker peptide (SEQ ID NO: 34) covalently linked to sugemalimab antibody heavy chain N-terminus. Abs-673 functional antibody is assembled by introducing a polynucleotide sequence (SEQ ID NO: 1135) vector expressing Abs-673 first polypeptide and a polynucleotide sequence (SEQ ID NO: 1160) vector expressing Abs-673 second polypeptide into a host cell for expression.

For example, the functional antibody Abs-674 comprises a chimeric multifunctional antibody first polypeptide (SEQ ID NO: 537) formed by CD86 IgV mutant Q25V/H90V C terminus via a linker peptide (SEQ ID NO: 34) covalently linked to sugemalimab antibody heavy chain N-terminus. Abs-674 functional antibody is assembled by introducing a polynucleotide sequence (SEQ ID NO: 1136) vector expressing Abs-674first polypeptide and a polynucleotide sequence (SEQ ID NO: 1160) vector expressing Abs-674 second polypeptide into a host cell for expression.

For example, the functional antibody Abs-675 comprises a chimeric multifunctional antibody first polypeptide (SEQ ID NO: 538) formed by CD86 IgV mutant Q25V/H90I C terminus via a linker peptide (SEQ ID NO: 34) covalently linked to sugemalimab antibody heavy chain N-terminus. Abs-675 functional antibody is assembled by introducing a polynucleotide sequence (SEQ ID NO: 1137) vector expressing Abs-675first polypeptide and a polynucleotide sequence (SEQ ID NO: 1244) vector expressing Abs-675 second polypeptide into a host cell for expression.

For example, the functional antibody Abs-676 comprises a chimeric multifunctional antibody first polypeptide (SEQ ID NO: 39) formed by unmutated CD86 IgV C terminus via a linker peptide (SEQ ID NO: 34) covalently linked to sugemalimab antibody heavy chain N-terminus. Abs-676 functional antibody is assembled by introducing a polynucleotide sequence (SEQ ID NO: 1138) vector expressing Abs-676first polypeptide and a polynucleotide sequence (SEQ ID NO: 1160) vector expressing Abs-676 second polypeptide into a host cell for expression.

For example, the functional antibody Abs-959 comprises a chimeric multifunctional antibody first polypeptide (SEQ ID NO: 626) formed by CD3 antibody CD3B219 heavy chain C-terminus via a linker peptide (SEQ ID NO: 34) covalently linked to CD86 IgV mutant Q25I/F33L/H90I. Abs-959 functional antibody is assembled by introducing a polynucleotide sequence (SEQ ID NO: 1155) vector expressing Abs-959first polypeptide and a polynucleotide sequence (SEQ ID NO: 1160) vector expressing Abs-959 second polypeptide into a host cell for expression.

For example, the functional antibody Abs-958 comprises a chimeric multifunctional antibody first polypeptide (SEQ ID NO: 633) formed by CD3 antibody CD3B219 heavy chain C-terminus via a linker peptide (SEQ ID NO: 34) covalently linked to unmutated CD86 IgV. Abs-958 functional antibody is assembled by introducing a polynucleotide sequence (SEQ ID NO: 1156) vector expressing Abs-958first polypeptide and a polynucleotide sequence (SEQ ID NO: 1160) vector expressing Abs-958 second polypeptide into a host cell for expression.

On the other hand, the present application provides a fusion polypeptide, which comprises a first domain, a second domain and a third domain, wherein the first domain may comprise a CD86 variant polypeptide, the second domain may comprise an antibody or an antigen-binding fragment thereof or other forms of polypeptides, and the third domain may comprise an antibody or an antigen-binding fragment thereof or a functional protein or an active fragment thereof that is the same as or different from the second domain.

For example, the first domain may comprise an amino acid substitution mutant of the IgV domain of human CD86.

For example, the first domain may comprise an amino acid substitution mutant of the IgV domain of human CD86 (SEQ ID NO: 3), and the amino acid substitution mutation site may comprise one or more of A13I, A13L, A13F, A13M, Q25A, Q25I, Q25V, Q25F, Q25M, F33A, F33L, F33V, F33M, M60R, I89A, I89L, I89V, I89F, I89M, H90I, H90V, H90M, and H90F. For example, the CD86 IgV mutant polypeptide includes a combination of 1, 2, 3, 4, 5 or more of the above-mentioned amino acid mutations.

For example, the first domain may comprise an amino acid substitution mutant of the IgV domain of CD86 (SEQ ID NO: 3), and the amino acid substitution mutant may be selected from the combination of amino acid mutation sites shown in the following group: Q25I/F33L/H90I, Q25V/F33L/H90I, Q25I/F33V/H90I, Q25V/F33V/H90I, Q25I/F33L/H90V, Q25V/F33L/H90V, Q25I/F33V/H90V, Q25V/F33V/H90V, A13L/Q25I/F33L/H90I, A13I/Q25I/F33L/H90I, A13L/Q25V/F33L/H90I, A13I/Q25V/F33L/H90I, Q25I/F33L/I89L/H90I, Q25I/F33L/M60R/H90I, Q25V/F33L/I89L/H90I, Q25V/F33L/M60R/H90I, Q25F/F33L/H90I, Q25I/F33L/H90F, Q25I/F33A/H90I, Q25I/H90I, Q25I, H90I, Q25V/H90V, Q25V/H90I, Q25F/H90I, Q25F/H90V, A13F/Q25I/F33L/H90I, A13M/Q25I/F33L/H90I, Q25A/F33L/H90I, Q25M/F33L/H90I, Q25I/F33M/H90I, Q25I/F33L/I89V/H90I, Q25I/F33L/I89F/H90I, Q25I/F33L/I89M/H90I and Q25I/F33L/H90M.

For example, the first domain may comprise the IgV domain amino acid substitution mutant Q25I/F33L/H90I of CD86.

For example, the first domain may comprise an amino acid substitution mutant of the extracellular domain of human CD86 (SEQ ID NO: 2).

For example, the first domain may comprise an amino acid substitution mutant of the extracellular domain of human CD86 (SEQ ID NO: 2), and the amino acid substitution mutant may comprise one or more of A13I, A13L, A13F, A13M, Q25A, Q25I, Q25V, Q25F, Q25M, F33A, F33L, F33V, F33M, M60R, I89A, I89L, I89V, I89F, I89M, H90I, H90V, H90M, and H90F. For example, the CD86 extracellular domain mutant polypeptide includes a combination of 1, 2, 3, 4, 5 or more of the above-mentioned amino acid mutations.

For example, the first domain may comprise an amino acid substitution mutant of the extracellular domain of human CD86 (SEQ ID NO: 2), and the amino acid substitution mutant of the extracellular domain of CD86 may be selected from the combination of amino acid mutation sites shown in the following group: Q25I/F33L/H90I, Q25V/F33L/H90I, Q25I/F33V/H90I, Q25V/F33V/H90I, Q25I/F33L/H90V, Q25V/F33L/H90V, Q25I/F33V/H90V, Q25V/F33V/H90V, A13L/Q25I/F33L/H90I, A13I/Q25I/F33L/H90I, A13L/Q25V/F33L/H90I, A13I/Q25V/F33L/H90I, Q25I/F33L/I89L/H90I, Q25I/F33L/M60R/H90I, Q25V/F33L/I89L/H90I, Q25V/F33L/M60R/H90I, Q25F/F33L/H90I, Q25I/F33L/H90F, Q25I/F33A/ H90I, Q25I/H90I, Q25I, H90I, Q25V/H90V, Q25V/H90I, Q25F/H901, Q25F/H90V, A13F/Q25I/F33L/H90I, A13M/Q25I/F33L/H90I, Q25A/F33L/H90I, Q25M/F33L/H90I, Q25I/F33M/H90I, Q25I/F33L/I89V/H90I, Q25I/F33L/I89F/H90I, Q25I/F33L/I89M/H90I and Q25I/F33L/H90M.

For example, the first domain may comprise the extracellular domain amino acid substitution mutant Q25I/F33L/H90I of a human CD86 variant polypeptide.

For example, the first domain may comprise an amino acid sequence selected from the group consisting of SEQ ID NO: 178 to SEQ ID NO: 243.

For example, the second domain may comprise an antibody or an antigen-binding fragment thereof.

For example, the second domain can bind to one or more of the targets shown in the following group: PD-L1, PD-L2, PD-1, OX40, 4-1BB, ICOS, TIGIT, CTLA4, LAG3, CD3, VEGF, VEGFR, CD47, HGF, Trop2, EpCAM, CCR8, CCR4, CCR5, GPRC5D, BCMA, CD19, CD20, HER-2 neu, DLL1, HER-3, HER-4, EGFR, PSMA, CEA, MUC-1 (mucin), MUC2, MUC3, MUC4, MUC5AC, MUC5B, MUC7, CD123, CD33, CD30, CD38, NKG2A, Nkp36 and Tim3.

For example, the second domain is capable of binding to PD-L1 and/or PD-1.

For example, the second domain may comprise HCDR3 of an antibody heavy chain, which may comprise an amino acid sequence selected from the group consisting of SEQ ID NO:4, SEQ ID NO:6, SEQ ID NO:8, SEQ ID NO:10, SEQ ID NO:12, SEQ ID NO:14, SEQ ID NO: 15 and SEQ ID NO: 17.

For example, the second domain may comprise HCDR2 of an antibody heavy chain, which may comprise an amino acid sequence selected from the group consisting of SEQ ID NO: SEQ ID NO:4, SEQ ID NO:6, SEQ ID NO:8, SEQ ID NO:10, SEQ ID NO:12, SEQ ID NO: 14, SEQ ID NO: 15 and SEQ ID NO: 17.

For example, the second domain may comprise HCDR1 of an antibody heavy chain, which may comprise an amino acid sequence selected from the group consisting of SEQ ID NO: SEQ ID NO:4, SEQ ID NO:6, SEQ ID NO:8, SEQ ID NO:10, SEQ ID NO:12, SEQ ID NO: 14, SEQ ID NO: 15 and SEQ ID NO: 17.

For example, the second domain may comprise a heavy chain variable region VH of an antibody heavy chain, and the antibody heavy chain variable region may comprise an amino acid sequence selected from the group consisting of SEQ ID NO:4, SEQ ID NO:6, SEQ ID NO:8, SEQ ID NO:10, SEQ ID NO:12, SEQ ID NO:14, SEQ ID NO:15 and SEQ ID NO:17.

For example, the second domain may comprise an antibody heavy chain, which may comprise an amino acid sequence selected from the group consisting of SEQ ID NO:4, SEQ ID NO:6, SEQ ID NO:8, SEQ ID NO:10, SEQ ID NO:12, SEQ ID NO:14, SEQ ID NO:15 and SEQ ID NO:17.

For example, the second domain may comprise LCDR3 of an antibody light chain, which may comprise an amino acid sequence selected from the group consisting of SEQ ID NO:5, SEQ ID NO:7, SEQ ID NO:9, SEQ ID NO:11, SEQ ID NO:13, SEQ ID NO:16 and SEQ ID NO:18.

For example, the second domain may comprise LCDR2 of an antibody light chain, which may comprise an amino acid sequence selected from the group consisting of SEQ ID NO:5, SEQ ID NO:7, SEQ ID NO:9, SEQ ID NO:11, SEQ ID NO:13, SEQ ID NO:16 and SEQ ID NO:18.

For example, the second domain may comprise LCDR1 of an antibody light chain, and the antibody light chain may comprise an amino acid sequence selected from the group consisting of SEQ ID NO:5, SEQ ID NO:7, SEQ ID NO:9, SEQ ID NO:11, SEQ ID NO:13, SEQ ID NO:16 and SEQ ID NO:18.

For example, the second domain may comprise a light chain variable region VL of an antibody light chain, and the antibody light chain variable region may comprise an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

For example, the second domain may comprise an antibody light chain, which may comprise an amino acid sequence selected from the group consisting of SEQ ID NO:5, SEQ ID NO:7, SEQ ID NO:9, SEQ ID NO:11, SEQ ID NO:13, SEQ ID NO:16 and SEQ ID NO:18.

For example, the second domain may comprise HCDR3, HCDR2 and HCDR1 of an antibody heavy chain, wherein the antibody heavy chain may comprise the amino acid sequence shown in SEQ ID NO:4. For example, the antibody can be Sugemalimab.

For example, the second domain may comprise LCDR3, LCDR2 and LCDR1 of an antibody light chain, wherein the antibody light chain may comprise the amino acid sequence shown in SEQ ID NO: 5. For example, the antibody can be Sugemalimab.

For example, the second domain may comprise HCDR3, HCDR2 and HCDR1 of an antibody heavy chain, wherein the antibody heavy chain may comprise the amino acid sequence shown in SEQ ID NO:4, and the second domain may comprise LCDR3, LCDR2 and LCDR1 of an antibody light chain, wherein the antibody light chain may comprise the amino acid sequence shown in SEQ ID NO:5. For example, the antibody can be Sugemalimab.

For example, the second domain may comprise the heavy chain variable region VH of the antibody heavy chain, and the antibody heavy chain may comprise the amino acid sequence shown in SEQ ID NO:4, and the second domain may comprise the light chain variable region VL of the antibody light chain, and the antibody light chain may comprise the amino acid sequence shown in SEQ ID NO:5. For example, the antibody can be Sugemalimab.

For example, the second domain may comprise HCDR3, HCDR2 and HCDR1 of an antibody heavy chain, wherein the antibody heavy chain may comprise the amino acid sequence shown in SEQ ID NO: 10. For example, the antibody can be Atezolizumab.

For example, the second domain may comprise LCDR3, LCDR2 and LCDR1 of an antibody light chain, wherein the antibody light chain may comprise the amino acid sequence shown in SEQ ID NO: 11. For example, the antibody can be Atezolizumab.

For example, the second domain may comprise HCDR3, HCDR2 and HCDR1 of an antibody heavy chain, wherein the antibody heavy chain may comprise the amino acid sequence shown in SEQ ID NO: 10, and the first domain may comprise LCDR3, LCDR2 and LCDR1 of an antibody light chain, wherein the antibody light chain may comprise the amino acid sequence shown in SEQ ID NO: 11. For example, the antibody can be Atezolizumab.

For example, the first domain may comprise a heavy chain variable region VH of an antibody heavy chain, and the antibody heavy chain may comprise an amino acid sequence as shown in SEQ ID NO: 10, and the first domain may comprise a light chain variable region VL of an antibody light chain, and the antibody light chain may comprise an amino acid sequence as shown in SEQ ID NO: 11. For example, the antibody can be Atezolizumab.

For example, the second domain may bind to CD3.

For example, the second domain may comprise a CD3B219 antibody or an antigen-binding fragment thereof.

For example, the second domain may comprise HCDR3, HCDR2 and HCDR1 of an antibody heavy chain, wherein the antibody heavy chain may comprise the amino acid sequence shown in SEQ ID NO:616. For example, the antibody may be a CD3B219 antibody.

For example, the second domain may comprise LCDR3, LCDR2 and LCDR1 of an antibody light chain, wherein the antibody light chain may comprise the amino acid sequence shown in SEQ ID NO:617. For example, the antibody may be a CD3B219 antibody.

For example, the second domain may comprise HCDR3, HCDR2 and HCDR1 of an antibody heavy chain, wherein the antibody heavy chain may comprise the amino acid sequence shown in SEQ ID NO:616, and the second domain may comprise LCDR3, LCDR2 and LCDR1 of an antibody light chain, wherein the antibody light chain may comprise the amino acid sequence shown in SEQ ID NO:617. For example, the antibody may be a CD3B219 antibody. For example, the second domain may comprise the heavy chain variable region VH of the antibody heavy chain, and the antibody heavy chain may comprise the amino acid sequence shown in SEQ ID NO:616, and the second domain may comprise the light chain variable region VL of the antibody light chain, and the antibody light chain may comprise the amino acid sequence shown in SEQ ID NO:617. For example, the antibody may be a CD3B219 antibody.

For example, the antibody can be selected from immunoglobulin antibodies, recombinant antibodies, chimeric antibodies, heavy chain antibodies, single domain antibodies and/or bispecific antibodies; the antigen-binding fragment can be selected from Fab, Fab', Fv, F(ab)2, F(ab')2, scFv, di-scFv, VHH and/or dAb.

For example, the second domain may comprise an immunoglobulin domain. For example, the second domain may comprise an immunoglobulin IgG antibody, and the immunoglobulin IgG antibody may comprise one or more selected from human IgG1, human IgG2, human IgG3, human IgG4, mouse IgG1, mouse IgG2a, mouse IgG2b and mouse IgG3.

For example, the second domain may comprise an immunoglobulin Fc domain. For example, the second domain may comprise the Fc domain of an immunoglobulin IgG antibody. For example, the second domain may comprise the Fc domain of an immunoglobulin IgG antibody, wherein the Fc domain of the immunoglobulin IgG antibody comprises a human IgG1 Fc domain, a human IgG2 Fc domain, a human IgG3 Fc domain, a human IgG4 Fc domain, a mouse IgG1 Fc domain, a mouse IgG2a Fc domain, a mouse IgG2b Fc domain or a mouse IgG3 Fc domain. For example, the second domain may be the Fc domain of human immunoglobulin IgG4.

For example, the second domain may comprise an amino acid sequence selected from the group consisting of SEQ ID NO: 4 to SEQ ID NO: 18, SEQ ID NO: 29 to SEQ ID NO: 30, SEQ ID NO: 634 to SEQ ID NO: 639, SEQ ID NO: 616 and SEQ ID NO: 617.

For example, the third domain may comprise a functional protein or an active fragment thereof, wherein the functional protein or the active fragment thereof is capable of activating an innate immune response.

For example, the third domain is capable of binding to an MHCII molecule on an antigen presenting cell.

For example, the third domain can be selected from: LAG3 or a functionally active fragment thereof derived from human and LAG3 or a functionally active fragment thereof derived from mouse.

For example, the third domain may comprise the extracellular domain of LAG3 or a functionally active fragment thereof.

For example, the third domain may comprise IgD1, IgD2, IgD3 and/or IgD4 of LAG3 or a functionally active fragment thereof.

For example, the third domain may comprise IgD1, IgD1-IgD2, IgD1-IgD2-IgD3, and/or IgD1-IgD2-IgD3-IgD4 of LAG3 or a functionally active fragment thereof.

For example, the third domain may comprise an amino acid sequence selected from the group consisting of SEQ ID NO: 20 to SEQ ID NO: 28.

For example, the third domain may comprise a LAG3 variant polypeptide.

For example, the third domain may comprise a LAG3 variant polypeptide, which may comprise a truncation and/or mutation based on human LAG3.

For example, the third domain may comprise a LAG3 variant polypeptide, which may have 0-124 amino acids truncated at the N-terminus based on a wild-type human LAG3 extracellular region polypeptide.

For example, the third domain may comprise a LAG3 variant polypeptide, and the LAG3 variant polypeptide may comprise a polypeptide starting from amino acids 23-146 of a wild-type human LAG3 extracellular region polypeptide.

For example, the third domain may comprise a LAG3 variant polypeptide, which may comprise 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117,118, 119, 120, 121, 122, 123 or 124 amino acids. For example, the third domain may comprise a LAG3 variant polypeptide, which may have 0, 5, 21, 37, 45, 60, 71, 74, 79, 84, 89, 94, 99, 104, 109 or 114 amino acids truncated at the N-terminus based on a wild-type human LAG3 extracellular region polypeptide. For example, the third domain may comprise a LAG3 variant polypeptide, which may have 0, 5, 21, 37, 45, 60, 71 or 74 amino acids truncated at the N-terminus based on a wild-type human LAG3 extracellular region polypeptide. For example, the third domain may comprise a LAG3 variant polypeptide, which may terminate at amino acid positions 122-167 at the C-terminus based on a wild-type human LAG3 extracellular region polypeptide.

For example, the third domain may comprise a LAG3 variant polypeptide, which may, based on a wild-type human LAG3 extracellular region polypeptide, terminate at any position corresponding to amino acid positions 122-167, 123-167, 124-167, 125-167, 126-167, 127-167, 128-167, 129-167, 130-167, 131-167, 132-167, 133-167, 134-167, 135-167, 136-167, 137-167, 138-167, 139-167, 140-167, 141-167, 142-167, 143-167, 144-167, 145-167, 146-167, 147-167, 148-167, 149-167, 150-167, 151-167, 152-167, 153-167, 154-167, 155-167, 156-167, 157-167, 158-167, 159-167, 160-167, 161-167, 162-167, 163-167, 164-167, 165-167 or 166-167 at C-terminus.

For example, the third domain may comprise a LAG3 variant polypeptide, which may, based on a wild-type human LAG3 extracellular region polypeptide, end at amino acid position 122, 129, 136, 146, 156, 157, 158, 159, 160, 161, 162, 163, 164, 165, 166 or 167 at the C-terminus.

For example, the third domain may comprise a LAG3 variant polypeptide, which may, based on a wild-type human LAG3 extracellular region polypeptide, terminate at amino acid position 122, 129, 136, 146, 156, 161 or 167 at the C-terminus.

For example, the third domain may comprise a LAG3 variant polypeptide, which may, based on a wild-type human LAG3 extracellular region polypeptide, terminate at amino acid position 156, 161 or 167 at the C-terminus.

For example, the third domain may comprise a LAG3 variant polypeptide, which may have 0, 5, 21, 37, 45, 60, 71 or 74 amino acids truncated at the N-terminus based on the wild-type human LAG3 extracellular region polypeptide, and terminate at amino acid position 122, 129, 136, 146, 156, 157, 158, 159, 160, 161, 162, 163, 164, 165, 166 or 167 at the C-terminus based on the wild-type human LAG3 extracellular region polypeptide.

For example, the third domain may comprise a LAG3 variant polypeptide, and the LAG3 variant polypeptide may comprise an amino acid mutation.

For example, the third domain may comprise a LAG3 variant polypeptide, and the LAG3 variant polypeptide may comprise an amino acid mutation, wherein the amino acid mutation site is an Arg amino acid at site 97.

For example, the third domain may comprise a LAG3 variant polypeptide, and the LAG3 variant polypeptide may comprise an amino acid mutation, wherein the amino acid mutation site is a mutation of the Arg amino acid at site 97 to a Glu amino acid.

For example, the third domain may comprise a LAG3 variant polypeptide, which may have 74 amino acids truncated at the N-terminus based on a wild-type human LAG3 extracellular region polypeptide. For example, the third domain may comprise an amino acid sequence selected from the group consisting of SEQ ID NO: 244 to SEQ ID NO: 259. For example, the third domain can be selected from the group consisting of the amino acid sequences shown in SEQ ID NO: 244, SEQ ID NO: 245, SEQ ID NO: 246, SEQ ID NO: 247, SEQ ID NO: 248 and SEQ ID NO: 253.

For example, the third domain may comprise a LAG3 variant polypeptide, which may, based on a wild-type human LAG3 extracellular region polypeptide, have 74 amino acids truncated at the N-terminus, and the Arg amino acid at position 97 is mutated to a Glu amino acid. For example, the third domain may comprise an amino acid sequence selected from the group consisting of SEQ ID NO: 260 to SEQ ID NO: 275. For example, the third domain can be selected from the group consisting of the amino acid sequences shown in SEQ ID NO:260, SEQ ID NO:261, SEQ ID NO:262, SEQ ID NO:263, SEQ ID NO:264 and SEQ ID NO:269.

For example, the third domain may comprise a LAG3 variant polypeptide, which may have 5 amino acids truncated at the N-terminus based on a wild-type human LAG3 extracellular region polypeptide. For example, the third domain may comprise an amino acid sequence selected from the group consisting of SEQ ID NO: 276 to SEQ ID NO: 278.

For example, the third domain may comprise a LAG3 variant polypeptide, which may, based on a wild-type human LAG3 extracellular region polypeptide, have 5 amino acids truncated at the N-terminus, and the Arg amino acid at position 97 is mutated to a Glu amino acid. The third domain may comprise an amino acid sequence selected from the group consisting of SEQ ID NO: 279 to SEQ ID NO: 281.

For example, the third domain may comprise a LAG3 variant polypeptide, which may, based on a wild-type human LAG3 extracellular region polypeptide, have 21 amino acids truncated at the N-terminus. For example, the third domain may comprise an amino acid sequence selected from the group consisting of SEQ ID NO: 282 to SEQ ID NO: 284.

For example, the third domain may comprise a LAG3 variant polypeptide, which may, based on a wild-type human LAG3 extracellular region polypeptide, have 21 amino acids truncated at the N-terminus, and the Arg amino acid at position 97 is mutated to a Glu amino acid. For example, the third domain may comprise an amino acid sequence selected from the group consisting of SEQ ID NO: 288 to SEQ ID NO: 290.

For example, the third domain may comprise a LAG3 variant polypeptide, which may have 37 amino acids turncated at the N-terminus based on a wild-type human LAG3 extracellular region polypeptide. For example, the third domain may comprise an amino acid sequence selected from the group consisting of SEQ ID NO: 276 to SEQ ID NO: 278.

For example, the third domain may comprise a LAG3 variant polypeptide, which may, based on a wild-type human LAG3 extracellular region polypeptide, chave 37 amino acids truncated at the N-terminus, and the Arg amino acid at position 97 is mutated to a Glu amino acid. For example, the third domain may comprise an amino acid sequence selected from the group consisting of SEQ ID NO: 291 to SEQ ID NO: 293.

For example, the third domain may comprise a LAG3 variant polypeptide, which may, based on a wild-type human LAG3 extracellular region polypeptide, chave 45 amino acids truncated at the N-terminus. For example, the third domain may comprise an amino acid sequence selected from the group consisting of SEQ ID NO: 294 to SEQ ID NO: 296.

For example, the third domain may comprise a LAG3 variant polypeptide, which may, based on a wild-type human LAG3 extracellular region polypeptide, have 45 amino acids truncated at the N-terminus, and the Arg amino acid at position 97 is mutated to a Glu amino acid. For example, the third domain may comprise an amino acid sequence selected from the group consisting of SEQ ID NO: 297 to SEQ ID NO: 299.

For example, the third domain may comprise a LAG3 variant polypeptide, which may, based on a wild-type human LAG3 extracellular region polypeptide, have 60 amino acids truncated at the N-terminus. For example, the third domain may comprise an amino acid sequence selected from the group consisting of SEQ ID NO: 300 to SEQ ID NO: 302.

For example, the third domain may comprise a LAG3 variant polypeptide, which may, based on a wild-type human LAG3 extracellular region polypeptide, have 60 amino acids truncated at the N-terminus, and the Arg amino acid at position 97 is mutated to a Glu amino acid. For example, the third domain may comprise an amino acid sequence selected from the group consisting of SEQ ID NO: 303 to SEQ ID NO: 305.

For example, the third domain may comprise a LAG3 variant polypeptide, which may, based on a wild-type human LAG3 extracellular region polypeptide, have 71 amino acids turncated at the N-terminus. For example, the third domain may comprise an amino acid sequence selected from the group consisting of SEQ ID NO: 306 to SEQ ID NO: 308.

For example, the third domain may comprise a LAG3 variant polypeptide, which may, based on a wild-type human LAG3 extracellular region polypeptide, have 71 amino acids truncated at the N-terminus, and the Arg amino acid at position 97 is mutated to a Glu amino acid. For example, the third domain may comprise an amino acid sequence selected from the group consisting of SEQ ID NO: 309 to SEQ ID NO: 311.

For example, the third domain may comprise a LAG3 variant polypeptide, which may, based on a wild-type human LAG3 extracellular region polypeptide, have 79 amino acids truncated at the N-terminus. For example, the third domain may comprise an amino acid sequence selected from the group consisting of SEQ ID NO: 312 to SEQ ID NO: 314.

For example, the third domain may comprise a LAG3 variant polypeptide, which may, based on a wild-type human LAG3 extracellular region polypeptide, have 79 amino acids truncated at the N-terminus, and the Arg amino acid at position 97 is mutated to a Glu amino acid. For example, the third domain may comprise an amino acid sequence selected from the group consisting of SEQ ID NO: 312 to SEQ ID NO: 314.

For example, the third domain may comprise a LAG3 variant polypeptide, which may, based on a wild-type human LAG3 extracellular region polypeptide, have 84 amino acids truncated at the N-terminus. For example, the third domain may comprise an amino acid sequence selected from the group consisting of SEQ ID NO: 315 to SEQ ID NO: 317.

For example, the third domain may comprise a LAG3 variant polypeptide, which may, based on a wild-type human LAG3 extracellular region polypeptide, have 84 amino acids truncated at the N-terminus, and the Arg amino acid at position 97 is mutated to a Glu amino acid.

For example, the third domain may comprise an amino acid sequence selected from the group consisting of SEQ ID NO: 315 to SEQ ID NO: 317.

For example, the third domain may comprise a LAG3 variant polypeptide, which may, based on a wild-type human LAG3 extracellular region polypeptide, have 89 amino acids truncated at the N-terminus. For example, the third domain may comprise an amino acid sequence selected from the group consisting of SEQ ID NO: 318 to SEQ ID NO: 320.

For example, the third domain may comprise a LAG3 variant polypeptide, which may, based on a wild-type human LAG3 extracellular region polypeptide, have 89 amino acids truncated at the N-terminus, and the Arg amino acid at position 97 is mutated to a Glu amino acid. For example, the third domain may comprise an amino acid sequence selected from the group consisting of SEQ ID NO: 318 to SEQ ID NO: 320.

For example, the third domain may comprise a LAG3 variant polypeptide, which may, based on a wild-type human LAG3 extracellular region polypeptide, have 94 amino acids truncated at the N-terminus. For example, the third domain may comprise an amino acid sequence selected from the group consisting of SEQ ID NO: 321 to SEQ ID NO: 323.

For example, the third domain may comprise a LAG3 variant polypeptide, which may, based on a wild-type human LAG3 extracellular region polypeptide, have 94 amino acids truncated at the N-terminus, and the Arg amino acid at position 97 is mutated to a Glu amino acid. For example, the third domain may comprise an amino acid sequence selected from the group consisting of SEQ ID NO: 321 to SEQ ID NO: 323.

For example, the third domain may comprise a LAG3 variant polypeptide, which may, based on a wild-type human LAG3 extracellular region polypeptide, have 99 amino acids truncated at the N-terminus. For example, the third domain may comprise an amino acid sequence selected from the group consisting of SEQ ID NO: 324 to SEQ ID NO: 326.

For example, the third domain may comprise a LAG3 variant polypeptide, which may, based on a wild-type human LAG3 extracellular region polypeptide, have 99 amino acids truncated at the N-terminus, and the Arg amino acid at position 97 is mutated to a Glu amino acid. For example, the third domain may comprise an amino acid sequence selected from the group consisting of SEQ ID NO: 324 to SEQ ID NO: 326.

For example, the third domain may comprise a LAG3 variant polypeptide, which may, based on a wild-type human LAG3 extracellular region polypeptide, have104 amino acids truncated at the N-terminus. For example, the third domain may comprise an amino acid sequence selected from the group consisting of SEQ ID NO: 327 to SEQ ID NO: 329.

For example, the third domain may comprise a LAG3 variant polypeptide, which may, based on a wild-type human LAG3 extracellular region polypeptide, have 104 amino acids truncated at the N-terminus, and the Arg amino acid at position 97 is mutated to a Glu amino acid. For example, the third domain may comprise an amino acid sequence selected from the group consisting of SEQ ID NO: 327 to SEQ ID NO: 329.

For example, the third domain may comprise a LAG3 variant polypeptide, which may, based on a wild-type human LAG3 extracellular region polypeptide, have 109 amino acids truncated at the N-terminus. For example, the third domain may comprise an amino acid sequence selected from the group consisting of SEQ ID NO: 330 to SEQ ID NO: 332.

For example, the third domain may comprise a LAG3 variant polypeptide, which may, based on a wild-type human LAG3 extracellular region polypeptide, have 109 amino acids truncated at the N-terminus, and the Arg amino acid at position 97 is mutated to a Glu amino acid. For example, the third domain may comprise an amino acid sequence selected from the group consisting of SEQ ID NO: 330 to SEQ ID NO: 332.

For example, the third domain may comprise a LAG3 variant polypeptide, which may, based on a wild-type human LAG3 extracellular region polypeptide, have 114 amino acids truncated at the N-terminus. For example, the third domain may comprise an amino acid sequence selected from the group consisting of SEQ ID NO: 333 to SEQ ID NO: 335.

For example, the third domain may comprise a LAG3 variant polypeptide, which may, based on a wild-type human LAG3 extracellular region polypeptide, have 114 amino acids truncated at the N-terminus, and the Arg amino acid at position 97 is mutated to a Glu amino acid. For example, the third domain may comprise an amino acid sequence selected from the group consisting of SEQ ID NO: 333 to SEQ ID NO: 335.

For example, the third domain may comprise a LAG3 variant polypeptide, which may, based on a wild-type human LAG3 extracellular region polypeptide, have 0 amino acids truncated at the N-terminus. For example, the third domain may comprise an amino acid sequence selected from the group consisting of SEQ ID NO: 336 to SEQ ID NO: 342.

For example, the third domain may comprise a LAG3 variant polypeptide, which may, based on a wild-type human LAG3 extracellular region polypeptide,have 0 amino acids truncated at the N-terminus, and the Arg amino acid at position 97 is mutated to a Glu amino acid. For example, the third domain may comprise an amino acid sequence selected from the group consisting of SEQ ID NO: 343 to SEQ ID NO: 349.

For example, the first domain may be directly or indirectly linked to the second domain.

For example, the first domain may be directly or indirectly connected to the C-terminus of the second domain.

For example, the first domain may be directly or indirectly connected to the N-terminus of the second domain.

For example, the second domain may comprise an antibody heavy chain, and the first domain may be directly or indirectly linked to the heavy chain of the second domain.

For example, the second domain may comprise an antibody heavy chain, and the first domain may be directly or indirectly linked to the N-terminus of the heavy chain of the second domain.

For example, the second domain may comprise an antibody heavy chain, and the first domain may be directly or indirectly linked to the C-terminus of the heavy chain of the second domain.

For example, the second domain may comprise an antibody light chain, and the first domain may be directly or indirectly linked to the light chain of the second domain.

For example, the second domain may comprise an antibody light chain, and the first domain may be directly or indirectly linked to the N-terminus of the light chain of the second domain.

For example, the second domain may comprise an antibody light chain, and the first domain may be directly or indirectly connected to the light chain C-terminus of the second domain.

For example, the first domain may be directly or indirectly linked to the third domain.

For example, the first domain may be directly or indirectly connected to the C-terminus of the third domain. For example, the first domain may be directly or indirectly connected to the N-terminus of the third domain.

For example, the second domain may be directly or indirectly linked to the third domain.

For example, the second domain may be directly or indirectly connected to the C-terminus of the third domain. For example, the second domain can be directly or indirectly connected to the N-terminus of the third domain.

For example, the second domain may comprise an antibody heavy chain, and the heavy chain of the second domain may be directly or indirectly linked to the N-terminus of the third domain.

For example, the second domain may comprise an antibody heavy chain, and the heavy chain of the second domain may be directly or indirectly linked to the C-terminus of the third domain.

For example, the second domain may comprise an antibody light chain, and the light chain of the second domain may be directly or indirectly linked to the N-terminus of the third domain.

For example, the second domain may comprise an antibody light chain, and the light chain of the second domain may be directly or indirectly linked to the C-terminus of the third domain.

For example, the first domain may be directly or indirectly linked to the second domain, and the first domain may be directly or indirectly linked to the third domain.

For example, the first domain may be directly or indirectly linked to the second domain, and the second domain may be directly or indirectly linked to the third domain.

For example, the first domain may be directly or indirectly linked to the third domain, and the second domain may be directly or indirectly linked to the third domain.

For example, the indirect connection comprises connection via a linker. For example, the linker comprises an amino acid sequence selected from the group consisting of SEQ ID NO:31, SEQ ID NO:32, SEQ ID NO:33, SEQ ID NO:34 and SEQ ID NO:35.

For example, the CD86 mutant fusion polypeptide complex includes a first polypeptide and a second polypeptide, and the second polypeptide may be present or absent.

In some embodiments, the second polypeptide comprises a light chain of a second domain, and the second binding domain light chain represents a light chain of an antibody or antigen-binding fragment thereof.

For example, the first polypeptide includes a second domain heavy chain, and the second binding domain heavy chain represents an Fc peptide chain or a heavy chain of an antibody or an antigen-binding fragment thereof.

For example, the first polypeptide is composed of a CD86 mutant polypeptide, a linker peptide, a second binding domain heavy chain, a linker peptide, and a third domain covalently connected in sequence from the N-terminus to the C-terminus, wherein the linker peptides may be the same or different, and may exist or not exist independently of each other.

For example, the first polypeptide is composed of a CD86 mutant polypeptide, a linker peptide, a second binding domain heavy chain, a linker peptide, and a third domain covalently connected in sequence from the N-terminus to the C-terminus, wherein the linker peptides may be the same or different, and may exist or not exist independently of each other.

For example, the first polypeptide is composed of a CD86 mutant polypeptide, a linker peptide, a second binding domain heavy chain, a linker peptide, and a third domain covalently connected in sequence from the N-terminus to the C-terminus, wherein the linker peptides may be the same or different, and may exist or not exist independently of each other.

For example, the first polypeptide is composed of a third domain, a linker peptide, a second binding domain heavy chain, a linker peptide, and a CD86 mutant polypeptide covalently connected in sequence from the N-terminus to the C-terminus, wherein the linker peptides may be the same or different, and may exist or not exist independently of each other.

For example, the first polypeptide is composed of a CD86 mutant polypeptide, a linker peptide, a third domain, a linker peptide, and a second binding domain heavy chain covalently connected in sequence from the N-terminus to the C-terminus, wherein the linker peptides may be the same or different, and may exist or not exist independently of each other.

For example, the first polypeptide is composed of a third domain, a linker peptide, a CD86 mutant polypeptide, a linker peptide, and a second binding domain heavy chain covalently connected in sequence from the N-terminus to the C-terminus, wherein the linker peptides may be the same or different, and may exist or not exist independently of each other.

For example, the first polypeptide is covalently connected from the N-terminus to the C-terminus by the second binding domain heavy chain, the linker peptide, the CD86 mutant polypeptide, the linker peptide, and the third domain, wherein the linker peptides may be the same or different and may exist or not exist independently.

For example, the first polypeptide is composed of a second binding domain heavy chain, a linker peptide, a third domain, a linker peptide, and a CD86 mutant polypeptide covalently connected in sequence from the N-terminus to the C-terminus, wherein the linker peptides may be the same or different, and may exist or not exist independently of each other.

For example, the second polypeptide is composed of a CD86 mutant polypeptide, a linker peptide, a light chain of an antibody or its antigen-binding fragment, a linker peptide, and a third domain covalently connected in sequence from the N-terminus to the C-terminus, wherein the linker peptides may be the same or different and may exist or not exist independently of each other.

For example, the first polypeptide is composed of a third domain, a linker peptide, a second binding domain light chain, a linker peptide, and a CD86 mutant polypeptide covalently connected in sequence from the N-terminus to the C-terminus, wherein the linker peptides may be the same or different, and may exist or not exist independently of each other.

For example, the first polypeptide is composed of a CD86 mutant polypeptide, a linker peptide, a third domain, a linker peptide, and a second binding domain light chain covalently connected in sequence from the N-terminus to the C-terminus, wherein the linker peptides may be the same or different, and may exist or not exist independently of each other.

For example, the first polypeptide is composed of a third domain, a linker peptide, a CD86 mutant polypeptide, a linker peptide, and a second binding domain light chain covalently connected in sequence from the N-terminus to the C-terminus, wherein the linker peptides may be the same or different, and may exist or not exist independently of each other.

For example, the first polypeptide is covalently connected from the N-terminus to the C-terminus by the second binding domain light chain, the linker peptide, the CD86 mutant polypeptide, the linker peptide, and the third domain, wherein the linker peptides may be the same or different and may exist or not exist independently.

For example, the first polypeptide is composed of a second binding domain light chain, a linker peptide, a third domain, a linker peptide, and a CD86 mutant polypeptide covalently connected in sequence from the N-terminus to the C-terminus, wherein the linker peptides may be the same or different, and may exist or not exist independently of each other.

For example, the fusion polypeptide may comprise an amino acid sequence selected from the group consisting of SEQ ID NO: 751 to SEQ ID NO: 1024, 1249, 1251, 1253 and 1255.

For example, the third domain may comprise human LAG-3 (SEQ ID NO: 125), an extracellular domain polypeptide thereof, an active fragment thereof, or a variant polypeptide thereof.

For example, the second domain can specifically target PD-L1, PD-L2 or PD-1.

For example, the second domain may be an anti-PD-L1 antibody or antigen-binding fragment, which may be Sugemalimab antibody or an antigen-binding fragment thereof.

For example, LAG3 mutant LAG3 97-156 polypeptide C-terminus connect to a linker peptide covalently linked to sugemalimab antibody heavy chain N-terminus, and sugemalimab antibody heavy chain C-terminus is via a linker peptide covalently linked to CD86 ECD mutant to form the functional fusion polypeptide complex first polypeptide containing amino acid sequence selected from SEQ ID NO: 753 to SEQ ID NO: 785. The first polypeptide is respectively assembled with sugemalimab antibody light chain second polypeptide (SEQ ID NO: 5) to form the functional fusion polypeptide complex.

For example, LAG3 mutant LAG3 R97E 97-156 polypeptide C-terminus connect to a linker peptide covalently linked to sugemalimab antibody heavy chain N-terminus, and sugemalimab antibody heavy chain C-terminus is via a linker peptide covalently linked to CD86 ECD mutant to form the functional fusion polypeptide complex first polypeptide containing amino acid sequence selected from SEQ ID NO: 787 to SEQ ID NO: 819. The first polypeptide is respectively assembled with sugemalimab antibody light chain second polypeptide (SEQ ID NO: 5) to form the functional fusion polypeptide complex.

For example, LAG3 mutant LAG3 97-156 polypeptide is covalently linked to sugemalimab antibody heavy chain N-terminus, and sugemalimab antibody heavy chain C-terminus is via a linker peptide covalently linked to CD86 IgV mutant to form the functional fusion polypeptide complex first polypeptide containing amino acid sequence selected from SEQ ID NO: 821 to SEQ ID NO: 853. The first polypeptide is respectively assembled with sugemalimab antibody light chain second polypeptide (SEQ ID NO: 5) to form the functional fusion polypeptide complex.

For example, LAG3 mutant LAG3 R97E 97-156 polypeptide C-terminus connect to a linker peptide covalently linked a sugemalimab antibody heavy chain N-terminus, and sugemalimab antibody heavy chain C-terminus is via a linker peptide covalently linked to CD86 IgV mutant to form the functional fusion polypeptide complex first polypeptide containing amino acid sequence selected from SEQ ID NO: 855 to SEQ ID NO: 887. The first polypeptide is respectively assembled with sugemalimab antibody light chain second polypeptide (SEQ ID NO: 5) to form the functional fusion polypeptide complex.

For example, LAG3 mutant LAG3 112-156 polypeptide C-terminus connect to a linker peptide covalently linked to sugemalimab antibody heavy chain N-terminus, and a sugemalimab antibody heavy chain C-terminus is via a linker peptide covalently linked to CD86 IgV mutant to form the functional fusion polypeptide complex first polypeptide containing amino acid sequence selected from SEQ ID NO: 957 to SEQ ID NO: 989. The first polypeptide is respectively assembled with sugemalimab antibody light chain second polypeptide (SEQ ID NO: 5) to form the functional fusion polypeptide complex.

For example, LAG3 mutant LAG3 122-156 polypeptide C-terminus connect to a linker peptide covalently linked to a sugemalimab antibody heavy chain N-terminus, and the sugemalimab antibody heavy chain C-terminus is through a linker peptide covalently linked to CD86 IgV mutant to form the functional fusion polypeptide complex first polypeptide containing amino acid sequence selected from SEQ ID NO: 991 to SEQ ID NO: 1023. The first polypeptide is assembled with the sugemalimab antibody light chain second polypeptide (SEQ ID NO: 5) to form the functional fusion polypeptide complex.

For example, the anti-PD-L1 antibody or antigen-binding fragment is atezolizumab antibody or its antigen-binding fragment.

For example, LAG3 mutant LAG3 97-156 polypeptide C-terminus connect to a linker peptide covalently linked to an atezolizumab antibody heavy chain N-terminus, and the atezolizumab antibody heavy chain C-terminus is through a linker peptide covalently linked to a CD86 IgV mutant to form the functional fusion polypeptide complex first polypeptide containing amino acid sequence selected from SEQ ID NO: 889 to SEQ ID NO: 921. The first polypeptide is respectively assembled with atezolizumab antibody light chain second polypeptide (SEQ ID NO: 11) to form the functional fusion polypeptide complex.

For example, LAG3 mutant LAG3 R97E 97-156 polypeptide C-terminus connect to a linker peptide covalently linked to an atezolizumab antibody heavy chain N-terminus, and an atezolizumab antibody heavy chain C-terminus is via a linker peptide covalently linked to a CD86 IgV mutant to form the functional fusion polypeptide complex first polypeptide containing amino acid sequence selected from SEQ ID NO: 923 to SEQ ID NO: 955. The first polypeptide is respectively assembled with atezolizumab antibody light chain second polypeptide (SEQ ID NO: 11) to form the functional fusion polypeptide complex.

For example, the functional antibody Abs-640 comprises a chimeric multifunctional antibody first polypeptide (SEQ ID NO: 821) (formed by LAG3 mutant LAG3 97-156 polypeptide C-terminus connecting linker peptide (SEQ ID NO: 34) covalently linked to sugemalimab antibody heavy chain N-terminus, and sugemalimab antibody heavy chain C-terminus connecting to linker peptide (SEQ ID NO: 34) covalently linked to CD86 IgV mutant Q25I/F33L/H90I). Abs-640 functional antibody is assembled by introducing a polynucleotide sequence (SEQ ID NO: 1163) vector expressing Abs-640 first polypeptide and a polynucleotide sequence (SEQ ID NO: 1158) vector expressing Abs-640 second polypeptide into a host cell for expression.

For example, the functional antibody Abs-641 comprises a chimeric multifunctional antibody first polypeptide (SEQ ID NO: 822) (formed by LAG3 mutant LAG3 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/F33L/H90I). Abs-641 functional antibody is assembled by introducing a polynucleotide sequence (SEQ ID NO: 1164) vector expressing Abs-641 first polypeptide and a polynucleotide sequence (SEQ ID NO: 1158) vector expressing Abs-641 second polypeptide into a host cell for expression.

For example, the functional antibody Abs-642 comprises a chimeric multifunctional antibody first polypeptide (SEQ ID NO: 823) (formed by LAG3 mutant LAG3 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25I/F33V/H90I). Abs-642 functional antibody is assembled by introducing a polynucleotide sequence (SEQ ID NO: 1165) vector expressing Abs-642first polypeptide and a polynucleotide sequence (SEQ ID NO: 1158) vector expressing Abs-642 second polypeptide into a host cell for expression.

For example, the functional antibody Abs-643 comprises a chimeric multifunctional antibody first polypeptide (SEQ ID NO: 824) (formed by LAG3 mutant LAG3 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/F33V/H90I). Abs-643 functional antibody is assembled by introducing a polynucleotide sequence (SEQ ID NO: 1166) vector expressing Abs-643first polypeptide and a polynucleotide sequence (SEQ ID NO: 1158) vector expressing Abs-643 second polypeptide into a host cell for expression.

For example, the functional antibody Abs-644 comprises a chimeric multifunctional antibody first polypeptide (SEQ ID NO: 825) (formed by LAG3 mutant LAG3 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25I/F33L/H90V). Abs-644 functional antibody is assembled by introducing a polynucleotide sequence (SEQ ID NO: 1167) vector expressing Abs-644 first polypeptide and a polynucleotide sequence (SEQ ID NO: 1158) vector expressing Abs-644 second polypeptide into a host cell for expression.

For example, the functional antibody Abs-645 comprises a chimeric multifunctional antibody first polypeptide (SEQ ID NO: 826) (formed by LAG3 mutant LAG3 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/F33L/H90V). Abs-645 functional antibody is assembled by introducing a polynucleotide sequence (SEQ ID NO: 1168) vector expressing Abs-645first polypeptide and a polynucleotide sequence (SEQ ID NO: 1158) vector expressing Abs-645 second polypeptide into a host cell for expression.

For example, the functional antibody Abs-646 comprises a chimeric multifunctional antibody first polypeptide (SEQ ID NO: 827) (formed by LAG3 mutant LAG3 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25I/F33V/H90V). expressing Abs-646first polypeptideto form a polynucleotide sequence (SEQ ID NO: 1169) vector and expressing Abs-646 second polypeptide to form a polynucleotide sequence (SEQ ID NO: 1158) vector into a host cell for expression is assembled by introducing Abs-646 functional antibody.

For example, the functional antibody Abs-647 comprises a chimeric multifunctional antibody first polypeptide (SEQ ID NO: 828) (formed by LAG3 mutant LAG3 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/F33V/H90V). expressing Abs-647first polypeptideto form a polynucleotide sequence (SEQ ID NO: 1170) vector and expressing Abs-647 second polypeptide to form a polynucleotide sequence (SEQ ID NO: 1158) vector into a host cell for expression is assembled by introducing Abs-647 functional antibody.

For example, the functional antibody Abs-648 comprises a chimeric multifunctional antibody first polypeptide (SEQ ID NO: 829) (formed by LAG3 mutant LAG3 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant A13L/Q25I/F33L/H90I). Abs-648 functional antibody is assembled by introducing a polynucleotide sequence (SEQ ID NO: 1171) vector expressing Abs-648first polypeptide and a polynucleotide sequence (SEQ ID NO: 1158) vector expressing Abs-648 second polypeptide into a host cell for expression.

For example, the functional antibody Abs-649 comprises a chimeric multifunctional antibody first polypeptide (SEQ ID NO: 830) (formed by LAG3 mutant LAG3 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant A13I/Q25I/F33L/H90I). Abs-649 functional antibody is assembled by introducing a polynucleotide sequence (SEQ ID NO: 1172) vector expressing Abs-649first polypeptide and a polynucleotide sequence (SEQ ID NO: 1158) vector expressing Abs-649 second polypeptide into a host cell for expression.

For example, the functional antibody Abs-650 comprises a chimeric multifunctional antibody first polypeptide (SEQ ID NO: 831) (formed by LAG3 mutant LAG3 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant A13L/Q25V/F33L/H90I). Abs-650 functional antibody is assembled by introducing a polynucleotide sequence (SEQ ID NO: 1173) vector expressing Abs-650first polypeptide and a polynucleotide sequence (SEQ ID NO: 1158) vector expressing Abs-650 second polypeptide into a host cell for expression.

For example, the functional antibody Abs-651 comprises a chimeric multifunctional antibody first polypeptide (SEQ ID NO: 832) (formed by LAG3 mutant LAG3 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant A13I/Q25V/F33L/H90I). Abs-651 functional antibody is assembled by introducing a polynucleotide sequence (SEQ ID NO: 1174) vector expressing Abs-651first polypeptide and a polynucleotide sequence (SEQ ID NO: 1158) vector expressing Abs-651 second polypeptide into a host cell for expression.

For example, the functional antibody Abs-652 comprises a chimeric multifunctional antibody first polypeptide (SEQ ID NO: 833) (formed by LAG3 mutant LAG3 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25I/F33L/I89L/H90I). Abs-652 functional antibody is assembled by introducing a polynucleotide sequence (SEQ ID NO: 1175) vector expressing Abs-652first polypeptide and a polynucleotide sequence (SEQ ID NO: 1158) vector expressing Abs-652 second polypeptide into a host cell for expression.

For example, the functional antibody Abs-653 comprises a chimeric multifunctional antibody first polypeptide (SEQ ID NO: 834) (formed by LAG3 mutant LAG3 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25I/F33L/M60R/H90I). Abs-653 functional antibody is assembled by introducing a polynucleotide sequence (SEQ ID NO: 1176) vector expressing Abs-653 first polypeptide and a polynucleotide sequence (SEQ ID NO: 1158) vector expressing Abs-653 second polypeptide into a host cell for expression.

For example, the functional antibody Abs-654 comprises a chimeric multifunctional antibody first polypeptide (SEQ ID NO: 835) (formed by LAG3 mutant LAG3 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/F33L/I89L/H90I). Abs-654 functional antibody is assembled by introducing a polynucleotide sequence (SEQ ID NO: 1177) vector expressing Abs-654first polypeptide and a polynucleotide sequence (SEQ ID NO: 1158) vector expressing Abs-654 second polypeptide into a host cell for expression.

For example, the functional antibody Abs-655 comprises a chimeric multifunctional antibody first polypeptide (SEQ ID NO: 836) (formed by LAG3 mutant LAG3 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/F33L/M60R/H90I). Abs-655 functional antibody is assembled by introducing a polynucleotide sequence (SEQ ID NO: 1178) vector expressing Abs-655first polypeptide and a polynucleotide sequence (SEQ ID NO: 1158) vector expressing Abs-655 second polypeptide into a host cell for expression.

For example, the functional antibody Abs-656 comprises a chimeric multifunctional antibody first polypeptide (SEQ ID NO: 837) (formed by LAG3 mutant LAG3 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25F/F33L/H90I). Abs-656 functional antibody is assembled by introducing a polynucleotide sequence (SEQ ID NO: 1179) vector expressing Abs-656first polypeptide and a polynucleotide sequence (SEQ ID NO: 1158) vector expressing Abs-656 second polypeptide into a host cell for expression.

For example, the functional antibody Abs-657 comprises a chimeric multifunctional antibody first polypeptide (SEQ ID NO: 838) (formed by LAG3 mutant LAG3 97-156 polypeptide C-terminus connecting to linker peptide (SEQ ID NO: 34) covalently linked to sugemalimab antibody heavy chain N-terminus, and sugemalimab antibody heavy chain C-terminus connecting to linker peptide (SEQ ID NO: 34) covalently linked to CD86 IgV mutant Q251/F33L/H90F). Abs-657 functional antibody is assembled by introducing a polynucleotide sequence (SEQ ID NO: 1180) vector expressing Abs-657first polypeptide and a polynucleotide sequence (SEQ ID NO: 1158) vector expressing Abs-657 second polypeptide into a host cell for expression.

For example, the functional antibody Abs-658 comprises a chimeric multifunctional antibody first polypeptide (SEQ ID NO: 839) (formed by LAG3 mutant LAG3 97-156 polypeptide C-terminus connecting to a linker peptide (SEQ ID NO: 34) covalently linked to sugemalimab antibody heavy chain N-terminus, and sugemalimab antibody heavy chain C-terminus connecting to linker peptide (SEQ ID NO: 34) covalently linked to CD86 IgV mutant Q25I/F33A/H90I). Abs-658 functional antibody is assembled by introducing a polynucleotide sequence (SEQ ID NO: 1181) vector expressing Abs-658 first polypeptide and a polynucleotide sequence (SEQ ID NO: 1158) vector expressing Abs-658 second polypeptide into a host cell for expression.

For example, the functional antibody Abs-659 comprises a chimeric multifunctional antibody first polypeptide (SEQ ID NO: 840) (formed by LAG3 mutant LAG3 97-156 polypeptide C-terminus connecting to a linker peptide (SEQ ID NO: 34) covalently linked to sugemalimab antibody heavy chain N-terminus, and sugemalimab antibody heavy chain C-terminus connecting to linker peptide (SEQ ID NO: 34) covalently linked to CD86 IgV mutant Q25I/H90I). Abs-659 functional antibody is assembled by introducing a polynucleotide sequence (SEQ ID NO: 1182) vector expressing Abs-659 first polypeptide and a polynucleotide sequence (SEQ ID NO: 1158) vector expressing Abs-659 second polypeptide into a host cell for expression.

For example, the functional antibody Abs-660 comprises a chimeric multifunctional antibody first polypeptide (SEQ ID NO: 841) (formed by LAG3 mutant LAG3 97-156 polypeptide C-terminus connecting to a linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/H90V). Abs-660 functional antibody is assembled by introducing a polynucleotide sequence (SEQ ID NO: 1183) vector expressing Abs-660 first polypeptide and a polynucleotide sequence (SEQ ID NO: 1158) vector expressing Abs-660 second polypeptide into a host cell for expression.

For example, the functional antibody Abs-661 comprises a chimeric multifunctional antibody first polypeptide (SEQ ID NO: 842) (formed by LAG3 mutant LAG3 97-156 polypeptide C-terminus connecting to a linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/H90I). Abs-661 functional antibody is assembled by introducing a polynucleotide sequence (SEQ ID NO: 1184) vector expressing Abs-661first polypeptide and a polynucleotide sequence (SEQ ID NO: 1158) vector expressing Abs-661 second polypeptide into a host cell for expression.

For example, the functional antibody Abs-662 comprises a chimeric multifunctional antibody first polypeptide (SEQ ID NO: 843) (formed by LAG3 mutant LAG3 97-156 polypeptide C-terminus connecting to a linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25F/H90I). Abs-662 functional antibody is assembled by introducing a polynucleotide sequence (SEQ ID NO: 1185) vector expressing Abs-662 first polypeptide and a polynucleotide sequence (SEQ ID NO: 1158) vector expressing Abs-662 second polypeptide into a host cell for expression.

For example, the functional antibody Abs-663 comprises a chimeric multifunctional antibody first polypeptide (SEQ ID NO: 844) (formed by LAG3 mutant LAG3 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25F/H90V). Abs-663 functional antibody is assembled by introducing a polynucleotide sequence (SEQ ID NO: 1186) vector expressing Abs-663first polypeptide and a polynucleotide sequence (SEQ ID NO: 1158) vector expressing Abs-663 second polypeptide into a host cell for expression.

For example, the functional antibody Abs-664 comprises a chimeric multifunctional antibody first polypeptide (SEQ ID NO: 854) (formed by LAG3 mutant LAG3 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to unmutated CD86 IgV). Abs-664 functional antibody is assembled by introducing a polynucleotide sequence (SEQ ID NO: 1187) vector expressing Abs-664 first polypeptide and a polynucleotide sequence (SEQ ID NO: 1158) vector expressing Abs-664 second polypeptide into a host cell for expression.

For example, the functional antibody Abs-881 comprises a chimeric multifunctional antibody first polypeptide (SEQ ID NO: 855) (formed by LAG3 mutant LAG3 R97E 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25I/F33L/H90I). Abs-881 functional antibody is assembled by introducing a polynucleotide sequence (SEQ ID NO: 1188) vector expressing Abs-881first polypeptide and a polynucleotide sequence (SEQ ID NO: 1158) vector expressing Abs-881 second polypeptide into a host cell for expression.

For example, the functional antibody Abs-882 comprises a chimeric multifunctional antibody first polypeptide (SEQ ID NO: 856) (formed by LAG3 mutant LAG3 R97E 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/F33L/H90I). Abs-882 functional antibody is assembled by introducing a polynucleotide sequence (SEQ ID NO: 1189) vector expressing Abs-882 first polypeptide and a polynucleotide sequence (SEQ ID NO: 1158) vector expressing Abs-882 second polypeptide into a host cell for expression.

For example, the functional antibody Abs-946 comprises a chimeric multifunctional antibody first polypeptide (SEQ ID NO: 857) (formed by LAG3 mutant LAG3 R97E 97-156 polypeptide C-terminus connecting to a linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25I/F33V/H90I). Abs-946 functional antibody is assembled by introducing a polynucleotide sequence (SEQ ID NO: 1190) vector expressing Abs-946 first polypeptide and a polynucleotide sequence (SEQ ID NO: 1158) vector expressing Abs-946 second polypeptide into a host cell for expression.

For example, the functional antibody Abs-883 comprises a chimeric multifunctional antibody first polypeptide (SEQ ID NO: 858) (formed by LAG3 mutant LAG3 R97E 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/F33V/H90I). Abs-883 functional antibody is assembled by introducing a polynucleotide sequence (SEQ ID NO: 1191) vector expressing Abs-883first polypeptide and a polynucleotide sequence (SEQ ID NO: 1158) vector expressing Abs-883 second polypeptide into a host cell for expression.

For example, the functional antibody Abs-947 comprises a chimeric multifunctional antibody first polypeptide (SEQ ID NO: 859) (formed by LAG3 mutant LAG3 R97E 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25I/F33L/H90V). Abs-947 functional antibody is assembled by introducing a polynucleotide sequence (SEQ ID NO: 1192) vector expressing Abs-947first polypeptide and a polynucleotide sequence (SEQ ID NO: 1158) vector expressing Abs-947 second polypeptide into a host cell for expression.

For example, the functional antibody Abs-917 comprises a chimeric multifunctional antibody first polypeptide (SEQ ID NO: 860) (formed by LAG3 mutant LAG3 R97E 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/F33L/H90V). Abs-917 functional antibody is assembled by introducing a polynucleotide sequence (SEQ ID NO: 1193) vector expressing Abs-917first polypeptide and a polynucleotide sequence (SEQ ID NO: 1158) vector expressing Abs-917 second polypeptide into a host cell for expression.

For example, the functional antibody Abs-916 comprises a chimeric multifunctional antibody first polypeptide (SEQ ID NO: 861) (formed by LAG3 mutant LAG3 R97E 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25I/F33V/H90V). Abs-916 functional antibody is assembled by introducing a polynucleotide sequence (SEQ ID NO: 1194) vector expressing Abs-916 first polypeptide and a polynucleotide sequence (SEQ ID NO: 1158) vector expressing Abs-916 second polypeptide into a host cell for expression.

For example, the functional antibody Abs-918 comprises a chimeric multifunctional antibody first polypeptide (SEQ ID NO: 862) (formed by LAG3 mutant LAG3 R97E 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/F33V/H90V). Abs-918 functional antibody is assembled by introducing a polynucleotide sequence (SEQ ID NO: 1195) vector expressing Abs-918first polypeptide and a polynucleotide sequence (SEQ ID NO: 1158) vector expressing Abs-918 second polypeptide into a host cell for expression.

For example, the functional antibody Abs-924 comprises a chimeric multifunctional antibody first polypeptide (SEQ ID NO: 863) (formed by LAG3 mutant LAG3 R97E 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant A13L/Q25I/F33L/H90I). Abs-924 functional antibody is assembled by introducing a polynucleotide sequence (SEQ ID NO: 1196) vector expressing Abs-924 first polypeptide and a polynucleotide sequence (SEQ ID NO: 1158) vector expressing Abs-924 second polypeptide into a host cell for expression.

For example, the functional antibody Abs-925 comprises a chimeric multifunctional antibody first polypeptide (SEQ ID NO: 864) (formed by LAG3 mutant LAG3 R97E 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant A13I/Q25I/F33L/H90I). Abs-925 functional antibody is assembled by introducing a polynucleotide sequence (SEQ ID NO: 1197) vector expressing Abs-925first polypeptide and a polynucleotide sequence (SEQ ID NO: 1158) vector expressing Abs-925 second polypeptide into a host cell for expression.

For example, the functional antibody Abs-926 comprises a chimeric multifunctional antibody first polypeptide (SEQ ID NO: 865) (formed by LAG3 mutant LAG3 R97E 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant A13L/Q25V/F33L/H90I). Abs-926 functional antibody is assembled by introducing a polynucleotide sequence (SEQ ID NO: 1198) vector expressing Abs-926first polypeptide and a polynucleotide sequence (SEQ ID NO: 1158) vector expressing Abs-926 second polypeptide into a host cell for expression.

For example, the functional antibody Abs-927 comprises a chimeric multifunctional antibody first polypeptide (SEQ ID NO: 866) (formed by LAG3 mutant LAG3 R97E 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant A13I/Q25V/F33L/H90I). Abs-927 functional antibody is assembled by introducing a polynucleotide sequence (SEQ ID NO: 1199) vector expressing Abs-927first polypeptide and a polynucleotide sequence (SEQ ID NO: 1158) vector expressing Abs-927 second polypeptide into a host cell for expression.

For example, the functional antibody Abs-928 comprises a chimeric multifunctional antibody first polypeptide (SEQ ID NO: 867) (formed by LAG3 mutant LAG3 R97E 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25I/F33L/I89L/H90I). Abs-928 functional antibody is assembled by introducing a polynucleotide sequence (SEQ ID NO: 1200) vector expressing Abs-928first polypeptide and a polynucleotide sequence (SEQ ID NO: 1158) vector expressing Abs-928 second polypeptide into a host cell for expression.

For example, the functional antibody Abs-929 comprises a chimeric multifunctional antibody first polypeptide (SEQ ID NO: 868) (formed by LAG3 mutant LAG3 R97E 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25I/F33L/M60R/H90I). Abs-929 functional antibody is assembled by introducing a polynucleotide sequence (SEQ ID NO: 1201) vector expressing Abs-929first polypeptide and a polynucleotide sequence (SEQ ID NO: 1158) vector expressing Abs-929 second polypeptide into a host cell for expression.

For example, the functional antibody Abs-930 comprises a chimeric multifunctional antibody first polypeptide (SEQ ID NO: 869) (formed by LAG3 mutant LAG3 R97E 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/F33L/I89L/H90I). Abs-930 functional antibody is assembled by introducing a polynucleotide sequence (SEQ ID NO: 1202) vector expressing Abs-930first polypeptide and a polynucleotide sequence (SEQ ID NO: 1158) vector expressing Abs-930 second polypeptide into a host cell for expression.

For example, the functional antibody Abs-931 comprises a chimeric multifunctional antibody first polypeptide (SEQ ID NO: 870) (formed by LAG3 mutant LAG3 R97E 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/F33L/M60R/H90I). Abs-931 functional antibody is assembled by introducing a polynucleotide sequence (SEQ ID NO: 1203) vector expressing Abs-931first polypeptide and a polynucleotide sequence (SEQ ID NO: 1158) vector expressing Abs-931 second polypeptide into a host cell for expression.

For example, the functional antibody Abs-934 comprises a chimeric multifunctional antibody first polypeptide (SEQ ID NO: 871) (formed by LAG3 mutant LAG3 R97E 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25F/F33L/H90I). Abs-934 functional antibody is assembled by introducing a polynucleotide sequence (SEQ ID NO: 1204) vector expressing Abs-934first polypeptide and a polynucleotide sequence (SEQ ID NO: 1158) vector expressing Abs-934 second polypeptide into a host cell for expression.

For example, the functional antibody Abs-935 comprises a chimeric multifunctional antibody first polypeptide (SEQ ID NO: 872) (formed by LAG3 mutant LAG3 R97E 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25I/F33L/H90F). Abs-935 functional antibody is assembled by introducing a polynucleotide sequence (SEQ ID NO: 1205) vector expressing Abs-935first polypeptide and a polynucleotide sequence (SEQ ID NO: 1158) vector expressing Abs-935 second polypeptide into a host cell for expression.

For example, the functional antibody Abs-936 comprises a chimeric multifunctional antibody first polypeptide (SEQ ID NO: 873) (formed by LAG3 mutant LAG3 R97E 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25I/F33A/H90I). Abs-936 functional antibody is assembled by introducing a polynucleotide sequence (SEQ ID NO: 1206) vector expressing Abs-936first polypeptide and a polynucleotide sequence (SEQ ID NO: 1158) vector expressing Abs-936 second polypeptide into a host cell for expression. 250102

For example, the functional antibody Abs-941 comprises a chimeric multifunctional antibody first polypeptide (SEQ ID NO: 84) (formed by LAG3 mutant LAG3 R97E 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25I/H90I). Abs-941 functional antibody is assembled by introducing a polynucleotide sequence (SEQ ID NO: 1207) vector expressing Abs-941first polypeptide and a polynucleotide sequence (SEQ ID NO: 1158) vector expressing Abs-941 second polypeptide into a host cell for expression.

For example, the functional antibody Abs-942 comprises a chimeric multifunctional antibody first polypeptide (SEQ ID NO: 875) (formed by LAG3 mutant LAG3 R97E 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/H90V). Abs-942 functional antibody is assembled by introducing a polynucleotide sequence (SEQ ID NO: 1208) vector expressing Abs-942first polypeptide and a polynucleotide sequence (SEQ ID NO: 1158) vector expressing Abs-942 second polypeptide into a host cell for expression.

For example, the functional antibody Abs-943 comprises a chimeric multifunctional antibody first polypeptide (SEQ ID NO: 876) (formed by LAG3 mutant LAG3 R97E 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/H90I). Abs-943 functional antibody is assembled by introducing a polynucleotide sequence (SEQ ID NO: 1209) vector expressing Abs-943first polypeptide and a polynucleotide sequence (SEQ ID NO: 1158) vector expressing Abs-943 second polypeptide into a host cell for expression.

For example, the functional antibody Abs-944 comprises a chimeric multifunctional antibody first polypeptide (SEQ ID NO: 877) (formed by LAG3 mutant LAG3 R97E 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25F/H90I). Abs-944 functional antibody is assembled by introducing a polynucleotide sequence (SEQ ID NO: 1210) vector expressing Abs-944first polypeptide and a polynucleotide sequence (SEQ ID NO: 1158) vector expressing Abs-944 second polypeptide into a host cell for expression.

For example, the functional antibody Abs-945 comprises a chimeric multifunctional antibody first polypeptide (SEQ ID NO: 878) (formed by LAG3 mutant LAG3 R97E 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25F/H90V). Abs-945 functional antibody is assembled by introducing a polynucleotide sequence (SEQ ID NO: 1211) vector expressing Abs-945first polypeptide and a polynucleotide sequence (SEQ ID NO: 1158) vector expressing Abs-945 second polypeptide into a host cell for expression.

For example, the functional antibody Abs-736 comprises a chimeric multifunctional antibody first polypeptide (SEQ ID NO: 888) formed by (LAG3 mutant LAG3 R97E 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to unmutated CD86 IgV). Abs-736 functional antibody is assembled by introducing a polynucleotide sequence (SEQ ID NO: 1212) vector expressing Abs-736 first polypeptide and polynucleotide sequence (SEQ ID NO: 1158) vector expressing Abs-736 second polypeptide into a host cell for expression.

For example, the functional antibody Abs-878 comprises a chimeric multifunctional antibody first polypeptide (SEQ ID NO: 957) (formed by LAG3 mutant LAG3 112-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25I/F33L/H90I). Abs-878 functional antibody is assembled by introducing a polynucleotide sequence (SEQ ID NO: 1139) vector expressing Abs-878first polypeptide and a polynucleotide sequence (SEQ ID NO: 1158) vector expressing Abs-878 second polypeptide into a host cell for expression.

For example, the functional antibody Abs-879 comprises a chimeric multifunctional antibody first polypeptide (SEQ ID NO: 958) (formed by LAG3 mutant LAG3 112-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/F33L/H90I). Abs-879 functional antibody is assembled by introducing a polynucleotide sequence (SEQ ID NO: 1140) vector expressing Abs-879 first polypeptide and a polynucleotide sequence (SEQ ID NO: 1158) vector expressing Abs-879 second polypeptide into a host cell for expression.

For example, the functional antibody Abs-887 comprises a chimeric multifunctional antibody first polypeptide (SEQ ID NO: 959) (formed by LAG3 mutant LAG3 112-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25I/F33V/H90I). Abs-887 functional antibody is assembled by introducing a polynucleotide sequence (SEQ ID NO: 1141) vector expressing Abs-887first polypeptide and a polynucleotide sequence (SEQ ID NO: 1158) vector expressing Abs-887 second polypeptide into a host cell for expression.

For example, the functional antibody Abs-880 comprises a chimeric multifunctional antibody first polypeptide (SEQ ID NO: 960) (formed by LAG3 mutant LAG3 112-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/F33V/H90I). Abs-880 functional antibody is assembled by introducing a polynucleotide sequence (SEQ ID NO: 1142) vector expressing Abs-880first polypeptide and a polynucleotide sequence (SEQ ID NO: 1158) vector expressing Abs-880 second polypeptide into a host cell for expression.

For example, the functional antibody Abs-890 comprises a chimeric multifunctional antibody first polypeptide (SEQ ID NO: 962) (formed by LAG3 mutant LAG3 112-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/F33L/H90V). Abs-890 functional antibody is assembled by introducing a polynucleotide sequence (SEQ ID NO: 1143) vector expressing Abs-890first polypeptide and a polynucleotide sequence (SEQ ID NO: 1158) vector expressing Abs-890 second polypeptide into a host cell for expression.

For example, the functional antibody Abs-917 comprises a chimeric multifunctional antibody first polypeptide (SEQ ID NO: 964) (formed by LAG3 mutant LAG3 112-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/F33V/H90V). Abs-917 functional antibody is assembled by introducing a polynucleotide sequence (SEQ ID NO: 1144) vector expressing Abs-917 first polypeptide and a polynucleotide sequence (SEQ ID NO: 1158) vector expressing Abs-917 second polypeptide into a host cell for expression.

For example, the functional antibody Abs-884 comprises a chimeric multifunctional antibody first polypeptide (SEQ ID NO: 991) (formed by LAG3 mutant LAG3 122-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25I/F33L/H90I). Abs-884 functional antibody is assembled by introducing a polynucleotide sequence (SEQ ID NO: 1145) vector expressing Abs-884 first polypeptide and a polynucleotide sequence (SEQ ID NO: 1158) vector expressing Abs-884 second polypeptide into a host cell for expression.

For example, the functional antibody Abs-885 comprises a chimeric multifunctional antibody first polypeptide (SEQ ID NO: 992) (formed by LAG3 mutant LAG3 122-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25V/F33L/H90I). Abs-885 functional antibody is assembled by introducing a polynucleotide sequence (SEQ ID NO: 1146) vector expressing Abs-885first polypeptide and a polynucleotide sequence (SEQ ID NO: 1158) vector expressing Abs-885 second polypeptide into a host cell for expression.

For example, the functional antibody Abs-886 comprises a chimeric multifunctional antibody first polypeptide (SEQ ID NO: 993) (formed by LAG3 mutant LAG3 122-156 polypeptide C-terminus connecting to linker peptide (SEQ ID NO: 34) covalently linked to sugemalimab antibody heavy chain N-terminus, and sugemalimab antibody heavy chain C-terminus via linker peptide (SEQ ID NO: 34) covalently linked to CD86 IgV mutant Q25I/F33V/H90I). Abs-886 functional antibody is assembled by introducing a polynucleotide sequence (SEQ ID NO: 1147) vector expressing Abs-886first polypeptide and a polynucleotide sequence (SEQ ID NO: 1158) vector expressing Abs-886 second polypeptide into a host cell for expression.

For example, the functional antibody Abs-891 comprises a chimeric multifunctional antibody first polypeptide (SEQ ID NO: 994) (formed by LAG3 mutant LAG3 122-156 polypeptide C-terminus connecting to a linker peptide (SEQ ID NO: 34) covalently linked to sugemalimab antibody heavy chain N-terminus, and sugemalimab antibody heavy chain C-terminus via linker peptide (SEQ ID NO: 34) covalently linked to CD86 IgV mutant Q25V/F33V/H90I). Abs-891 functional antibody is assembled by introducing a polynucleotide sequence (SEQ ID NO: 1148) vector expressing Abs-891first polypeptide and a polynucleotide sequence (SEQ ID NO: 1158) vector expressing Abs-891 second polypeptide into a host cell for expression.

For example, the functional antibody Abs-888 comprises a chimeric multifunctional antibody first polypeptide (SEQ ID NO: 996) (formed by LAG3 mutant LAG3 122-156 polypeptide C-terminus connecting to a linker peptide (SEQ ID NO: 34) covalently linked to sugemalimab antibody heavy chain N-terminus, and sugemalimab antibody heavy chain C-terminus via linker peptide (SEQ ID NO: 34) covalently linked to CD86 IgV mutant Q25V/F33L/H90V). Abs-888 functional antibody is assembled by introducing a polynucleotide sequence (SEQ ID NO: 1149) vector expressing Abs-889first polypeptide and a polynucleotide sequence (SEQ ID NO: 1158) vector expressing Abs-888 second polypeptide into a host cell for expression.

For example, the functional antibody Abs-889 comprises a chimeric multifunctional antibody first polypeptide (SEQ ID NO: 998) (formed by LAG3 mutant LAG3 122-156 polypeptide C-terminus connecting to linker peptide (SEQ ID NO: 34) covalently linked to sugemalimab antibody heavy chain N-terminus, and sugemalimab antibody heavy chain C-terminus via linker peptide (SEQ ID NO: 34) covalently linked to CD86 IgV mutant Q25V/F33V/H90V). Abs-889 functional antibody is assembled by introducing a polynucleotide sequence (SEQ ID NO: 1150) vector expressing Abs-889first polypeptide and a polynucleotide sequence (SEQ ID NO: 1158) vector expressing Abs-889 second polypeptide into a host cell for expression.

For example, the functional antibody Abs-975 comprises a chimeric multifunctional antibody first polypeptide (SEQ ID NO: 1249) (formed by LAG3 mutant LAG3 R97E 97-156 polypeptide C-terminus connecting linker peptide (SEQ ID NO: 34) covalently linked to sugemalimab antibody heavy chain N-terminus, and sugemalimab antibody heavy chain C-terminus via linker peptide (SEQ ID NO: 34) covalently linked to CD86 IgV mutant Q25I). Abs-975 functional antibody is assembled by introducing a polynucleotide sequence (SEQ ID NO: 1250) vector expressing Abs-975first polypeptide and a polynucleotide sequence (SEQ ID NO: 1158) vector expressing Abs-975 second polypeptide into a host cell for expression.

For example, the functional antibody Abs-976 comprises a chimeric multifunctional antibody first polypeptide (SEQ ID NO: 1251) (formed by LAG3 mutant LAG3 R97E 97-156 polypeptide C-terminus connecting linker peptide (SEQ ID NO: 34) covalently linked to sugemalimab antibody heavy chain N-terminus, and sugemalimab antibody heavy chain C-terminus via linker peptide (SEQ ID NO: 34) covalently linked to CD86 IgV mutant H90I). Abs-976 functional antibody is assembled by introducing a polynucleotide sequence (SEQ ID NO: 1252) vector expressing Abs-976first polypeptide and a polynucleotide sequence (SEQ ID NO: 1158) vector expressing Abs-976 second polypeptide into a host cell for expression.

For example, the functional antibody Abs-977 comprises a chimeric multifunctional antibody first polypeptide (SEQ ID NO: 1253) (formed by LAG3 mutant LAG3 97-156 polypeptide C-terminus connecting to linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant Q25I). Abs-977 functional antibody is assembled by introducing a polynucleotide sequence (SEQ ID NO: 1254) vector expressing Abs-977first polypeptide and a polynucleotide sequence (SEQ ID NO: 1158) vector expressing Abs-977 second polypeptide into a host cell for expression.

For example, the functional antibody Abs-978 comprises a chimeric multifunctional antibody first polypeptide (SEQ ID NO: 1255) (formed by LAG3 mutant LAG3 97-156 polypeptide C-terminus connecting linker peptide (SEQ ID NO: 34) covalently linked to sugemalimab antibody heavy chain N-terminus, and a sugemalimab antibody heavy chain C-terminus connecting to linker peptide (SEQ ID NO: 34) covalently linked to CD86 IgV mutant H90I). Abs-978 functional antibody is assembled by introducing a polynucleotide sequence (SEQ ID NO: 1256) vector expressing Abs-978first polypeptide and a polynucleotide sequence (SEQ ID NO: 1158) vector expressing Abs-978 second polypeptide into a host cell for expression.

For example, the functional antibody Abs-962 comprises a chimeric multifunctional antibody first polypeptide (SEQ ID NO: 889) (formed by LAG3 mutant LAG3 97-156 polypeptide C-terminus connecting to linker peptide (SEQ ID NO: 34) covalently linked to atezolizumab antibody heavy chain N-terminus, and atezolizumab antibody heavy chain C-terminus connecting to linker peptide (SEQ ID NO: 34) covalently linked to CD86 IgV mutant Q25I/F33L/H90I). Abs-962 functional antibody is assembled by introducing a polynucleotide sequence (SEQ ID NO: 1151) vector expressing Abs-962 first polypeptide and a polynucleotide sequence (SEQ ID NO: 1160) vector expressing Abs-962 second polypeptide into a host cell for expression.

For example, the functional antibody Abs-963 comprises a chimeric multifunctional antibody first polypeptide (SEQ ID NO: 922) (formed by LAG3 mutant LAG3 97-156 polypeptide C-terminus connecting linker peptide (SEQ ID NO: 34) covalently linked to atezolizumab antibody heavy chain N-terminus, and atezolizumab antibody heavy chain C-terminus through linker peptide (SEQ ID NO: 34) covalently linked to unmutated CD86 IgV). Abs-963 functional antibody is assembled by introducing a polynucleotide sequence (SEQ ID NO: 1152) vector expressing Abs-963first polypeptide and a polynucleotide sequence (SEQ ID NO: 1160) vector expressing Abs-963 second polypeptide into a host cell for expression.

For example, the functional antibody Abs-960 comprises a chimeric multifunctional antibody first polypeptide (SEQ ID NO: 923) (formed by LAG3 mutant LAG3 R97E 97-156 polypeptide C-terminus connecting linker peptide (SEQ ID NO: 34) covalently linked to atezolizumab antibody heavy chain N-terminus, and atezolizumab antibody heavy chain C-terminus connecting to linker peptide (SEQ ID NO: 34) covalently linked to CD86 IgV mutant Q25I/F33L/H90I). Abs-960 functional antibody is assembled by introducing a polynucleotide sequence (SEQ ID NO: 1153) vector expressing Abs-960first polypeptide and a polynucleotide sequence (SEQ ID NO: 1160) vector expressing Abs-960 second polypeptide into a host cell for expression.

For example, the functional antibody Abs-961 comprises a chimeric multifunctional antibody first polypeptide (SEQ ID NO: 956) (formed by LAG3 mutant LAG3 R97E 97-156 polypeptide C-terminus connecting to linker peptide (SEQ ID NO: 34) covalently linked to atezolizumab antibody heavy chain N-terminus, and atezolizumab antibody heavy chain C-terminus via linker peptide (SEQ ID NO: 34) covalently linked to unmutated CD86 IgV). Abs-961 functional antibody is assembled by introducing a polynucleotide sequence (SEQ ID NO: 1154) vector expressing Abs-961first polypeptide and a polynucleotide sequence (SEQ ID NO: 1160) vector expressing Abs-961 second polypeptide into a host cell for expression.

For example, the antibody Abs-16 comprises sugemalimab antibody heavy chain first polypeptide (SEQ ID NO: 4) and sugemalimab antibody light chain second polypeptide (SEQ ID NO: 5). Abs-16 antibody is assembled by introducing a polynucleotide sequence (SEQ ID NO: 1157) vector expressing Abs-16 first polypeptide and a polynucleotide sequence (SEQ ID NO: 1158) vector expressing Abs-16 second polypeptide into a host cell for expression.

For example, the antibody Abs-23 comprises atezolizumab antibody heavy chain first polypeptide (SEQ ID NO: 810) and atezolizumab antibody light chain second polypeptide (SEQ ID NO: 11). Abs-23 antibody is assembled by introducing a polynucleotide sequence (SEQ ID NO: 1159) vector expressing Abs-23 first polypeptide and a polynucleotide sequence (SEQ ID NO: 1160) vector expressing Abs-23 second polypeptide into a host cell for expression.

For example, the antibody Abs-03 comprises CD3B219 antibody heavy chain first polypeptide (SEQ ID NO: 616) and CD3B219 antibody light chain second polypeptide (SEQ ID NO: 617). Abs-03 antibody is assembled by introducing a polynucleotide sequence (SEQ ID NO: 1161) vector expressing Abs-03first polypeptide and a polynucleotide sequence (SEQ ID NO: 1162) vector expressing Abs-03 second polypeptide into a host cell for expression.

LAG3 is a type I transmembrane protein in immune checkpoints, mainly expressed on activated NK cells and T cells. LAG3 is expressed on CD8+T cells in tumor failure and is an effective target for tumor immunotherapy; however, its function is dual-sided. On the one hand, LAG3 plays a negative regulatory role in T cell proliferation and activation. After binding to its ligand, major histocompatibility complex II (MHCII) or ligand fibrinogen-like protein 1 (FGL1), LAG3 participates in inhibiting T cell activation and function; on the other hand, soluble LAG3 polypeptide participates in inducing the activation of antigen-presenting cells, such as upregulating the expression of CD83/CD40/CD80/CD86, expressing effector cytokines such as IL-12 and TNFa, secreting chemokines such as CCL4, CCL2 and CCL5 to recruit T cells, etc., and participates in assisting T cell activation. Antagonistic LAG3 antibodies can directly relieve LAG3-mediated T cell inhibition, but also block LAG3-mediated antigen-presenting cell activation, so they cannot fully enhance LAG3-related immune activation effects. LAG3 polypeptides have the effect of activating antigen-presenting cells, so they have potential therapeutic prospects in the fields of tumors and infections.

However, the expression yield of the complete LAG3 polypeptide in mammalian cells is low and it is very easy to form a highly aggregated state. At the same time, the LAG3 polypeptide Fc fusion protein LAG3-Fc is extremely unstable in the body and is very easy to be quickly cleared, which limits the efficacy of the LAG3 polypeptide in the body. Therefore, there is a need to develop new LAG3 variant polypeptides.

The present invention provides a functional LAG3 variant polypeptide obtained by innovatively modifying a LAG3 polypeptide, which can be conveniently produced in mammalian cells with high expression levels and can exhibit good function and stability in vivo and in vitro. The LAG3 variant polypeptide can be used to regulate the immune response of antigen-presenting cells, thereby achieving the treatment or prevention of diseases such as infection and tumors caused by the inhibition of immune cell function.

In one aspect, the present application provides a LAG3 mutant, wherein the LAG3 variant polypeptide may comprise truncations and/or mutations based on human LAG3.

For example, the LAG3 variant polypeptide may have 0-124 amino acids truncated at the N-terminus based on the wild-type human LAG3 extracellular region polypeptide.

For example, the LAG3 variant polypeptide may have 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75 , 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62 , 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 104, 106, 107, 108, 109, 110, 111, 112, 113, 114, 115, 116, 117, 118, 119, 120, 121, 122, 123, or 124 amino acids truncated at the N-terminus based on the wild-type human LAG3 extracellular region polypeptide.

For example, the LAG3 variant polypeptide may have 0, 5, 21, 37, 45, 60, 71, 74, 79, 84, 89, 94, 99, 104, 109 or 114 amino acids truncated at the N-terminus based on the wild-type human LAG3 extracellular region polypeptide.

For example, the LAG3 variant polypeptide may have 0, 5, 21, 37, 45, 60, 71 or 74 amino acids truncated at the N-terminus based on the wild-type human LAG3 extracellular region polypeptide.

For example, the LAG3 variant polypeptide may terminate at amino acid positions 122-167 at the C-terminus based on a wild-type human LAG3 extracellular region polypeptide.

For example, the LAG3 variant polypeptide may, based on a wild-type human LAG3 extracellular region polypeptide, based onterminate at any position corresponding to amino acid positions 122-167, 123-167, 124-167, 125-167, 126-167, 127-167, 128-167, 129-167, 130-167, 131-167, 132-167, 133-167, 134-167, 135-167, 136-167, 137-167, 138-167, 139-167, 140-167, 141-167, 142 -167, 143-167, 144-167, 145-167, 146-167, 147-167, 148-167, 149-167, 150-167, 151-167, 152-167, 153-167, 154-167, 155-167, 156-167, 157-167, 158-167, 159-167, 160-167, 161-167, 162-167, 163-167, 164-167, 165-167 or 166-167 at C-terminus.

For example, the LAG3 variant polypeptide may, based on a wild-type human LAG3 extracellular region polypeptide, terminate at amino acid site 122, 129, 136, 146, 156, 157, 158, 159, 160, 161, 162, 163, 164, 165, 166 or 167 based onat the C-terminus.

For example, the LAG3 variant polypeptide may, based on a wild-type human LAG3 extracellular region polypeptide, terminate at amino acid position 122, 129, 136, 146, 156, 161 or 167 at the C-terminus.

For example, the LAG3 variant polypeptide may comprise, based on a wild-type human LAG3 extracellular region polypeptide, end at amino acid position 156, 161 or 167 at the C-terminus.

For example, the LAG3 variant polypeptide may have 0, 5, 21, 37, 45, 60, 71 or 74 amino acids truncated at the N-terminus based on the wild-type human LAG3 extracellular region polypeptide, and terminate at amino acid position 122, 129, 136, 146, 156, 157, 158, 159, 160, 161, 162, 163, 164, 165, 166 or 167 at the C-terminus based on the wild-type human LAG3 extracellular region polypeptide.

For example, the LAG3 variant polypeptide may comprise an amino acid mutation.

For example, the LAG3 variant polypeptide may comprise an amino acid mutation, wherein the amino acid mutation site is an Arg amino acid at position 97.

For example, the LAG3 variant polypeptide may comprise an amino acid mutation, wherein the amino acid mutation site is a mutation of the Arg amino acid at site 97 to a Glu amino acid.

For example, the LAG3 variant polypeptide may have 74 amino acids truncated at the N-terminus based on a wild-type human LAG3 extracellular region polypeptide. For example, the LAG3 variant polypeptide may comprise an amino acid sequence selected from the group consisting of SEQ ID NO: 244 to SEQ ID NO: 259. For example, the LAG3 variant polypeptide can be selected from the group consisting of the amino acid sequences shown in SEQ ID NO:244, SEQ ID NO:245, SEQ ID NO:246, SEQ ID NO:247, SEQ ID NO:248 and SEQ ID NO:253.

For example, the LAG3 variant polypeptide may, based on a wild-type human LAG3 extracellular region polypeptide, have 74 amino acids truncated at the N-terminus, and the Arg amino acid at position 97 is mutated to a Glu amino acid. For example, the LAG3 variant polypeptide may comprise an amino acid sequence selected from the group consisting of SEQ ID NO: 260 to SEQ ID NO: 275. For example, the LAG3 variant polypeptide can be selected from the group consisting of the amino acid sequences shown in SEQ ID NO:260, SEQ ID NO:261, SEQ ID NO:262, SEQ ID NO:263, SEQ ID NO:264 and SEQ ID NO:269.

For example, the LAG3 variant polypeptide may have 5 amino acids truncated at the N-terminus based on a wild-type human LAG3 extracellular region polypeptide. For example, the LAG3 variant polypeptide may comprise an amino acid sequence selected from the group consisting of SEQ ID NO: 276 to SEQ ID NO: 278.

For example, the LAG3 variant polypeptide may, based on a wild-type human LAG3 extracellular region polypeptide, have 5 amino acids truncated at the N-terminus, and the Arg amino acid at position 97 is mutated to a Glu amino acid. The LAG3 variant polypeptide may comprise an amino acid sequence selected from the group consisting of SEQ ID NO: 279 to SEQ ID NO: 281.

For example, the LAG3 variant polypeptide may, based on a wild-type human LAG3 extracellular region polypeptide, have 21 amino acids truncated at the N-terminus. For example, the LAG3 variant polypeptide may comprise an amino acid sequence selected from the group consisting of SEQ ID NO: 282 to SEQ ID NO: 284.

For example, the LAG3 variant polypeptide may, based on a wild-type human LAG3 extracellular region polypeptide, have 21 amino acids truncated at the N-terminus, and the Arg amino acid at position 97 is mutated to a Glu amino acid. For example, the LAG3 variant polypeptide may comprise an amino acid sequence selected from the group consisting of SEQ ID NO: 288 to SEQ ID NO: 290.

For example, the LAG3 variant polypeptide may, based on a wild-type human LAG3 extracellular region polypeptide, have 37 amino acids truncated at the N-terminusbased on. For example, the LAG3 variant polypeptide may comprise an amino acid sequence selected from the group consisting of SEQ ID NO: 276 to SEQ ID NO: 278.

For example, the LAG3 variant polypeptide may, based on a wild-type human LAG3 extracellular region polypeptide, have 37 amino acids truncated at the N-terminus, and the Arg amino acid at position 97 mutated to a Glu amino acid. For example, the LAG3 variant polypeptide may comprise an amino acid sequence selected from the group consisting of SEQ ID NO: 291 to SEQ ID NO: 293.

For example, the LAG3 variant polypeptide may have 45 amino acids truncated at the N-terminus based on the wild-type human LAG3 extracellular region polypeptide. For example, the LAG3 variant polypeptide may comprise an amino acid sequence selected from the group consisting of SEQ ID NO: 294 to SEQ ID NO: 296.

For example, the LAG3 variant polypeptide may, based on a wild-type human LAG3 extracellular region polypeptide, have 45 amino acids truncated at the N-terminus, and the Arg amino acid at position 97 mutated to a Glu amino acid. For example, the LAG3 variant polypeptide may comprise an amino acid sequence selected from the group consisting of SEQ ID NO: 297 to SEQ ID NO: 299.

For example, the LAG3 variant polypeptide may have 60 amino acids truncated at the N-terminus based on the wild-type human LAG3 extracellular region polypeptide. For example, the LAG3 variant polypeptide may comprise an amino acid sequence selected from the group consisting of SEQ ID NO: 300 to SEQ ID NO: 302.

For example, the LAG3 variant polypeptide may, based on a wild-type human LAG3 extracellular region polypeptide, have 60 amino acids truncated at the N-terminus, and the Arg amino acid at position 97 mutated to a Glu amino acid. For example, the LAG3 variant polypeptide may comprise an amino acid sequence selected from the group consisting of SEQ ID NO: 303 to SEQ ID NO: 305.

For example, the LAG3 variant polypeptide may have 71 amino acids truncated at the N-terminus based on the wild-type human LAG3 extracellular region polypeptide. For example, the LAG3 variant polypeptide may comprise an amino acid sequence selected from the group consisting of SEQ ID NO: 306 to SEQ ID NO: 308.

For example, the LAG3 variant polypeptide may, based on a wild-type human LAG3 extracellular region polypeptide, have 71 amino acids truncated at the N-terminus, and the Arg amino acid at position 97 mutated to a Glu amino acid. For example, the LAG3 variant polypeptide may comprise an amino acid sequence selected from the group consisting of SEQ ID NO: 309 to SEQ ID NO: 311.

For example, the LAG3 variant polypeptide may have 79 amino acids truncated at the N-terminus based on the wild-type human LAG3 extracellular region polypeptide. For example, the LAG3 variant polypeptide may comprise an amino acid sequence selected from the group consisting of SEQ ID NO: 312 to SEQ ID NO: 314.

For example, the LAG3 variant polypeptide may, based on a wild-type human LAG3 extracellular region polypeptide, have 79 amino acids truncated at the N-terminus, and the Arg amino acid at position 97 mutated to a Glu amino acid. For example, the LAG3 variant polypeptide may comprise an amino acid sequence selected from the group consisting of SEQ ID NO: 312 to SEQ ID NO: 314.

For example, the LAG3 variant polypeptide may have 84 amino acids truncated at the N-terminus based on the wild-type human LAG3 extracellular region polypeptide. For example, the LAG3 variant polypeptide may comprise an amino acid sequence selected from the group consisting of SEQ ID NO: 315 to SEQ ID NO: 317.

For example, the LAG3 variant polypeptide may, based on a wild-type human LAG3 extracellular region polypeptide, have 84 amino acids truncated at the N-terminus, and the Arg amino acid at position 97 mutated to a Glu amino acid. For example, the LAG3 variant polypeptide may comprise an amino acid sequence selected from the group consisting of SEQ ID NO: 315 to SEQ ID NO: 317.

For example, the LAG3 variant polypeptide may have 89 amino acids truncated at the N-terminus based on the wild-type human LAG3 extracellular region polypeptide. For example, the LAG3 variant polypeptide may comprise an amino acid sequence selected from the group consisting of SEQ ID NO: 318 to SEQ ID NO: 320.

For example, the LAG3 variant polypeptide may, based on a wild-type human LAG3 extracellular region polypeptide, have 89 amino acids truncated at the N-terminus, and the Arg amino acid at position 97 mutated to a Glu amino acid. For example, the LAG3 variant polypeptide may comprise an amino acid sequence selected from the group consisting of SEQ ID NO: 318 to SEQ ID NO: 320.

For example, the LAG3 variant polypeptide may have 94 amino acids truncated at the N-terminus based on the wild-type human LAG3 extracellular region polypeptide. For example, the LAG3 variant polypeptide may comprise an amino acid sequence selected from the group consisting of SEQ ID NO: 321 to SEQ ID NO: 323.

For example, the LAG3 variant polypeptide may, based on a wild-type human LAG3 extracellular region polypeptide, have 94 amino acids truncated at the N-terminus, and the Arg amino acid at position 97 mutated to a Glu amino acid. For example, the LAG3 variant polypeptide may comprise an amino acid sequence selected from the group consisting of SEQ ID NO: 321 to SEQ ID NO: 323.

For example, the LAG3 variant polypeptide may have 99 amino acids truncated at the N-terminus based on the wild-type human LAG3 extracellular region polypeptide. For example, the LAG3 variant polypeptide may comprise an amino acid sequence selected from the group consisting of SEQ ID NO: 324 to SEQ ID NO: 326.

For example, the LAG3 variant polypeptide may, based on a wild-type human LAG3 extracellular region polypeptide, have 99 amino acids truncated at the N-terminus, and the Arg amino acid at position 97 mutated to a Glu amino acid. For example, the LAG3 variant polypeptide may comprise an amino acid sequence selected from the group consisting of SEQ ID NO: 324 to SEQ ID NO: 326.

For example, the LAG3 variant polypeptide may have 104 amino acids truncated at the N-terminus based on the wild-type human LAG3 extracellular region polypeptide. For example, the LAG3 variant polypeptide may comprise an amino acid sequence selected from the group consisting of SEQ ID NO: 327 to SEQ ID NO: 329.

For example, the LAG3 variant polypeptide may, based on a wild-type human LAG3 extracellular region polypeptide, have 104 amino acids truncated at the N-terminus, and the Arg amino acid at position 97 mutated to a Glu amino acid. For example, the LAG3 variant polypeptide may comprise an amino acid sequence selected from the group consisting of SEQ ID NO: 327 to SEQ ID NO: 329.

For example, the LAG3 variant polypeptide may have 109 amino acids truncated at the N-terminus based on the wild-type human LAG3 extracellular region polypeptide. For example, the LAG3 variant polypeptide may comprise an amino acid sequence selected from the group consisting of SEQ ID NO: 330 to SEQ ID NO: 332.

For example, the LAG3 variant polypeptide may, based on a wild-type human LAG3 extracellular region polypeptide, have 109 amino acids truncated at the N-terminus, and the Arg amino acid at position 97 mutated to a Glu amino acid. For example, the LAG3 variant polypeptide may comprise an amino acid sequence selected from the group consisting of SEQ ID NO: 330 to SEQ ID NO: 332.

For example, the LAG3 variant polypeptide may have 114 amino acids truncated at the N-terminus based on the wild-type human LAG3 extracellular region polypeptide. For example, the LAG3 variant polypeptide may comprise an amino acid sequence selected from the group consisting of SEQ ID NO: 333 to SEQ ID NO: 335.

For example, the LAG3 variant polypeptide may, based on a wild-type human LAG3 extracellular region polypeptide, have 114 amino acids truncated at the N-terminus, and the Arg amino acid at position 97 mutated to a Glu amino acid. For example, the LAG3 variant polypeptide may comprise an amino acid sequence selected from the group consisting of SEQ ID NO: 333 to SEQ ID NO: 335.

For example, the LAG3 variant polypeptide may have 0 amino acids truncated at the N-terminus based on the wild-type human LAG3 extracellular region polypeptide. For example, the LAG3 variant polypeptide may comprise an amino acid sequence selected from the group consisting of SEQ ID NO: 336 to SEQ ID NO: 342.

For example, the LAG3 variant polypeptide may, based on a wild-type human LAG3 extracellular region polypeptide, have 0 amino acids truncated at the N-terminus, and the Arg amino acid at position 97 mutated to a Glu amino acid. For example, the LAG3 variant polypeptide may comprise an amino acid sequence selected from the group consisting of SEQ ID NO: 343 to SEQ ID NO: 349.

On the one hand, after extensive research, the present invention unexpectedly discovered a class of LAG3 variant polypeptides, which are truncated forms of different fragment sizes based on the wild-type human LAG3 extracellular region (23-450) polypeptide (SEQ ID NO: 1) and have one of the following characteristics: (1) a LAG3 polypeptide variant having 81 amino acids truncated at the N-terminus , terminating at amino acid position 121 at the C-terminus, and having mutations at the R110, and/or R113, and/or R119. (2) a LAG3 polypeptide variant having 99 amino acids truncated at the N-terminus, terminating at amino acid position 146 at the C-terminus, and having mutations at R129, and/or G130, and/or R141. (3) a LAG3 polypeptide variant having 81 amino acids truncated at N-terminus, terminating at amino acid position 146 at C-terminus, and having mutations at R110, and/or R113, and/or R119, and/or R129, and/or G130, and/or R141.

In certain preferred embodiments, the R110 position is mutated to K110.

In certain preferred embodiments, the R113 position is mutated to K113.

In certain preferred embodiments, the R119 position is mutated to K119.

In certain preferred embodiments, the R129 position is mutated to K129.

In certain preferred embodiments, the G130 position is mutated to P130, or A130, or T130, or Y130, or S130.

In certain preferred embodiments, the R141 position is mutated to K141.

In some embodiments, the amino acid sequence of the LAG3 variant polypeptide is selected from 104-121 R110K (SEQ ID NO: 1285), 104-121 R113K (SEQ ID NO: 1286), 104-121 R110K/R113K (SEQ ID NO: 1287), 104-121 R119K (SEQ ID NO: 1288), 104-121 R110K/R119K (SEQ ID NO: 1289), 104-121 R113K/R119K (SEQ ID NO: 1290), 104-121 R110K/R113K/R119K (SEQ ID NO: 1291), 122-146 R129K (SEQ ID NO: 1292), 122-146 G130P(SEQ ID NO: 1293), 122-146 G130A (SEQ ID NO:1294), 122-146 G130T (SEQ ID NO:1295), 122-146 G130Y (SEQ ID NO:1296), 122-146 G130S (SEQ ID NO:1297), 122-146 R141K (SEQ ID NO:1298), 122-146 R129K/G130P (SEQ ID NO:1299), 122-146 R129K/R141K (SEQ ID NO:1300), 122-146 G130P/R141K (SEQ ID NO:1301), 122-146 G130A/R141K (SEQ ID NO:1302), 122-146 G130T/R141K (SEQ ID NO:1303), 122-146 G130Y/R141K (SEQ ID NO:1304), 122-146 G130S/R141K (SEQ ID NO:1305), 104-146 R110K (SEQ ID NO:1306), 104-146 R113K (SEQ ID NO:1307), 104-146 R129K (SEQ ID NO:1308), 104-146 G130P (SEQ ID NO:1309), 104-146 G130A(SEQ ID NO:1310), 104-146 G130T (SEQ ID NO:1311), 104-146 G130Y (SEQ ID NO:1312), 104-146 G130S (SEQ ID NO:1313), 104-146 R141K (SEQ ID NO:1314), 104-146 R110K/G130P (SEQ ID NO:1315), 104-146 G130P/R141K (SEQ ID NO:1316), 104-146 R110K/R141K (SEQ ID NO:1317), 104-146 R110K/G130P/R141K (SEQ ID NO:1318).

In another aspect, the present invention provides a LAG3 polypeptide variant fusion polypeptide complex comprising the LAG3 variant polypeptide and a second domain.

In some embodiments, the second domain is an immunoglobulin Fc fragment, and the LAG3 variant polypeptide can be directly or indirectly linked to the N-terminus of the second domain.

In some embodiments, the LAG3 variant polypeptide is covalently linked to the second domain with or without a linker peptide. For example, the indirect connection may comprise connection via a linker. For example, the linker may comprise an amino acid sequence selected from the group consisting of SEQ ID NO:31, SEQ ID NO:32, SEQ ID NO:33, SEQ ID NO:34 and SEQ ID NO:35.

In some embodiments, the Fc domain is selected from human IgG1 Fc domain (SEQ ID NO:29), human IgG2 Fc domain (SEQ ID NO:634), human IgG3 Fc domain (SEQ ID NO:635), human IgG4 Fc domain (SEQ ID NO:30), mouse IgG1 Fc domain (SEQ ID NO:636), mouse IgG2a Fc domain (SEQ ID NO:637), mouse IgG2b Fc domain (SEQ ID NO:638) or mouse IgG3 Fc domain (SEQ ID NO:639).

In some embodiments, the LAG3 variant polypeptide is covalently linked to the N-terminus of the immunoglobulin Fc domain with or without a linker peptide.

In some embodiments, the amino acid sequence of the LAG3 variant fusion polypeptide complex is selected from 104-121 R110K-Fc (SEQ ID NO:1319), 104-121 R113K-Fc (SEQ ID NO:1320), 104-121 R110K/R113K-Fc (SEQ ID NO:1321), 104-121 R119K-Fc (SEQ ID NO:1322), 104-121 R110K/R119K-Fc (SEQ ID NO:1323), 104-121 R113K/R119K-Fc (SEQ ID NO:1324), 104-121 R110K/R113K/R119K-Fc (SEQ ID NO:1325), 122-146 R129K-Fc (SEQ ID NO:1326), 122-146 G130P-Fc (SEQ ID NO:1327), 122-146 G130A-Fc (SEQ ID NO:1328), 122-146 G130T-Fc (SEQ ID NO:1329), 122-146 G130Y-Fc (SEQ ID NO:1330), 122-146 G130S-Fc (SEQ ID NO:1331), 122-146 R141K-Fc (SEQ ID NO:1332), 122-146 R129K/G130P-Fc (SEQ ID NO:1333), 122-146 R129K/R141K-Fc (SEQ ID NO:1334), 122-146 G130P/R141K-Fc (SEQ ID NO:1335), 122-146 G130A/R141K-Fc (SEQ ID NO:1336), 122-146 G130T/R141K-Fc (SEQ ID NO:1337), 122-146 G130Y/R141K-Fc (SEQ ID NO:1338), 122-146 G130S/R141K-Fc (SEQ ID NO:1339), 104-146 R110K-Fc (SEQ ID NO:1340), 104-146 R113K-Fc (SEQ ID NO:1341), 104-146 R129K-Fc (SEQ ID NO:1342), 104-146 G130P-Fc (SEQ ID NO:1343), 104-146 G130A-Fc (SEQ ID NO:1344), 104-146 G130T-Fc (SEQ ID NO:1345), 104-146 G130Y-Fc (SEQ ID NO:1346), 104-146 G130S-Fc (SEQ ID NO:1347), 104-146 R141K-Fc (SEQ ID NO:1348), 104-146 R110K/G130P-Fc (SEQ ID NO:1349), 104-146 G130P/R141K-Fc (SEQ ID NO:1350), 104-146 R110K/R141K-Fc (SEQ ID NO:1351), 104-146 R110K/G130P/R141K-Fc (SEQ ID NO:1352).

In other embodiments, the second domain is an antibody or an antigen-binding fragment thereof, and the LAG3 variant polypeptide can be directly or indirectly linked to the N-terminus of the second domain.

In some embodiments, the antibody is selected from an immunoglobulin IgG antibody, a recombinant antibody, a chimeric antibody, a heavy chain antibody, a single domain antibody, and/or a bispecific antibody.

In some embodiments, the immunoglobulin IgG antibody is selected from human IgG1, human IgG2, human IgG3, human IgG4, mouse IgG1, mouse IgG2a, mouse IgG2b or mouse IgG3.

In some embodiments, the antigen binding fragment is selected from Fab, Fab', Fv, F(ab)2, F(ab')2, scFv, VHH, di-scFv and/or dAb.

In some embodiments, the LAG3 variant polypeptide is covalently linked to the second domain with or without a linker peptide.

For example, the second domain can bind to one or more of the targets shown in the following group: PD-L1, PD-L2, PD-1, OX40, 4-1BB, ICOS, TIGIT, CTLA4, LAG3, CD3, VEGF, VEGFR, CD47, HGF, Trop2, EpCAM, CCR8, CCR4, CCR5, GPRC5D, BCMA, CD19, CD20, HER-2 neu, DLL1, HER-3, HER-4, EGFR, PSMA, CEA, MUC-1 (mucin), MUC2, MUC3, MUC4, MUC5AC, MUC5B, MUC7, CD123, CD33, CD30, CD38, NKG2A, Nkp36 and Tim3.

For example, the second domain is capable of binding to PD-L1 and/or PD-1.

For example, the second domain is an antibody that binds to PD-L1 and/or PD-1, including but not limited to Sugemalimab, Durvalumab, Atezolizumab, Avelumab, Envafolimab, Permbrolizumab and Nivolumab.

In some embodiments, the heavy chain amino acid sequence of the LAG3 variant fusion Sugemalimab antibody polypeptide complex is selected from 104-121 R110K-Sugemalimab (SEQ ID NO: 1353), 104-121 R113K-Sugemalimab (SEQ ID NO: 1354), 104-121 R110K/R113K-Sugemalimab (SEQ ID NO: 1355), 104-121 R119K-Sugemalimab (SEQ ID NO: 1356), 104-121 R110K/R119K-Sugemalimab (SEQ ID NO: 1357), 104-121 R113K/R119K-Sugemalimab (SEQ ID NO: 1358), 104-121 R110K/R113K/R119K-Sugemalimab (SEQ ID NO: 1359), 122-146 R129K-Sugemalimab (SEQ ID NO:1360), 122-146 G130P-Sugemalimab (SEQ ID NO:1361), 122-146 G130A-Sugemalimab (SEQ ID NO:1362), 122-146 G130T-Sugemalimab (SEQ ID NO:1363), 122-14 G130Y-Sugemalimab (SEQ ID NO:1364), 122-146 G130S-Sugemalimab (SEQ ID NO:1365), 122-146 R141K-Sugemalimab (SEQ ID NO:1366), 122-146 R129K/G130P-Sugemalimab (SEQ ID NO:1367), 122-146 R129K/R141K-Sugemalimab (SEQ ID NO:1368), 122-146 G130P/R141K-Sugemalimab (SEQ ID NO:1369), 122-146 G130A/R141K-Sugemalimab (SEQ ID NO:1370), 122-146 G130T/R141K-Sugemalimab (SEQ ID NO:1371), 122-146 G130Y/R141K-Sugemalimab (SEQ ID NO:1372), 122-146 G130S/R141K-Sugemalimab (SEQ ID NO:1373), 104-146 R110K-Sugemalimab (SEQ ID NO:1374), 104-146 R113K-Sugemalimab (SEQ ID NO:1375), 104-146 R129K-Sugemalimab (SEQ ID NO:1376), 104-146 G130P-Sugemalimab (SEQ ID NO:1377), 104-146 G130A-Sugemalimab (SEQ ID NO:1378), 104-146 G130T-Sugemalimab (SEQ ID NO:1379), 104-146 G130Y-Sugemalimab (SEQ ID NO:1380), 104-146 G130S-Sugemalimab (SEQ ID NO:1381), 104-146 R141K-Sugemalimab (SEQ ID NO:1382), 104-146 R110K/G130P-Sugemalimab (SEQ ID NO:1383), 104-146 G130P/R141K-Sugemalimab (SEQ ID NO:1384), 104-146 R110K/R141K-Sugemalimab (SEQ ID NO:1385), 104-146 R110K/G130P/R141K-Sugemalimab (SEQ ID NO:1386).

The LAG3 variant fused Sugemalimab antibody heavy chain polypeptide is combined with the Sugemalimab antibody light chain polypeptide (SEQ ID NO: 5) to form a LAG3 variant fused Sugemalimab antibody polypeptide complex.

**In** another aspect, the present invention provides a LAG3 polypeptide variant fusion polypeptide complex that may also have the following characteristics:
For example, the second domain may comprise an antibody heavy chain, and the LAG3 variant polypeptide may be directly or indirectly linked to the antibody heavy chain of the second domain.

For example, the second domain may comprise an antibody heavy chain, and the LAG3 variant polypeptide may be directly or indirectly linked to the C-terminus of the antibody heavy chain of the second domain.

For example, the second domain may comprise an antibody heavy chain, and the N-terminus of the LAG3 variant polypeptide may be directly or indirectly linked to the C-terminus of the antibody heavy chain of the second domain.

For example, the second domain may comprise an antibody heavy chain, and the LAG3 variant polypeptide may be directly or indirectly linked to the N-terminus of the antibody heavy chain of the second domain.

For example, the second domain may comprise an antibody heavy chain, and the C-terminus of the LAG3 variant polypeptide may be directly or indirectly linked to the N-terminus of the antibody heavy chain of the second domain.

For example, the second domain may comprise an antibody light chain, and the LAG3 variant polypeptide may be directly or indirectly linked to the antibody light chain of the second domain.

For example, the second domain may comprise an antibody light chain, and the LAG3 variant polypeptide may be directly or indirectly linked to the C-terminus of the antibody light chain of the second domain.

For example, the second domain may comprise an antibody light chain, and the N-terminus of the LAG3 variant polypeptide may be directly or indirectly linked to the C-terminus of the antibody light chain of the second domain.

For example, the second domain may comprise an antibody light chain, and the LAG3 variant polypeptide may be directly or indirectly linked to the N-terminus of the antibody light chain of the second domain.

For example, the second domain may comprise an antibody light chain, and the C-terminus of the LAG3 variant polypeptide may be directly or indirectly linked to the N-terminus of the antibody light chain of the second domain.

In another aspect, the present invention provides a multifunctional LAG3 polypeptide variant fusion polypeptide complex, which comprises the LAG3 variant polypeptide, the second domain and the third domain.

In some specific embodiments, the second domain is capable of binding to PD-L1 and/or PD-1, and the third domain is a CD86 mutant.

The CD86 mutant may include an amino acid substitution mutant of the IgV domain (SEQ ID NO: 3) of human CD86, and the amino acid substitution mutation site may include one or more of A13I, A13L, A13F, A13M, Q25A, Q25I, Q25V, Q25F, Q25M, F33A, F33L, F33V, F33M, M60R, I89A, I89L, I89V, I89F, I89M, H90I, H90V, H90M, and H90F. For example, the CD86 IgV mutant polypeptide includes a combination of 1, 2, 3, 4, 5 or more of the above-mentioned amino acid mutations.

For example, the amino acid substitution mutant of the IgV domain of CD86 can be selected from the combination of amino acid mutation sites shown in the following group: Q25I/F33L/H90I, Q25V/F33L/H90I, Q25I/F33V/H90I, Q25V/F33V/H90I, Q25I/F33L/H90V, Q25V/F33L/H90V, Q25I/F33V/H90V, Q25V/F33V/H90V, A13L/Q25I/F33L/H90I, A13I/Q25I/F33L/H90I, A13L/Q25V/F33L/H90I, A13I/Q25V/F33L/H90I, Q25I/F33L/I89L/H90I, Q25I/F33L/M60R/H90I, Q25V/F33L/I89L/H90I, Q25V/F33L/M60R/H90I, Q25F/F33L/H90I, Q25I/F33L/H90F, Q25I/F33A/H90I, Q25I/H90I, Q25I, H90I, Q25V/H90V, Q25V/H90I, Q25F/H90I, Q25F/H90V, A13F/Q25I/F33L/H90I, A13M/Q25I/F33L/H90I, Q25A/F33L/H90I, Q25M/F33L/H90I, Q25I/F33M/H90I, Q25I/F33L/I89V/H90I, Q25I/F33L/I89F/H90I, Q25I/F33L/I89M/H90I and Q25I/F33L/H90M.

For example, the variant polypeptide may comprise the IgV domain amino acid substitution mutant Q25I/F33L/H90I of CD86.

In some embodiments, the heavy chain amino acid sequence of the multifunctional LAG3 variant fused Sugemalimab-CD86 IgV Q25I/F33L/H90I antibody polypeptide complex is selected from: 104-121 R110K-Sugemalimab (SEQ ID NO:1387), 104-121 R113K-Sugemalimab-CD86 IgV Q25I/F33L/H90I (SEQ ID NO:1388), 104-121 R110K/R113K-Sugemalimab-CD86 IgV Q25I/F33L/H90I (SEQ ID NO:1389), 104-121 R119K-Sugemalimab-CD86 IgV Q25I/F33L/H90I (SEQ ID NO:1390), 104-121 R110K/R119K-Sugemalimab-CD86 IgV Q25I/F33L/H90I (SEQ ID NO:1391), 104-121 R113K/R119K-Sugemalimab-CD86 IgV Q25I/F33L/H90I (SEQ ID NO:1392), 104-121 R110K/R113K/R119K-Sugemalimab-CD86 IgV Q25I/F33L/H90I (SEQ ID NO:1393), 122-146 R129K-Sugemalimab-CD86 IgV Q25I/F33L/H90I (SEQ ID NO:1394), 122-146 G130P-Sugemalimab-CD86 IgV Q25I/F33L/H90I (SEQ ID NO:1395), 122-146 G130A-Sugemalimab-CD86 IgV Q25I/F33L/H90I (SEQ ID NO:1396), 122-146 G130T-Sugemalimab-CD86 IgV Q25I/F33L/H90I (SEQ ID NO:1397), 122-146 G130Y-Sugemalimab-CD86 IgV Q25I/F33L/H90I (SEQ ID NO:1398), 122-146 G130S-Sugemalimab-CD86 IgV Q25I/F33L/H90I (SEQ ID NO:1399), 122-146 R141K-Sugemalimab-CD86 IgV Q25I/F33L/H90I (SEQ ID NO:1400), 122-146 R129K/G130P-Sugemalimab-CD86 IgV Q25I/F33L/H90I (SEQ ID NO:1401), 122-146 R129K/R141K-Sugemalimab-CD86 IgV Q25I/F33L/H90I (SEQ ID NO:1402), 122-146 G130P/R141K-Sugemalimab-CD86 IgV Q25I/F33L/H90I (SEQ ID NO:1403), 122-146 G130A/R141K-Sugemalimab-CD86 IgV Q25I/F33L/H90I (SEQ ID NO:1404), 122-146 G130T/R141K-Sugemalimab-CD86 IgV Q25I/F33L/H90I (SEQ ID NO:1405), 122-146 G130Y/R141K-Sugemalimab-CD86 IgV Q25I/F33L/H90I (SEQ ID NO:1406), 122-146 G130S/R141K-Sugemalimab-CD86 IgV Q25I/F33L/H90I (SEQ ID NO:1407), 104-146 R110K-Sugemalimab-CD86 IgV Q25I/F33L/H90I (SEQ ID NO:1408), 104-146 R113K-Sugemalimab-CD86 IgV Q25I/F33L/H90I (SEQ ID NO:1409), 104-146 R129K-Sugemalimab-CD86 IgV Q25I/F33L/H90I (SEQ ID NO:1410), 104-146 G130P-Sugemalimab-CD86 IgV Q25I/F33L/H90I (SEQ ID NO:1411), 104-146 G130A-Sugemalimab-CD86 IgV Q25I/F33L/H90I (SEQ ID NO:1412), 104-146 G130T-Sugemalimab-CD86 IgV Q25I/F33L/H90I (SEQ ID NO:1413), 104-146 G130Y-Sugemalimab-CD86 IgV Q25I/F33L/H90I (SEQ ID NO:1414), 104-146 G130S-Sugemalimab-CD86 IgV Q25I/F33L/H90I (SEQ ID NO:1415), 104-146 R141K-Sugemalimab-CD86 IgV Q25I/F33L/H90I (SEQ ID NO:1416), 104-146 R110K/G130P-Sugemalimab-CD86 IgV Q25I/F33L/H90I (SEQ ID NO:1417), 104-146 G130P/R141K-Sugemalimab-CD86 IgV Q25I/F33L/H90I (SEQ ID NO:1418), 104-146 R110K/R141K-Sugemalimab-CD86 IgV Q25I/F33L/H90I (SEQ ID NO:1419), 104-146 R110K/G130P/R141K-Sugemalimab-CD86 IgV Q25I/F33L/H90I (SEQ ID NO:1420).

The multifunctional LAG3 variant fusion Sugemalimab-CD86 IgV Q25I/F33L/H90I antibody heavy chain polypeptide is combined with the Sugemalimab antibody light chain polypeptide (SEQ ID NO: 5) to form a multifunctional LAG3 variant fusion Sugemalimab-CD86 IgV Q25I/F33L/H90I multifunctional complex.

For example, the multifunctional antibody Abs-1022 comprises a chimeric multifunctional antibody first polypeptide (SEQ ID NO: 1421) (formed by covalently linking the C-terminus of the LAG3 variant LAG3 101-121 polypeptide to the N-terminus of the sugemalimab antibody heavy chain via a linker peptide (SEQ ID NO: 34), and the C-terminus of the sugemalimab antibody heavy chain further covalently linked to the CD86 IgV mutant Q25I/F33L/H90I via a linker peptide (SEQ ID NO: 34)). The functional antibody Abs-1022 is assembled by introducing a polynucleotide sequence (SEQ ID NO: 1422) vector expressing the first polypeptide of Abs-1022 and a polynucleotide sequence (SEQ ID NO: 1423) vector expressing the second polypeptide of Abs-1022 into host cells for expression.

For example, the multifunctional antibody Abs-1045 comprises a chimeric multifunctional antibody first polypeptide (SEQ ID NO: 1387) (formed by covalently linking the C-terminus of the LAG3 variant LAG3 104-121 R110K polypeptide to the N-terminus of the sugemalimab antibody heavy chain via a linker peptide (SEQ ID NO: 35), and the C-terminus of the sugemalimab antibody heavy chain further covalently linked to the CD86 IgV mutant Q25I/F33L/H90I via a linker peptide (SEQ ID NO: 35)). The functional antibody Abs-1045 is assembled by introducing a polynucleotide sequence (SEQ ID NO: 1424) vector expressing the first polypeptide of Abs-1045 and a polynucleotide sequence (SEQ ID NO: 1423) vector expressing the second polypeptide of Abs-1045 into host cells for expression.

For example, the multifunctional antibody Abs-1036 comprises a chimeric multifunctional antibody first polypeptide (SEQ ID NO: 1393) (formed by covalently linking the C-terminus of the LAG3 variant LAG3 104-121 R110K/R113K/R119K polypeptide to the N-terminus of the sugemalimab antibody heavy chain via a linker peptide (SEQ ID NO: 35), and the C-terminus of the sugemalimab antibody heavy chain further covalently linked to the CD86 IgV mutant Q25I/F33L/H90I via a linker peptide (SEQ ID NO: 35)). The functional antibody Abs-1036 is assembled by introducing a polynucleotide sequence (SEQ ID NO: 1425) vector expressing the first polypeptide of Abs-1036 and a polynucleotide sequence (SEQ ID NO: 1423) vector expressing the second polypeptide of Abs-1036 into host cells for expression.

For example, the multifunctional antibody Abs-1000 comprises a chimeric multifunctional antibody first polypeptide (SEQ ID NO: 1426) (formed by covalently linking the C-terminus of the LAG3 variant LAG3 122-146 polypeptide to the N-terminus of the sugemalimab antibody heavy chain via a linker peptide (SEQ ID NO: 35), and the C-terminus of the sugemalimab antibody heavy chain further covalently linked to the CD86 IgV mutant Q25I/F33L/H90I via a linker peptide (SEQ ID NO: 35)). The functional antibody Abs-1000 is assembled by introducing a polynucleotide sequence (SEQ ID NO: 1427) vector expressing the first polypeptide of Abs-1000 and a polynucleotide sequence (SEQ ID NO: 1423) vector expressing the second polypeptide of Abs-1000 into host cells for expression.

For example, the multifunctional antibody Abs-1028 comprises a chimeric multifunctional antibody first polypeptide (SEQ ID NO: 1395) (formed by covalently linking the C-terminus of the LAG3 variant LAG3 122-146 G130P polypeptide to the N-terminus of the sugemalimab antibody heavy chain via a linker peptide (SEQ ID NO: 35), and the C-terminus of the sugemalimab antibody heavy chain further covalently linked to the CD86 IgV mutant Q25I/F33L/H90I via a linker peptide (SEQ ID NO: 35)). The functional antibody Abs-1028 is assembled by introducing a polynucleotide sequence (SEQ ID NO: 1428) vector expressing the first polypeptide of Abs-1028 and a polynucleotide sequence (SEQ ID NO: 1423) vector expressing the second polypeptide of Abs-1028 into host cells for expression.

For example, the multifunctional antibody Abs-1038 comprises a chimeric multifunctional antibody first polypeptide (SEQ ID NO: 1403) (formed by covalently linking the C-terminus of the LAG3 variant LAG3 122-146 G130P/R141K polypeptide to the N-terminus of the sugemalimab antibody heavy chain via a linker peptide (SEQ ID NO: 35), and the C-terminus of the sugemalimab antibody heavy chain further covalently linked to the CD86 IgV mutant Q25I/F33L/H90I via a linker peptide (SEQ ID NO: 35)). The functional antibody Abs-1038 is assembled by introducing a polynucleotide sequence (SEQ ID NO: 1429) vector expressing the first polypeptide of Abs-1038 and a polynucleotide sequence (SEQ ID NO: 1423) vector expressing the second polypeptide of Abs-1038 into host cells for expression.

For example, the multifunctional antibody Abs-1026 comprises a chimeric multifunctional antibody first polypeptide (SEQ ID NO: 1402) (formed by covalently linking the C-terminus of the LAG3 variant LAG3 122-146 R129K/R141K polypeptide to the N-terminus of the sugemalimab antibody heavy chain via a linker peptide (SEQ ID NO: 35), and the C-terminus of the sugemalimab antibody heavy chain further covalently linked to the CD86 IgV mutant Q25I/F33L/H90I via a linker peptide (SEQ ID NO: 35)). The functional antibody Abs-1026 is assembled by introducing a polynucleotide sequence (SEQ ID NO: 1430) vector expressing the first polypeptide of Abs-1026 and a polynucleotide sequence (SEQ ID NO: 1423) vector expressing the second polypeptide of Abs-1026 into host cells for expression.

For example, the multifunctional antibody Abs-1027 comprises a chimeric multifunctional antibody first polypeptide (SEQ ID NO: 1404) (formed by covalently linking the C-terminus of the LAG3 variant LAG3 122-146 G130A/R141K polypeptide to the N-terminus of the sugemalimab antibody heavy chain via a linker peptide (SEQ ID NO: 35), and the C-terminus of the sugemalimab antibody heavy chain further covalently linked to the CD86 IgV mutant Q25I/F33L/H90I via a linker peptide (SEQ ID NO: 35)). The functional antibody Abs-1027 is assembled by introducing a polynucleotide sequence (SEQ ID NO: 1431) vector expressing the first polypeptide of Abs-1027 and a polynucleotide sequence (SEQ ID NO: 1423) vector expressing the second polypeptide of Abs-1027 into host cells for expression.

For example, the multifunctional antibody Abs-1024 comprises a chimeric multifunctional antibody first polypeptide (SEQ ID NO: 1396) (formed by covalently linking the C-terminus of the LAG3 variant LAG3 122-146 G130A polypeptide to the N-terminus of the sugemalimab antibody heavy chain via a linker peptide (SEQ ID NO: 35), and the C-terminus of the sugemalimab antibody heavy chain further covalently linked to the CD86 IgV mutant Q25I/F33L/H90I via a linker peptide (SEQ ID NO: 35)). The functional antibody Abs-1024 is assembled by introducing a polynucleotide sequence (SEQ ID NO: 1432) vector expressing the first polypeptide of Abs-1024 and a polynucleotide sequence (SEQ ID NO: 1423) vector expressing the second polypeptide of Abs-1024 into host cells for expression.

For example, the multifunctional antibody Abs-1046 comprises a chimeric multifunctional antibody first polypeptide (SEQ ID NO: 1420) (formed by covalently linking the C-terminus of the LAG3 variant LAG3 104-146 R110K/G130P/R141K polypeptide to the N-terminus of the sugemalimab antibody heavy chain via a linker peptide (SEQ ID NO: 35), and the C-terminus of the sugemalimab antibody heavy chain further covalently linked to the CD86 IgV mutant Q25I/F33L/H90I via a linker peptide (SEQ ID NO: 35)). The functional antibody Abs-1046 is assembled by introducing a polynucleotide sequence (SEQ ID NO: 1433) vector expressing the first polypeptide of Abs-1046 and a polynucleotide sequence (SEQ ID NO: 1423) vector expressing the second polypeptide of Abs-1046 into host cells for expression.

For example, the LAG3 variant fusion protein Abs-1034 is expressed by combining LAG3 variants 104-121 R113K-Fc (SEQ ID NO: 1320).

For example, the LAG3 variant fusion protein Abs-1036 is expressed by combining LAG3 variants 104-121 R110K/R113K/R119K-Fc (SEQ ID NO: 1325).

For example, the LAG3 variant fusion protein Abs-1036 is expressed by combining LAG3 variants 122-146 G130P/R141K-Fc (SEQ ID NO: 1335).

In another aspect, the present invention provides a multifunctional LAG3 polypeptide variant fusion polypeptide complex that may also have the following characteristics:
For example, the fusion polypeptide complex can include a first polypeptide and a second polypeptide. For example, the second polypeptide may be absent.

For example, the first polypeptide of the fusion polypeptide complex may comprise a second domain, the second domain may comprise an antibody or an antigen-binding fragment thereof, and the second polypeptide may comprise a light chain of the antibody or an antigen-binding fragment thereof.

For example, the first polypeptide of the fusion polypeptide complex may comprise a second domain, the second domain may comprise a heavy chain of an antibody or an antigen-binding fragment thereof, and the second polypeptide may comprise a light chain of the antibody or an antigen-binding fragment thereof.

For example, the fusion polypeptide complex may comprise a second domain, the second domain may comprise a heavy chain, and the second domain heavy chain may represent an Fc peptide chain or a heavy chain of an antibody or an antigen-binding fragment thereof.

For example, the first polypeptide comprises a second binding domain heavy chain, wherein the second binding domain heavy chain represents an Fc peptide chain or a heavy chain of an antibody or antigen-binding fragment thereof.

For example, the first polypeptide of the fusion polypeptide complex may include, from N-terminus to C-terminus, a LAG3 variant polypeptide, a linker peptide, a second binding domain heavy chain, a linker peptide, and a third binding domain, wherein the linker peptides are independently present or absent.

For example, the first polypeptide of the fusion polypeptide complex includes, from N-terminus to C-terminus, a third binding domain, a linker peptide, a second binding domain heavy chain, a linker peptide and a LAG3 variant polypeptide, wherein the linker peptides are independently present or absent.

For example, the first polypeptide of the fusion polypeptide complex includes, from N-terminus to C-terminus, a LAG3 variant polypeptide, a linker peptide, a third binding domain, a linker peptide and a second binding domain heavy chain, wherein the linker peptides are independently present or absent.

For example, the first polypeptide of the fusion polypeptide complex includes, from N-terminus to C-terminus, a third binding domain, a linker peptide, a LAG3 variant polypeptide, a linker peptide and a second binding domain heavy chain, wherein the linker peptides are independently present or absent.

For example, the first polypeptide of the fusion polypeptide complex includes, from N-terminus to C-terminus, a second binding domain heavy chain, a linker peptide, a LAG3 variant polypeptide, a linker peptide and a third binding domain, wherein the linker peptides are independently present or absent.

For example, the first polypeptide of the fusion polypeptide complex includes, from N-terminus to C-terminus, a second binding domain heavy chain, a linker peptide, a third binding domain, a linker peptide and a LAG3 variant polypeptide, wherein the linker peptides are independently present or absent.

In another aspect, the present invention relates to a polynucleotide encoding the above-mentioned variant polypeptide or a variant thereof and a vector containing the polynucleotide.

In another aspect, the present invention relates to a polynucleotide encoding the above-mentioned truncated protein or a variant thereof, a vector containing the polynucleotide, and a pharmaceutically acceptable carrier.

Vectors that can be used to insert a polynucleotide of interest are well known in the art and include, but are not limited to, cloning vectors and expression vectors. In one embodiment, the vector is, for example, a plasmid.

In another aspect, the present invention also relates to a host cell comprising the above polynucleotide or vector. Such host cells include, but are not limited to, eukaryotic cells such as animal cells (eg, mammalian cells, such as mouse cells, human cells, etc.), yeast cells, insect cells and plant cells; particularly preferably, the host cell of the present invention is a mammalian cell CHO cell.

The present invention provides a method for preparing and purifying the above-mentioned variant fusion polypeptide complex, comprising culturing the host cell to express the LAG3 variant fusion polypeptide complex, and purifying to obtain the variant fusion polypeptide complex.

The present invention provides a method for regulating the activity of antigen presenting cells, comprising contacting the LAG3 variant fusion polypeptide complex with antigen presenting cells to stimulate their activation.

The pharmaceutical composition of the present invention can be administered by methods known in the art, such as but not limited to administration by injection. In certain preferred embodiments, the pharmaceutical compositions of the present invention are administered in unit dosage form.

In another aspect, the present invention provides the use of the LAG3 variant polypeptide or its fusion polypeptide complex, immunoconjugate, composition, polynucleotide, vector or host cell of the present invention to prepare a medicament, wherein the medicament is used to prevent, improve and/or treat tumors or cancers. Wherein, the tumor includes solid tumor and hematological tumor.

In another aspect, the LAG3 variant polypeptides and fusion polypeptide complexes disclosed in the present invention have anti-infection effects, including but not limited to infections by bacteria, viruses and parasites.

On the other hand, the present application provides a fusion polypeptide, which comprises a first domain and a second domain, wherein the first domain may comprise a LAG3 variant polypeptide, and the second domain may comprise an antibody or an antigen-binding fragment thereof or other forms of polypeptides.

In one embodiment, the second domain is a PD1/PDL1 antibody or an antigen-binding fragment thereof. The fusion polypeptide complex can simultaneously interfere with, inhibit, or block the programmed death ligand-1 (PD-L1) antibody/programmed death-1 (PD-1) signal transduction pathway and/or synergistically stimulate antigen-presenting cell activation and/or T-cell activation, effectively activating T cells to enhance immune responses and/or stimulating antigen-presenting cells. It may also have potential tumor-killing effects, thus enabling therapeutic applications in diseases caused by T-cell dysfunction, such as tumors. Notably, it may improve efficacy in tumor patients who exhibit no or weak responses to anti-PD-1 or anti-PD-L1 antibodies. Preferably, the PD1/PDL1 antibody or antigen-binding fragment thereof can be sugemalimab, atezolizumab, Durvalumab, Avelumab, Envafolimab, Permbrolizumab, Nivolumab or antigen-binding fragment thereof. More preferably, the PD1/PDL1 antibody or antigen-binding fragment thereof is sugemalimab or an antigen-binding fragment thereof.

For example, the first domain may comprise a LAG3 variant polypeptide.

For example, the first domain may comprise a LAG3 variant polypeptide, which may comprise a truncation and/or mutation based on human LAG3.

For example, the first domain may comprise a LAG3 variant polypeptide, which may have 0-124 amino acids truncated at the N-terminus based on a wild-type human LAG3 extracellular region polypeptide.

For example, the first domain may comprise a LAG3 variant polypeptide, which may, based on a wild-type human LAG3 extracellular region polypeptide, have 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75 , 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 , 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95 , 96, 97, 98, 99, 100, 101, 102, 103, 104, 105, 106, 107, 108, 109, 110, 111, 112, 113, 114, 115, 116, 117, 118, 119, 120, 121, 122, 123 or 124 amino acids truncated at N-terminus.

For example, the first domain may comprise a LAG3 variant polypeptide, which may have 0, 5, 21, 37, 45, 60, 71, 74, 79, 84, 89, 94, 99, 104, 109 or 114 amino acids truncated at the N-terminus based on the wild-type human LAG3 extracellular region polypeptide.

For example, the first domain may comprise a LAG3 variant polypeptide, which may, based on a wild-type human LAG3 extracellular region polypeptide, have 0, 5, 21, 37, 45, 60, 71 or 74 amino acids truncated at the N-terminusbased on.

For example, the first domain may comprise a LAG3 variant polypeptide, which may, based on a wild-type human LAG3 extracellular region polypeptide, terminate at amino acid positions 122-167 at the C-terminus.

For example, the first domain may comprise a LAG3 variant polypeptide, which may, based on a wild-type human LAG3 extracellular region polypeptide, terminate at any position corresponding to amino acid positions 122-167, 123-167, 124-167, 125-167, 126-167, 127-167, 128-167, 129-167, 130-167, 131-167, 132-167, 133-167, 134-167, 135-167, 136-167, 137-167, 138-167, 139-167, 140-167, 141-167, 142-167, 143-167, 144-167, 145-167, 146-167, 147-167, 148-167, 149-167, 150-167, 151-167, 152-167, 153-167, 154-167, 155-167, 156-167, 157-167, 158-167, 159-167, 160-167, 161-167, 162-167, 163-167, 164-167, 165-167 or 166-167 at C-terminus.

For example, the first domain may comprise a LAG3 variant polypeptide, which may, based on a wild-type human LAG3 extracellular region polypeptide, terminate at amino acid position 122, 129, 136, 146, 156, 157, 158, 159, 160, 161, 162, 163, 164, 165, 166 or 167 at the C-terminus.

For example, the first domain may comprise a LAG3 variant polypeptide, which may, based on a wild-type human LAG3 extracellular region polypeptide, terminate at amino acid position 122, 129, 136, 146, 156, 161 or 167 at the C-terminus.

For example, the first domain may comprise a LAG3 variant polypeptide, which may, based on a wild-type human LAG3 extracellular region polypeptide, terminate at amino acid position 156, 161 or 167 at the C-terminus.

For example, the first domain may comprise a LAG3 variant polypeptide, which may, based on a wild-type human LAG3 extracellular region polypeptide, have 0, 5, 21, 37, 45, 60, 71 or 74 amino acids truncated at the N-terminus, and terminate at amino acid position 122, 129, 136, 146, 156, 157, 158, 159, 160, 161, 162, 163, 164, 165, 166 or 167 at the C-terminus.

For example, the first domain may comprise a LAG3 variant polypeptide, and the LAG3 variant polypeptide may comprise an amino acid mutation.

For example, the first domain may comprise a LAG3 variant polypeptide, and the LAG3 variant polypeptide may comprise an amino acid mutation, wherein the amino acid mutation site is an Arg amino acid at site 97.

For example, the first domain may comprise a LAG3 variant polypeptide, and the LAG3 variant polypeptide may comprise an amino acid mutation, wherein the amino acid mutation site is a mutation of the Arg amino acid at site 97 to a Glu amino acid.

For example, the first domain may comprise a LAG3 variant polypeptide, which may, based on a wild-type human LAG3 extracellular region polypeptide, have 74 amino acids truncated at the N-terminus. For example, the first domain may comprise an amino acid sequence selected from the group consisting of SEQ ID NO: 244 to SEQ ID NO: 259. For example, the first domain can be selected from the group consisting of the amino acid sequences shown in SEQ ID NO: 244, SEQ ID NO: 245, SEQ ID NO: 246, SEQ ID NO: 247, SEQ ID NO: 248 and SEQ ID NO: 253.

For example, the first domain may comprise a LAG3 variant polypeptide, which may, based on a wild-type human LAG3 extracellular region polypeptide, have 74 amino acids truncated at the N-terminus, and the Arg amino acid at position 97 is mutated to a Glu amino acid. For example, the first domain may comprise an amino acid sequence selected from the group consisting of SEQ ID NO: 260 to SEQ ID NO: 275. For example, the first domain can be selected from the group consisting of the amino acid sequences shown in SEQ ID NO:260, SEQ ID NO:261, SEQ ID NO:262, SEQ ID NO:263, SEQ ID NO:264 and SEQ ID NO:269.

For example, the first domain may comprise a LAG3 variant polypeptide, which may, based on a wild-type human LAG3 extracellular region polypeptide, have 5 amino acids truncated at the N-terminus. For example, the first domain may comprise an amino acid sequence selected from the group consisting of SEQ ID NO: 276 to SEQ ID NO: 278.

For example, the first domain may comprise a LAG3 variant polypeptide, which may, based on a wild-type human LAG3 extracellular region polypeptide, have 5 amino acids truncated at the N-terminus, and the Arg amino acid at position 97 is mutated to a Glu amino acid. The first domain may comprise an amino acid sequence selected from the group consisting of SEQ ID NO: 279 to SEQ ID NO: 281.

For example, the first domain may comprise a LAG3 variant polypeptide, which may, based on a wild-type human LAG3 extracellular region polypeptide, have 21 amino acids truncated at the N-terminus. For example, the first domain may comprise an amino acid sequence selected from the group consisting of SEQ ID NO: 282 to SEQ ID NO: 284.

For example, the first domain may comprise a LAG3 variant polypeptide, which may, based on a wild-type human LAG3 extracellular region polypeptide, have 21 amino acids truncated at the N-terminus, and the Arg amino acid at position 97 is mutated to a Glu amino acid. For example, the first domain may comprise an amino acid sequence selected from the group consisting of SEQ ID NO: 288 to SEQ ID NO: 290.

For example, the first domain may comprise a LAG3 variant polypeptide, which may, based on a wild-type human LAG3 extracellular region polypeptide, have 37 amino acids truncated at the N-terminus. For example, the first domain may comprise an amino acid sequence selected from the group consisting of SEQ ID NO: 276 to SEQ ID NO: 278.

For example, the first domain may comprise a LAG3 variant polypeptide, which may, based on a wild-type human LAG3 extracellular region polypeptide, have 37 amino acids truncated at the N-terminus, and the Arg amino acid at position 97 is mutated to a Glu amino acid. For example, the first domain may comprise an amino acid sequence selected from the group consisting of SEQ ID NO: 291 to SEQ ID NO: 293.

For example, the first domain may comprise a LAG3 variant polypeptide, which may, based on a wild-type human LAG3 extracellular region polypeptide, have 45 amino acids truncated at the N-terminus. For example, the first domain may comprise an amino acid sequence selected from the group consisting of SEQ ID NO: 294 to SEQ ID NO: 296.

For example, the first domain may comprise a LAG3 variant polypeptide, which may, based on a wild-type human LAG3 extracellular region polypeptide, have 45 amino acids truncated at the N-terminus, and the Arg amino acid at position 97 is mutated to a Glu amino acid. For example, the first domain may comprise an amino acid sequence selected from the group consisting of SEQ ID NO: 297 to SEQ ID NO: 299.

For example, the first domain may comprise a LAG3 variant polypeptide, which may, based on a wild-type human LAG3 extracellular region polypeptide, have 60 amino acids truncated at the N-terminus. For example, the first domain may comprise an amino acid sequence selected from the group consisting of SEQ ID NO: 300 to SEQ ID NO: 302.

For example, the first domain may comprise a LAG3 variant polypeptide, which may, based on a wild-type human LAG3 extracellular region polypeptide, have 60 amino acids truncaterd at the N-terminus, and the Arg amino acid at position 97 is mutated to a Glu amino acid. For example, the first domain may comprise an amino acid sequence selected from the group consisting of SEQ ID NO: 303 to SEQ ID NO: 305.

For example, the first domain may comprise a LAG3 variant polypeptide, which may, based on a wild-type human LAG3 extracellular region polypeptide, have 71 amino acids truncated at the N-terminus. For example, the first domain may comprise an amino acid sequence selected from the group consisting of SEQ ID NO: 306 to SEQ ID NO: 308.

For example, the first domain may comprise a LAG3 variant polypeptide, which may, based on a wild-type human LAG3 extracellular region polypeptide, have 71 amino acids truncated at the N-terminus, and the Arg amino acid at position 97 is mutated to a Glu amino acid. For example, the first domain may comprise an amino acid sequence selected from the group consisting of SEQ ID NO: 309 to SEQ ID NO: 311.

For example, the first domain may comprise a LAG3 variant polypeptide, which may, based on a wild-type human LAG3 extracellular region polypeptide, have 79 amino acids truncated at the N-terminus. For example, the first domain may comprise an amino acid sequence selected from the group consisting of SEQ ID NO: 312 to SEQ ID NO: 314.

For example, the first domain may comprise a LAG3 variant polypeptide, which may, based on a wild-type human LAG3 extracellular region polypeptide, have 79 amino acids truncated at the N-terminus, and the Arg amino acid at position 97 is mutated to a Glu amino acid. For example, the first domain may comprise an amino acid sequence selected from the group consisting of SEQ ID NO: 312 to SEQ ID NO: 314.

For example, the first domain may comprise a LAG3 variant polypeptide, which may, based on a wild-type human LAG3 extracellular region polypeptide, have 84 amino acids truncated at the N-terminus. For example, the first domain may comprise an amino acid sequence selected from the group consisting of SEQ ID NO: 315 to SEQ ID NO: 317.

For example, the first domain may comprise a LAG3 variant polypeptide, which may, based on a wild-type human LAG3 extracellular region polypeptide, have 84 amino acids truncated at the N-terminus, and the Arg amino acid at position 97 is mutated to a Glu amino acid. For example, the first domain may comprise an amino acid sequence selected from the group consisting of SEQ ID NO: 315 to SEQ ID NO: 317.

For example, the first domain may comprise a LAG3 variant polypeptide, which may, based on a wild-type human LAG3 extracellular region polypeptide, have 89 amino acids truncated at the N-terminus. For example, the first domain may comprise an amino acid sequence selected from the group consisting of SEQ ID NO: 318 to SEQ ID NO: 320.

For example, the first domain may comprise a LAG3 variant polypeptide, which may, based on a wild-type human LAG3 extracellular region polypeptide, have 89 amino acids truncated at the N-terminus, and the Arg amino acid at position 97 is mutated to a Glu amino acid. For example, the first domain may comprise an amino acid sequence selected from the group consisting of SEQ ID NO: 318 to SEQ ID NO: 320.

For example, the first domain may comprise a LAG3 variant polypeptide, which may, based on a wild-type human LAG3 extracellular region polypeptide, have 94 amino acids truncated at the N-terminus. For example, the first domain may comprise an amino acid sequence selected from the group consisting of SEQ ID NO: 321 to SEQ ID NO: 323.

For example, the first domain may comprise a LAG3 variant polypeptide, which may, based on a wild-type human LAG3 extracellular region polypeptide, have 94 amino acids truncated at the N-terminus, and the Arg amino acid at position 97 is mutated to a Glu amino acid. For example, the first domain may comprise an amino acid sequence selected from the group consisting of SEQ ID NO: 321 to SEQ ID NO: 323.

For example, the first domain may comprise a LAG3 variant polypeptide, which may, based on a wild-type human LAG3 extracellular region polypeptide, have 99 amino acids truncated at the N-terminus. For example, the first domain may comprise an amino acid sequence selected from the group consisting of SEQ ID NO: 324 to SEQ ID NO: 326.

For example, the first domain may comprise a LAG3 variant polypeptide, which may, based on a wild-type human LAG3 extracellular region polypeptide, have 99 amino acids truncated at the N-terminus, and the Arg amino acid at position 97 is mutated to a Glu amino acid. For example, the first domain may comprise an amino acid sequence selected from the group consisting of SEQ ID NO: 324 to SEQ ID NO: 326.

For example, the first domain may comprise a LAG3 variant polypeptide, which may, based on a wild-type human LAG3 extracellular region polypeptide, have 104 amino acids truncated at the N-terminus. For example, the first domain may comprise an amino acid sequence selected from the group consisting of SEQ ID NO: 327 to SEQ ID NO: 329.

For example, the first domain may comprise a LAG3 variant polypeptide, which may, based on a wild-type human LAG3 extracellular region polypeptide, have 104 amino acids truncated at the N-terminus, and the Arg amino acid at position 97 is mutated to a Glu amino acid. For example, the first domain may comprise an amino acid sequence selected from the group consisting of SEQ ID NO: 327 to SEQ ID NO: 329.

For example, the first domain may comprise a LAG3 variant polypeptide, which may, based on a wild-type human LAG3 extracellular region polypeptide, have 109 amino acids truncated at the N-terminus. For example, the first domain may comprise an amino acid sequence selected from the group consisting of SEQ ID NO: 330 to SEQ ID NO: 332.

For example, the first domain may comprise a LAG3 variant polypeptide, which may, based on a wild-type human LAG3 extracellular region polypeptide, have 109 amino acids truncated at the N-terminus, and the Arg amino acid at position 97 is mutated to a Glu amino acid. For example, the first domain may comprise an amino acid sequence selected from the group consisting of SEQ ID NO: 330 to SEQ ID NO: 332.

For example, the first domain may comprise a LAG3 variant polypeptide, which may, based on a wild-type human LAG3 extracellular region polypeptide, have 114 amino acids truncated at the N-terminus. For example, the first domain may comprise an amino acid sequence selected from the group consisting of SEQ ID NO: 333 to SEQ ID NO: 335.

For example, the first domain may comprise a LAG3 variant polypeptide, which may, based on a wild-type human LAG3 extracellular region polypeptide, have 114 amino acids truncated at the N-terminus, and the Arg amino acid at position 97 is mutated to a Glu amino acid. For example, the first domain may comprise an amino acid sequence selected from the group consisting of SEQ ID NO: 333 to SEQ ID NO: 335.

For example, the first domain may comprise a LAG3 variant polypeptide, which may, based on a wild-type human LAG3 extracellular region polypeptide, have 0 amino acids truncated at the N-terminus. For example, the first domain may comprise an amino acid sequence selected from the group consisting of SEQ ID NO: 336 to SEQ ID NO: 342.

For example, the first domain may comprise a LAG3 variant polypeptide, which may, based on a wild-type human LAG3 extracellular region polypeptide, have 0 amino acids truncated at the N-terminus, and the Arg amino acid at position 97 is mutated to a Glu amino acid. For example, the first domain may comprise an amino acid sequence selected from the group consisting of SEQ ID NO: 343 to SEQ ID NO: 349.

For example, the second domain may comprise an antibody or an antigen-binding fragment thereof.

For example, the second domain can bind to one or more of the targets shown in the following group: PD-L1, PD-L2, PD-1, OX40, 4-1BB, ICOS, TIGIT, CTLA4, LAG3, CD3, VEGF, VEGFR, CD47, HGF, Trop2, EpCAM, CCR8, CCR4, CCR5, GPRC5D, BCMA, CD19, CD20, HER-2 neu, DLL1, HER-3, HER-4, EGFR, PSMA, CEA, MUC-1 (mucin), MUC2, MUC3, MUC4, MUC5AC, MUC5B, MUC7, CD123, CD33, CD30, CD38, NKG2A, Nkp36 and Tim3.

For example, the second domain is capable of binding to PD-L1 and/or PD-1.

For example, the second domain may comprise HCDR3 of an antibody heavy chain, which may comprise an amino acid sequence selected from the group consisting of SEQ ID NO:4, SEQ ID NO:6, SEQ ID NO:8, SEQ ID NO:10, SEQ ID NO:12, SEQ ID NO:14, SEQ ID NO: 15 and SEQ ID NO: 17.

For example, the second domain may comprise HCDR2 of an antibody heavy chain, which may comprise an amino acid sequence selected from the group consisting of SEQ ID NO: SEQ ID NO:4, SEQ ID NO:6, SEQ ID NO:8, SEQ ID NO:10, SEQ ID NO:12, SEQ ID NO: 14, SEQ ID NO: 15 and SEQ ID NO: 17.

For example, the second domain may comprise HCDR1 of an antibody heavy chain, which may comprise an amino acid sequence selected from the group consisting of SEQ ID NO: SEQ ID NO:4, SEQ ID NO:6, SEQ ID NO:8, SEQ ID NO:10, SEQ ID NO:12, SEQ ID NO: 14, SEQ ID NO: 15 and SEQ ID NO: 17.

For example, the second domain may comprise a heavy chain variable region VH of an antibody heavy chain, and the antibody heavy chain variable region may comprise an amino acid sequence selected from the group consisting of SEQ ID NO:4, SEQ ID NO:6, SEQ ID NO:8, SEQ ID NO: 10, SEQ ID NO: 12, SEQ ID NO: 14, SEQ ID NO: 15 and SEQ ID NO: 17.

For example, the second domain may comprise an antibody heavy chain, which may comprise an amino acid sequence selected from the group consisting of SEQ ID NO:4, SEQ ID NO:6, SEQ ID NO:8, SEQ ID NO:10, SEQ ID NO:12, SEQ ID NO:14, SEQ ID NO:15 and SEQ ID NO: 17.

For example, the second domain may comprise LCDR3 of an antibody light chain, which may comprise an amino acid sequence selected from the group consisting of SEQ ID NO:5, SEQ ID NO:7, SEQ ID NO:9, SEQ ID NO: 11, SEQ ID NO: 13, SEQ ID NO:16 and SEQ ID NO:18.

For example, the second domain may comprise LCDR2 of an antibody light chain, which may comprise an amino acid sequence selected from the group consisting of SEQ ID NO:5, SEQ ID NO:7, SEQ ID NO:9, SEQ ID NO: 11, SEQ ID NO: 13, SEQ ID NO:16 and SEQ ID NO:18.

For example, the second domain may comprise LCDR1 of an antibody light chain, and the antibody light chain may comprise an amino acid sequence selected from the group consisting of SEQ ID NO:5, SEQ ID NO:7, SEQ ID NO:9, SEQ ID NO: 11, SEQ ID NO:13, SEQ ID NO:16 and SEQ ID NO:18.

For example, the second domain may comprise a light chain variable region VL of an antibody light chain, and the antibody light chain variable region may comprise an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

For example, the second domain may comprise an antibody light chain, which may comprise an amino acid sequence selected from the group consisting of SEQ ID NO:5, SEQ ID NO:7, SEQ ID NO:9, SEQ ID NO: 11, SEQ ID NO: 13, SEQ ID NO: 16 and SEQ ID NO: 18.

For example, the second domain may comprise HCDR3, HCDR2 and HCDR1 of an antibody heavy chain, wherein the antibody heavy chain may comprise the amino acid sequence shown in SEQ ID NO:4. For example, the antibody can be Sugemalimab.

For example, the second domain may comprise LCDR3, LCDR2 and LCDR1 of an antibody light chain, wherein the antibody light chain may comprise the amino acid sequence shown in SEQ ID NO: 5. For example, the antibody can be Sugemalimab.

For example, the second domain may comprise HCDR3, HCDR2 and HCDR1 of an antibody heavy chain, wherein the antibody heavy chain may comprise the amino acid sequence shown in SEQ ID NO:4, and the second domain may comprise LCDR3, LCDR2 and LCDR1 of an antibody light chain, wherein the antibody light chain may comprise the amino acid sequence shown in SEQ ID NO:5. For example, the antibody can be Sugemalimab.

For example, the second domain may comprise the heavy chain variable region VH of the antibody heavy chain, and the antibody heavy chain may comprise the amino acid sequence shown in SEQ ID NO:4, and the second domain may comprise the light chain variable region VL of the antibody light chain, and the antibody light chain may comprise the amino acid sequence shown in SEQ ID NO:5. For example, the antibody can be Sugemalimab.

For example, the antibody can be selected from immunoglobulin antibodies, recombinant antibodies, chimeric antibodies, heavy chain antibodies, single domain antibodies and/or bispecific antibodies; the antigen-binding fragment can be selected from Fab, Fab', Fv, F(ab)2, F(ab')2, scFv, di-scFv, VHH and/or dAb.

For example, the second domain may comprise an immunoglobulin domain. For example, the second domain may comprise an immunoglobulin IgG antibody, and the immunoglobulin IgG antibody may comprise one or more selected from human IgG1, human IgG2, human IgG3, human IgG4, mouse IgG1, mouse IgG2a, mouse IgG2b and mouse IgG3.

For example, the second domain may comprise an immunoglobulin Fc domain. For example, the second domain may comprise the Fc domain of an immunoglobulin IgG antibody. For example, the second domain may comprise the Fc domain of an immunoglobulin IgG antibody, wherein the Fc domain of the immunoglobulin IgG antibody comprises a human IgG1 Fc domain, a human IgG2 Fc domain, a human IgG3 Fc domain, a human IgG4 Fc domain, a mouse IgG1 Fc domain, a mouse IgG2a Fc domain, a mouse IgG2b Fc domain or a mouse IgG3 Fc domain. For example, the second domain may be the Fc domain of human immunoglobulin IgG4.

For example, the first domain may be directly or indirectly linked to the second domain.

For example, the second domain may include an immunoglobulin Fc domain, and the first domain may be directly or indirectly connected to the C-terminus of the second domain.

For example, the second domain may include an immunoglobulin Fc domain, and the first domain may be directly or indirectly connected to the N-terminus of the second domain.

For example, the second domain may comprise an antibody heavy chain, and the first domain may be directly or indirectly linked to the antibody heavy chain of the second domain.

For example, the second domain may comprise an antibody heavy chain, and the first domain may be directly or indirectly linked to the antibody heavy chain C-terminus of the second domain.

For example, the second domain may comprise an antibody heavy chain, and the N-terminus of the first domain may be directly or indirectly connected to the antibody heavy chain C-terminus of the second domain.

For example, the second domain may comprise an antibody heavy chain, and the first domain may be directly or indirectly linked to the N-terminus of the antibody heavy chain of the second domain.

For example, the second domain may comprise an antibody heavy chain, and the C-terminus of the first domain may be directly or indirectly connected to the N-terminus of the antibody heavy chain of the second domain.

For example, the second domain may comprise an antibody light chain, and the first domain may be directly or indirectly linked to the antibody light chain of the second domain.

For example, the second domain may comprise an antibody light chain, and the first domain may be directly or indirectly connected to the antibody light chain C-terminus of the second domain.

For example, the second domain may comprise an antibody light chain, and the N-terminus of the first domain may be directly or indirectly connected to the antibody light chain C-terminus of the second domain.

For example, the second domain may comprise an antibody light chain, and the first domain may be directly or indirectly linked to the N-terminus of the antibody light chain of the second domain.

For example, the second domain may comprise an antibody light chain, and the C-terminus of the first domain may be directly or indirectly connected to the N-terminus of the antibody light chain of the second domain.

For example, the indirect connection may comprise connection via a linker. For example, the linker may comprise an amino acid sequence selected from the group consisting of SEQ ID NO:31, SEQ ID NO:32, SEQ ID NO:33, SEQ ID NO:34 and SEQ ID NO:35.

For example, the fusion polypeptide complex can include a first polypeptide and a second polypeptide. For example, the second polypeptide may be absent.

For example, the first polypeptide of the fusion polypeptide complex may comprise a second domain, the second domain may comprise an antibody or an antigen-binding fragment thereof, and the second polypeptide may comprise a light chain of the antibody or an antigen-binding fragment thereof.

For example, the first polypeptide of the fusion polypeptide complex may comprise a second domain, the second domain may comprise a heavy chain of an antibody or an antigen-binding fragment thereof, and the second polypeptide may comprise a light chain of the antibody or an antigen-binding fragment thereof.

For example, the fusion polypeptide complex may comprise a second domain, the second domain may comprise a heavy chain, and the second domain heavy chain may represent an Fc peptide chain or a heavy chain of an antibody or an antigen-binding fragment thereof.

For example, the first polypeptide comprises a second binding domain heavy chain, wherein the second domain heavy chain represents an Fc peptide chain or a heavy chain of an antibody or antigen-binding fragment thereof.

For example, the first polypeptide of the fusion polypeptide complex may include, from N-terminus to C-terminus, a LAG3 variant polypeptide, a linker peptide, a second domain heavy chain, a linker peptide, and a third domain, wherein the linker peptides are independently present or absent.

For example, the first polypeptide of the fusion polypeptide complex includes, from N-terminus to C-terminus, a third domain, a linker peptide, a second domain heavy chain, a linker peptide and a LAG3 variant polypeptide, wherein the linker peptides are independently present or absent.

For example, the first polypeptide of the fusion polypeptide complex includes, from N-terminus to C-terminus, a LAG3 variant polypeptide, a linker peptide, a third domain, a linker peptide and a second domain heavy chain, wherein the linker peptides are independently present or absent.

For example, the first polypeptide of the fusion polypeptide complex includes, from N-terminus to C-terminus, a third domain, a linker peptide, a LAG3 variant polypeptide, a linker peptide and a second domain heavy chain, wherein the linker peptides are independently present or absent.

For example, the first polypeptide of the fusion polypeptide complex includes, from N-terminus to C-terminus, a second domain heavy chain, a linker peptide, a LAG3 variant polypeptide, a linker peptide and a third domain, wherein the linker peptides are independently present or absent.

For example, the first polypeptide of the fusion polypeptide complex includes, from N-terminus to C-terminus, a second domain heavy chain, a linker peptide, a third domain, a linker peptide and a LAG3 variant polypeptide, wherein the linker peptides are independently present or absent.

For example, the fusion polypeptide may comprise an amino acid sequence selected from the group consisting of SEQ ID NO: 640 to SEQ ID NO: 745 and SEQ ID NO: 747 to SEQ ID NO: 750.

For example, the wild-type LAG3 extracellular region polypeptide complex AN_0322 can be composed of the AN_0322 polypeptide (SEQ ID NO: 746), and the AN_0322 polypeptide complex can be assembled by introducing a vector expressing the AN_0322 polypeptide polynucleotide (SEQ ID NO: 1214) into a host cell for expression.

For example, the LAG3 variant polypeptide complex AN_0320 can be composed of the AN_0320 polypeptide (SEQ ID NO: 743), and the assembly into the AN_0320 polypeptide complex is achieved by introducing a vector expressing the AN_0320 polypeptide polynucleotide (SEQ ID NO: 1213) into a host cell for expression.

For example, the LAG3 variant polypeptide complex AN_0324 can be composed of the AN_0324 polypeptide (SEQ ID NO: 744), and the AN_0324 polypeptide complex can be assembled by introducing a vector expressing the AN_0324 polypeptide polynucleotide (SEQ ID NO: 1215) into a host cell for expression.

For example, the LAG3 variant polypeptide complex AN_0325 can be composed of the AN_0325 polypeptide (SEQ ID NO: 745), and the assembly into the AN_0325 polypeptide complex is achieved by introducing a vector expressing the AN_0325 polypeptide polynucleotide (SEQ ID NO: 1216) into a host cell for expression.

For example, the LAG3 variant polypeptide complex AN_0912 can be composed of the AN_0912 polypeptide (SEQ ID NO: 660), and the AN_0912 polypeptide complex can be assembled by introducing a vector expressing the AN_0912 polypeptide polynucleotide (SEQ ID NO: 1217) into a host cell for expression.

For example, the LAG3 variant polypeptide complex AN_0913 can be composed of the AN_0913 polypeptide (SEQ ID NO: 644), and the AN_0913 polypeptide complex can be assembled by introducing a vector expressing the AN_0913 polypeptide polynucleotide (SEQ ID NO: 1218) into a host cell for expression.

For example, the LAG3 variant polypeptide complex AN_0914 can be composed of the AN_0914 polypeptide (SEQ ID NO: 708), and the assembly into the AN_0914 polypeptide complex is achieved by introducing a polynucleotide (SEQ ID NO: 1219) vector expressing the AN_0914 polypeptide into a host cell for expression.

For example, the LAG3 variant polypeptide complex AN_0915 can be composed of the AN_0915 polypeptide (SEQ ID NO: 714), and the assembly into the AN_0915 polypeptide complex is achieved by introducing a polynucleotide (SEQ ID NO: 1220) vector expressing the AN_0915 polypeptide into a host cell for expression.

For example, the LAG3 variant polypeptide complex AN_0916 can be composed of the AN_0916 polypeptide (SEQ ID NO: 720), and the assembly into the AN_0916 polypeptide complex is achieved by introducing a vector polypeptide polynucleotide (SEQ ID NO: 1221) expressing the AN_0916 into a host cell for expression.

For example, the LAG3 variant polypeptide complex AN_0398 can be composed of the AN_0398 polypeptide (SEQ ID NO: 742), and the AN_0398 polypeptide complex can be assembled by introducing a polynucleotide (SEQ ID NO: 1222) vector expressing the AN_0398 polypeptide into a host cell for expression).

For example, the LAG3 variant polypeptide complex AN_0399 can be composed of the AN_0399 polypeptide (SEQ ID NO: 741), and the AN_0399 polypeptide complex can be assembled by introducing a polynucleotide (SEQ ID NO: 1223) vector expressing the AN_0399 polypeptide into a host cell for expression.

For example, the LAG3 variant polypeptide complex AN_0400 can be composed of the AN_0400 polypeptide (SEQ ID NO: 740), and the assembly into the AN_0400 polypeptide complex is achieved by introducing a polynucleotide (SEQ ID NO: 1224) vector expressing the AN_0400 polypeptide into a host cell for expression.

For example, the LAG3 variant polypeptide complex AN_0401 can be composed of the AN_0401 polypeptide (SEQ ID NO: 739), and the AN_0401 polypeptide complex can be assembled by introducing a vector polynucleotide (SEQ ID NO: 1225) expressing the AN_0401 polypeptide into a host cell for expression.

For example, the LAG3 variant polypeptide complex AN 0411 can be composed of the AN_0411 polypeptide (SEQ ID NO: 682), and the AN_0411 polypeptide complex can be assembled by introducing a vector polynucleotide (SEQ ID NO: 1226) expressing the AN 0411 polypeptide into a host cell for expression.

For example, the LAG3 variant polypeptide complex AN_0412 can be composed of the AN_0412 polypeptide (SEQ ID NO: 688), and the AN_0412 polypeptide complex can be assembled by introducing a vector polynucleotide (SEQ ID NO: 1227) expressing the AN_0412 polypeptide into a host cell for expression.

For example, the LAG3 variant polypeptide complex AN_0413 can be composed of the AN_0413 polypeptide (SEQ ID NO: 694), and the AN_0413 polypeptide complex can be assembled by introducing a vector polynucleotide (SEQ ID NO: 1228) expressing the AN_0413 polypeptide into a host cell for expression.

For example, the LAG3 variant polypeptide complex AN_0414 can be composed of the AN_0414 polypeptide (SEQ ID NO: 700), and the AN_0414 polypeptide complex can be assembled by introducing a vector polynucleotide (SEQ ID NO: 1229) expressing the AN_0414 polypeptide into a host cell for expression.

For example, the LAG3 variant polypeptide complex AN_0415 can be composed of the AN_0415 polypeptide (SEQ ID NO: 706), and the assembly into the AN_0415 polypeptide complex is achieved by introducing a vector polynucleotide (SEQ ID NO: 1230) expressing the AN_0415 polypeptide into a host cell for expression.

For example, the LAG3 variant polypeptide complex AN_0551 can be composed of the AN_0551 polypeptide (SEQ ID NO: 676), and the assembly into the AN_0551 polypeptide complex is achieved by introducing a polynucleotide (SEQ ID NO: 1231) vector expressing the AN_0551 polypeptide into a host cell for expression.

For example, the LAG3 variant polypeptide complex AN_0714 can be composed of the AN_0714 polypeptide (SEQ ID NO: 709), and the AN_0714 polypeptide complex can be assembled by introducing a vector polynucleotide (SEQ ID NO: 1232) expressing the AN_0714 polypeptide into a host cell for expression.

For example, the LAG3 variant polypeptide complex AN_0715 can be composed of the AN_0715 polypeptide (SEQ ID NO: 712), and the assembly into the AN_0715 polypeptide complex is achieved by introducing a vector polynucleotide (SEQ ID NO: 1233) expressing the AN_0715 polypeptide into a host cell for expression.

For example, the LAG3 variant polypeptide complex AN_0716 can be composed of the AN_0716 polypeptide (SEQ ID NO: 715), and the assembly into the AN_0716 polypeptide complex is achieved by introducing a vector polynucleotide (SEQ ID NO: 1234) expressing the AN_0716 polypeptide into a host cell for expression.

For example, the LAG3 variant polypeptide complex AN_0717 can be composed of the AN_0717 polypeptide (SEQ ID NO: 718), and the assembly into the AN_0717 polypeptide complex is achieved by introducing a vector polynucleotide (SEQ ID NO: 1235) expressing the AN_0717 polypeptide into a host cell for expression.

For example, the LAG3 variant polypeptide complex AN_0718 can be composed of the AN_0718 polypeptide (SEQ ID NO: 721), and the assembly into the AN_0718 polypeptide complex is achieved by introducing a polynucleotide (SEQ ID NO: 1236) vector expressing the AN_0718 polypeptide into a host cell for expression.

For example, the LAG3 variant polypeptide complex AN_0738 can be composed of the AN_0738 polypeptide (SEQ ID NO: 710), and the assembly into the AN_0738 polypeptide complex is achieved by introducing a polynucleotide (SEQ ID NO: 1237) vector expressing the AN_0738 polypeptide into a host cell for expression.

For example, the LAG3 variant polypeptide complex AN_0739 can be composed of the AN_0739 polypeptide (SEQ ID NO: 711), and the AN_0739 polypeptide complex can be assembled by introducing a polynucleotide (SEQ ID NO: 1238) vector expressing the AN_0739 polypeptide into a host cell for expression.

For example, the LAG3 variant polypeptide complex AN_0741 can be composed of the AN_0741 polypeptide (SEQ ID NO: 720), and the AN_0741 polypeptide complex can be assembled by introducing a polynucleotide (SEQ ID NO: 1239) vector expressing the AN_0741 polypeptide into a host cell for expression.

For example, the LAG3 variant polypeptide complex AN_0826 can be composed of the AN_0826 polypeptide (SEQ ID NO: 724), and the assembly into the AN_0826 polypeptide complex is achieved by introducing a polynucleotide (SEQ ID NO: 1240) vector expressing the AN_0826 polypeptide into a host cell for expression.

For example, the LAG3 variant polypeptide complex AN_0827 can be composed of an AN_0827 polypeptide (SEQ ID NO: 727), and the assembly into the AN_0827 polypeptide complex is achieved by introducing a polynucleotide (SEQ ID NO: 1241) vector expressing an AN_0827 polypeptide into a host cell for expression.

For example, the LAG3 variant polypeptide complex AN_0828 can be composed of the AN_0828 polypeptide (SEQ ID NO: 730), and the AN_0828 polypeptide complex can be assembled by introducing a polynucleotide (SEQ ID NO: 1242) vector expressing the AN_0828 polypeptide into a host cell for expression.

For example, the LAG3 variant polypeptide complex AN_0993 can be composed of the AN_0993 polypeptide (SEQ ID NO: 1257), and the assembly into the AN_0993 polypeptide complex is achieved by introducing a polynucleotide (SEQ ID NO: 1270) vector expressing the AN_0993 polypeptide into a host cell for expression.

For example, the LAG3 variant polypeptide complex AN_0994 can be composed of the AN_0994 polypeptide (SEQ ID NO: 1258), and the AN_0994 polypeptide complex can be assembled by introducing a polynucleotide (SEQ ID NO: 1271) vector expressing the AN_0994 polypeptide into a host cell for expression.

For example, the LAG3 variant polypeptide complex AN_0995 can be composed of the AN_0995 polypeptide (SEQ ID NO: 1259), and the assembly into the AN_0995 polypeptide complex is achieved by introducing a polynucleotide (SEQ ID NO: 1272) vector expressing the AN_0995 polypeptide into a host cell for expression.

For example, the LAG3 variant polypeptide complex AN_0996 can be composed of the AN_0996 polypeptide (SEQ ID NO: 1260), and the assembly into the AN_0996 polypeptide complex is achieved by introducing a polynucleotide (SEQ ID NO: 1273) vector expressing the AN_0996 polypeptide into a host cell for expression.

For example, the LAG3 variant polypeptide complex AN_0997 can be composed of the AN_0997 polypeptide (SEQ ID NO: 634), and the assembly into the AN_0997 polypeptide complex is achieved by introducing a polynucleotide (SEQ ID NO: 1274) vector expressing the AN_0997 polypeptide into a host cell for expression.

For example, the LAG3 variant polypeptide complex AN_0998 can be composed of the AN_0998 polypeptide (SEQ ID NO: 1261), and the assembly into the AN_0998 polypeptide complex is achieved by introducing a polynucleotide (SEQ ID NO: 1275) vector expressing the AN_0998 polypeptide into a host cell for expression.

For example, the LAG3 variant polypeptide complex AN_0999 can be composed of the AN_0999 polypeptide (SEQ ID NO: 1262), and the assembly into the AN_0999 polypeptide complex is achieved by introducing a polynucleotide (SEQ ID NO: 1276) vector expressing the AN_0999 polypeptide into a host cell for expression.

For example, the LAG3 variant polypeptide complex AN_1000 can be composed of the AN_1000 polypeptide (SEQ ID NO: 1263), and the assembly into the AN_1000 polypeptide complex is achieved by introducing a polynucleotide (SEQ ID NO: 1277) vector expressing the AN_1000 polypeptide into a host cell for expression.

For example, the LAG3 variant polypeptide complex AN_1001 can be composed of the AN_1001 polypeptide (SEQ ID NO: 1264), and the assembly into the AN_1001 polypeptide complex is achieved by introducing a polynucleotide (SEQ ID NO: 1278) vector expressing the AN_1001 polypeptide into a host cell for expression.

For example, the LAG3 variant polypeptide complex AN_1002 can be composed of the AN_1002 polypeptide (SEQ ID NO: 1265), and the assembly into the AN_1002 polypeptide complex is achieved by introducing a polynucleotide (SEQ ID NO: 1279) vector expressing the AN_1002 polypeptide into a host cell for expression.

For example, the LAG3 variant polypeptide complex AN_1010 can be composed of the AN_1010 polypeptide (SEQ ID NO: 1266), and the assembly into the AN_1010 polypeptide complex is achieved by introducing a polynucleotide (SEQ ID NO: 1280) vector expressing the AN_1010 polypeptide into a host cell for expression.

For example, the LAG3 variant polypeptide complex AN_1011 can be composed of the AN_1011 polypeptide (SEQ ID NO: 1267), and the assembly into the AN_1011 polypeptide complex is achieved by introducing a polynucleotide (SEQ ID NO: 1281) vector expressing the AN_1011 polypeptide into a host cell for expression.

For example, the LAG3 variant polypeptide complex AN_1012 can be composed of the AN_1012 polypeptide (SEQ ID NO: 1268), and the assembly into the AN_1012 polypeptide complex is achieved by introducing a polynucleotide (SEQ ID NO: 1282) vector expressing the AN_1012 polypeptide into a host cell for expression.

For example, the LAG3 variant polypeptide complex AN_1013 can be composed of the AN_1013 polypeptide (SEQ ID NO: 641), and the assembly into the AN_1013 polypeptide complex is achieved by introducing a polynucleotide (SEQ ID NO: 1283) vector expressing the AN_1013 polypeptide into a host cell for expression.

For example, the LAG3 variant polypeptide complex AN_1029 can be composed of the AN_1029 polypeptide (SEQ ID NO: 1269), and the assembly into the AN_1029 polypeptide complex is achieved by introducing a polynucleotide (SEQ ID NO: 1284) vector expressing the AN_1029 polypeptide into a host cell for expression.

For example, the bifunctional antibody AN_0737 can be composed of a LAG3 variant polypeptide hLAG3 R97E 97-156 C-terminal connecting linker peptide (SEQ ID NO: 34) covalently linked to the N-terminus of the sugemalimab heavy chain to form a chimeric antibody AN_0737 first polypeptide (SEQ ID NO: 747) and a sugemalimab light chain second polypeptide (SEQ ID NO: 5), and the assembly into the AN_0737 functional antibody is achieved by introducing a vector expressing the AN_0737 first polypeptide polynucleotide sequence (SEQ ID NO: 1243) and a vector expressing the AN_0737 second polypeptide polynucleotide sequence (SEQ ID NO: 1158) into a host cell for expression.

For example, the bifunctional antibody AN_0740 can be composed of a chimeric antibody AN_0740 first polypeptide (SEQ ID NO: 748) (formed by a LAG3 variant polypeptide hLAG3 112-156 C-terminus connecting to linker peptide (SEQ ID NO: 34) covalently linked to the N-terminus of the sugemalimab heavy chain) and a sugemalimab light chain second polypeptide (SEQ ID NO: 5), and the assembly into the AN_0740 functional antibody is achieved by introducing a polynucleotide sequence (SEQ ID NO: 1244) vector expressing the AN_0740 first polypeptide and a polynucleotide sequence (SEQ ID NO: 1158) vector expressing the AN_0740 second polypeptide into a host cell for expression.

For example, the bifunctional antibody AN_0742 can be composed of a chimeric antibody AN_0742 first polypeptide (SEQ ID NO: 749) (formed by LAG3 variant polypeptide hLAG3 122-156 C-terminus connecting to linker peptide (SEQ ID NO: 34) covalently linked to the N-terminus of the sugemalimab heavy chain) and a sugemalimab light chain second polypeptide (SEQ ID NO: 5), and the assembly into the AN_0742 functional antibody is achieved by introducing a polynucleotide sequence (SEQ ID NO: 1245) vector expressing the AN_0742 first polypeptide and a polynucleotide sequence (SEQ ID NO: 1158) vector expressing the AN_0742 second polypeptide into a host cell for expression.

For example, the bifunctional antibody AN_0673 can be composed of a chimeric antibody AN_0673 first polypeptide (SEQ ID NO: 750) (to formed by LAG3 variant polypeptide hLAG3 R97E 97-161 C-terminus connecting to linker peptide (SEQ ID NO: 34) covalently linked to the N-terminus of the sugemalimab heavy chain) and a sugemalimab light chain second polypeptide (SEQ ID NO: 5), and the assembly into the AN_0673 functional antibody is achieved by introducing a polynucleotide sequence (SEQ ID NO: 1246) vector expressing the AN_0673 first polypeptide and a polynucleotide sequence (SEQ ID NO: 1158) vector expressing the AN_0673 second polypeptide into a host cell for expression.

For example, the multifunctional antibody AN_0736 can be composed of a LAG3 variant polypeptide hLAG3 R97E 97-156 C-terminal connecting linker peptide (SEQ ID NO: 34) covalently linked to the N-terminus of the sugemalimab heavy chain, and a sugemalimab heavy chain C-terminal connecting linker peptide (SEQ ID NO: 34) covalently linked to the IgV region of the CD86 extracellular domain to form a chimeric antibody AN_0736 first polypeptide (SEQ ID NO: 751) and a sugemalimab light chain second polypeptide (SEQ ID NO: 5), and the assembly into the AN_0736 multifunctional antibody is achieved by introducing a vector expressing the AN_0736 first polypeptide polynucleotide (SEQ ID NO: 1247) and a vector expressing the AN_0736 second polypeptide polynucleotide (SEQ ID NO: 1158) into a host cell for expression.

For example, the multifunctional antibody AN_0685 can be composed of a LAG3 variant polypeptide hLAG3 R97E 97-161 C-terminal connecting linker peptide (SEQ ID NO: 34) covalently linked to the N-terminus of the sugemalimab heavy chain, and a sugemalimab heavy chain C-terminal connecting linker peptide (SEQ ID NO: 34) covalently linked to the IgV region of the CD86 extracellular domain to form a chimeric antibody AN_0685 first polypeptide (SEQ ID NO: 752) and a sugemalimab light chain second polypeptide (SEQ ID NO: 5), and the assembly into the AN_0685 multifunctional antibody is achieved by introducing a vector expressing the AN_0685 first polypeptide polynucleotide (SEQ ID NO: 1248) and a vector expressing the AN_0685 second polypeptide polynucleotide (SEQ ID NO: 1158) into a host cell for expression.

On the other hand, the present application provides an immunoconjugate comprising the fusion polypeptide described in the present application, the CD86 variant polypeptide described in the present application and/or the LAG3 variant described in the present application.

On the other hand, the present application provides a nucleic acid molecule encoding the fusion polypeptide described in the present application, the CD86 variant polypeptide described in the present application and/or the LAG3 variant described in the present application.

On the other hand, the present application provides a vector comprising the nucleic acid molecule described in the present application.

On the other hand, the present application provides a cell, which contains and/or expresses the fusion polypeptide described in the present application, the CD86 variant polypeptide described in the present application, the LAG3 variant described in the present application, the immunoconjugate described in the present application, the nucleic acid molecule described in the present application, and/or the vector described in the present application.

On the other hand, the present application provides a composition comprising the fusion polypeptide described herein, the immunoconjugate described herein, the nucleic acid molecule described herein, the vector described herein, and/or the cell described herein, and optionally a pharmaceutically acceptable carrier.

On the other hand, the present application provides a method for preparing the fusion polypeptide described in the present application, the CD86 variant polypeptide described in the present application, and the LAG3 variant described in the present application, which comprises culturing the cell described in the present application under conditions that allow the fusion polypeptide to be expressed.

On the other hand, the present application provides a method for blocking the interaction between PD-L1 protein and PD-1, which comprises administering an effective amount of the fusion polypeptide described in the present application, the CD86 variant polypeptide described in the present application, the LAG3 variant described in the present application, the immunoconjugate described in the present application, the nucleic acid molecule described in the present application, the vector described in the present application, the cell described in the present application, and/or the composition described in the present application.

On the other hand, the present application provides a method for stimulating antigen-presenting cells and/or activating T cells, which comprises administering an effective amount of the fusion polypeptide described herein, the CD86 variant polypeptide described herein, the LAG3 variant described herein, the immunoconjugate described herein, the nucleic acid molecule described herein, the vector described herein, the cell described herein, and/or the composition described herein.

According to the method described in the present application, the stimulation of antigen presenting cells comprises a step selected from the group consisting of: increasing the expression of co-stimulatory molecules in antigen presenting cells, causing morphological changes and maturation of antigen presenting cells, increasing the secretion of chemokines in antigen presenting cells, and enhancing the phagocytic ability of antigen presenting cells.

On the other hand, the present application provides a method for inhibiting the growth and/or proliferation of tumors or tumor cells, which comprises administering an effective amount of the fusion polypeptide described herein, the CD86 variant polypeptide described herein, the LAG3 variant described herein, the immunoconjugate described herein, the nucleic acid molecule described herein, the vector described herein, the cell described herein, and/or the composition described herein.

On the other hand, the present application provides the use of the fusion polypeptide described in the present application, the CD86 variant polypeptide described in the present application, the LAG3 variant described in the present application, the immunoconjugate described in the present application, the nucleic acid molecule described in the present application, the vector described in the present application, the cell described in the present application, and/or the composition described in the present application in the preparation of a medicament, wherein the medicament is used to prevent, improve and/or treat tumors.

According to the use described in the present application, the tumor comprises a solid tumor and/or a hematological tumor.

According to the use described in the present application, the tumor is selected from the following group: colon tumor, breast tumor, lung tumor, gastric tumor, melanoma, head and neck tumor, lymphoma, nasopharyngeal tumor, cervical tumor, esophageal tumor, kidney tumor, skin squamous cell carcinoma, endometrial tumor, liver tumor, bladder tumor, urothelial tumor and skin tumor.

On the other hand, the present application provides the fusion polypeptide described in the present application, the immunoconjugate described in the present application, the nucleic acid molecule described in the present application, the vector described in the present application, the cell described in the present application, and/or the composition described in the present application, which are used to prevent, improve and/or treat tumors.

On the other hand, the present application provides the fusion polypeptide described in the present application, the immunoconjugate described in the present application, the nucleic acid molecule described in the present application, the vector described in the present application, the cell described in the present application, and/or the composition described in the present application, which are used to prevent, improve and/or treat tumors, wherein the tumors include solid tumors and hematological tumors.

On the other hand, the present application provides the fusion polypeptide described in the present application, the immunoconjugate described in the present application, the nucleic acid molecule described in the present application, the vector described in the present application, the cell described in the present application, and/or the composition described in the present application, which are used to prevent, improve and/or treat tumors, wherein the tumor is selected from the following group: colon tumors, breast tumors, lung tumors, gastric tumors, melanoma, head and neck tumors, lymphomas, nasopharyngeal tumors, cervical tumors, esophageal tumors, kidney tumors, skin squamous cell carcinoma, endometrial tumors, liver tumors, bladder tumors, urothelial tumors and skin tumors.

On the other hand, the present application provides a method for preventing, improving and/or treating tumors, which may comprise administering the fusion polypeptide described herein, the immunoconjugate described herein, the nucleic acid molecule described herein, the vector described herein, the cell described herein, and/or the composition described herein to a subject in need thereof.

According to the method described in the present application, the tumor includes solid tumors and hematological tumors.

According to the method described in the present application, the tumor is selected from the following group: colon tumor, breast tumor, lung tumor, gastric tumor, melanoma, head and neck tumor, lymphoma, nasopharyngeal tumor, cervical tumor, esophageal tumor, kidney tumor, skin squamous cell carcinoma, endometrial tumor, liver tumor, bladder tumor, urothelial tumor and skin tumor.

Without intending to be bound by any theory, the following embodiments are merely intended to illustrate the products, preparation methods and uses of the present application, and are not intended to limit the scope of the present invention.

### Example

### Example 1

### Expression and purification of fusion peptide complexes

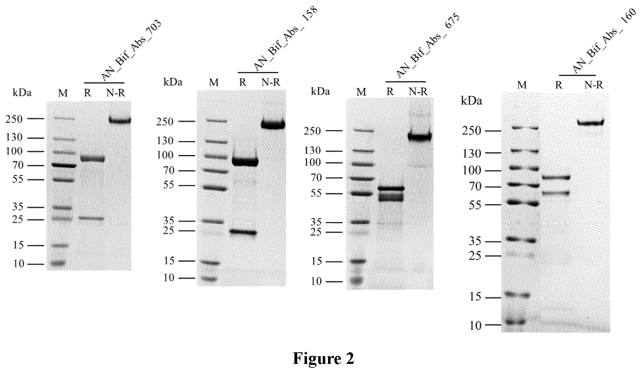

In order to identify the antibodies obtained by screening, it is necessary to express the fusion polypeptide complex in mammalian cells. Therefore, firstly, an expression plasmid vector for expressing the heavy chain of the multi/bifunctional antibody and an expression plasmid vector containing the light chain were constructed. About 24 h before plasmid transfection, Expi293 cells were passaged to a cell density of about 2.4×10⁶ cells/ml. When the cell density is 6×10⁶ cells/ml and the viability is >95%, 25ug of the plasmid vector expressing the mixed heavy chain and light chain is taken and transfected into 25ml of Expi293 cells with Expifectamine 293, and cultured at 37°C, 130rpm, 8% CO2 in a shaking incubator for 7 days. The cell culture product is centrifuged and the supernatant is taken. After filtering with a 0.45 MCE filter, the target antibody is purified and collected using a Capturem protein A Maxiprep column, and then concentrated by centrifugation with a Vivaspin 20 Centrifugal Concentrator 50K, and quantified by the NanoDrop 2000 method for determining A280. The antibody purity is determined by SDS-PAGE and SEC-HPLC. FIG2 shows the result of using SDS-PAGE to detect the expression of the fusion polypeptide complex of the present application, and the result shows that the fusion polypeptide complex of the present application can be expressed and purified.

### Example 2

### Binding affinity determination

In a specific embodiment, the present application provides the binding of AN_Bif_Abs_0016, and/or AN_Bif_Abs_00703 and/or AN_Bif_Abs_0158, and/or AN_Bif_Abs_0675, and/or AN_Bif_Abs_0676, and/or AN_Bif_Abs_0031, and/or AN_Bif_Abs_0130, and/or AN_Bif_Abs_0131, and/or AN_Bif_Abs_0160, and/or AN_Bif_Abs_0161, and/or AN_Bif_Abs_0646 to human PD-L1 protein, human CTLA4 protein, and human CD28 protein.

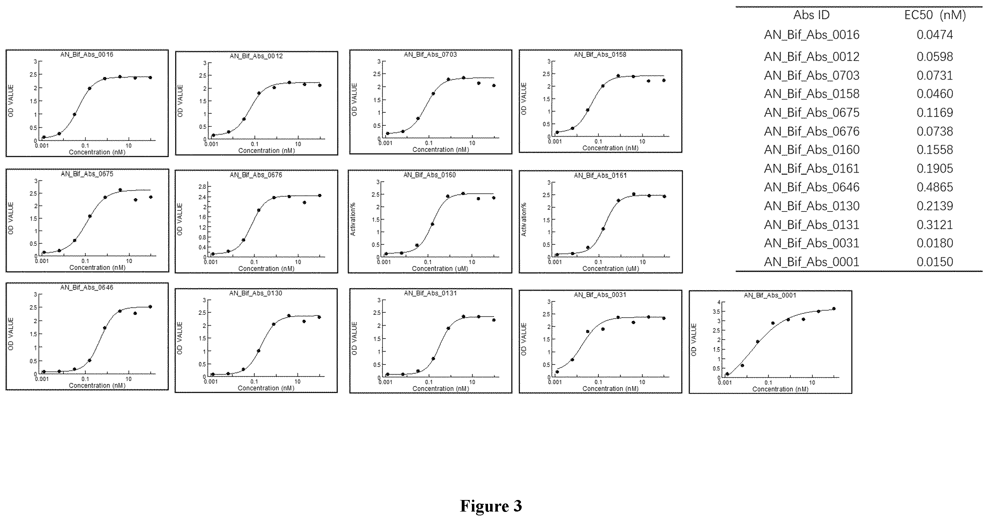

Specifically, human PD-L1 protein (5 µg/mL in PBS; Acro, PD1-H5258) was incubated in a flat-bottom 96-well Immuno detachable transparent ELISA plate (MaxiSorp) (Thermo Scientific, 446469) at 4°C overnight; then, after washing, 2% BSA(in PBS; VWR Life Science, 0332-1KG) was added to each well. Incubate at room temperature for 1 hour, wash and add serial dilutions of AN_Bif_Abs_0016, and/or AN_Bif_Abs_0012, and/or AN_Bif_Abs_0703, and/or AN_Bif_Abs_0158, and/or AN_Bif_Abs_0675, and/or AN_Bif_Abs_0676, and/or AN Bif Abs_0031, and/or AN Bif Abs_0130, and/or AN Bif Abs_0131, and/or AN_Bif_Abs_0160, and/or AN_Bif_Abs_0161, and/or AN_Bif_Abs_0646, and/or AN_Bif_Abs_0001 to the final test concentration. Incubate at room temperature for 2 hours, wash and add Goat Abs_0016, and/or AN_Bif_Abs_0012 to each well. Anti-Human Lambda-HRP (SouthernBiotech, 2060-05) was incubated at room temperature for 30 min. After washing, the binding affinity of the candidate multi-/bifunctional fusion peptides to the human PD-L1 protein was detected using the Soluble TMB Kit (CWBIO, CW0050S). The specific operation was performed according to the manufacturer's instructions. FIG3 shows the results of using ELISA to detect the binding between the fusion polypeptide complex of the present application and the human PDL1 protein.

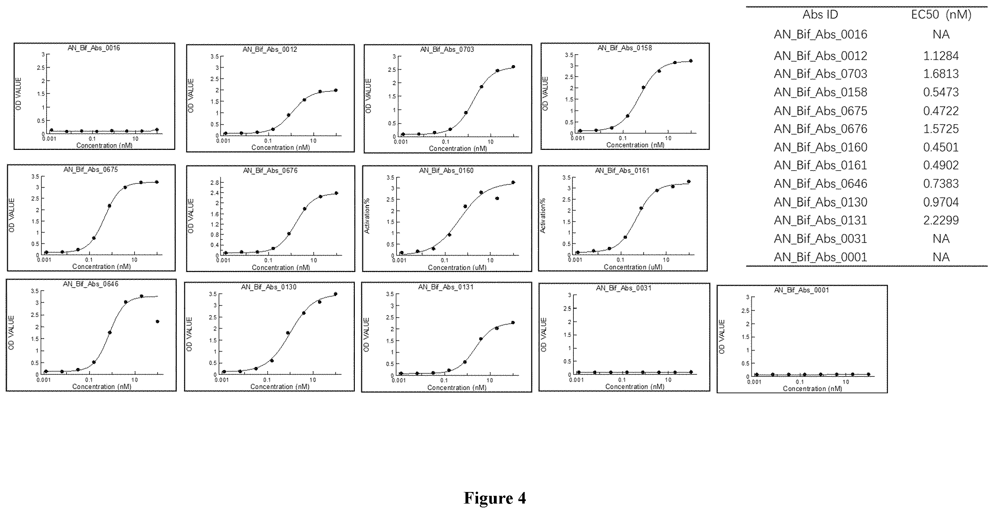

Similarly, human CTLA4 protein (5 µg/mL in PBS; Acro) was plated in a flat-bottom 96-well Immuno removable transparent ELISA plate (MaxiSorp) (Thermo Scientific, 446469) and incubated at 4°C overnight; the binding affinity of AN_Bif_Abs_0016, and/or AN_Bif_Abs_0012, and/or AN_Bif_Abs_0703, and/or AN_Bif_Abs_0158, and/or AN_Bif_Abs_0675, and/or AN_Bif_Abs_0676, and/or AN_Bif_Abs_0031, and/or AN_Bif_Abs_0130, and/or AN_Bif_Abs_0131, and/or AN_Bif_Abs_0160, and/or AN_Bif_Abs_0161, and/or AN_Bif_Abs_0646, and/or AN_Bif_Abs_0001 to human CTLA4 protein was detected by a similar method to that of the binding affinity of the candidate multi-/bifunctional fusion peptides to the human PD-L1 protein . FIG. 4 shows the results of using ELISA to detect the binding of the fusion polypeptide complex of the present application to the human CTLA4 protein.

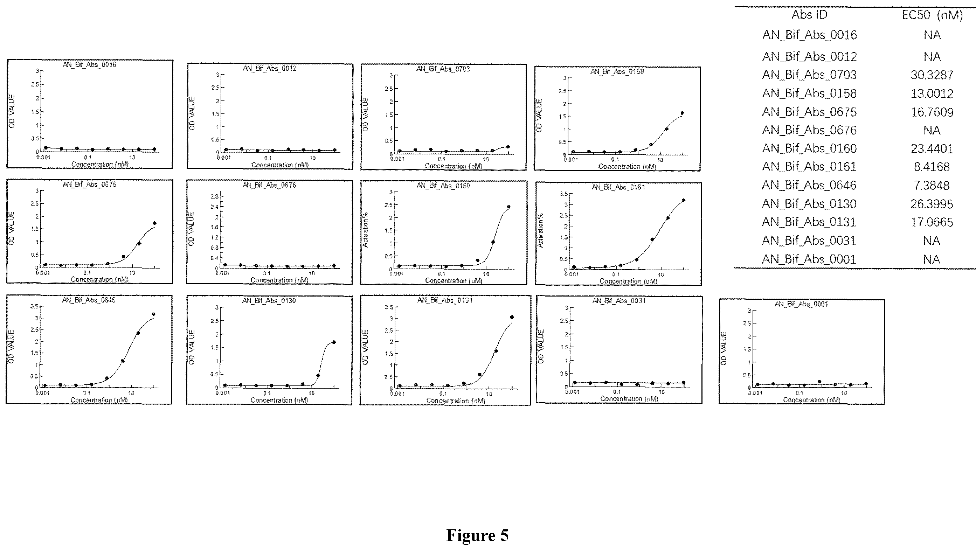

Similarly, human CD28 protein (5 µg/mL in PBS; Acro) was plated in flat-bottom 96-well Immuno removable transparent ELISA plates (MaxiSorp) (Thermo Scientific, 446469) and incubated at 4°C overnight; the binding affinity of AN_Bif_Abs_0016, and/or AN_Bif_Abs_0012, and/or AN_Bif_Abs_0703, and/or AN_Bif_Abs_0158, and/or AN_Bif_Abs_0675, and/or AN_Bif_Abs_0676, and/or AN_Bif_Abs_0031, and/or AN_Bif_Abs_0130, and/or AN_Bif_Abs_0131, and/or AN_Bif_Abs_0160, and/or AN_Bif_Abs_0161, and/or AN_Bif_Abs_0646, and/or AN_Bif_Abs_0001 to human CD28 protein was detected by a similar method to that of the binding affinity of the candidate multi-/bifunctional fusion peptides to the human PD-L1 protein . FIG5 shows the results of using ELISA to detect the binding between the fusion polypeptide complex of the present application and the human CD28 protein.

### Example 3

### T cell activation immune response assay

In a specific embodiment, the present application provides situations in which AN_Bif_Abs_0016, and/or AN_Bif_Abs_0161, and/or AN_Bif_Abs_0160, and/or AN_Bif_Abs_0675, and/or AN_Bif_Abs_0676, and/or AN_Bif_Abs_0130, and/or AN_Bif_Abs_0131, and/or AN_Bif_Abs_0646 regulate T cell immune responses.

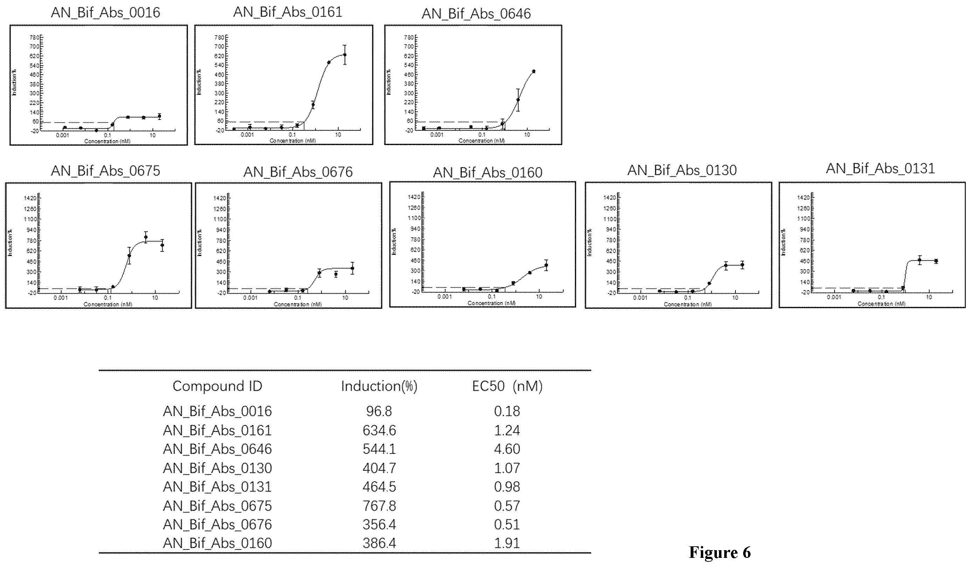

Specifically, on the first day, TCR activator PD-L1-CHO cells (BPS bioscience) were digested and collected using 0.05% trypsin, counted, and plated: fresh culture medium was added to mix the cells and the cell density was adjusted to 3.5*10⁵cells/ml. Add cells to a sterile 96-well flat-bottom plate (CORNING, 3599) at 100ul/well, for a total of 35,000 cells/well; grow overnight in a 37°C incubator until 80%; the next day, remove the culture medium of TCR activator PD-L1- CHO cells in the 96-well flat-bottom plate, and add 50ul of fresh culture medium containing the corresponding concentration of test substances (serial dilutions) AN_Bif_Abs_0016, and/or AN_Bif_Abs_0161, and/or AN_Bif_Abs_0160, and/or AN_Bif_Abs_0675, and/or AN_Bif_Abs_0676, and/or AN_Bif_Abs_0130, and/or AN_Bif_Abs_0131, and/or AN_Bif_Abs_0646 to each well. After 30 minutes of treatment, collect Jurkat-Lucia^{™} TCR-hPD-1 cells (Invivogen) and count; add fresh culture medium to mix the cells and adjust the cell density to 4*10⁵ cells/ml; add 50ul Jurkat-Lucia^{™} TCR-hPD-1 cells to a final cell density of 2*10⁴ /well, and incubate in a 96-well flat-bottom plate in a 37°C incubator for 5-6h. After incubation, observe cell morphology under a microscope and detect chemiluminescence using QUANTI-Luc^{™} (Invivogen; rep-qlc1) according to the manufacturer's instructions. FIG6 shows the result of the fusion polypeptide complex of the present application regulating the activation of Jurkat T cells. The fusion polypeptide complex of the present application can have the ability to regulate the immune response of T cells.

### Example 4

### Determination of costimulatory molecules upregulated by antigen presenting cells

In a specific embodiment, the present application provides the expression of co-stimulatory molecules induced by AN_Bif_Abs_0016, and/or AN_Bif_Abs_0031, and/or AN_Bif_Abs_0130, and/or AN_Bif_Abs_0131, and/or AN_Bif_Abs_0160, and/or AN_Bif_Abs_0161, and/or AN_Bif_Abs_0646 in antigen presenting cells.

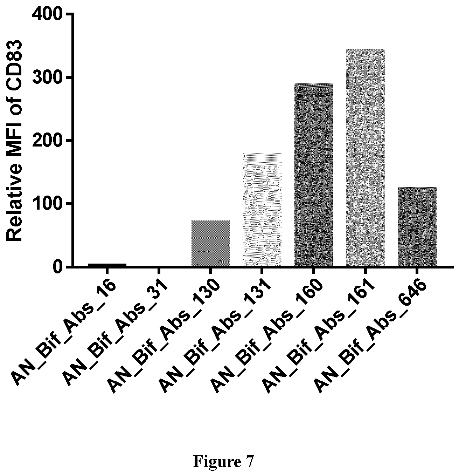

Specifically, THP-1 cells (ATCC) were collected, counted, and plated: fresh culture medium was added to mix the cells and the cell density was adjusted to 1*10⁵ cells/ml, and 100 ul of cells were plated in each well. 100ul of fresh culture medium containing 20nM test substances AN_Bif_Abs_0016, and/or AN_Bif_Abs_0031, and/or AN_Bif_Abs_0130, and/or AN_Bif_Abs_0131, and/or AN_Bif_Abs_0160, and/or AN_Bif_Abs_0161, and/or AN_Bif_Abs_0646 was added to each well. After 3 days of culture, the cells were collected and the level of co-stimulatory molecule CD83 expressed on the surface of THP-1 cells was detected by flow cytometry. The specific operation was carried out according to the instruction manual of flow cytometry. FIG7 shows the result of the fusion polypeptide complex of the present application regulating the up-regulation of the expression of costimulatory molecules in antigen presenting cells. The fusion polypeptide complex of the present application can have the ability to induce antigen presenting cells to express costimulatory molecules.

### Example 5

### Determination of the morphological changes of antigen presenting cells during maturation

In a specific embodiment, the present application provides AN_Bif_Abs_0016, and/or AN_Bif_Abs_0031, and/or AN_Bif_Abs_0130, and/or AN_Bif_Abs_0131, and/or AN_Bif_Abs_0160, and/or AN_Bif_Abs_0161, and/or AN_Bif_Abs_0646 that induce antigen presenting cells to differentiate and mature and undergo morphological changes.

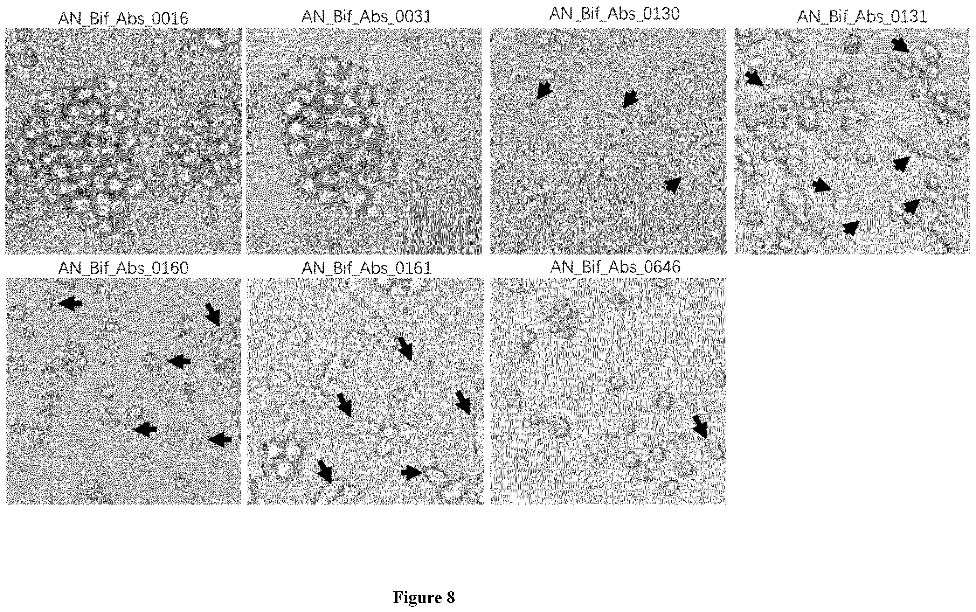

Specifically, THP-1 cells (ATCC) were collected, counted, and plated: fresh culture medium was added to mix the cells and the cell density was adjusted to 1*10⁵ cells/ml, and 100 ul of cells were plated in each well. 100ul of fresh culture medium containing 20nM test substances AN_Bif_Abs_0016, and/or AN_Bif_Abs_0031, and/or AN_Bif_Abs_0130, and/or AN_Bif_Abs_0131, and/or AN_Bif_Abs_0160, and/or AN_Bif_Abs_0161, and/or AN_Bif_Abs_0646 was added to each well. After 3 days of culture, the cells were collected and the changes in THP-1 cell morphology were detected using an inverted microscope (Leica). The specific operations were carried out according to the instruction manual of the inverted microscope. FIG8 shows the result of the fusion polypeptide complex of the present application regulating the morphological differentiation of antigen-presenting cells. The fusion polypeptide complex of the present application can have the ability to induce the differentiation and maturation of antigen-presenting cells.

### Example 6

### Assay for chemokine secretion by antigen presenting cells

In a specific embodiment, the present invention discloses that AN_Bif_Abs_0016, and/or AN_Bif_Abs_0031, and/or AN_Bif_Abs_0130, and/or AN_Bif_Abs_0131, and/or AN_Bif_Abs_0160, and/or AN_Bif_Abs_0161, and/or AN_Bif_Abs_0646 induce antigen presenting cells to secrete chemokine CCL4.

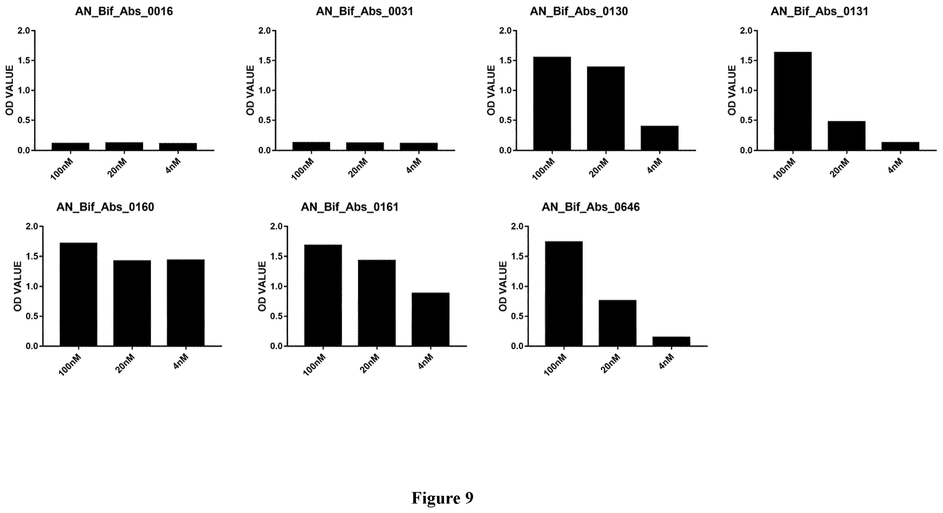

Specifically, THP-1 cells (ATCC) were collected, counted, and plated: fresh culture medium was added to mix the cells and the cell density was adjusted to 1*10⁶ cells/ml, and 100 ul of cells were plated in each well. 100ul of fresh culture medium containing AN_Bif_Abs_0016, and/or AN_Bif_Abs_0031, and/or AN_Bif_Abs_0130, and/or AN_Bif_Abs_0131, and/or AN_Bif_Abs_0160, and/or AN_Bif_Abs_0161, and/or AN_Bif_Abs_0646 of the corresponding concentration of the test substance was added to each well. After 6h, the cell culture supernatant was collected and the expression level of CCL4 was detected using a CCL4 detection kit (PROTEINTECH). The specific operations were carried out according to the manufacturer's instructions. FIG9 shows the result of the fusion polypeptide complex of the present application regulating antigen presentation and secretion of CCL4. The fusion polypeptide complex of the present application can have the ability to induce antigen presenting cells to secrete the chemokine CCL4.

### Example 7

### Antibody expression and purification

### Expression and purification of peptide complexes

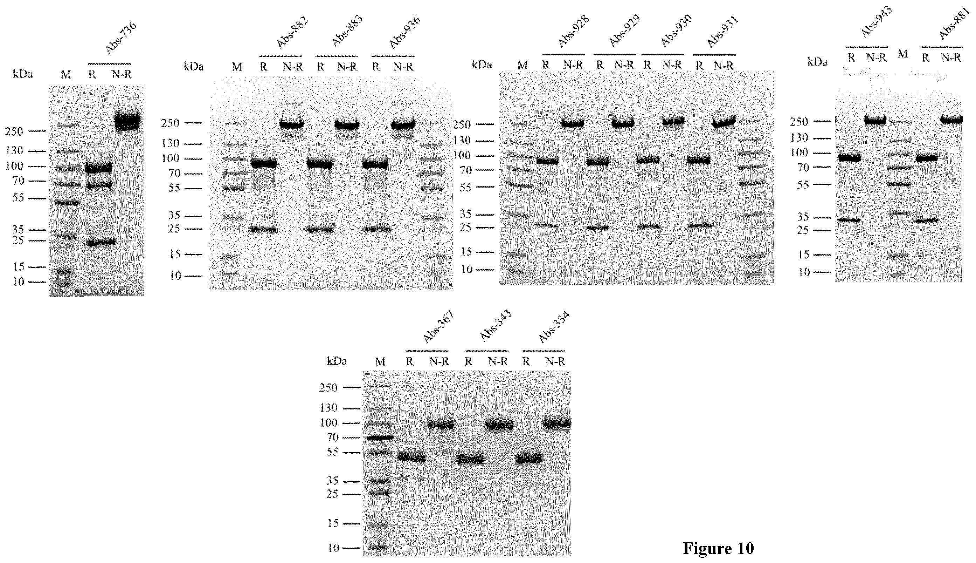

In order to further identify the screened antibodies, it is necessary to express the fusion polypeptide complex of the present application in mammalian cells. Therefore, an expression plasmid vector for expressing the polypeptide complex was first constructed. About 24 h before plasmid transfection, passage ExpiCHO cells to a cell density of about 3-4×10⁶ cells/ml. When the cell density was 6×10⁶ cells/ml and the viability was >95%, 25ug of the plasmid vector expressing the mixed heavy chain and light chain was taken and transfected into 25ml of ExpiCHO cells with ExpiFectamine^{™} CHO, and cultured in a shaking incubator at 37°C, 130rpm, and 8% CO2 for 7 days. The cell culture product was centrifuged and the supernatant was taken. After filtering with a 0.45 MCE filter, the target polypeptide complex was purified and collected using a Capturem protein A Maxiprep column, and then concentrated by centrifugation with a Vivaspin 20 Centrifugal Concentrator 50K. The A280 was determined by NanoDrop 2000 for quantification, and the antibody purity was determined by SDS-PAGE and Superdex 200-AKTA. FIG. 10 shows the result of using SDS-PAGE to detect the expression of the polypeptide complex of the present application.

### Example 8

### Protein electrophoresis analysis and AKTA analysis

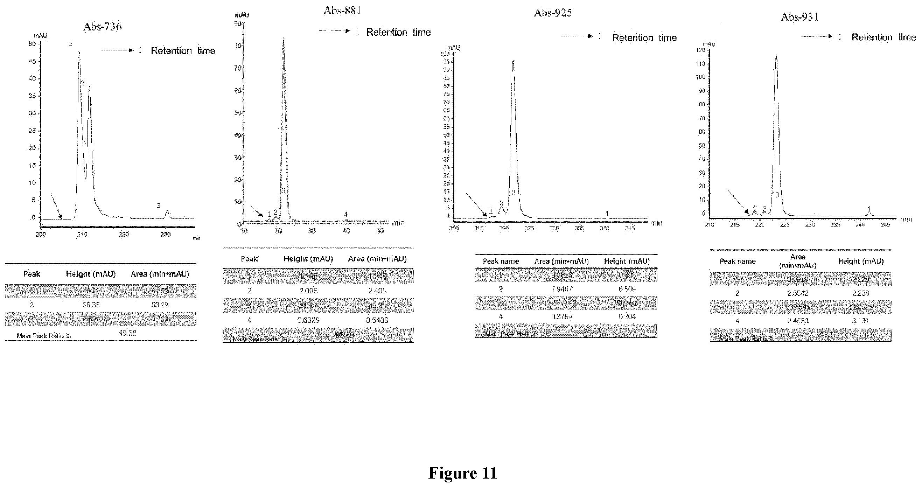

SDS-PAGE analysis of the CD86 IgV mutant fusion polypeptide complex selected and purified under reducing conditions (N-R) and non-reducing conditions (N) is shown in Figure 1; AKTA analysis of the purity of the CD86 IgV mutant fusion polypeptide complex is shown in Figure 11 .

The results showed that the antibody assembly of CD86 IgV mutant fusion polypeptide complex was superior to that of unmutated CD80 IgV fusion polypeptide complex.

### Example 9

### Binding affinity determination

In a specific embodiment, the present invention discloses the binding of CD86 variant polypeptide fusion polypeptide complex to human PD-L1 protein, human CTLA4 protein and human CD28.

Specifically, human CD28 protein (1 µg/mL in PBS; Acro, CD8-H525a) was incubated in a flat-bottom 96-well Immuno detachable transparent ELISA plate (MaxiSorp) (Thermo Scientific, 446469) at 4°C overnight; the affinity of the test substance to human CD28 protein was detected using a similar method as described above and expressed as EC₅₀. The test results are shown in the following table.

| ID | CD28 ELISA binding |
|---|---|
| | EC50 (nM) |
| Abs-881 | 1.9708 |
| Abs-882 | 3.8569 |
| Abs-946 | 2.707 |
| Abs-883 | 2.8446 |
| Abs-947 | 1.8692 |
| Abs-917 | 11.4173 |
| Abs-916 | 25.8436 |
| Abs-918 | 1.7979 |
| Abs-924 | 3.3682 |
| Abs-925 | 1.2986 |
| Abs-926 | 10.8937 |
| Abs-927 | 1.2975 |
| Abs-928 | 1.9387 |
| Abs-929 | 2.1569 |
| Abs-930 | 1.6937 |
| Abs-931 | 1.2596 |
| Abs-934 | 2.3695 |
| Abs-935 | 25.9283 |
| Abs-936 | 45.6932 |
| Abs-941 | 2.3695 |
| Abs-942 | 1.8362 |
| Abs-943 | 3.3368 |
| Abs-944 | 1.6983 |
| Abs-945 | 4.5973 |
| Abs-736 | 31.4285 |
| Abs-300 | 0.2894 |
| Abs-301 | 0.4893 |
| Abs-302 | 0.2684 |
| Abs-303 | 0.3024 |
| Abs-304 | 0.3964 |
| Abs-305 | 4.9854 |
| Abs-306 | 8.9517 |
| Abs-307 | 0.3057 |
| Abs-308 | 0.8291 |
| Abs-309 | 0.1882 |
| Abs-310 | 3.2975 |
| Abs-311 | 0.2976 |
| Abs-312 | 0.2789 |
| Abs-313 | 0.3987 |
| Abs-314 | 0.2683 |
| Abs-315 | 0.3891 |
| Abs-316 | 0.4057 |
| Abs-317 | 5.9734 |
| Abs-318 | 20.9813 |
| Abs-319 | 0.4137 |
| Abs-320 | 0.8497 |
| Abs-321 | 0.7931 |
| Abs-322 | 0.9057 |
| Abs-323 | 0.8937 |
| Abs-333 | 10.0415 |
| Abs-334 | 0.0315 |
| Abs-335 | 0.0897 |
| Abs-336 | 0.0327 |
| Abs-337 | 0.0531 |
| Abs-338 | 0.0302 |
| Abs-339 | 2.2591 |
| Abs-340 | 4.4024 |
| Abs-341 | 0.0432 |
| Abs-342 | 0.0493 |
| Abs-343 | 0.0242 |
| Abs-344 | 2.3971 |
| Abs-345 | 0.0249 |
| Abs-346 | 0.0418 |
| Abs-347 | 0.0407 |
| Abs-348 | 0.0318 |
| Abs-349 | 0.0493 |
| Abs-350 | 0.039 |
| Abs-351 | 2.971 |
| Abs-352 | 10.527 |
| Abs-353 | 0.0487 |
| Abs-354 | 0.0331 |
| Abs-355 | 0.0297 |
| Abs-356 | 0.0647 |
| Abs-357 | 0.0814 |
| Abs-367 | 5.9766 |
| Abs-16 | NA |
| Abs-23 | NA |
| Abs-958 | 30.697 |
| Abs-959 | 1.8793 |
| Abs-960 | 1.9547 |
| Abs-961 | 29.987 |

| | |
|---|---|
| NA: indicates no binding to the target protein | |

In the above table, the binding experiment of CD86 variant polypeptide fusion polypeptide complex with human CD28 antigen shows that the binding activity of CD80 mutant fusion polypeptide complex with CD28 antigen is higher than that of wild-type CD86 fusion polypeptide complex.

Similarly, human CTLA4 protein (1 µg/mL in PBS; Acro, CT4-H52H9) was incubated in a flat-bottom 96-well Immuno detachable transparent ELISA plate (MaxiSorp) (Thermo Scientific, 446469) at 4°C overnight; the affinity of the test substance to the human CTLA4 protein was detected using a similar method as above and expressed as EC₅₀. The test results are shown in the following table.

| ID | CTLA4 ELISA binding |
|---|---|
| | EC50 (nM) |
| Abs-881 | 0.7774 |
| Abs-882 | 1.5046 |
| Abs-946 | 0.6286 |
| Abs-883 | 0.7255 |
| Abs-947 | 0.9534 |
| Abs-917 | 3.924 |
| Abs-916 | 22.8002 |
| Abs-918 | 0.8414 |
| Abs-924 | 1.2684 |
| Abs-925 | 0.7987 |
| Abs-926 | 3.1684 |
| Abs-927 | 0.8192 |
| Abs-928 | 0.6167 |
| Abs-929 | 0.8561 |
| Abs-930 | 0.5224 |
| Abs-931 | 0.4941 |
| Abs-934 | 1.9015 |
| Abs-935 | 10.369 |
| Abs-936 | 19.6797 |
| Abs-941 | 0.5295 |
| Abs-942 | 4.7299 |
| Abs-943 | 0.5465 |
| Abs-944 | 0.8094 |
| Abs-945 | 3.2267 |
| Abs-736 | 2.9042 |
| Abs-300 | 0.0793 |
| Abs-301 | 0.1267 |
| Abs-302 | 0.0849 |
| Abs-303 | 0.0893 |
| Abs-304 | 0.0926 |
| Abs-305 | 0.5317 |
| Abs-306 | 0.6971 |
| Abs-307 | 0.0874 |
| Abs-308 | 0.1935 |
| Abs-309 | 0.0565 |
| Abs-310 | 0.4937 |
| Abs-311 | 0.0789 |
| Abs-312 | 0.0831 |
| Abs-313 | 0.0793 |
| Abs-314 | 0.0903 |
| Abs-315 | 0.0819 |
| Abs-316 | 0.0798 |
| Abs-317 | 2.6317 |
| Abs-318 | 4.2971 |
| Abs-319 | 0.0791 |
| Abs-320 | 0.2981 |
| Abs-321 | 0.0938 |
| Abs-322 | 0.1208 |
| Abs-323 | 0.1387 |
| Abs-333 | 0.2544 |
| Abs-334 | 0.5214 |
| Abs-335 | 0.7061 |
| Abs-336 | 0.6019 |
| Abs-337 | 0.5917 |
| Abs-338 | 0.4297 |
| Abs-339 | 1.0694 |
| Abs-340 | 1.8934 |
| Abs-341 | 0.4971 |
| Abs-342 | 0.5137 |
| Abs-343 | 0.3133 |
| Abs-344 | 0.9173 |
| Abs-345 | 0.3473 |
| Abs-346 | 0.493 |
| Abs-347 | 0.4097 |
| Abs-348 | 0.437 |
| Abs-349 | 0.3284 |
| Abs-350 | 0.437 |
| Abs-351 | 1.097 |
| Abs-352 | 5.971 |
| Abs-353 | 0.2971 |
| Abs-354 | 0.4197 |
| Abs-355 | 0.312 |
| Abs-356 | 0.598 |
| Abs-357 | 0.779 |
| Abs-367 | 0.7939 |
| Abs-16 | NA |
| Abs-23 | NA |
| Abs-958 | 2.8947 |
| Abs-959 | 0.7834 |
| Abs-960 | 0.7683 |
| Abs-961 | 3.1827 |

| | |
|---|---|
| NA: indicates no binding to the target protein | |

In the above table, the binding experiment of CD86 variant polypeptide fusion polypeptide complex with human CTLA4 antigen shows that the binding activity of CD86 variant polypeptide fusion polypeptide complex with CTLA4 antigen is higher than that of wild-type CD86 fusion polypeptide complex.

Similarly, human PD-L1 protein (1 µg/mL in PBS; Acro, PD1-H5258) was incubated in a flat-bottom 96-well Immuno detachable transparent ELISA plate (MaxiSorp) (Thermo Scientific, 446469) at 4°C overnight; then, after washing, 2% BSA(in PBS; VWR Life Science, 0332-1KG) was added to each well and incubated at room temperature for 1 hour. After washing, serial dilutions of the test substance were added to each well and added to the final test concentration and incubated at room temperature for 2 hours. After washing, Goat Anti-Human Lambda-HRP (Southern Biotech, 2060-05) was added to each well and incubated at room temperature for 30 minutes. After washing, Soluble TMB was used Kit (CW0050S, CWBIO) detection showed that the specific operation was carried out according to the manufacturer's instructions, and the affinity between the candidate multi-/bifunctional fusion protein and the human PD-L1 protein was expressed by EC50. The test results are shown in the following table.

| ID | PDL1 ELISA binding |
|---|---|
| | EC50 (nM) |
| Abs-881 | 0.058 |
| Abs-882 | 0.1754 |
| Abs-946 | 0.1504 |
| Abs-883 | 0.1009 |
| Abs-947 | 0.0753 |
| Abs-917 | 0.1305 |
| Abs-916 | 0.074 |
| Abs-918 | 0.0741 |
| Abs-924 | 0.0631 |
| Abs-925 | 0.0922 |
| Abs-926 | 0.0453 |
| Abs-927 | 0.0592 |
| Abs-928 | 0.053 |
| Abs-929 | 0.0889 |
| Abs-930 | 0.096 |
| Abs-931 | 0.0727 |
| Abs-934 | 0.1285 |
| Abs-935 | 0.0751 |
| Abs-936 | 0.0912 |
| Abs-941 | 0.0551 |
| Abs-942 | 0.0695 |
| Abs-943 | 0.0813 |
| Abs-944 | 0.0677 |
| Abs-945 | 0.0655 |
| Abs-736 | 0.0731 |
| Abs-16 | 0.0767 |
| Abs-23 | 0.1807 |
| Abs-960 | 0.1918 |
| Abs-961 | 0.1592 |

In the above table, in the binding experiment between CD86 variant polypeptide fusion polypeptide complex and human PDL1 antigen, the binding activity of CD86 variant polypeptide fusion polypeptide complex and PDL1 antigen is equivalent to the binding activity of PDL1 control antibody, indicating that CD86 variant polypeptide fusion polypeptide complex does not affect the affinity of PDL1 antibody.

### Example 10

### Determination of the release of PD-1/PD-L1-mediated T cell immunosuppression

In a specific embodiment, the present invention discloses that the test substance releases the PD-1/PD-L1-mediated T cell inhibitory response.

Specifically, on the first day, TCR activator PD-L1-CHO cells (BPS bioscience) were digested and collected using 0.05% trypsin, counted, and plated: fresh culture medium was added to mix the cells and the cell density was adjusted to 3.5*10⁵cells/ml. Add cells to a sterile 96-well flat-bottom plate (CORNING, 3599) at 100ul/well, for a total of 35,000 cells/well; grow overnight in a 37°C incubator until the cell density reaches 80%; the next day, remove the culture medium of TCR activator PD-L1- CHO cells in the 96-well flat-bottom plate, add 50ul of fresh culture medium containing the corresponding concentration of test substance (serial dilution) to each well, and after 30 minutes of treatment, collect Jurkat-Lucia^{™} TCR-hPD-1 cells (Invivogen) and count; add fresh culture medium to mix the cells and adjust the cell density to 4* 10⁵cells/ml; add 50ul Jurkat-Lucia^{™} TCR-hPD-1 cells to a final cell density of 2* 10⁴ /well, and incubate in a 96-well flat-bottom plate in a 37°C incubator for 5-6h. After incubation, the cell morphology was observed under a microscope and the chemiluminescence value was detected using QUANTI-Luc^{™} (Invivogen; rep-qlc1). The specific operation was carried out according to the manufacturer's instructions. The activity was expressed as EC50 and the highest induction fold. The test results are shown in the table below.

| ID | Jurkat-NFAT-Luc/CHO-PDL1 | |
|---|---|---|
| | EC50 (nM) | Top induction relative to 10nM sugemalimab |
| Abs-881 | 0.05 | 589% |
| Abs-882 | 0.07 | 513% |
| Abs-883 | 0.14 | 576% |
| Abs-925 | 0.08 | 601% |
| Abs-927 | 0.05 | 579% |
| Abs-928 | 0.11 | 572% |
| Abs-929 | 0.07 | 574% |
| Abs-930 | 0.11 | 535% |
| Abs-931 | 0.10 | 598% |
| Abs-934 | 0.06 | 558% |
| Abs-935 | 0.13 | 207% |
| Abs-936 | 0.10 | 270% |
| Abs-941 | 0.06 | 511% |
| Abs-942 | 0.13 | 505% |
| Abs-943 | 0.09 | 409% |
| Abs-944 | 0.05 | 547% |
| Abs-945 | 0.05 | 388% |
| Abs-736 | 0.13 | 201% |
| Abs-665 | 0.16 | 1113% |
| Abs-676 | 0.20 | 522% |
| Abs-500 | 0.12 | 591% |
| Abs-501 | 0.20 | 581% |
| Abs-502 | 0.12 | 531% |
| Abs-503 | 0.10 | 339% |
| Abs-507 | 0.16 | 564% |
| Abs-519 | 0.12 | 550% |
| Abs-520 | 0.10 | 521% |
| Abs-521 | 0.13 | 538% |
| Abs-533 | 0.15 | 210% |
| Abs-16 | 0.09 | 100% |
| Abs-960 | 0.16 | 519% |
| Abs-961 | 0.19 | 220% |
| Abs-23 | 0.36 | 135% |
| Abs-975 | 0.25 | 416% |
| Abs-976 | 0.21 | 544% |
| Abs-977 | 0.24 | 423% |
| Abs-978 | 0.22 | 550% |

| | | |
|---|---|---|
| NT: Not tested, NA: No stimulatory effect | | |

In the above table, the experiment of regulating Jurkat T cell activation by CD86 variant polypeptide fusion polypeptide complex shows that the ability of CD86 variant polypeptide fusion polypeptide complex to regulate Jurkat T cell activation is higher than that of wild-type CD86 fusion polypeptide complex.

### Example 11

### T cell activation immune response assay-1

In a specific embodiment, the present invention discloses that the test substance regulates T cell immune response.

Specifically, Jurkat-Lucia^{™} TCR cells (Invivogen) were collected and counted; fresh culture medium containing 2ug/ml anti-CD3 antibody (Invitrogen 16-0037-81) was added to mix the cells and adjust the cell density to 4*10⁵cells/ml; 100ul of fresh culture medium containing the corresponding concentration of test substance (serial dilution) was added to each well, and incubated in a 96-well flat-bottom plate in a 37°C incubator for 5-6h. After incubation, cell morphology was observed under a microscope and chemiluminescence was detected using QUANTI-Luc^{™} (Invivogen; rep-qlc1) according to the manufacturer's instructions. The results are shown in the following table.

| Antibody ID | Jurkat-Lucia^{™} TCR | |
|---|---|---|
| | EC50 (nM) | Top induction relative to IgG control |
| Abs-300 | 24.86 | 276.40% |
| Abs-319 | 28.23 | 233.50% |
| Abs-321 | 21.49 | 229.14% |
| Abs-333 | 22.84 | 146.60% |

In the above table, the CD86 variant polypeptide fusion polypeptide complex T cell activation immune response assay test shows that the CD86 variant polypeptide fusion polypeptide complex has a higher ability to activate T cell immune response than the wild-type CD86 fusion polypeptide complex.

### T cell activation immune response assay-2

In a specific embodiment, the present invention discloses that the test substance regulates T cell immune response.

Specifically, Jurkat-Lucia^{™} TCR cells (Invivogen) were collected and counted; fresh culture medium was added to mix the cells and the cell density was adjusted to 4*105 cells/ml; 100ul of fresh culture medium containing the corresponding concentration of the test substance (serially diluted) was added to each well and incubated in a 96-well flat-bottom plate in a 37°C incubator for 5-6h. After incubation, cell morphology was observed under a microscope and chemiluminescence was detected using QUANTI-Luc^{™} (Invivogen; rep-qlc1) according to the manufacturer's instructions. The results are shown in the following table.

| Antibody ID | Jurkat-Lucia^{™} TCR | |
|---|---|---|
| | EC50 (nM) | Top induction relative to 10nM Abs-3 |
| Abs-3 | 0.34 | 100% |
| Abs-958 | 24.1 | 526% |
| Abs-959 | 0.23 | 1480% |

In the above table, the CD86 variant polypeptide fusion polypeptide complex T cell activation immune response assay test shows that the CD86 variant polypeptide fusion polypeptide complex has a higher ability to activate T cell immune response than the wild-type CD86 fusion polypeptide complex.

### Example 12

### Primary PBMC immune response assay

In a specific embodiment, the present invention discloses the activation of T cell immunity in primary PBMC by the test substance. Specifically, PBMCs were collected, counted, and plated: fresh culture medium was added to mix the cells and the cell density was adjusted to 1*10⁵ cells/ml. 50ul of fresh culture medium containing the test substance (serial dilutions) at the corresponding concentration was added to each well, and 40ng/ml SEB (Toxin BT202) was added. After incubation for 3 days, the culture supernatant was collected and the secretion of IL-2 in the supernatant was detected using an IL-2 ELISA detection kit (R&D, DY202). The specific operation was carried out according to the manufacturer's instructions. The results are shown in the following table.

| ID | human PBMC IL-2 | |
|---|---|---|
| | EC50 (nM) | Top induction relative to IgG control |
| Abs-881 | 0.2206 | 448% |
| Abs-882 | 0.1609 | 352% |
| Abs-925 | 0.1685 | 413% |
| Abs-928 | 0.1138 | 339% |
| Abs-929 | 0.138 | 388% |
| Abs-931 | 0.1104 | 403% |
| Abs-934 | 0.1486 | 379% |
| Abs-936 | 1.9718 | 241% |
| Abs-941 | 0.175 | 451% |
| Abs-943 | 0.0828 | 446% |
| Abs-944 | 0.3171 | 449% |
| Abs-500 | 0.1334 | 408% |
| Abs-501 | 0.1261 | 399% |
| Abs-503 | 0.2637 | 319% |
| Abs-736 | 0.2059 | 221% |
| Abs-533 | 0.2629 | 236% |
| Abs-16 | 0.5078 | 192% |

In the above table, the ability of CD86 variant polypeptide fusion polypeptide complex to activate T cells in primary PBMC is higher than that of wild-type CD86 fusion polypeptide complex.

### Example 13

### Assay for chemokine secretion by antigen presenting cells

In a specific embodiment, the present invention discloses that a CD86 variant polypeptide fusion polypeptide complex induces antigen presenting cells to secrete the chemokine CCL4.

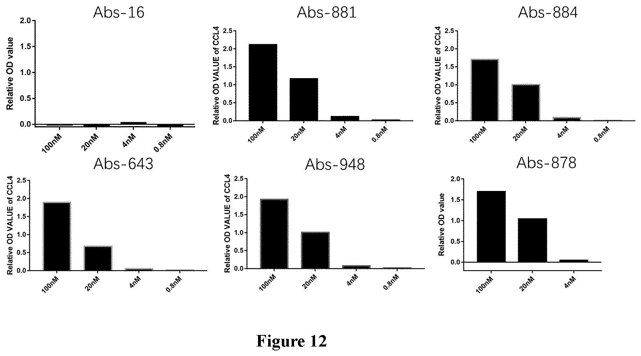

Specifically, THP-1 cells (ATCC) were collected, counted, and plated: fresh culture medium was added to mix the cells and the cell density was adjusted to 1*10⁶ cells/ml, and 100 ul of cells were plated in each well. 100ul of fresh culture medium containing the test substance at the corresponding concentration was added to each well. After 6h, the cell culture supernatant was collected and the expression level of CCL4 was detected using a CCL4 detection kit (PROTEINTECH). The specific operation was performed according to the manufacturer's instructions. As shown in Figure 12, CD86 variant polypeptide fusion polypeptide complexes Abs-881, Abs-884, Abs-643, Abs-948, Abs-878, etc. can induce antigen-presenting cells to secrete chemokine CCL4.

### Example 14

### Determination of antitumor activity

In a specific embodiment, the present invention discloses the anti-tumor activity of Abs-881.

Specifically, the genetically engineered mouse colon cancer cell line MC38 (MC38/hPD-L1) expressing human PD-L1 was used to detect and evaluate the anti-tumor activity of CD86 variant polypeptide fusion polypeptide.

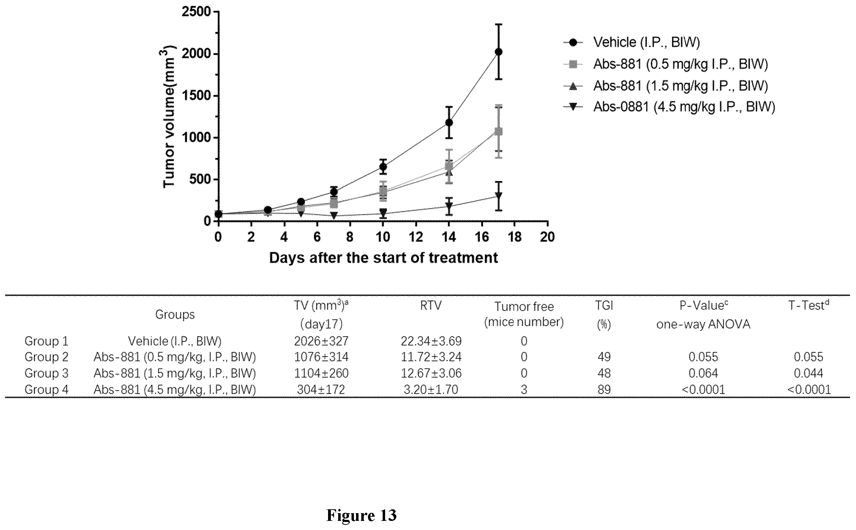

Specifically, on the first day, the MC38/hPD-L1 cells were subcutaneously implanted into C57BL/6 mice, with eight mice in each group. When the average volume of the subcutaneous tumor of the mice grew to 80 mm³, the control article (PBS buffer) or the test article Abs-881 (0.5 mg/kg, 1.5 mg/kg, 4.5 mg/kg) was intraperitoneally injected, with an interval of twice a week for two weeks. The individual tumor volumes measured over time are shown in the figure; ip = intraperitoneal, sc = subcutaneous. FIG. 13 shows the tumor inhibition curve results of the fusion polypeptide complex of the present application in the MC38/hPD-L1 tumor model.

As shown in Figure 13, 4.5 mg/kg Abs-881 significantly inhibited tumor growth, with an inhibition rate of 89%, and 37.5% (3/8) of the tumors in this group completely regressed. 0.5 mg/kg and 1.5 mg/kg Abs-881 also showed good tumor inhibition effects.

### Example 15

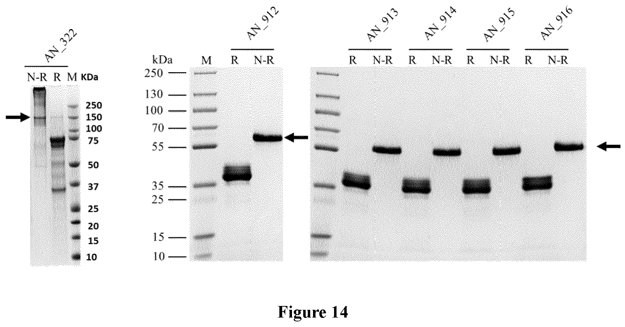

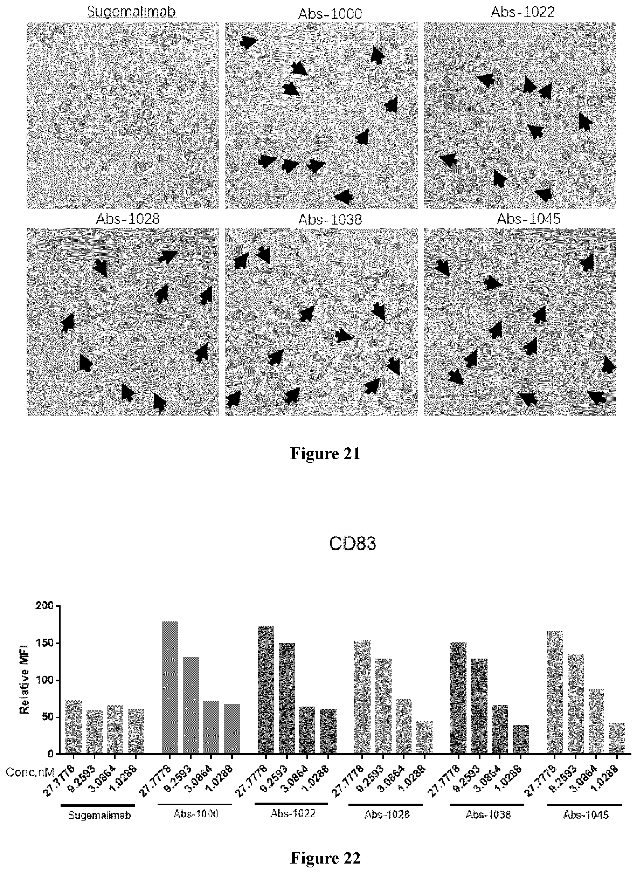

In order to further identify the screened antibodies, it is necessary to express the fusion polypeptide complex of the present application in mammalian cells. Therefore, an expression plasmid vector for expressing the polypeptide complex was first constructed. About 24 h before plasmid transfection, passage ExpiCHO cells to a cell density of about 3-4×10⁶ cells/ml. When the cell density was 6×10⁶ cells/ml and the viability was >95%, 25ug of the plasmid vector expressing the mixed heavy chain and light chain was taken and transfected into 25ml of ExpiCHO cells with ExpiFectamine^{™} CHO, and cultured in a shaking incubator at 37°C, 130rpm, and 8% CO2 for 7 days. The cell culture product was centrifuged and the supernatant was taken. After filtering with a 0.45 MCE filter, the target polypeptide complex was purified and collected using a Capturem protein A Maxiprep column, and then concentrated by centrifugation with a Vivaspin 20 Centrifugal Concentrator 50K, and quantified by the NanoDrop 2000 method for determining A280, and the antibody purity was determined by SDS-PAGE. Figures 14 and 21 show the results of using SDS-PAGE to detect the expression of the polypeptide complex of the present application. The results show that the LAG3 variant polypeptide complex does not produce high aggregates and has a higher yield and purity than the wild-type LAG3 polypeptide complex.

### Example 16

### Assay for chemokine secretion by antigen presenting cells

In a specific embodiment, the present invention discloses that the LAG3 variant polypeptide complex induces antigen presenting cells to secrete the chemokine CCL4.

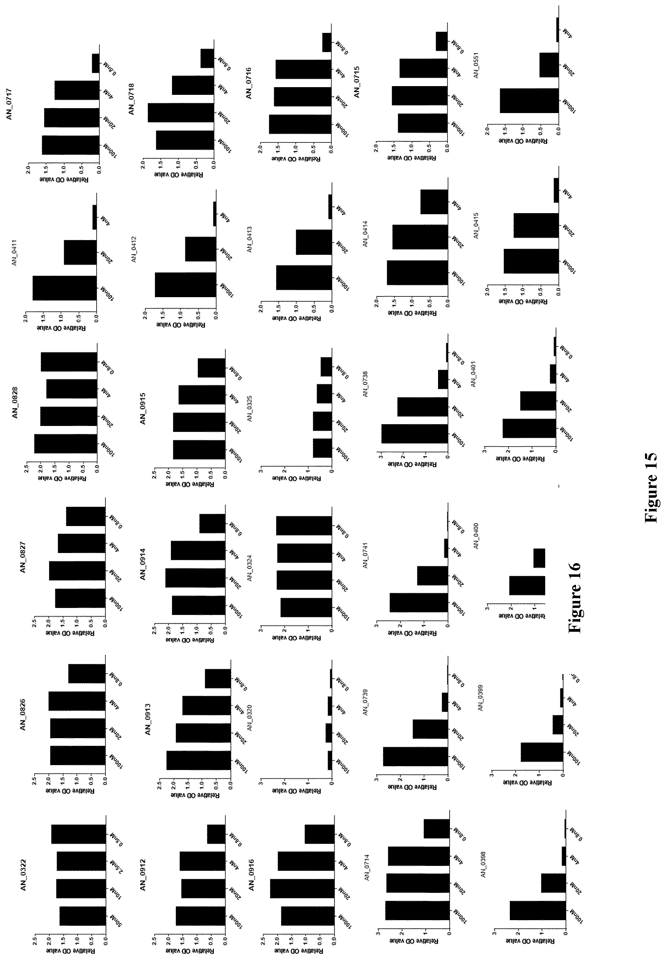

Specifically, THP-1 cells (ATCC) were collected, counted, and plated: fresh culture medium was added to mix the cells and the cell density was adjusted to 1*10⁶ cells/ml, and 100ul of cells were plated in each well. 100 ul of fresh culture medium containing the test substance at the corresponding concentration was added to each well. After 6 h, the cell culture supernatant was collected and the expression level of CCL4 was detected using a CCL4 detection kit (PROTEINTECH). The specific operation was carried out according to the manufacturer's instructions. The following Table 1, Table 2 and Figure 15 show the results of the fusion polypeptide complex of the present application regulating antigen presentation and secretion of CCL4. The LAG3 variant polypeptide complex of the present application can have the ability to induce antigen presenting cells to secrete the chemokine CCL4.

**Table 1 LAG3 variant polypeptide complex stimulates THP-1 cells to secrete CCL4**

| ID | CCL4 production (@20nM) |
|---|---|
| | Relative to AN_0322 (%) |
| AN_0320 | 9.51 |
| AN_0324 | 89.23 |
| AN_0325 | 30.01 |
| AN_0912 | 88.96 |
| AN_0913 | 110.70 |
| AN_0914 | 121.22 |
| AN_0915 | 105.67 |
| AN_0916 | 129.72 |
| AN_0398 | 39.35 |
| AN_0399 | 16.05 |
| AN_0400 | 39.97 |
| AN_0401 | 57.20 |
| AN 0411 | 47.75 |
| AN_0412 | 46.05 |
| AN_0413 | 53.31 |
| AN_0414 | 82.10 |
| AN_0415 | 66.25 |
| AN_0551 | 27.86 |
| AN_0714 | 101.81 |
| AN_0715 | 87.03 |
| AN_0716 | 91.22 |
| AN_0717 | 87.94 |
| AN_0718 | 105.27 |
| AN_0738 | 86.16 |
| AN_0739 | 55.93 |
| AN_0741 | 48.39 |
| AN_0826 | 111.00 |
| AN_0827 | 113.37 |
| AN_0828 | 115.85 |
| AN_0322 | 100.00 |
| AN_0737 | 101.83 |
| AN_0740 | 101.06 |
| AN_0742 | 122.40 |
| AN_0673 | 100.19 |
| AN_0736 | 98.36 |
| AN_0685 | 114.62 |
| AN_0997 | 94.73 |
| AN_1000 | 94.21 |
| AN_1012 | 94.73 |
| AN_1013 | 89.47 |
| IgG control | 0.00 |

**Table 2 LAG3 variant polypeptide complex stimulates THP-1 cells to secrete CCL4**

| ID | CCL4 production (@100nM) Relative to sugemalimab (%) |
|---|---|
| Abs-1022 | 1120 |
| Abs-1045 | 1113 |
| Abs-1036 | 466 |
| Abs-1000 | 1100 |
| Abs-1028 | 1118 |
| Abs-1038 | 1191 |
| Abs-1027 | 156 |
| Abs-1024 | 129 |
| Abs-1046 | 1050 |
| Abs-1034 | 778.32 |
| Abs-1036 | 529.55 |
| Abs-1052 | 1140.82 |
| IgG isotype | 100 |

### Example 17

### Chemokine secretion assay of primary PBMC cells

In a specific embodiment, the present invention discloses that the LAG3 variant polypeptide complex induces primary PBMC cells to secrete the chemokine CCL4.

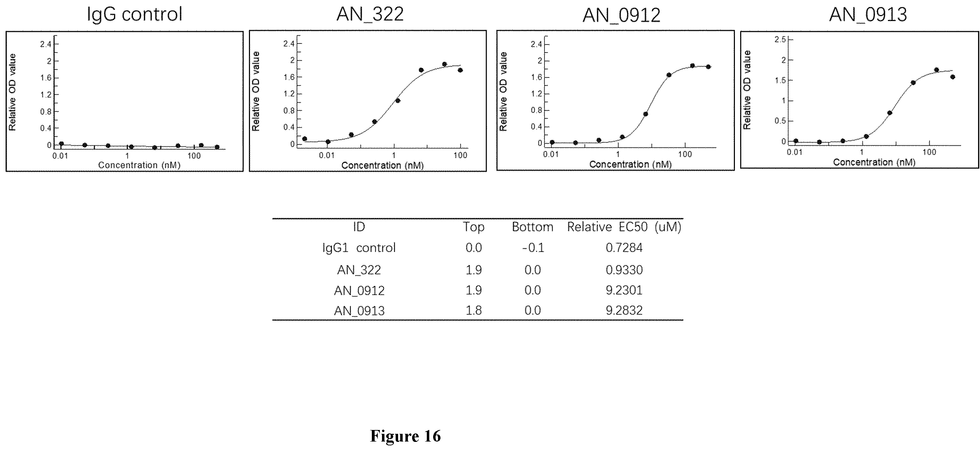

Specifically, primary PBMC cells (SAILYBIO XFWBC6089W) were collected, counted and plated: fresh culture medium was added to mix the cells and the cell density was adjusted to 1*10⁶ cells/ml, and 100ul of cells were plated in each well. 100 ul of fresh culture medium containing the test substance at the corresponding concentration was added to each well. After 6 h, the cell culture supernatant was collected and the expression level of CCL4 was detected using a CCL4 detection kit (PROTEINTECH). The specific operation was carried out according to the manufacturer's instructions. FIG. 16 shows the results of the fusion polypeptide complex of the present application regulating antigen presentation and secretion of CCL4. The fusion polypeptide complex of the present application can have the ability to induce primary PBMC cells to secrete the chemokine CCL4.

### Example 18

### Determination of morphological changes in the maturation of primary dendritic cells (DCs)

In a specific embodiment, the present invention discloses that the LAG3 variant polypeptide complex induces DCs cell differentiation and maturation to cause morphological changes.

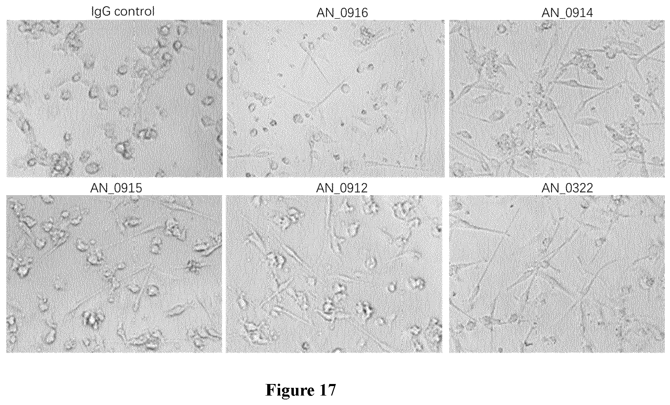

Specifically, monocytes derived from PBMC were obtained, counted and plated: fresh culture medium was added to mix the cells and the cell density was adjusted to 1*10⁶ cells/ml. After 5 days of inducing differentiation by adding IL-4 and GM-CSF, immature DCs cells were collected and then the cells were adjusted to 1*10⁵ cells/ml and 100ul of cells were plated in each well. DCs cells were stimulated with 10 nM LAG3 variant polypeptide complex test substance. After 6 h of culture, the changes in DCs cell morphology were detected using an inverted microscope (Leica). The specific operation was performed according to the instruction manual of the inverted microscope. Figures 17 and 21 show the results of the fusion polypeptide complex of the present application regulating the morphological differentiation of DCs cells. The fusion polypeptide complex of the present application can have the ability to induce the differentiation and maturation of primary DCs cells.

### Example 19

### Primary dendritic cells (DCs) upregulate the expression of co-stimulatory molecules

In a specific embodiment, the present invention discloses that the LAG3 variant polypeptide complex induces DCs cell differentiation and maturation and upregulates the expression of co-stimulatory molecules

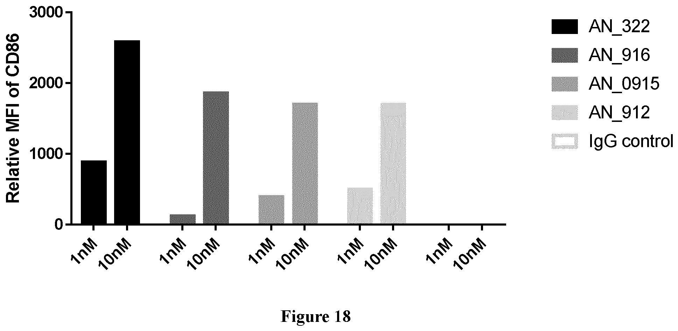

Specifically, immature DCs cells were collected, counted and plated: fresh culture medium was added to mix the cells and the cell density was adjusted to 1*10⁵ cells/ml, and 100ul of cells were plated in each well. 100ul of fresh culture medium containing LAG3 variant polypeptide complexes with corresponding concentrations of test substances (serial dilutions) was added to each well. After culturing for 3 days, the cells were collected and the levels of co-stimulatory molecule CD86 expressed on the surface of DCs were detected by flow cytometry. The specific operation was carried out according to the instruction manual of flow cytometry. Figures 18 and 22 show the results of the fusion polypeptide complex of the present application regulating DCs cells to up-regulate the expression of costimulatory molecules. The fusion polypeptide complex of the present application can have the ability to induce primary DCs cells to express costimulatory molecules.

### Example 20

### Primary dendritic cells (DCs) upregulate the expression of cytokine TNF-α

In a specific embodiment, the present invention discloses that the LAG3 variant polypeptide complex induces DCs cell differentiation and maturation and upregulates the expression of cytokines

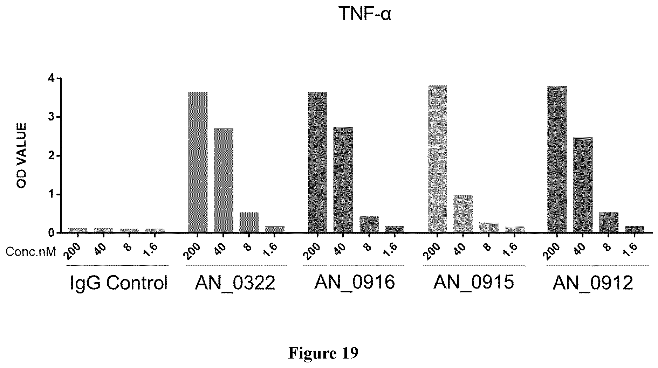

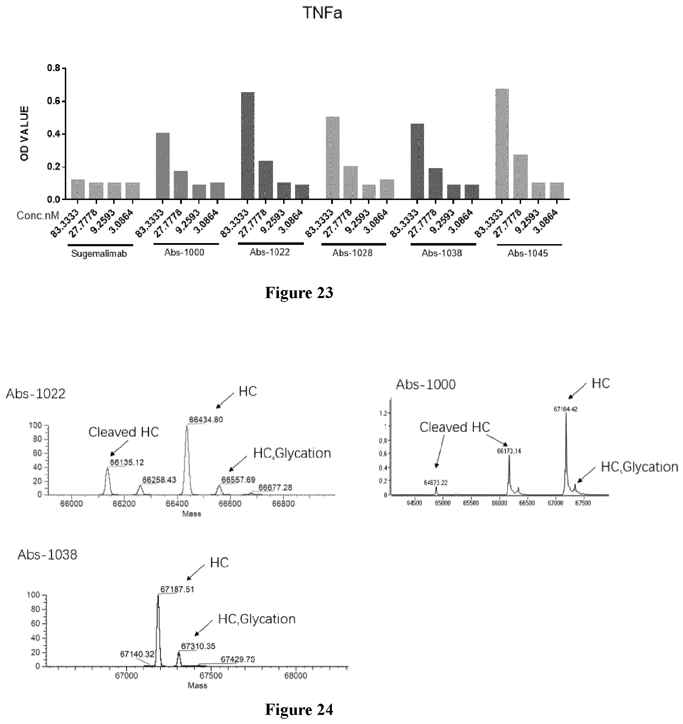

Specifically, immature DCs cells were collected, counted and plated: fresh culture medium was added to mix the cells and the cell density was adjusted to 1*10⁵ cells/ml, and 100ul of cells were plated in each well. 100ul of fresh culture medium containing LAG3 variant polypeptide complexes with corresponding concentrations of test substances (serial dilutions) was added to each well. After 3 days of culture, the culture supernatant was collected and cultured using human TNF-α DuoSet ELISA (R&D DY210). Specific operations were performed according to the instruction manual. Figures 19 and 23 show the results of the fusion polypeptide complex of the present application regulating the secretion of TNF-α by DCs cells. The fusion polypeptide complex of the present application can have the ability to induce increased secretion of TNF-α by primary DCs cells.

### Example 21

### T cell activation immune response assay

In a specific embodiment, the present invention discloses that the LAG3 variant polypeptide complex regulates T cell immune response.

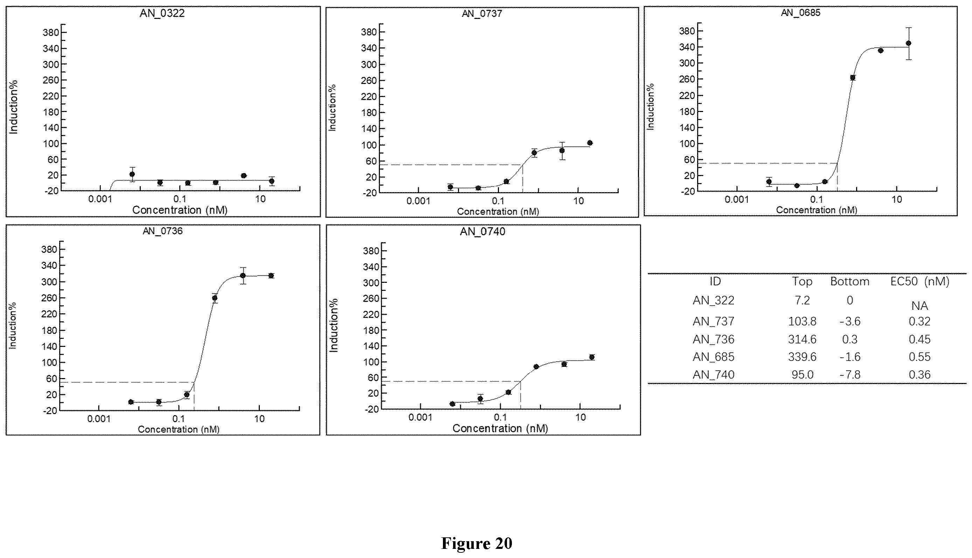

Specifically, on the first day, TCR activator PD-L1-CHO cells (BPS bioscience) were digested and collected using 0.05% trypsin, counted, and plated: fresh culture medium was added to mix the cells and the cell density was adjusted to 3.5*10⁵cells/ml. Add cells to a sterile 96-well flat-bottom plate (CORNING, 3599) at 100ul/well, for a total of 35,000 cells/well; grow overnight in a 37°C incubator to 80%; the next day, remove the culture medium of TCR activator PD-L1- CHO cells in the 96-well flat-bottom plate, add 50ul of fresh culture medium containing the corresponding concentration of test substance (serial dilution) to each well, and after 30 minutes of treatment, collect Jurkat-Lucia^{™} TCR-hPD-1 cells (Invivogen) and count; add fresh culture medium to mix the cells and adjust the cell density to 4*10⁵cells/ml; add 50ul Jurkat-Lucia^{™} TCR-hPD-1 cells to a final cell density of 2*10⁴ /well, and incubate in a 96-well flat-bottom plate in a 37°C incubator for 5-6h. After incubation, observe cell morphology under a microscope and detect chemiluminescence using QUANTI-Luc^{™} (Invivogen; rep-qlc1) according to the manufacturer's instructions. FIG20 shows the result of regulating T cell activation by the fusion polypeptide complex of the present application. The fusion polypeptide complex of the present application has the ability to induce increased secretion of IL-2 by activated T cells.

### Example 22

### Octet R8 molecular interaction detection of PDL1, CD28 and CTLA4 affinity

In a specific embodiment, the present invention discloses the binding affinity of LAG3 variant polypeptide complexes to PDL1, CD28 and CTLA4.

Soak the Protein A probe in PBST for 10 minutes; prepare LAG3 variant polypeptide complex antibody with a final concentration of 5ug/mL; configure hPD-L1, hCTLA-4, and hCD28 at a concentration of 50nM (diluent is PBST), use PBST as the diluent, and dilute them in a 96-well plate in a 2-fold gradient, with a total of 8 concentration gradients, of which the 8th concentration is 0nM (PBST); after establishing the operating method, add the required reagents and antibodies to the configured equilibration solution, binding solution, and dissociation solution in turn according to the operating method for detection, run the method, perform detection, and use Octet R8 system analysis software for data analysis and export. Table 3 shows the Kd values of the binding affinity of the fusion polypeptide complex of the present application to PDL1, CD28 and CTLA4.

**Table 3 Kd values of LAG3 variant polypeptide complexes binding to PDL1, CD28 and CTLA4**

| ID | PDL1 Kd(M) | CD28 Kd(M) | CTLA4 Kd(M) |
|---|---|---|---|
| Abs-1022 | 6.309E-10 | 4.652E-09 | 5.162E-10 |
| Abs-1045 | 8.936E-10 | 3.409E-09 | 4.265E-10 |
| Abs-1036 | 5.174E-10 | 3.664E-09 | 4.260E-10 |
| Abs-1000 | 3.807E-10 | 2.563E-09 | 4.168E-10 |
| Abs-1028 | 4.965E-10 | 2.902E-09 | 4.863E-10 |
| Abs-1038 | 4.197E-10 | 2.699E-09 | 4.045E-10 |
| Abs-1027 | 4.389E-10 | 2.834E-09 | 4.432E-10 |
| Abs-1024 | 5.987E-10 | 3.135E-09 | 4.210E-10 |
| Abs-1046 | 6.591E-10 | 3.781E-09 | 4.542E-10 |
| sugemalimab | 5.811E-10 | NA | NA |

| | | | |
|---|---|---|---|
| NA indicates no binding activity. | | | |

### Example 23

### Deglycosylated reduced molecular weight LC-MS analysis

Samples for protein or peptide mass spectrometry detection can also be subjected to deglycosylation or reduction treatment as required. Deglycosylation: Take a part of the test sample and react with PNGase F enzyme at 37°C for 2 hours. Reduction: Take a portion of the test sample and reduce it with 10 mM DTT or TCEP for 0.5 h. After treatment, the samples were separated using the U3000 (ultra-high performance liquid chromatography) system. Liquid A was 0.1% formic acid in water, and liquid B was 0.1% formic acid in acetonitrile. The column temperature was 35 °C, and the flow rate was 0.5 mL/min. The mobile phase gradient is shown below. The treated samples were separated by ultra-high performance liquid phase and then detected and scanned by Orbitrap Fusion high-resolution mass spectrometer. The analysis time was 10 minutes in positive ion mode. All raw data files after mass spectrometry acquisition were characterized and analyzed by PEAKS software for protein molecular weight. FIG24 shows the results of deglycosylated reduced molecular weight of the fusion polypeptide complex of the present application. After the mutation of LAG3 in the fusion polypeptide complex of the present application, the generation of LAG3 fragments during the production process (such as Abs-1038) can be prevented, and the complete molecule can be effectively produced.

## Claims

1. A fusion polypeptide comprising a first domain and a second domain, wherein the first domain is capable of blocking PD-1/PD-L1 signal, and the second domain comprises CD86 or a functionally active fragment thereof.

2. The fusion polypeptide according to claim 1, wherein the first domain is capable of binding to PD-L1 and/or PD-1.

3. The fusion polypeptide according to any one of claims 1 to 2, wherein the first domain comprises an antibody or an antigen-binding fragment thereof.

4. The fusion polypeptide according to claim 3, wherein the antibody is selected from the group consisting of a recombinant antibody, a single domain antibody, a heavy chain antibody, a chimeric antibody and a bispecific antibody.

5. The fusion polypeptide according to claim 3, wherein the antigen-binding fragment is selected from one or more of the group consisting of Fab, Fab', Fv fragment, F(ab')₂, F(ab)₂, scFv, di-scFv, VHH and dAb.

6. The fusion polypeptide according to any one of claims 1 to 5, wherein the first domain comprises an antibody or an antigen-binding fragment thereof selected from the group consisting of Sugemalimab, Durvalumab, Atezolizumab, Avelumab, Envafolimab, Permbrolizumab and Nivolumab.

7. The fusion polypeptide according to any one of claims 1 to 6, wherein the first domain comprises HCDR1, HCDR2 and/or HCDR3 of an antibody heavy chain, and the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15 and SEQ ID NO: 17.

8. The fusion polypeptide according to any one of claims 1 to 7, wherein the first domain comprises a heavy chain variable region VH of an antibody heavy chain, and the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15 and SEQ ID NO: 17.

9. The fusion polypeptide according to any one of claims 1 to 8, wherein the first domain comprises an antibody heavy chain, and the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15 and SEQ ID NO: 17.

10. The fusion polypeptide according to any one of claims 1 to 9, wherein the first domain comprises LCDR1, LCDR2 and/or LCDR3 of a light chain of an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

11. The fusion polypeptide according to any one of claims 1 to 10, wherein the first domain comprises a light chain variable region VL of an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

12. The fusion polypeptide according to any one of claims 1 to 11, wherein the first domain comprises an antibody light chain, and the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

13. The fusion polypeptide according to any one of claims 1 to 12, wherein the first domain comprises Sugemalimab or an antigen-binding fragment thereof.

14. The fusion polypeptide according to any one of claims 1 to 13, wherein the second domain is capable of binding to CD28 and/or CTLA4.

15. The fusion polypeptide according to any one of claims 1 to 14, wherein the second domain is selected from the group consisting of CD86 derived from humans or a functionally active fragment thereof and CD86 derived from mice or a functionally active fragment thereof.

16. The fusion polypeptide according to any one of claims 1 to 15, wherein the second domain comprises the IgV domain of CD86 or a functionally active fragment thereof.

17. The fusion polypeptide according to any one of claims 1 to 16, wherein the second domain comprises the extracellular domain of CD86 or a functionally active fragment thereof.

18. The fusion polypeptide according to any one of claims 1 to 17, wherein the second domain comprises the amino acid sequence shown in SEQ ID NO: 2 or SEQ ID NO: 3.

19. The fusion polypeptide according to any one of claims 1 to 15, wherein the CD86 or a functionally active fragment thereof comprises a CD86 variant polypeptide.

20. The fusion polypeptide according to claim 19, wherein the CD86 variant polypeptide comprises an amino acid substitution mutation of human CD86.

21. The CD86 variant polypeptide according to claim 20, comprising an amino acid substitution mutant of the IgV domain of the human CD86 extracellular domain.

22. The fusion polypeptide according to any one of claims 19 to 21, wherein the CD86 variant polypeptide comprises an amino acid substitution mutant of the IgV domain of CD86, and the mutation site of the amino acid substitution mutant of the IgV domain of CD86 comprises one or more of the amino acid mutations selected from the group consisting of A13I, A13L, A13F, A13M, Q25A, Q25I, Q25V, Q25F, Q25M, F33A, F33L, F33V, F33M, M60R, I89A, I89L, I89V, I89F, I89M, H90I, H90V, H90M, and H90F.

23. The fusion polypeptide according to any one of claims 19 to 22, wherein the CD86 variant polypeptide comprises a combination of 1, 2, 3, 4, 5 or more amino acid mutations selected from the group consisting of A13I, A13L, A13F, A13M, Q25A, Q25I, Q25V, Q25F, Q25M, F33A, F33L, F33V, F33M, M60R, I89A, I89L, I89V, I89F, I89M, H90I, H90V, H90M, and H90F.

24. The fusion polypeptide according to any one of claims 19 to 23, wherein the CD86 variant polypeptide comprises an amino acid substitution mutant of the IgV domain of CD86, wherein the amino acid substitution mutant comprises one or more groups selected from the combination shown in the following group: Q25I/F33L/H90I, Q25V/F33L/H90I, Q25I/F33V/H90I, Q25V/F33V/H90I, Q25I/F33L/H90V, Q25V/F33L/H90V, Q25I/F33V/H90V, Q25V/F33V/H90V, A13L/Q25I/F33L/H90I, A13I/Q25I/F33L/H90I, A13L/Q25V/F33L/H90I, A13I/Q25V/F33L/H90 I. Q25I/F33L/I89L/H90I, Q25I/F33L/M60R/H90I, Q25V/F33L/I89L/H90I, Q25V/F33L/M60R/H90I, Q25F/F33L/H90I, Q25I/F33L/H90F, Q25I/F33A/H90I, Q25I/H90I, Q25I, H90I, Q25V/H90V, Q25V/H90I, Q25F/H90I, Q2SF/H90V, A13F/Q25I/F33L/H90I, A13M/Q25I/F33L/H90I, Q25A/F33L/H90I, Q25M/F33L/H90I, Q25I/F33M/H90I, Q25I/F33L/I89V/H90I, Q25I/F33L/I89F/H90I, Q25I/F33L/I89M/H90I and Q25I/F33L/H90M.

25. The fusion polypeptide according to any one of claims 19 to 24, wherein the CD86 variant polypeptide comprises the IgV domain amino acid substitution mutant Q25I/F33L/H90I of CD86.

26. The fusion polypeptide according to any one of claims 19 to 25, wherein the CD86 variant polypeptide comprises an amino acid substitution mutant of the extracellular domain of human CD86.

27. The fusion polypeptide according to any one of claims 19 to 26, wherein the CD86 variant polypeptide comprises an amino acid substitution mutant of the extracellular domain of CD86, and the amino acid substitution mutant of the extracellular domain of CD86 comprises one or more mutations in the amino acid sites selected from the group consisting of A13I, A13L, A13F, A13M, Q25A, Q25I, Q25V, Q25F, Q25M, F33A, F33L, F33V, F33M, M60R, I89A, I89L, I89V, I89F, I89M, H90I, H90V, H90M, and H90F.

28. The fusion polypeptide according to any one of claims 19 to 27, wherein the CD86 variant polypeptide comprises an amino acid substitution mutant of the extracellular domain of CD86, wherein the amino acid substitution mutant comprises one or more groups selected from the combination shown in the following group: Q25I/F33L/H90I, 25V/F33L/H90I, Q25I/F33V/H90I, Q25V/F33V/H90I, Q25I/F33L/H90V, Q25V/F33L/H90V, Q25I/F33V/H90V, Q25V/F33V/H90V, A13L/Q25I/F33L/H90I, A131/Q251/F33 L/H90I, A13L/Q25V/F33L/H90I, A13I/Q25V/F33L/H90I, Q25I/F33L/I89L/H90I, Q25I/F33L/M6 0R/H90I, Q25V/F33L/I89L/H90I, Q25V/F33L/M60R/H90I, Q25F/F33L/H90I, Q25I/F33L/H90F, Q25I/F33A/H90I, Q25I/H90I, Q25I, H90I, Q25V/H90V, Q25V/H90I, Q25F/H90I, Q25F/H90V, A13 F/Q25I/F33L/H90I, A13M/Q25I/F33L/H90I, Q25A/F33L/H90I, Q25M/F33L/H90I, Q25I/F33M/H 90I, Q25I/F33L/I89V/H90I, Q25I/F33L/I89F/H90I, Q25I/F33L/I89M/H90I and Q25I/F33L/H90M.

29. The fusion polypeptide according to any one of claims 19 to 28, wherein the CD86 variant polypeptide comprises the extracellular domain amino acid substitution mutant Q25I/F33L/H90I of CD86.

30. The fusion polypeptide according to any one of claims 19 to 29, wherein the CD86 variant polypeptide comprises an amino acid sequence selected from the group consisting of SEQ ID NO: 178 to SEQ ID NO: 243.

31. The fusion polypeptide according to any one of claims 1 to 30, wherein the first domain is directly or indirectly connected to the second domain.

32. The fusion polypeptide according to any one of claims 1 to 31, wherein the first domain comprises an antibody heavy chain, and the first domain antibody heavy chain is directly or indirectly connected to the second domain.

33. The fusion polypeptide according to any one of claims 1 to 32, wherein the first domain comprises an antibody heavy chain, and the C-terminus of the antibody heavy chain of the first domain is directly or indirectly connected to the N-terminus of the second domain.

34. The fusion polypeptide according to any one of claims 1 to 33, wherein the first domain comprises an antibody heavy chain, and the N-terminus of the antibody heavy chain of the first domain is directly or indirectly connected to the C-terminus of the second domain.

35. The fusion polypeptide according to any one of claims 1 to 34, wherein the first domain comprises an antibody light chain, and the first domain antibody light chain is directly or indirectly connected to the second domain.

36. The fusion polypeptide according to any one of claims 1 to 35, wherein the first domain comprises an antibody light chain, and the C-terminus of the antibody light chain of the first domain is directly or indirectly connected to the N-terminus of the second domain.

37. The fusion polypeptide according to any one of claims 1 to 36, wherein the first domain comprises an antibody light chain, and the N-terminus of the antibody light chain of the first domain is directly or indirectly connected to the C-terminus of the second domain.

38. The fusion polypeptide according to any one of claims 1 to 37, wherein the first domain comprises an antibody heavy chain and an antibody light chain, the C-terminus of the antibody heavy chain of the first domain is directly or indirectly connected to the N-terminus of the second domain, or the C-terminus of the antibody light chain of the first domain is directly or indirectly connected to the N-terminus of the second domain.

39. The fusion polypeptide according to any one of claims 1 to 38, wherein the first domain comprises an antibody heavy chain and an antibody light chain, the N-terminus of the antibody heavy chain of the first domain is directly or indirectly connected to the C-terminus of the second domain, or the N-terminus of the antibody light chain of the first domain is directly or indirectly connected to the C-terminus of the second domain.

40. The fusion polypeptide according to any one of claims 1 to 39, wherein the indirect connection comprises connection through a linker.

41. The fusion polypeptide according to claim 40, wherein the linker comprises an amino acid sequence selected from the group consisting of SEQ ID NO: 31, SEQ ID NO: 32, SEQ ID NO: 33, SEQ ID NO: 34 and SEQ ID NO: 35.

42. The fusion polypeptide according to any one of claims 1 to 41, wherein the fusion polypeptide comprises an amino acid sequence selected from the group consisting of SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82. SEQ ID NO: 83, SEQ ID NO: 84, SEQ ID NO: 85, SEQ ID NO: 86 and SEQ ID NO: 87.

43. The fusion polypeptide according to any one of claims 1 to 42, wherein the fusion polypeptide comprises an amino acid sequence selected from the group consisting of: SEQ ID NO:418 to SEQ ID NO:450, SEQ ID NO:451 to SEQ ID NO:483, SEQ ID NO:484 to SEQ ID NO:615.

44. The fusion polypeptide according to any one of claims 1 to 43, further comprising a third domain, wherein the third domain is capable of activating an innate immune response.

45. According to the fusion polypeptide of claim 44, the third domain is capable of binding to the MHCII molecule on the antigen presenting cell.

46. The fusion polypeptide according to any one of claims 44 to 45, wherein the third domain comprises LAG3 or a functionally active fragment thereof.

47. The fusion polypeptide according to any one of claims 44 to 46, wherein the third domain is selected from the group consisting of human-derived LAG3 or a functionally active fragment thereof and mouse-derived LAG3 or a functionally active fragment thereof.

48. The fusion polypeptide according to any one of claims 44 to 47, wherein the third domain comprises the extracellular domain of LAG3 or a functionally active fragment thereof.

49. The fusion polypeptide according to any one of claims 44 to 48, wherein the third domain comprises IgD1, IgD2, IgD3 and/or IgD4 of LAG3 or a functionally active fragment thereof.

50. The fusion polypeptide according to any one of claims 44 to 49, wherein the third domain comprises IgD1, IgD1-IgD2, IgD1-IgD2-IgD3, and/or IgD1-IgD2-IgD3-IgD4 of LAG3 or a functionally active fragment thereof.

51. The fusion polypeptide according to any one of claims 44 to 50, wherein the third domain comprises an amino acid sequence selected from the group consisting of SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27 and SEQ ID NO: 28.

52. The fusion polypeptide according to any one of claims 44 to 51, wherein the LAG3 or a functionally active fragment thereof comprises a LAG3 variant polypeptide, and the LAG3 variant polypeptide comprises a truncation and/or mutation based on human LAG3.

53. The fusion polypeptide according to claim 52, wherein the LAG3 variant polypeptide comprises a truncation based on a wild-type human LAG3 extracellular region polypeptide.

54. The fusion polypeptide according to any one of claims 52 to 53, wherein the LAG3 variant polypeptide has 0-124 amino acids truncated at the N-terminus based onbased on the wild-type human LAG3 extracellular region polypeptide, and terminates at amino acid positions 121-167 at the C-terminus based onbased on the wild-type human LAG3 extracellular region polypeptide.

55. The fusion polypeptide according to any one of claims 52 to 54, wherein the LAG3 variant polypeptide has 0, 5, 21, 37, 45, 60, 71, 74, 79, 84, 89, 94, 99, 104, 109 or 114 amino acids truncated at the N-terminus based on the wild-type human LAG3 extracellular region polypeptide.

56. The fusion polypeptide according to any one of claims 52 to 55, wherein the LAG3 variant polypeptide has 0, 5, 21, 37, 45, 60, 71, 74, 81 or 99 amino acids truncated at the N-terminus based on the wild-type human LAG3 extracellular region polypeptide.

57. The fusion polypeptide according to any one of claims 52 to 56, wherein the LAG3 variant polypeptide has 0, 5, 21, 37, 45, 60, 71 or 74 amino acids truncated at the N-terminus based on the wild-type human LAG3 extracellular region polypeptide.

58. The fusion polypeptide according to any one of claims 52-57, wherein the LAG3 variant polypeptide terminates at amino acid position 121, 122, 129, 136, 146, 156, 157, 158, 159, 160, 161, 162, 163, 164, 165, 166 or 167 at the C-terminus based on the wild-type human LAG3 extracellular region polypeptide.

59. The fusion polypeptide according to any one of claims 52 to 58, wherein the LAG3 variant polypeptide terminates at amino acid position 121, 122, 129, 136, 146, 156, 161 or 167 at the C-terminus based on the wild-type human LAG3 extracellular region polypeptide.

60. The fusion polypeptide according to any one of claims 52-59, wherein the LAG3 variant polypeptide terminates at amino acid position 121, 146, 156, 161 or 167 at the C-terminus based on the wild-type human LAG3 extracellular region polypeptide.

61. The fusion polypeptide according to any one of claims 52-60, wherein the LAG3 variant polypeptide terminates at amino acid position 156, 161 or 167 at the C-terminus based on the wild-type human LAG3 extracellular region polypeptide.

62. The fusion polypeptide according to any one of claims 52 to 61, wherein the LAG3 variant polypeptide has 0, 5, 21, 37, 45, 60, 71, 74, 81 or 99 amino acids truncated at the N-terminus based on the wild-type human LAG3 extracellular region polypeptide, and terminates at amino acid position 121, 122, 129, 136, 146, 156, 157, 158, 159, 160, 161, 162, 163, 164, 165, 166 or 167 at the C-terminus based on the wild-type human LAG3 extracellular region polypeptide.

63. The fusion polypeptide according to any one of claims 52 to 62, wherein the LAG3 variant polypeptidehas 0, 5, 21, 37, 45, 60, 71 or 74 amino acids truncated at the N-terminus based on the wild-type human LAG3 extracellular region polypeptide, and terminates at amino acid position 122, 129, 136, 146, 156, 157, 158, 159, 160, 161, 162, 163, 164, 165, 166 or 167 at the C-terminus based on the wild-type human LAG3 extracellular region polypeptide.

64. The fusion polypeptide according to any one of claims 52 to 63, wherein the LAG3 variant polypeptide comprises an amino acid mutation.

65. The fusion polypeptide according to any one of claims 52 to 64, wherein the LAG3 variant polypeptide comprises an amino acid mutation, and the amino acid mutation site is the Arg amino acid at position 97.

66. The fusion polypeptide according to any one of claims 52 to 65, wherein the LAG3 variant polypeptide comprises an amino acid mutation, wherein the amino acid mutation site is a mutation of the Arg amino acid at site 97 to a Glu amino acid.

67. The fusion polypeptide according to any one of claims 52 to 66, wherein the LAG3 variant polypeptide is selected from the amino acid sequences shown by SEQ ID NO: 244 to SEQ ID NO: 349.

68. The fusion polypeptide according to any one of claims 52 to 67, wherein the LAG3 variant polypeptide comprises an amino acid mutation, and the amino acid mutation site is R110, R113, R119, R129, G130 and/or R141.

69. The fusion polypeptide according to any one of claims 52 to 68, wherein the LAG3 variant polypeptide is truncated by 81 amino acids at the N-terminus and terminates at amino acid position 121 at the C-terminus, and a mutation is introduced at R110, and/or R113, and/or R119.

70. The fusion polypeptide according to any one of claims 52 to 69, wherein the LAG3 variant polypeptide is truncated by 99 amino acids at the N-terminus and terminates at amino acid position 146 at the C-terminus, and a mutation is introduced at R129, and/or G130, and/or R141.

71. The fusion polypeptide according to any one of claims 52 to 70, wherein the LAG3 variant polypeptide is truncated by 81 amino acids at the N-terminus and terminates at amino acid position 146 at the C-terminus, and a mutation is introduced at R110, and/or R113, and/or R119, and/or R129, and/or G130, and/or R141.

72. The fusion polypeptide according to any one of claims 52 to 71, wherein the LAG3 variant polypeptide comprises an amino acid mutation, and the R110 site is mutated to K110.

73. The fusion polypeptide according to any one of claims 52 to 72, wherein the LAG3 variant polypeptide comprises an amino acid mutation, and the R113 site is mutated to K113.

74. The fusion polypeptide according to any one of claims 52 to 73, wherein the LAG3 variant polypeptide comprises an amino acid mutation, and the R119 site is mutated to K119.

75. The fusion polypeptide according to any one of claims 52 to 74, wherein the LAG3 variant polypeptide comprises an amino acid mutation, and the R129 site is mutated to K129.

76. The fusion polypeptide according to any one of claims 52 to 75, wherein the LAG3 variant polypeptide comprises an amino acid mutation, and the G130 site is mutated to P130, or A130, or T130, or Y130, or S130.

77. The fusion polypeptide according to any one of claims 52 to 76, wherein the LAG3 variant polypeptide comprises an amino acid mutation, and the R141 site is mutated to K141.

78. The fusion polypeptide of any one of claims 52 to 77, wherein the amino acid sequence of the LAG3 variant polypeptide is selected from SEQ ID NO: 1285 -SEQ ID NO: 1318.

79. According to any one of claims 44 to 78, the first domain is directly or indirectly connected to the third domain.

80. The fusion polypeptide according to any one of claims 44 to 79, wherein the first domain comprises an antibody heavy chain, and the first domain antibody heavy chain is directly or indirectly connected to the third domain.

81. The fusion polypeptide according to any one of claims 44 to 80, wherein the first domain comprises an antibody heavy chain, and the C-terminus of the antibody heavy chain of the first domain is directly or indirectly connected to the N-terminus of the third domain.

82. The fusion polypeptide according to any one of claims 44 to 81, wherein the first domain comprises an antibody heavy chain, and the N-terminus of the antibody heavy chain of the first domain is directly or indirectly connected to the C-terminus of the third domain.

83. The fusion polypeptide according to any one of claims 44 to 82, wherein the first domain comprises an antibody light chain, and the first domain light chain is directly or indirectly connected to the third domain.

84. The fusion polypeptide according to any one of claims 44 to 83, wherein the first domain comprises an antibody light chain, and the C-terminus of the light chain of the first domain is directly or indirectly connected to the N-terminus of the third domain.

85. The fusion polypeptide according to any one of claims 44 to 84, wherein the first domain comprises an antibody light chain, and the N-terminus of the light chain of the first domain is directly or indirectly connected to the C-terminus of the third domain.

86. The fusion polypeptide according to any one of claims 44 to 85, wherein the second domain and the third domain are directly or indirectly connected.

87. The fusion polypeptide according to any one of claims 44 to 86, wherein the C-terminus of the second domain is directly or indirectly connected to the N-terminus of the third domain.

88. The fusion polypeptide according to any one of claims 44 to 87, wherein the N-terminus of the second domain is directly or indirectly connected to the C-terminus of the third domain.

89. The fusion polypeptide according to any one of claims 44 to 88, wherein the first domain is directly or indirectly connected to the second domain, and the second domain is directly or indirectly connected to the third domain.

90. The fusion polypeptide according to any one of claims 44 to 89, wherein the C-terminus of the first domain is directly or indirectly connected to the N-terminus of the second domain, and the C-terminus of the second domain is directly or indirectly connected to the N-terminus of the third domain.

91. The fusion polypeptide according to any one of claims 44 to 90, wherein the first domain comprises an antibody heavy chain, the C-terminus of the antibody heavy chain of the first domain is directly or indirectly connected to the N-terminus of the second domain, and the C-terminus of the second domain is directly or indirectly connected to the N-terminus of the third domain.

92. The fusion polypeptide according to any one of claims 44 to 91, wherein the first domain comprises an antibody light chain, the C-terminus of the antibody light chain of the first domain is directly or indirectly connected to the N-terminus of the second domain, and the C-terminus of the second domain is directly or indirectly connected to the N-terminus of the third domain.

93. The fusion polypeptide according to any one of claims 44 to 92, wherein the N-terminus of the first domain is directly or indirectly connected to the C-terminus of the second domain, and the N-terminus of the second domain is directly or indirectly connected to the C-terminus of the third domain.

94. The fusion polypeptide according to any one of claims 44 to 93, wherein the first domain comprises an antibody heavy chain, the N-terminus of the antibody heavy chain of the first domain is directly or indirectly connected to the C-terminus of the second domain, and the N-terminus of the second domain is directly or indirectly connected to the C-terminus of the third domain.

95. The fusion polypeptide according to any one of claims 44 to 94, wherein the first domain comprises an antibody light chain, the N-terminus of the antibody light chain of the first domain is directly or indirectly connected to the C-terminus of the second domain, and the N-terminus of the second domain is directly or indirectly connected to the C-terminus of the third domain.

96. The fusion polypeptide according to any one of claims 44 to 95, wherein the first domain is directly or indirectly connected to the third domain, and the third domain is directly or indirectly connected to the second domain.

97. The fusion polypeptide according to any one of claims 44 to 96, wherein the C-terminus of the first domain is directly or indirectly connected to the N-terminus of the third domain, and the C-terminus of the third domain is directly or indirectly connected to the N-terminus of the second domain.

98. The fusion polypeptide according to any one of claims 44 to 97, wherein the first domain comprises an antibody heavy chain, the C-terminus of the first domain antibody heavy chain is directly or indirectly connected to the N-terminus of the third domain, and the C-terminus of the third domain is directly or indirectly connected to the N-terminus of the second domain.

99. The fusion polypeptide according to any one of claims 44 to 98, wherein the first domain comprises an antibody light chain, the C-terminus of the antibody light chain of the first domain is directly or indirectly connected to the N-terminus of the third domain, and the C-terminus of the third domain is directly or indirectly connected to the N-terminus of the second domain.

100. The fusion polypeptide according to any one of claims 44 to 99, wherein the N-terminus of the first domain is directly or indirectly connected to the C-terminus of the third domain, and the N-terminus of the third domain is directly or indirectly connected to the C-terminus of the second domain.

101. The fusion polypeptide according to any one of claims 44 to 100, wherein the first domain comprises an antibody heavy chain, the N-terminus of the antibody heavy chain of the first domain is directly or indirectly connected to the C-terminus of the third domain, and the N-terminus of the third domain is directly or indirectly connected to the C-terminus of the second domain.

102. The fusion polypeptide according to any one of claims 44 to 101, wherein the first domain comprises an antibody light chain, the N-terminus of the antibody light chain of the first domain is directly or indirectly connected to the C-terminus of the third domain, and the N-terminus of the third domain is directly or indirectly connected to the C-terminus of the second domain.

103. The fusion polypeptide according to any one of claims 44 to 102, wherein the first domain is directly or indirectly connected to the second domain, and the first domain is directly or indirectly connected to the third domain.

104. The fusion polypeptide according to any one of claims 44 to 103, wherein the C-terminus of the first domain is directly or indirectly connected to the N-terminus of the second domain, and the N-terminus of the first domain is directly or indirectly connected to the C-terminus of the third domain.

105. The fusion polypeptide according to any one of claims 44 to 104, wherein the first domain comprises an antibody heavy chain, the C-terminus of the first domain antibody heavy chain is directly or indirectly connected to the N-terminus of the second domain, and the N-terminus of the first domain heavy chain is directly or indirectly connected to the C-terminus of the third domain.

106. The fusion polypeptide according to any one of claims 44 to 105, wherein the first domain comprises an antibody light chain, the C-terminus of the first domain antibody light chain is directly or indirectly connected to the N-terminus of the second domain, and the N-terminus of the first domain light chain is directly or indirectly connected to the C-terminus of the third domain.

107. The fusion polypeptide according to any one of claims 44 to 106, wherein the first domain comprises an antibody heavy chain, the C-terminus of the first domain antibody heavy chain is directly or indirectly connected to the N-terminus of the second domain, and the N-terminus of the first domain light chain is directly or indirectly connected to the C-terminus of the third domain.

108. The fusion polypeptide according to any one of claims 44 to 107, wherein the first domain comprises an antibody light chain, the C-terminus of the first domain antibody light chain is directly or indirectly connected to the N-terminus of the second domain, and the N-terminus of the first domain heavy chain is directly or indirectly connected to the C-terminus of the third domain.

109. The fusion polypeptide according to any one of claims 44 to 108, wherein the N-terminus of the first domain is directly or indirectly connected to the C-terminus of the second domain, and the C-terminus of the first domain is directly or indirectly connected to the N-terminus of the third domain.

110. The fusion polypeptide according to any one of claims 44 to 109, wherein the first domain comprises an antibody heavy chain, the N-terminus of the first domain antibody heavy chain is directly or indirectly connected to the C-terminus of the second domain, and the C-terminus of the first domain heavy chain is directly or indirectly connected to the N-terminus of the third domain.

111. The fusion polypeptide according to any one of claims 44 to 110, wherein the first domain comprises an antibody light chain, the N-terminus of the first domain antibody light chain is directly or indirectly connected to the C-terminus of the second domain, and the C-terminus of the first domain light chain is directly or indirectly connected to the N-terminus of the third domain.

112. The fusion polypeptide according to any one of claims 44 to 111, wherein the first domain comprises an antibody heavy chain and an antibody light chain, the N-terminus of the antibody heavy chain of the first domain is directly or indirectly connected to the C-terminus of the second domain, and the C-terminus of the antibody light chain of the first domain is directly or indirectly connected to the N-terminus of the third domain.

113. The fusion polypeptide according to any one of claims 44 to 112, wherein the first domain comprises an antibody heavy chain and an antibody light chain, the N-terminus of the antibody light chain of the first domain is directly or indirectly connected to the C-terminus of the second domain, and the C-terminus of the antibody heavy chain of the first domain is directly or indirectly connected to the N-terminus of the third domain.

114. The fusion polypeptide according to any one of claims 44 to 113, wherein the indirect connection comprises connection via a linker.

115. The fusion polypeptide according to claim 114, wherein the linker comprises an amino acid sequence selected from the group consisting of SEQ ID NO: 31, SEQ ID NO: 32, SEQ ID NO: 33, SEQ ID NO: 34 and SEQ ID NO: 35.

116. The fusion polypeptide according to any one of claims 44 to 115, wherein the fusion polypeptide comprises an amino acid sequence selected from the group consisting of SEQ ID NO: 88, SEQ ID NO: 89, SEQ ID NO: 90, SEQ ID NO: 91, SEQ ID NO: 92, SEQ ID NO: 93, SEQ ID NO: 94, SEQ ID NO: 95, SEQ ID NO: 96, SEQ ID NO: 97, SEQ ID NO: 98, SEQ ID NO: 99, SEQ ID NO: 100, SEQ ID NO: 101, SEQ ID NO: 102, SEQ ID NO: 103, SEQ ID NO: 104, SEQ ID NO: 105, SEQ ID NO: 106, SEQ ID NO: 107, SEQ ID NO: 108, SEQ ID NO: 109, SEQ ID NO: 110, SEQ ID NO: 111, SEQ ID NO: 112, SEQ ID NO: 113, SEQ ID NO: 114, SEQ ID NO: 115, SEQ ID NO: 116, SEQ ID NO: 117, SEQ ID NO: 118, SEQ ID NO: 119, SEQ ID NO: 120, SEQ ID NO: 121, SEQ ID NO: 122, SEQ ID NO: 123, SEQ ID NO: 124, SEQ ID NO: 125, SEQ ID NO: 126, SEQ ID NO: 127, SEQ ID NO: 128, SEQ ID NO: 129, SEQ ID NO: 130, SEQ ID NO: 131, SEQ ID NO: 132, SEQ ID NO: 133, SEQ ID NO: 134, SEQ ID NO: 135, SEQ ID NO: 171, SEQ ID NO: 173.

117. The fusion polypeptide according to any one of claims 44 to 116, comprising an amino acid sequence selected from the group consisting of SEQ ID NO: 751 to SEQ ID NO: 1024, SEQ ID NO: 1249, SEQ ID NO: 1251, SEQ ID NO: 1253 and SEQ ID NO: 1255.

118. The fusion polypeptide according to any one of claims 44 to 117, wherein the fusion polypeptide comprises a heavy chain of the multifunctional antibody polypeptide complex of LAG3 variant fused Sugemalimab-CD86 IgV Q25I/F33L/H90I, and the heavy chain amino acid sequence is selected from SEQ ID NO: 1387-SEQ ID NO: 1420.

119. The fusion polypeptide according to any one of claims 44 to 118, comprising the following sequences: SEQ ID NO: 1421 or SEQ ID NO: 1426.

120. A CD86 variant polypeptide comprising an amino acid substitution mutation of human CD86.

121. The CD86 variant polypeptide according to claim 120, comprising an amino acid substitution mutant of the IgV domain of the human CD86 extracellular domain.

122. The CD86 variant polypeptide according to any one of claims 120-121, comprising an amino acid substitution mutant of the IgV domain of CD86, wherein the mutation site of the amino acid substitution mutant of the IgV domain of CD86 comprises one or more of the amino acid mutations selected from the group consisting of A13I, A13L, A13F, A13M, Q25A, Q25I, Q25V, Q25F, Q25M, F33A, F33L, F33V, F33M, M60R, I89A, I89L, I89V, I89F, I89M, H90I, H90V, H90M, and H90F.

123. The CD86 variant polypeptide according to any one of claims 120 to 122, comprising a combination of 1, 2, 3, 4, 5 or more amino acid mutations selected from the group consisting of A13I, A13L, A13F, A13M, Q25A, Q25I, Q25V, Q25F, Q25M, F33A, F33L, F33V, F33M, M60R, I89A, I89L, I89V, I89F, I89M, H90I, H90V, H90M, and H90F.

124. The CD86 variant polypeptide according to any one of claims 120 to 123, comprising an amino acid substitution mutant of the IgV domain of CD86, wherein the amino acid substitution mutant comprises one or more groups selected from the combination shown in the following group: Q25I/F33L/H90I, Q25V/F33L/H90I, Q25I/F33V/H90I, Q25V/F33V/H90I, Q25I/F33L/H90V, Q25V/F33L/H90V, Q25I/F33V/H90V, Q25V/F33V/H90V, A13L/Q25I/F33L/H90I, A13I/Q25I/F33L/H90I, A13L/Q25V/F33L/H90I, A13I/Q25V/F33L/H90I, Q25I/F33L/I89L/H90I, Q25I/F33L/M60R/H90I, Q25V/F33L/I89L/H90I, Q25V/F33L/M60R/H90I, Q25F/F33L/H90I, Q25I/F33L/H90F, Q25I/F33A/H90I, Q25I/H90I, Q25I, H90I, Q25V/H90V, Q25V/H90I, Q25F/H90I, Q25F/H90V, A13F/Q25I/F33L/H90I, A13M/Q25I/F33L/H90I, Q25A/F33L/H90I, Q25M/F33L/H90I, Q25I/F33M/H90I, Q25I/F33L/I89V/H90I, Q25I/F33L/I89F/H90I, Q25I/F33L/I89M/H90I, and Q25I/F33L/H90M.

125. The CD86 variant polypeptide according to any one of claims 120 to 124, comprising the IgV domain amino acid substitution mutant Q25I/F33L/H90I of CD86.

126. The CD86 variant polypeptide according to any one of claims 120 to 125, comprising an amino acid substitution mutant of the extracellular domain of human CD86.

127. The CD86 variant polypeptide according to any one of claims 120 to 126, comprising an amino acid substitution mutant of the extracellular domain of CD86, wherein the amino acid substitution mutant of the extracellular domain of CD86 comprises one or more mutations in the amino acid sites selected from the group consisting of A13I, A13L, A13F, A13M, Q25A, Q25I, Q25V, Q25F, Q25M, F33A, F33L, F33V, F33M, M60R, I89A, I89L, I89V, I89F, I89M, H90I, H90V, H90M, and H90F.

128. The CD86 variant polypeptide according to any one of claims 120 to 127, comprising an amino acid substitution mutant of the extracellular domain of CD86, wherein the amino acid substitution mutant comprises one or more groups selected from the combination shown in the following group: Q25I/F33L/H90I, 25V/F33L/H90I, Q25I/F33V/H90I, Q25V/F33V/H90I, Q25I/F33L/H90I 0V, Q25V/F33L/H90V, Q25I/F33V/H90V, Q25V/F33V/H90V, A13L/Q25I/F33L/H90I, A13I/Q25I/F33L/H90I, A13L/Q25V/F33L/H90I, A13I/Q25V/F33L/H90I, Q25I/F33L/I89L/H90I, Q25I/F33L/M60R/H90I, Q25V/F33L/I89L/H90I, Q25V/F33L/M60R/H90I, Q25F/F33L/H90I, Q25I/F33L/H90F, Q25I/F33A/H90I, Q25I/H90I, Q25I, H90I, Q25V/H90V, Q25V/H90I, Q25F/H90I, Q25F/H90V, A13F/Q25I/F33L/H90I, A13M/Q25I/F33L/H90I, Q25A/F33L/H90I, Q25M/F33L/H90I, Q25I/F33M/H90I, Q25I/F33L/I89V/H90I, Q25I/F33L/I89F/H90I, Q25I/F33L/I89M/H90I, and Q25I/F33L/H90M.

129. The CD86 variant polypeptide according to any one of claims 120 to 128, comprising the extracellular domain amino acid substitution mutant Q25I/F33L/H90I of CD86.

130. The CD86 variant polypeptide according to any one of claims 120 to 129, comprising an amino acid sequence selected from the group consisting of SEQ ID NO: 178 to SEQ ID NO: 243.

131. A fusion polypeptide comprising a first domain and a second domain, wherein the first domain comprises the CD86 variant polypeptide according to any one of claims 120-130, and the second domain comprises an antibody or an antigen-binding fragment thereof or an immunoglobulin Fc domain.

132. The fusion polypeptide according to claim 131, wherein the second domain comprises an antibody or an antigen-binding fragment thereof.

133. The fusion polypeptide according to any one of claims 131 to 132, wherein the antibody is selected from the group consisting of an immunoglobulin IgG antibody, a recombinant antibody, a chimeric antibody, a heavy chain antibody, a single domain antibody, and a bispecific antibody.

134. The fusion polypeptide of any one of claims 131-133, wherein the second domain is capable of binding to one or more of the targets shown in the following group: PD-L1, PD-L2, PD-1, OX40, 4-1BB, ICOS, TIGIT, CTLA4, LAG3, CD3, VEGF, VEGFR, CD47, HGF, Trop2, EpCAM, CCR8, CCR4, CCR5, GPRC5D, BCMA, CD19, CD20, HER-2 neu, DLL1, HER-3, HER-4, EGFR, PSMA, CEA, MUC-1 (mucin), MUC2, MUC3, MUC4, MUC5AC, MUC5B, MUC7, CD123, CD33, CD30, CD38, NKG2A, Nkp36 and Tim3.

135. The fusion polypeptide according to any one of claims 131-134, wherein the second domain is capable of binding to CD3.

136. The fusion polypeptide according to any one of claims 131 to 135, wherein the second domain comprises a CD3B219 antibody or an antigen-binding fragment thereof.

137. The fusion polypeptide according to any one of claims 131 to 136, wherein the second domain comprises HCDR1, HCDR2 and/or HCDR3 of an antibody heavy chain, and the antibody heavy chain comprises the amino acid sequence shown in SEQ ID NO: 616.

138. The fusion polypeptide according to any one of claims 131 to 137, wherein the second domain comprises a heavy chain variable region VH of an antibody, and the antibody heavy chain comprises the amino acid sequence shown in SEQ ID NO: 616.

139. The fusion polypeptide according to any one of claims 131 to 138, wherein the second domain comprises an antibody heavy chain, and the antibody heavy chain comprises the amino acid sequence shown in SEQ ID NO: 616.

140. The fusion polypeptide according to any one of claims 131 to 139, wherein the second domain comprises LCDR1, LCDR2 and/or LCDR3 of an antibody light chain, and the antibody light chain comprises the amino acid sequence shown in SEQ ID NO: 617.

141. The fusion polypeptide according to any one of claims 131 to 140, wherein the second domain comprises a light chain variable region VL of an antibody, and the antibody light chain comprises the amino acid sequence shown in SEQ ID NO: 617.

142. The fusion polypeptide according to any one of claims 131 to 141, wherein the second domain comprises an antibody light chain, and the antibody light chain comprises the amino acid sequence shown in SEQ ID NO: 617.

143. The fusion polypeptide according to any one of claims 131-142, wherein the immunoglobulin Fc domain comprises an IgG antibody Fc domain, and the immunoglobulin IgG antibody comprises one or more selected from human IgG1, human IgG2, human IgG3, human IgG4, mouse IgG1, mouse IgG2a, mouse IgG2b and mouse IgG3.

144. The fusion polypeptide according to any one of claims 131 to 143, comprising an amino acid sequence selected from the group consisting of SEQ ID NO: 350 to SEQ ID NO: 382, SEQ ID NO: 384 to SEQ ID NO: 416, SEQ ID NO: 618 to SEQ ID NO: 624 and SEQ ID NO: 626 to SEQ ID NO: 632.

145. The fusion polypeptide according to any one of claims 131 to 144, further comprising a third domain, wherein the third domain is an antibody or antigen-binding fragment thereof, or a functional protein or active fragment thereof, which is the same as or different from the second domain.

146. The fusion polypeptide according to any one of claims 131 to 145, wherein the third domain is a functional protein or an active fragment thereof, and the functional protein or the active fragment thereof can activate the innate immune response.

147. The fusion polypeptide according to any one of claims 131 to 146, wherein the third domain is capable of binding to the MHCII molecule on an antigen presenting cell.

148. The fusion polypeptide according to any one of claims 131 to 147, wherein the third domain comprises LAG3 or a functionally active fragment thereof.

149. A LAG3 variant polypeptide comprising truncations and/or mutations based on human LAG3.

150. The fusion polypeptide or LAG3 variant polypeptide according to claim 149, wherein the LAG3 variant polypeptide comprises a truncation based on a wild-type human LAG3 extracellular region polypeptide.

151. The LAG3 variant polypeptide according to any one of claims 149 to 150, wherein the LAG3 variant polypeptide has 0-124 amino acids truncated at the N-terminus based on the wild-type human LAG3 extracellular region polypeptide, and terminates at amino acid positions 121-167 at the C-terminus.

152. The LAG3 variant polypeptide according to any one of claims 149 to 151, wherein the LAG3 variant polypeptidehas 0, 5, 21, 37, 45, 60, 71, 74, 79, 84, 89, 94, 99, 104, 109 or 114 amino acids truncated at the N-terminus based on the wild-type human LAG3 extracellular region polypeptide.

153. The LAG3 variant polypeptide according to any one of claims 149 to 152, wherein the LAG3 variant polypeptide has 0, 5, 21, 37, 45, 60, 71, 74, 81 or 99 amino acids truncated at the N-terminus based on the wild-type human LAG3 extracellular region polypeptide.

154. The LAG3 variant polypeptide according to any one of claims 149 to 153, wherein the LAG3 variant polypeptide has 0, 5, 21, 37, 45, 60, 71 or 74 amino acids truncated at the N-terminus based on the wild-type human LAG3 extracellular region polypeptide.

155. The LAG3 variant polypeptide according to any one of claims 149-154, wherein the LAG3 variant polypeptide terminates at amino acid position 121, 122, 129, 136, 146, 156, 157, 158, 159, 160, 161, 162, 163, 164, 165, 166 or 167 at the C-terminus based on the wild-type human LAG3 extracellular region polypeptide.

156. The LAG3 variant polypeptide according to any one of claims 149-155, wherein the LAG3 variant polypeptide terminates at amino acid position 121, 122, 129, 136, 146, 156, 161 or 167 at the C-terminus based on the wild-type human LAG3 extracellular region polypeptide.

157. The LAG3 variant polypeptide according to any one of claims 149-156, wherein the LAG3 variant polypeptide terminates at amino acid position 121, 146, 156, 161 or 167 at the C-terminus based on the wild-type human LAG3 extracellular region polypeptide.

158. The LAG3 variant polypeptide according to any one of claims 149-157, wherein the LAG3 variant polypeptide terminates at amino acid position 156, 161 or 167 at the C-terminus based on the wild-type human LAG3 extracellular region polypeptide.

159. The LAG3 variant polypeptide of any one of claims 149-158, wherein the LAG3 variant polypeptide has 0, 5, 21, 37, 45, 60, 71, 74, 81 or 99 amino acids truncated at the N-terminus based on the wild-type human LAG3 extracellular region polypeptide, and terminates at amino acid position 121, 122, 129, 136, 146, 156, 157, 158, 159, 160, 161, 162, 163, 164, 165, 166 or 167 at the C-terminus based on the wild-type human LAG3 extracellular region polypeptide.

160. The LAG3 variant polypeptide according to any one of claims 149-159, wherein the LAG3 variant polypeptide has 0, 5, 21, 37, 45, 60, 71 or 74 amino acids truncated at the N-terminus based on the wild-type human LAG3 extracellular region polypeptide, and terminates at amino acid position 122, 129, 136, 146, 156, 157, 158, 159, 160, 161, 162, 163, 164, 165, 166 or 167 at the C-terminus based on the wild-type human LAG3 extracellular region polypeptide.

161. The LAG3 variant polypeptide according to any one of claims 149-160, wherein the LAG3 variant polypeptide comprises an amino acid mutation.

162. The LAG3 variant polypeptide according to any one of claims 149 to 161, wherein the LAG3 variant polypeptide comprises an amino acid mutation, wherein the amino acid mutation site is an Arg amino acid at position 97.

163. The LAG3 variant polypeptide according to any one of claims 149 to 162, wherein the LAG3 variant polypeptide comprises an amino acid mutation, wherein the amino acid mutation site is a mutation of the Arg amino acid at site 97 to a Glu amino acid.

164. The LAG3 variant polypeptide according to any one of claims 149 to 163, wherein the LAG3 variant polypeptide is selected from the amino acid sequences shown by SEQ ID NO: 244 to SEQ ID NO: 349.

165. The LAG3 variant polypeptide according to any one of claims 149 to 164, wherein the LAG3 variant polypeptide comprises an amino acid mutation at a site of R110, R113, R119, R129, G130 and/or R141.

166. The LAG3 variant polypeptide according to any one of claims 149 to 165, wherein the LAG3 variant polypeptide is truncated by 81 amino acids at the N-terminus, terminates at amino acid position 121 at the C-terminus, and a mutation is introduced at R110, and/or R113, and/or R119.

167. The LAG3 variant polypeptide according to any one of claims 149 to 165, wherein the LAG3 variant polypeptide is truncated by 99 amino acids at the N-terminus, terminates at amino acid position 146 at the C-terminus, and a mutation is introduced at R129, and/or G130, and/or R141.

168. The LAG3 variant polypeptide according to any one of claims 149 to 165, wherein the LAG3 variant polypeptide is truncated by 81 amino acids at the N-terminus and terminates at amino acid position 146 at the C-terminus, and a mutation is introduced at R110, and/or R113, and/or R119, and/or R129, and/or G130, and/or R141.

169. The LAG3 variant polypeptide according to any one of claims 149 to 168, wherein the LAG3 variant polypeptide comprises an amino acid mutation, wherein the R110 site is mutated to K110.

170. The LAG3 variant polypeptide according to any one of claims 149 to 169, wherein the LAG3 variant polypeptide comprises an amino acid mutation, wherein the R113 site is mutated to K113.

171. The LAG3 variant polypeptide according to any one of claims 149 to 170, wherein the LAG3 variant polypeptide comprises an amino acid mutation, wherein the R119 site is mutated to K119.

172. The LAG3 variant polypeptide according to any one of claims 149 to 171, wherein the LAG3 variant polypeptide comprises an amino acid mutation, wherein the R129 site is mutated to K129.

173. The LAG3 variant polypeptide according to any one of claims 149 to 172, wherein the LAG3 variant polypeptide comprises an amino acid mutation, wherein the G130 site is mutated to P130, or A130, or T130, or Y130, or S130.

174. The LAG3 variant polypeptide according to any one of claims 149 to 173, wherein the LAG3 variant polypeptide comprises an amino acid mutation, wherein the R141 site is mutated to K141.

175. The LAG3 variant polypeptide of any one of claims 149 to 174, wherein the amino acid sequence of the LAG3 variant polypeptide is selected from SEQ ID NO: 1285 -SEQ ID NO: 1318.

176. A fusion polypeptide comprising a first domain and a second domain, wherein the first domain comprises the LAG3 variant polypeptide according to any one of claims 149 to 175, and the second domain comprises an antibody or an antigen-binding fragment thereof or an immunoglobulin Fc domain.

177. The fusion polypeptide according to claim 176, wherein the second domain comprises an antibody or an antigen-binding fragment thereof.

178. The fusion polypeptide according to any one of claims 176 to 177, wherein the antibody is selected from the group consisting of an immunoglobulin antibody, a recombinant antibody, a chimeric antibody, a heavy chain antibody, a single domain antibody, and a bispecific antibody.

179. The fusion polypeptide according to any one of claims 176 to 178, wherein the antigen binding fragment is selected from one or more of the group consisting of: Fab, Fab', Fv fragment, F(ab')₂, F(ab)₂, scFv, di-scFv, VHH and dAb.

180. The fusion polypeptide of any one of claims 176-179, wherein the second domain is capable of binding to one or more of the targets shown in the following group: PD-L1, PD-L2, PD-1, OX40, 4-1BB, ICOS, TIGIT, CTLA4, LAG3, CD3, VEGF, VEGFR, CD47, HGF, Trop2, EpCAM, CCR8, CCR4, CCR5, GPRC5D, BCMA, CD19, CD20, HER-2 neu, DLL1, HER-3, HER-4, EGFR, PSMA, CEA, MUC-1 (mucin), MUC2, MUC3, MUC4, MUC5AC, MUC5B, MUC7, CD123, CD33, CD30, CD38, NKG2A, Nkp36 and Tim3.

181. The fusion polypeptide according to any one of claims 176 to 180, wherein the second domain is capable of binding to PD-L1, PD-1 and/or PD-L2.

182. The fusion polypeptide according to any one of claims 176 to 181, wherein the second domain comprises HCDR1, HCDR2 and/or HCDR3 of an antibody heavy chain, preferably the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15 and SEQ ID NO: 17.

183. The fusion polypeptide according to any one of claims 176 to 182, wherein the second domain comprises the heavy chain variable region VH of an antibody heavy chain, and preferably the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15, and SEQ ID NO: 17.

184. The fusion polypeptide according to any one of claims 176 to 183, wherein the second domain comprises an antibody heavy chain, preferably the antibody heavy chain comprises an amino acid sequence selected from the group consisting of SEQ ID NO: 4, SEQ ID NO: 6, SEQ ID NO: 8, SEQ ID NO: 10, SEQ ID NO: 12, SEQ ID NO: 14, SEQ ID NO: 15 and SEQ ID NO: 17.

185. The fusion polypeptide according to any one of claims 176 to 184, wherein the second domain comprises LCDR1, LCDR2 and/or LCDR3 of an antibody light chain, and preferably the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

186. The fusion polypeptide according to any one of claims 176 to 185, wherein the second domain comprises a light chain variable region VL of an antibody light chain, and preferably the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

187. The fusion polypeptide according to any one of claims 176 to 186, wherein the second domain comprises an antibody light chain, preferably the antibody light chain comprises an amino acid sequence selected from the group consisting of SEQ ID NO: 5, SEQ ID NO: 7, SEQ ID NO: 9, SEQ ID NO: 11, SEQ ID NO: 13, SEQ ID NO: 16 and SEQ ID NO: 18.

188. The fusion polypeptide according to any one of claims 176 to 187, wherein the second domain comprises an antibody or an antigen-binding fragment thereof selected from the group consisting of Sugemalimab, Durvalumab, Atezolizumab, Avelumab, Envafolimab, Permbrolizumab and Nivolumab.

189. The fusion polypeptide according to any one of claims 176 to 188, wherein the second domain comprises Sugemalimab or an antigen-binding fragment thereof.

190. The fusion polypeptide according to any one of claims 176-189, wherein the fusion polypeptide comprises a heavy chain, and the heavy chain amino acid sequence is selected from SEQ ID NO: 1353 to SEQ ID NO: 1386.

191. The fusion polypeptide according to any one of claims 176 to 190, wherein the fusion polypeptide comprises a light chain, and the light chain comprises an antibody light chain polypeptide, and preferably the light chain sequence of the fusion polypeptide is selected from the group consisting of: SEQ ID NO: 5.

192. The fusion polypeptide according to any one of claims 176 to 191, wherein the fusion polypeptide heavy chain and the fusion polypeptide light chain are combined into the fusion polypeptide.

193. The fusion polypeptide according to any one of claims 176 to 192, wherein the first domain is directly or indirectly connected to the second domain.

194. The fusion polypeptide according to any one of claims 176 to 193, wherein the first domain is directly or indirectly connected to the N-terminus of the second domain.

195. The fusion polypeptide according to any one of claims 176 to 194, wherein the first domain is directly or indirectly connected to the C-terminus of the second domain.

196. The fusion polypeptide according to any one of claims 176 to 195, wherein the second domain may comprise an antibody heavy chain, and the LAG3 variant polypeptide may be directly or indirectly linked to the antibody heavy chain of the second domain.

197. The fusion polypeptide according to any one of claims 176 to 196, wherein the second domain may comprise an antibody heavy chain, and the LAG3 variant polypeptide may be directly or indirectly linked to the C-terminus of the antibody heavy chain of the second domain.

198. The fusion polypeptide according to any one of claims 176 to 197, wherein the second domain may comprise an antibody heavy chain, and the N-terminus of the LAG3 variant polypeptide may be directly or indirectly connected to the C-terminus of the antibody heavy chain of the second domain.

199. The fusion polypeptide according to any one of claims 176 to 198, wherein the second domain may comprise an antibody heavy chain, and the LAG3 variant polypeptide may be directly or indirectly linked to the N-terminus of the antibody heavy chain of the second domain.

200. The fusion polypeptide according to any one of claims 176 to 199, wherein the second domain may comprise an antibody heavy chain, and the C-terminus of the LAG3 variant polypeptide may be directly or indirectly connected to the N-terminus of the antibody heavy chain of the second domain.

201. The fusion polypeptide according to any one of claims 176-200, wherein the second domain may comprise an antibody light chain, and the LAG3 variant polypeptide may be directly or indirectly linked to the antibody light chain of the second domain.

202. The fusion polypeptide according to any one of claims 176 to 201, the second domain may comprise an antibody light chain, and the LAG3 variant polypeptide may be directly or indirectly linked to the C-terminus of the antibody light chain of the second domain.

203. The fusion polypeptide according to any one of claims 176 to 202, wherein the second domain may comprise an antibody light chain, and the N-terminus of the LAG3 variant polypeptide may be directly or indirectly connected to the C-terminus of the antibody light chain of the second domain.

204. The fusion polypeptide according to any one of claims 176 to 203, the second domain may comprise an antibody light chain, and the LAG3 variant polypeptide may be directly or indirectly linked to the N-terminus of the antibody light chain of the second domain.

205. The fusion polypeptide according to any one of claims 176 to 204, the second domain may comprise an antibody light chain, and the C-terminus of the LAG3 variant polypeptide may be directly or indirectly connected to the N-terminus of the antibody light chain of the second domain.

206. The fusion polypeptide according to any one of claims 176 to 205, wherein the indirect connection comprises connection via a linker.

207. The fusion polypeptide according to any one of claims 176 to 206, wherein the second domain comprises an immunoglobulin Fc domain.

208. The fusion polypeptide according to claim 207, wherein the immunoglobulin Fc domain comprises an IgG antibody Fc domain, and the immunoglobulin IgG antibody comprises one or more selected from human IgG1, human IgG2, human IgG3, human IgG4, mouse IgG1, mouse IgG2a, mouse IgG2b and mouse IgG3.

209. The fusion polypeptide according to any one of claims 176-208, wherein the second domain comprises an immunoglobulin Fc domain.

210. The fusion polypeptide according to any one of claims 176 to 209, wherein the second domain comprises an Fc domain of an immunoglobulin IgG antibody.

211. The fusion polypeptide according to any one of claims 176 to 210, wherein the second domain comprises the Fc domain of an immunoglobulin IgG antibody, and the Fc domain of the immunoglobulin IgG antibody comprises a human IgG1 Fc domain, a human IgG2 Fc domain, a human IgG3 Fc domain, a human IgG4 Fc domain, a mouse IgG1 Fc domain, a mouse IgG2a Fc domain, a mouse IgG2b Fc domain or a mouse IgG3 Fc domain.

212. The fusion polypeptide according to any one of claims 176 to 211, wherein the second domain comprises the Fc domain of human immunoglobulin IgG4.

213. The fusion polypeptide according to any one of claims 176 to 212, wherein the second domain comprises an amino acid sequence selected from the group consisting of SEQ ID NO: 4 to SEQ ID NO: 18, SEQ ID NO: 29 to SEQ ID NO: 30, and SEQ ID NO: 634 to SEQ ID NO: 639.

214. The fusion polypeptide according to any one of claims 176 to 213, wherein the second domain is selected from the group consisting of amino acid sequences shown in SEQ ID NO: 1319 to SEQ ID NO: 1352.

215. The fusion polypeptide according to any one of claims 176 to 214, wherein the first domain is directly or indirectly connected to the second domain.

216. The fusion polypeptide according to any one of claims 176 to 215, wherein the first domain is directly or indirectly connected to the N-terminus of the second domain.

217. The fusion polypeptide according to any one of claims 176 to 216, wherein the first domain is directly or indirectly connected to the C-terminus of the second domain.

218. The fusion polypeptide according to any one of claims 176 to 217, wherein the indirect connection comprises connection via a linker.

219. The fusion polypeptide according to claim 218, wherein the linker comprises an amino acid sequence selected from the group consisting of SEQ ID NO: 31, SEQ ID NO: 32, SEQ ID NO: 33, SEQ ID NO: 34 and SEQ ID NO: 35.

220. The fusion polypeptide according to any one of claims 176-219, comprising an amino acid sequence selected from the group consisting of SEQ ID NO: 640 to SEQ ID NO: 745, SEQ ID NO: 747 to SEQ ID NO: 750, and SEQ ID NO: 1353 to SEQ ID NO: 1386.

221. The fusion polypeptide according to any one of claims 176-220, which also includes a third domain, wherein the third domain is an antibody or antigen-binding fragment or a functional protein or an active fragment thereof that is the same as or different from the second domain.

222. The fusion polypeptide according to claim 221, wherein the third domain is a functional protein or an active fragment thereof, and the functional protein or the active fragment thereof can activate the innate immune response.

223. The fusion polypeptide according to any one of claims 221-222, wherein the third domain is capable of binding to CD28 and/or CTLA4.

224. The fusion polypeptide according to any one of claims 221 to 223, wherein the third domain comprises CD80, CD86 or an active fragment thereof.

225. An immunoconjugate comprising the fusion polypeptide of any one of claims 1-119, 131-148 and 176-224, the CD86 variant polypeptide of any one of claims 120-130 and/or the LAG3 variant of any one of claims 149-175.

226. A nucleic acid molecule encoding the fusion polypeptide of any one of claims 1-119, 131-148 and 176-224, the CD86 variant polypeptide of any one of claims 120-130 and/or the LAG3 variant of any one of claims 149-175.

227. A vector comprising the nucleic acid molecule of claim 226.

228. A cell comprising and/or expressing the fusion polypeptide of any one of claims 1-119, 131-148 and 176-224, the CD86 variant polypeptide of any one of claims 120-130, the LAG3 variant of any one of claims 149-175, the immunoconjugate of claim 225, the nucleic acid molecule of claim 226, and/or the vector of claim 227.

229. A composition comprising the fusion polypeptide of any one of claims 1-119, 131-148 and 176-224, the CD86 variant polypeptide of any one of claims 120-130, the LAG3 variant of any one of claims 149-175, the immunoconjugate of claim 225, the nucleic acid molecule of claim 226, the vector of claim 227, and/or the cell of claim 228, and optionally a pharmaceutically acceptable carrier.

230. A method for preparing the fusion polypeptide of any one of claims 1-119, 131-148 and 176-224, the CD86 variant polypeptide of any one of claims 120-130, and the LAG3 variant of any one of claims 149-175, comprising culturing the cell of claim 228 under conditions that allow the fusion polypeptide to be expressed.

231. A method for blocking the interaction between PD-L1 protein and PD-1, comprising administering an effective amount of the fusion polypeptide according to any one of claims 1-119, 131-148 and 176-224.

232. A method for stimulating antigen presenting cells and/or activating T cells, comprising the LAG3 variant according to any one of claims 149-175.

233. The method according to claim 232, the stimulation of antigen-presenting cells comprises a step selected from the group consisting of increasing the expression of co-stimulatory molecules in antigen-presenting cells, causing morphological changes and maturation of antigen-presenting cells, increasing the secretion of chemokines in antigen-presenting cells, and enhancing the phagocytic ability of antigen-presenting cells.

234. A method for inhibiting tumor or tumor cell growth and/or proliferation, comprising administering an effective amount of the fusion polypeptide of any one of claims 1-119, 131-148 and 176-224, the CD86 variant polypeptide of any one of claims 120-130, the LAG3 variant of any one of claims 149-175, the immunoconjugate of claim 225, the nucleic acid molecule of claim 226, the vector of claim 227, the cell of claim 228, and/or the composition of claim 229.

235. Use of the fusion polypeptide of any one of claims 1-119, 131-148 and 176-224, the CD86 variant polypeptide of any one of claims 120-130, the LAG3 variant of any one of claims 149-175, the immunoconjugate of claim 225, the nucleic acid molecule of claim 226, the vector of claim 227, the cell of claim 228, and/or the composition of claim 229 in the preparation of a medicament, wherein the medicament is used to prevent, improve and/or treat tumors.

236. The use according to claim 235, wherein the tumor comprises a solid tumor and/or a hematological tumor.

237. The use according to any one of claims 235-236, wherein the tumor is selected from the group consisting of colon tumors, breast tumors, lung tumors, gastric tumors, melanomas, head and neck tumors, lymphomas, nasopharyngeal tumors, cervical tumors, esophageal tumors, kidney tumors, skin squamous cell carcinoma, endometrial tumors, liver tumors, bladder tumors, urothelial tumors and skin tumors.
